(19) Europäisches Patentamt
European Patent Office
Office européen des brevets

(11) EP 4 772 499 A1

(12) EUROPEAN PATENT APPLICATION
published in accordance with Art. 153(4) EPC

(43) Date of publication:
08.07.2026 Bulletin 2026/28

(21) Application number: 24858713.1

(22) Date of filing: 30.08.2024

(51) International Patent Classification (IPC):
*C07D 239/48* (2006.01) *C07D 417/14* (2006.01)
*C07D 417/02* (2006.01) *C07D 401/12* (2006.01)
*C07D 417/12* (2006.01) *C07D 471/10* (2006.01)
*C07D 239/34* (2006.01) *C07D 405/14* (2006.01)
*A61K 31/506* (2006.01) *A61P 35/00* (2006.01)

(52) Cooperative Patent Classification (CPC):
**A61K 31/506; A61P 35/00; C07D 239/34; C07D 239/48; C07D 401/12; C07D 405/14; C07D 417/02; C07D 417/12; C07D 417/14; C07D 471/10**

(86) International application number:
**PCT/CN2024/115733**

(87) International publication number:
**WO 2025/045182 (06.03.2025 Gazette 2025/10)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **01.09.2023 CN 202311138740**

(71) Applicant: **Dovetree Medicines Unus, Inc.**
**Prides Crossing, MA 01965 (US)**

(72) Inventors:
- **HUA, Zihao**
  **Shenzhen, Guangdong 518017 (CN)**
- **SUN, Yina**
  **Shenzhen, Guangdong 518017 (CN)**
- **ZHAO, Chuanfang**
  **Shenzhen, Guangdong 518017 (CN)**
- **CHEN, Chong**
  **Shenzhen, Guangdong 518017 (CN)**

(74) Representative: **Latscha Schöllhorn Partner AG**
**Grellingerstrasse 60**
**4052 Basel (CH)**

# (54) ULK1 INHIBITOR, PREPARATION METHOD THEREFOR AND USE THEREOF

(57) Provided are a ULK1 inhibitor, a preparation method therefor and a use thereof. Specifically, provided are a compound of formula 1 or a pharmaceutically acceptable salt, a stereoisomer, a tautomer, a polymorph, a solvate, a hydrate, an N-oxide, an isotope labeled compound, a metabolite, an ester or a prodrug thereof, wherein $X_1$, $X_2$, $X_3$, $R_1$, $R_2$, $R_3$, R are as defined herein. Also provided are a pharmaceutical composition containing the compound, a treatment method using the compound, and a method for preparing the compound.

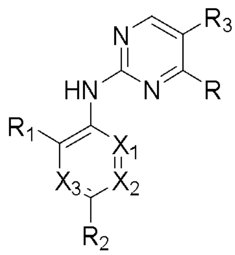

(1)

EP 4 772 499 A1

## Description

Reference to Related Applications

This application claims priority to Chinese Patent Application No. 202311138740.7, filed on September 1, 2023, the contents of which are incorporated herein by reference in their entirety and for all purposes.

## Field of the Invention

The present invention relates to a novel ULK1 inhibitor, a pharmaceutical composition containing the same, a preparation method therefor and a use thereof in the preparation of a medicament for a ULK1-mediated disease.

## Background

Autophagy is a central cellular mechanism that eliminates damaged proteins, protein complexes, and organelles. This conserved process plays a crucial role in the cellular response to nutrient deprivation and other stresses, in addition to maintaining appropriate cellular and tissue homeostasis during embryonic development and defending against pathogens. Defects in the autophagic pathway are associated with certain human pathologies, including an infectious disease, a neurodegenerative disorder, and a cancer. Despite possessing these highly conserved fundamental cellular functions, little is known about the molecular and biochemical details of how different carriers initiate autophagy, and the coordination of the steps from autophagosome initiation to eventual fusion with a lysosome.

Provided herein is an inhibitor of Unc-51-like autophagy-activated kinase (ULK) protein. In many cases, ULK1 and ULK2 are important proteins that regulate autophagy in mammalian cells. In certain cases, ULK1 and ULK2 are activated by several upstream signals under a nutrient deprivation condition, which then initiate autophagy. The demand for ULK1 and ULK2 in the initiation of autophagy has been studied in the context of nutrient deprivation. Although ULK1 appears to be the most essential for autophagy, in certain cases, ULK1 and ULK2 exhibit a high functional redundancy. The kinase domains of ULK1 and ULK2 share 78% sequence homology, indicating that in certain cases, ULK2 may compensate for the loss of ULK1. Therefore, nutrient-dependent autophagy may only be eliminated if both ULK1 and ULK2 are inhibited. However, in certain cases, ULK1 inhibition alone is sufficient to provide a therapeutic benefit, such as normalizing autophagy in any of the methods provided herein, or other beneficial outcomes. Inhibition of ULK1 and ULK2 produces a therapeutic benefit, such as tumor shrinkage, tumor cell death, or a reduction in the rate of tumor growth, there remains a need to provide a novel ULK1 inhibitor for the treatment of a ULK1-mediated disease.

## Summary of the Invention

In one aspect, the present invention provides a pyrimidine compound that has a specific inhibitory effect on ULK1, and thus has a good therapeutic effect on a ULK1-mediated disease. The compound is a compound of formula 1 or a pharmaceutically acceptable salt, a stereoisomer, a tautomer, a polymorph, a solvate, a hydrate, an N-oxide, an isotope labeled compound, a metabolite, an ester, or a prodrug thereof:

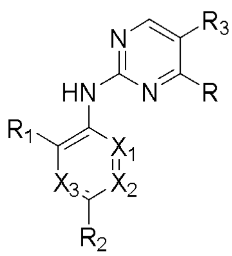

(1)

wherein $X_1$ is CH or N; $X_2$ is N or $CR_4$; $X_3$ is N or $CR_5$;

$R_1$ is selected from H, halogen, cyano, nitro, $C_1$-$C_6$ alkyl, $C_2$-$C_6$ alkenyl, $C_2$-$C_6$ alkynyl, $C_1$-$C_6$ alkoxy, $C_3$-$C_{10}$ cycloalkyl, 3-12-membered heterocyclyl, $C_6$-$C_{10}$ aryl and 5-10-membered heteroaryl, wherein each of the $C_1$-$C_6$ alkyl, $C_2$-$C_6$ alkenyl, $C_2$-$C_6$ alkynyl, $C_1$-$C_6$ alkoxy, $C_3$-$C_{10}$ cycloalkyl, 3-12-membered heterocyclyl, $C_6$-$C_{10}$ aryl or 5-10-membered heteroaryl is optionally substituted by one or more $R_6$;

$R_2$ is selected from H, halogen, cyano, nitro, $C_2$-$C_6$ alkenyl, $C_2$-$C_6$ alkynyl, $C_3$-$C_{10}$ cycloalkyl, 3-12-membered heterocyclyl, $C_6$-$C_{10}$ aryl, 5-10-membered heteroaryl, $R_7O$-, $-SR_7$, $-NR_7R_8$, $-NHCONR_7R_8$, $-NHCOR_7$, $-CONR_7R_8$, and $-SO_2R_7$, wherein each of the $C_2$-$C_6$ alkenyl, $C_2$-$C_6$ alkynyl, $C_3$-$C_{10}$ cycloalkyl, 3-12-membered heterocyclyl,

$C_6$-$C_{10}$ aryl, or 5-10-membered heteroaryl is optionally substituted by one or more $R_6$;

$R_3$ is selected from H, cyano, halogen, $C_1$-$C_6$ alkyl, $C_1$-$C_6$ hydroxyalkyl, $C_1$-$C_6$ aminoalkyl, $C_1$-$C_6$ haloalkyl, $C_1$-$C_6$ heteroalkyl, $C_2$-$C_6$ alkynyl, and $C_3$-$C_{10}$cycloalkyl;

$R_4$ is selected from H, halogen, cyano, nitro, $C_1$-$C_6$ alkyl, $C_2$-$C_6$ alkenyl, $C_2$-$C_6$ alkynyl, $C_3$-$C_{10}$ cycloalkyl, 3-12-membered heterocyclyl, $C_6$-$C_{10}$ aryl, 5-10-membered heteroaryl, $R_7O$-, -$NR_7R_8$, and -$NHCONR_7R_8$, wherein each of the $C_1$-$C_6$ alkyl, $C_2$-$C_6$ alkenyl, $C_2$-$C_6$ alkynyl, $C_3$-$C_{10}$ cycloalkyl, 3-12-membered heterocyclyl, $C_6$-$C_{10}$ aryl, or 5-10-membered heteroaryl is optionally substituted by one or more $R_6$;

or, $R_2$ and $R_4$ together with the groups that they are attached to, form 3-12-membered monocyclic or spiro heterocycle, $C_6$-$C_{10}$ aromatic ring, or 5-10-membered heteroaromatic ring, wherein each of the 3-12-membered monocyclic or spiro heterocycle, $C_6$-$C_{10}$ aromatic ring, or 5-10-membered heteroaromatic ring is substituted by one or more $R_6$;

$R_5$ is selected from H, $C_1$-$C_6$ alkyl, $C_2$-$C_6$ alkenyl, $C_2$-$C_6$ alkynyl, $C_3$-$C_{10}$ cycloalkyl, 3-12-membered heterocyclyl, $C_6$-$C_{10}$ aryl, and 5-10-membered heteroaryl;

or, $R_1$ and $R_5$ together with the groups that they are attached to, form 5-6-membered heterocycle, benzene ring, or 5-6-membered heteroaromatic ring;

each $R_6$ is independently selected from halogen, cyano, nitro, amino, -NHCO($C_1$-$C_6$ alkyl), $C_1$-$C_6$ alkyl, $C_2$-$C_6$ alkenyl, $C_2$-$C_6$ alkynyl, $C_3$-$C_{10}$ cycloalkyl, 3-12-membered heterocyclyl, $C_6$-$C_{10}$ aryl, and 5-10-membered heteroaryl, wherein each of the $C_3$-$C_{10}$ cycloalkyl, 3-12-membered heterocyclyl, $C_6$-$C_{10}$ aryl, or 5-10-membered heteroaryl is optionally substituted by one or more halogen, hydroxyl, cyano, $C_1$-$C_6$ alkyl, $C_1$-$C_6$ alkoxy;

$R_7$, $R_8$ are each independently selected from H, $C_1$-$C_6$ alkyl, $C_3$-$C_{10}$ cycloalkyl, 3-12-membered heterocyclyl, and $C_6$-$C_{10}$ aryl, wherein each of the $C_1$-$C_6$ alkyl, $C_3$-$C_{10}$ cycloalkyl, 3-12-membered heterocyclyl, or $C_6$-$C_{10}$ aryl is optionally substituted by one or more $R_6$;

or $R_7$ and $R_8$ together with the groups that they are attached to, form 5-6-membered heterocycle or 5-6-membered heteroaromatic ring;

R is selected from 3-12-membered heterocyclyl, 5-10-membered heteroaryl, and -L-$R_9$, wherein each of the 3-12-membered heterocyclyl or 5-10-membered heteroaryl is optionally substituted by one or more $R_{10}$;

L is $-(L_1)_m-(L_2)_n-(L_3)_p-$;

$L_1$ is selected from -O-, -N($R_{12}$)-, -C($R_{12}$)($R_{13}$)-O-, -N($R_{12}$)CO-, and -S-;

$L_2$ is selected from $C_1$-$C_6$ alkylene, $C_1$-$C_6$ heteroalkylene, $C_3$-$C_{10}$ cycloalkylene, and 3-10-membered heterocyclylene;

$L_3$ is selected from -N($R_{12}$)-, -N($R_{12}$)C(O)-, and -C(O)-;

m, n, and p are each independently selected from 0, 1, and 2, and m, n, and p are not simultaneously 0;

$R_9$ is selected from $C_3$-$C_{10}$ cycloalkyl, $C_6$-$C_{10}$ aryl, 5-10-membered heteroaryl, and 3-12-membered heterocyclyl, each of the $C_3$-$C_{10}$ cycloalkyl, $C_6$-$C_{10}$ aryl, 5-10-membered heteroaryl, or 3-12-membered heterocyclyl is optionally substituted by one or more $R_{14}$;

each $R_{10}$ is independently selected from $C_1$-$C_6$ alkyl, oxo, 5-10-membered heteroaryl, 3-12-membered heterocyclyl, $R_{11}$C(O)-, $R_{11}$C(O)N($R_{12}$)-, and -$NR_{12}R_{13}$, wherein each of the $C_1$-$C_6$ alkyl, 5-10-membered heteroaryl, or 3-12-membered heterocyclyl is optionally substituted by one or more $R_{15}$;

$R_{11}$ is selected from $C_1$-$C_6$ alkyl and $C_3$-$C_{10}$ cycloalkyl;

$R_{12}$ and $R_{13}$ are each independently selected from H, $C_1$-$C_6$ alkyl, and $C_3$-$C_{10}$ cycloalkyl;

each $R_{14}$ is independently selected from H, halogen, cyano, oxo, $C_1$-$C_6$ alkyl, $C_1$-$C_6$ alkoxy, -$CONR_{12}R_{13}$, and $R_{11}$C(O)-; and

each $R_{15}$ is independently selected from H, halogen, cyano, oxo, $C_1$-$C_6$ alkyl, $C_3$-$C_{10}$ cycloalkyl, and 3-12-membered heterocyclyl, wherein each of the $C_1$-$C_6$ alkyl, $C_3$-$C_{10}$ cycloalkyl, or 3-12-membered heterocyclyl is optionally substituted by one or more substituents selected from $C_1$-$C_6$ alkyl and oxo;

with the proviso that:

(1) when $R_9$ is $C_3$-$C_{10}$ cycloalkyl, $C_6$-$C_{10}$ aryl, or 5-10-membered heteroaryl, then n is not 0;

(2) when L is $-(L_1)_m-(L_2)_n-(L_3)_p-$, $L_1$ is selected from -N($R_{12}$)-, m is 1, $L_2$ is selected from $C_1$-$C_6$ alkylene, and n is 1, then $L_3$ is not -N($R_{12}$)C(O)-;

(3) when L is $-(L_1)_m-(L_2)_{a}-(L_3)_p-$, $L_1$ is selected from -N($R_{12}$)-, m is 1, $L_2$ is selected from $C_1$-$C_6$ alkylene, n is 1, p is 0, and $R_9$ is 3-12-membered heterocyclyl, then the 3-12-membered heterocyclyl is substituted by oxo; and

(4) when L is $-(L_1)_m-(L_2)_{a}-(L_3)_p-$, $L_1$ is selected from -N($R_{12}$)-, m is 1, $L_2$ is selected from $C_1$-$C_6$ alkylene, n is 1, p is 0, and $R_9$ is 5-10-membered heteroaryl, then $R_2$ is not morpholinyl.

In another aspect, the present invention provides a pharmaceutical composition, comprising an effective amount of the compound or pharmaceutically acceptable salt, stereoisomer, tautomer, polymorph, solvate, hydrate, N-oxide, isotope labeled compound, metabolite, ester, or prodrug thereof of the present invention, and one or more pharmaceutically acceptable carriers.

In yet another aspect, the present invention provides use of the compound or pharmaceutically acceptable salt, stereoisomer, tautomer, polymorph, solvate, hydrate, N-oxide, isotope labeled compound, metabolite, ester, or prodrug thereof of the present invention, or the pharmaceutical composition of the present invention in the preparation of a medicament for treating a ULK1-mediated disease in a subject in need thereof.

In another aspect, the present invention provides a method for treating a ULK1 -mediated disease in a subject in need thereof, the method comprising administering to the subject the compound or pharmaceutically acceptable salt, stereoisomer, tautomer, polymorph, solvate, hydrate, N-oxide, isotope labeled compound, metabolite, ester, or prodrug thereof of the present invention, or the pharmaceutical composition of the present invention.

In yet another aspect, the present invention provides the compound or pharmaceutically acceptable salt, stereoisomer, tautomer, polymorph, solvate, hydrate, N-oxide, isotope labeled compound, metabolite, ester, or prodrug thereof of the present invention, or the pharmaceutical composition of the present invention, for use in treating a ULK1 -mediated disease in a subject in need thereof.

In a further aspect, the present invention provides a method for preparing the compound of the present invention, comprising the steps shown in the following synthetic route A or B:

Synthetic route A

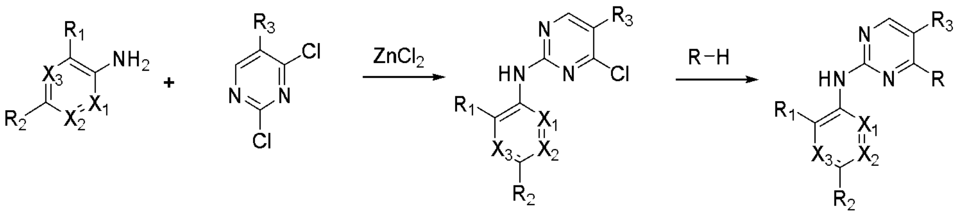


1-0 1-1 1-2 (1) or

Synthetic route B

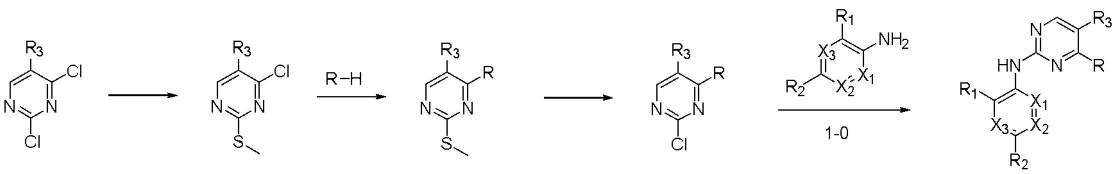


2-0 2-1 2-2 2-3 (1)

wherein: $R_1$, $R_2$, $R_3$, R, $X_1$, $X_2$, $X_3$ are as defined hereinbefore.

## Detailed Description of the Invention

### Definitions

Unless otherwise defined below, all technical and scientific terms used herein are intended to have the same meaning as commonly understood by those skilled in the art. Reference to a technique used herein is intended to refer to a technique as commonly understood in the art, including those technological changes or equivalent replacements that are obvious to those skilled in the art. While it is believed that the following terms will be well understood by those skilled in the art, the following definitions are set forth in order to better explain the present invention.

As used herein, the terms "including", "comprising", "having", "containing", or "involving" and their other variant forms herein, are inclusive or open-ended and do not exclude other unlisted elements or method steps.

As used herein, the term "alkyl" is defined as a straight or branched chain saturated aliphatic hydrocarbon group. For example, as used herein, the term "$C_{1-6}$ alkyl" refers to a straight or branched chain group having 1 to 6 carbon atoms, for example, methyl, ethyl, n-propyl, isopropyl, n-butyl, isobutyl, sec-butyl, tert-butyl, n-pentyl, n-hexyl, isopentyl, and isobutyl.

As used herein, the term "alkylene" refers to a straight or branched chain divalent alkyl.

As used herein, the term "alkenyl" refers to a straight or branched chain aliphatic hydrocarbon group having 1 and above unsaturated double bond. For example, as used herein, the term "$C_2$-$C_6$ alkenyl" refers to an alkenyl having 2 to 6 carbon atoms, for example, vinyl, 1-propenyl, 2-propenyl, 2-methyl-1-propenyl, 2-methyl-2-propenyl, 2-buten-1-yl, 3-buten-1-yl, 2-penten-1-yl, 3-penten-1-yl, 4-penten-1-yl, 5-hexen-1-yl, 4-hexen-1-yl, 3-hexen-1-yl, 2-hexen-1-yl, 3-methyl-2-buten-1-yl, 3-methyl-3-penten-1-yl, 3-methyl-2-penten-1-yl, 4-methyl-3-penten-1-yl, 4-methyl-2-penten-1-yl, and 2-methyl-2-penten-1-yl, etc. It is preferable to have one double bond.

As used herein, the term "alkynyl" refers to a straight or branched chain aliphatic hydrocarbon group having 1 or more unsaturated triple bonds. For example, as used herein, the term "$C_2$-$C_6$ alkynyl" refers to an alkenyl having 2 to 6 carbon atoms, for example, ethynyl, 1-propyn-1-yl, 2-propyn-1-yl, 2-butyn-1-yl, 3-butyn-1-yl, 2-pentyn-1-yl, 3-pentyn-1-yl, 4-pentyn-1-yl, 5-hexyn-1-yl, 4-hexyn-1-yl, 3-hexyn-1-yl, and 2-hexyn-1-yl, etc. It is preferable to have one triple bond.

As used herein, the term "alkoxy" means a group in which an oxygen atom is inserted at any reasonable position in an alkyl (as defined hereinbefore), for example, $C_{1-8}$ alkoxy, $C_{1-6}$ alkoxy, $C_{1-4}$ alkoxy, or $C_{1-3}$ alkoxy. Representative examples of $C_{1-6}$ alkoxy include, but are not limited to, methoxy, ethoxy, n-propoxy, isopropoxy, n-butoxy, isobutoxy, tert-butoxy, pentyloxy, hexyloxy, etc.

As used herein, the term "halo" or "halogen" group is defined as including fluorine, chlorine, bromine, or iodine.

As used herein, the term "haloalkyl" refers to an alkyl substituted by one or more (such as 1 to 3) identical or different halogen atoms. For example, the term "$C_{1-6}$ haloalkyl" refers to a haloalkyl having 1 to 6 carbon atoms, for example, $-CF_3$, $-C_2F_5$, $-CHF_2$, $-CH_2F$, $-CH_2CF_3$, $-CH_2Cl$, or $-CH_2CH_2CF_3$, etc.

As used herein, the term "hydroxyalkyl" refers to an alkyl substituted by one or more (such as 1 to 3) hydroxyl groups. For example, the term "$C_{1-6}$ hydroxyalkyl" refers to a hydroxyalkyl having 1 to 6 carbon atoms, for example, $-CH_2OH$, $-CH_2CH_2OH$, etc.

As used herein, the term "aminoalkyl" refers to an alkyl substituted by one or more (such as 1 to 3) amino groups. For example, the term "$C_{1-6}$ aminoalkyl" refers to an aminoalkyl having 1 to 6 carbon atoms, for example, $-CH_2NH_2$, $-CH_2CH_2NH_2$, etc.

As used herein, the term "heteroalkyl" refers to a straight or branched chain alkyl interrupted by one or more heteroatoms independently selected from N, O or $S(O)_q$ (wherein q is 0, 1, or 2). For example, the term "$C_{1-6}$ heteroalkyl" refers to a heteroalkyl having 1 to 6 carbon atoms.

As used herein, the term "heteroalkylene" refers to a straight or branched chain alkylene interrupted by one or more heteroatoms independently selected from N, O or $S(O)_q$ (wherein q is 0, 1, or 2). For example, the term "$C_{1-6}$ heteroalkylene" refers to a heteroalkylene having 1 to 6 carbon atoms.

As used herein, the term "cycloalkyl" refers to a saturated or partially unsaturated nonaromatic monocyclic or polycyclic (such as bicyclic) hydrocarbon ring, for example, a monocyclic ring, such as cyclopropyl, cyclobutyl, cyclopentyl, cyclohexyl, cycloheptyl, cyclooctyl, cyclononyl, or a bicyclic ring, including a spiro, fused (fused-ring), or bridged (bridged-ring) system, such as bicyclo[1.1.1]pentyl, bicyclo[2.2.1]heptyl, bicyclo[3.2.1]octyl, or bicyclo[5.2.0]nonyl, decalinyl, etc. The cycloalkyl has 3 to 15, for example, 3 to 10 carbon atoms. For example, the term "$C_{3-10}$ cycloalkyl" refers to a saturated or partially unsaturated non-aromatic monocyclic or polycyclic (such as bicyclic) hydrocarbon ring having 3 to 10 ring-forming carbon atoms, for example, cyclopropyl, cyclobutyl, cyclopentyl or cyclohexyl.

As used herein, the term "heterocyclyl" or "heterocycle" refers to a saturated or partially unsaturated monocyclic or polycyclic (including a spiro, fused (fused-ring) or bridged (bridged-ring) system) group, for example it has 2, 3, 4, 5, 6, 7, 8, or 9 carbon atoms and one or more (e.g., 1, 2, 3, or 4) heteroatoms independently selected from N, O, or $S(O)_q$ (wherein q is 0, 1, or 2) in the ring, for example, a 3-12-membered heterocyclyl, a 3-10-membered heterocyclyl, a 3-6-membered heterocyclyl, a 5-6-membered heterocyclyl, etc. Representative examples of heterocyclyl include, but are not limited to, oxiranyl, aziridinyl, azetidinyl, oxetanyl, tetrahydrofuranyl, pyrrolidinyl, pyrrolidinonyl, imidazolidinyl, pyrazolidinyl, tetrahydropyranyl, piperidinyl, morpholinyl, dithianyl, thiomorpholinyl, piperazinyl, trithianyl, etc. As used herein, the term "nitrogen-containing heterocycle" refers to a heterocycle having at least one N atom and optionally other heteroatoms in the ring, including a monocyclic or polycyclic (including a spiro, fused (fused-ring) or bridged (bridged-ring) system) heterocycle.

As used herein, the term "aryl" or "aromatic ring" refers to an all-carbon monocyclic or fused-ring polycyclic aromatic group having a conjugated $\pi$-electron system. For example, the term "$C_{6-10}$ aryl" or "$C_{6-10}$ aromatic ring" refer to an aromatic group containing 6 to 10 carbon atoms, such as phenyl (ring) or naphthalenyl (ring).

As used herein, the term "heteroaryl" or "heteroaromatic ring" refers to a monocyclic, bicyclic or tricyclic aromatic ring system containing at least one heteroatom selected from N, O and S, for example having 5, 6, 8, 9, 10, 11, 12, 13, or 14 ring atoms, particularly containing 1 or 2 or 3 or 4 or 5 or 6 or 9 or 10 carbon atoms, and, furthermore, in each case, it can be benzofused. For example, examples of heteroaryl or heteroaromatic ring include, but are not limited to, thienyl (ring), furanyl (ring), pyrrolyl (ring), oxazolyl (ring), thiazolyl (ring), imidazolyl (ring), pyrazolyl (ring), isoxazolyl (ring), isothiazolyl (ring), oxadiazolyl (ring), triazolyl (ring), thiadiazolyl (ring), etc., as well as their benzo derivatives; or pyridinyl (ring), pyridazinyl (ring), pyrimidinyl (ring), pyrazinyl (ring), triazinyl (ring), etc., as well as their benzo derivatives.

The term "substitution" refers to a selective replacement of one or more (e.g., 1, 2, 3, or 4) hydrogens on a specified atom by a designated group, with the proviso that the normal valence of the specified atom in the present case is not exceeded and the substitution forms a stable compound. A combination of substituents and/or variables is permitted only when this combination forms a stable compound.

If a substituent is described as "optionally substituted by ......", the substituent may be (1) unsubstituted or (2) substituted. If the carbon of the substituent is described as being optionally substituted by one or more of the substituents in the list, one or more hydrogens on the carbon (to the extent that any hydrogens are present) may be substituted individually and/or together by an independently selected substituent or not substituted. If the nitrogen of the substituent is described as being optionally substituted by one or more of the substituents in the list, each of one or more hydrogens on the nitrogen (to the extent that any hydrogens are present) may be substituted by an independently selected substituent or not substituted.

If a substituent is described as being "independently selected from" a group of groups, each substituent is selected independently of the other. Therefore, each substituent may be the same as or different from another (other) substituent.

As used herein, the term "one or more" means 1 or more than 1 under a reasonable condition, for example, 2, 3, 4, 5, 6, 7, 8, 9, or 10.

Unless otherwise specified, as used herein, the point of attachment of a substituent may be from any suitable position of the substituent.

When the bond of a substituent is shown as passing through a bond connecting two atoms in a ring, such a substituent may be bonded to any ring-forming atom in the substitutable ring.

The present invention also includes all pharmaceutically acceptable isotope labeled compounds that are identical to the compounds of the present invention, except that one or more atoms are replaced by atoms having the same atomic number but with an atomic mass or mass number different from which predominates in nature. Examples of an isotope suitable for inclusion in the compound of the present invention include, but are not limited to, a hydrogen isotope (e.g., $^{2}H$, $^{3}H$, deuterium D, tritium T); a carbon isotope (e.g., $^{11}C$, $^{13}C$, and $^{14}C$); a chlorine isotope (e.g., $^{37}Cl$); a fluorine isotope (e.g., $^{18}F$); an iodine isotope (e.g., $^{123}I$ and $^{125}I$); a nitrogen isotope (e.g., $^{13}N$ and $^{15}N$); an oxygen isotope (e.g., $^{15}O$, $^{17}O$ and $^{18}O$); a phosphorus isotope (e.g., $^{32}P$); and a sulfur isotope (e.g., $^{35}S$). Certain isotope labeled compounds of the present invention (e.g., those incorporating a radioisotope) can be used in a pharmaceutical and/or substrate tissue distribution study (e.g., analysis). Radioisotope tritium (i.e., $^{3}H$) and carbon-14 (i.e., $^{14}C$) are particularly useful for this purpose due to their ease of incorporation and detection. Substitution with a positron emission isotope (e.g., $^{11}C$, $^{18}F$, $^{15}O$, and $^{13}N$) can be used to examine substrate receptor occupancy in a positron emission tomography (PET) study. An isotope labeled compound of the present invention can be prepared by a method similar to those described in the accompanying routes and/or an example and preparation, by using an appropriate isotope labeled reagent instead of a previously used unlabeled reagent. The pharmaceutically acceptable solvate of the present invention includes those in which a crystallization solvent can be substituted by an isotope, for example, $D_2O$, acetone-$d_6$ or DMSO-$d_6$.

The term "stereoisomer" represents an isomer formed due to at least one asymmetric center. In compounds with one or more (e.g., 1, 2, 3, or 4) asymmetric centers, racemic mixtures, single enantiomers, diastereomer mixtures, and individual diastereomers can be produced. A specific individual molecule can also exist as a geometric isomer (cis/trans).

Similarly, the compound of the present invention can exist as a mixture of two or more structurally different forms in rapid equilibrium (commonly referred to as tautomers). Representative examples of tautomers include keto-enol tautomers, phenol-keto tautomers, nitroso-oxime tautomers, imine-enamine tautomers, etc. It should be understood that the scope of the present application covers all such isomers or mixtures thereof in any proportion (e.g., 60%, 65%, 70%, 75%, 80%, 85%, 90%, 95%, 96%, 97%, 98%, 99%).

The present invention covers all possible crystalline forms or polymorphs of the compound of the present invention, which may be a single polymorph or a mixture of more than one polymorphs in any proportion.

It should also be understood that certain compounds of the present invention may exist in a free form for treatment, or where appropriate, exist in their pharmaceutically acceptable derivative forms. In the present invention, a pharmaceutically acceptable derivative includes, but is not limited to, a pharmaceutically acceptable salt, solvate, metabolite or prodrug that, when administered to a patient in need thereof, can directly or indirectly provide the compound of the present invention or its metabolite or residue. Therefore, when referring to "the compound of the present invention" herein, it is also intended to cover the various derivative forms of the compound described above.

The pharmaceutically acceptable salt of the compound of the present invention includes an acid addition salt and a base addition salt thereof. A suitable acid addition salt is formed from an acid that forms a pharmaceutically acceptable salt. A suitable base addition salt is formed from a base that forms a pharmaceutically acceptable salt. For a review of a suitable salt, see "Handbook of Pharmaceutical Salts: Properties, Selection, and Use" by Stahl and Wermuth (Wiley-VCH, 2002). The methods for preparing a pharmaceutically acceptable salt of the compound of the present invention are known to those skilled in the art.

The compound of the present invention may exist in the form of a solvate (preferably a hydrate), wherein the compound of the present invention contains a polar solvent, particularly, for example, water, methanol, or ethanol, as a structural element of the lattice of the compound. The amount of the polar solvent, particularly water, can exist in a stoichiometric or non-stoichiometric ratio.

Those skilled in the art will understand that not all nitrogen-containing heterocycles are capable of forming N-oxides because nitrogen requires available lone pairs of electrons to be oxidized to an oxide; those skilled in the art will identify nitrogen-containing heterocycles that are capable of forming N-oxides. Those skilled in the art will also recognize that tertiary amines can form N-oxides. The synthetic methods for preparing N-oxides of heterocycles and tertiary amines are well known to those skilled in the art, including the oxidation of heterocycles and tertiary amines with peroxy acids such as peracetic acid and m-chloroperoxybenzoic acid (MCPBA), hydrogen peroxide, alkyl hydrogen peroxide such as tert-butyl hydrogen peroxide, sodium perborate, and dioxirane such as dimethyldioxirane. These methods for preparing N-oxides have been extensively described and reviewed in literatures, see, for example: T. L. Gilchrist, Comprehensive Organic Synthesis, vol. 7, pp 748-750; A. R. Katritzky and A. J. Boulton, Eds., Academic Press; and G. W. H. Cheeseman and E. S. G. Werstiuk, Advances in Heterocyclic Chemistry, vol. 22, pp 390-392, A. R. Katritzky and A. J. Boulton, Eds., Academic Press.

The scope of the present invention also includes a metabolite of the compound of the present invention, i.e., a substance formed in the body upon administration of the compound of the present invention. Such products can be generated, for example, by oxidation, reduction, hydrolysis, amidation, deamidation, esterification, enzymatic hydrolysis, etc., of the administered compound. Therefore, the present invention includes a metabolite of the compound of the present invention, including a compound prepared by a method of contacting the compound of the present invention with a mammal for a time sufficient to produce its metabolite.

The present invention further includes, within its scope, prodrugs of the compound of the present invention, which are certain derivatives of the compound of the present invention that may themselves have a little or no pharmacological activity, and which, when administered to or on the body, can be converted, for example, by hydrolytic cleavage into the compound of the present invention having the desired activity. Typically, such a prodrug will be a functional group derivative of the compound, which is readily converted in vivo into a compound with the desired therapeutic activity. Other information regarding the use of prodrugs can be found in "Pro-drugs as Novel Delivery Systems", Vol. 14, ACS Symposium Series (T. Higuchi and V. Stella), and "Bioreversible Carriers in Drug Design," Pergamon Press, 1987 (edited by E. B. Roche, American Pharmaceutical Association). The prodrug of the present invention can be prepared, for example, by replacing an appropriate functional group present in the compound of the present invention with a certain moiety known to those skilled in the art as "pro-moiety (e.g., described in "Design of Prodrugs", H. Bundgaard (Elsevier, 1985))".

The present invention also covers the compound of the present invention containing a protecting group. In any process for preparing the compound of the present invention, it may be necessary and/or desirable to protect sensitive or reactive groups on any relevant molecules, thereby forming a chemically protected form of the compound of the present invention. This can be achieved by a conventional protecting group, for example, those protecting groups described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991, these references are incorporated herein by reference. The protecting group can be removed at an appropriate subsequent stage using methods known in the art.

The term "about" refers to within the range of 10%, preferably ±5%, more preferably ±2% of the stated numerical value.

## Compounds

One object of the present invention is to provide a compound of formula 1 or a pharmaceutically acceptable salt, a stereoisomer, a tautomer, a polymorph, a solvate, a hydrate, an N-oxide, an isotope labeled compound, a metabolite, an ester, or a prodrug thereof:

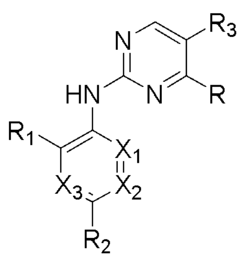

(1)

wherein $X_1$ is CH or N; $X_2$ is N or $CR_4$; $X_3$ is N or $CR_5$;

$R_1$ is selected from H, halogen, cyano, nitro, $C_1$-$C_6$ alkyl, $C_2$-$C_6$ alkenyl, $C_2$-$C_6$ alkynyl, $C_1$-$C_6$ alkoxy, $C_3$-$C_{10}$ cycloalkyl, 3-12-membered heterocyclyl, $C_6$-$C_{10}$ aryl and 5-10-membered heteroaryl, wherein each of the $C_1$-$C_6$ alkyl, $C_2$-$C_6$ alkenyl, $C_2$-$C_6$ alkynyl, $C_1$-$C_6$ alkoxy, $C_3$-$C_{10}$ cycloalkyl, 3-12-membered heterocyclyl, $C_6$-$C_{10}$ aryl or 5-10-membered heteroaryl is optionally substituted by one or more $R_6$;

$R_2$ is selected from H, halogen, cyano, nitro, C2-C6 alkenyl, C2-C6 alkynyl, C3-C10 cycloalkyl, 3-12-membered

heterocyclyl, C6-C10 aryl, 5-10-membered heteroaryl, $R_7O$-, - $SR_7$, $-NR_7R_8$, $-NHCONR_7R_8$, $-NHCOR_7$, $-CONR_7R_8$, and $-SO_2R_7$, wherein each of the C2-C6 alkenyl, C2-C6 alkynyl, C3-C10 cycloalkyl, 3-12-membered heterocyclyl, C6-C10 aryl, or 5-10-membered heteroaryl is optionally substituted by one or more R6;

$R_3$ is selected from H, cyano, halogen, $C_1$-$C_6$ alkyl, $C_1$-$C_6$ hydroxyalkyl, $C_1$-$C_6$ aminoalkyl, $C_1$-$C_6$ haloalkyl, $C_1$-$C_6$ heteroalkyl, $C_2$-$C_6$ alkynyl, and $C_3$-$C_{10}$cycloalkyl;

$R_4$ is selected from H, halogen, cyano, nitro, $C_1$-$C_6$ alkyl, $C_2$-$C_6$ alkenyl, $C_2$-$C_6$ alkynyl, $C_3$-$C_{10}$ cycloalkyl, 3-12-membered heterocyclyl, $C_6$-$C_{10}$ aryl, 5-10-membered heteroaryl, $R_7O$-, $-NR_7R_8$, and $-NHCONR_7R_8$, wherein each of the $C_1$-$C_6$ alkyl, $C_2$-$C_6$ alkenyl, $C_2$-$C_6$ alkynyl, $C_3$-$C_{10}$ cycloalkyl, 3-12-membered heterocyclyl, $C_6$-$C_{10}$ aryl, or 5-10-membered heteroaryl is optionally substituted by one or more $R_6$;

or, $R_2$ and $R_4$ together with the groups that they are attached to, form 3-12-membered monocyclic or spiro heterocycle, $C_6$-$C_{10}$ aromatic ring, or 5-10-membered heteroaromatic ring, wherein each of the 3-12-membered monocyclic or spiro heterocycle, $C_6$-$C_{10}$ aromatic ring, or 5-10-membered heteroaromatic ring is substituted by one or more $R_6$;

$R_5$ is selected from H, $C_1$-$C_6$ alkyl, $C_2$-$C_6$ alkenyl, $C_2$-$C_6$ alkynyl, $C_3$-$C_{10}$ cycloalkyl, 3-12-membered heterocyclyl, $C_6$-$C_{10}$ aryl, and 5-10-membered heteroaryl;

or, $R_1$ and $R_5$ together with the groups that they are attached to, form 5-6-membered heterocycle, benzene ring, or 5-6-membered heteroaromatic ring;

each $R_6$ is independently selected from halogen, cyano, nitro, amino, -NHCO($C_1$-$C_6$ alkyl), $C_1$-$C_6$ alkyl, $C_2$-$C_6$ alkenyl, $C_2$-$C_6$ alkynyl, $C_3$-$C_{10}$ cycloalkyl, 3-12-membered heterocyclyl, $C_6$-$C_{10}$ aryl, and 5-10-membered heteroaryl, wherein each of the $C_3$-$C_{10}$ cycloalkyl, 3-12-membered heterocyclyl, $C_6$-$C_{10}$ aryl, or 5-10-membered heteroaryl is optionally substituted by one or more halogen, hydroxyl, cyano, $C_1$-$C_6$ alkyl, $C_1$-$C_6$ alkoxy;

$R_7$, $R_8$ are each independently selected from H, $C_1$-$C_6$ alkyl, $C_3$-$C_{10}$ cycloalkyl, 3-12-membered heterocyclyl, and $C_6$-$C_{10}$ aryl, wherein each of the $C_1$-$C_6$ alkyl, $C_3$-$C_{10}$ cycloalkyl, 3-12-membered heterocyclyl, or $C_6$-$C_{10}$ aryl is optionally substituted by one or more $R_6$;

or $R_7$ and $R_8$ together with the groups that they are attached to, form 5-6-membered heterocycle or 5-6-membered heteroaromatic ring;

R is selected from 3-12-membered heterocyclyl, 5-10-membered heteroaryl, and $-L-R_9$, wherein each of the 3-12-membered heterocyclyl or 5-10-membered heteroaryl is optionally substituted by one or more $R_{10}$;

L is $-(L_1)_m-(L_2)_n-(L_3)_p-$;

$L_1$ is selected from -O-, $-N(R_{12})-$, $-C(R_{12})(R_{13})-O-$, $-N(R_{12})CO-$, and -S-;

$L_2$ is selected from $C_1$-$C_6$ alkylene, $C_1$-$C_6$ heteroalkylene, $C_3$-$C_{10}$ cycloalkylene, and 3-10-membered heterocyclylene;

$L_3$ is selected from $-N(R_{12})-$, $-N(R_{12})C(O)-$, and -C(O)-;

m, n, and p are each independently selected from 0, 1, and 2, and m, n, and p are not simultaneously 0;

$R_9$ is selected from $C_3$-$C_{10}$ cycloalkyl, $C_6$-$C_{10}$ aryl, 5-10-membered heteroaryl, and 3-12-membered heterocyclyl, each of the $C_3$-$C_{10}$ cycloalkyl, $C_6$-$C_{10}$ aryl, 5-10-membered heteroaryl, or 3-12-membered heterocyclyl is optionally substituted by one or more $R_{14}$;

each $R_{10}$ is independently selected from $C_1$-$C_6$ alkyl, oxo, 5-10-membered heteroaryl, 3-12-membered heterocyclyl, $R_{11}C(O)$-, $R_{11}C(O)N(R_{12})$-, and $-NR_{12}R_{13}$, wherein each of the $C_1$-$C_6$ alkyl, 5-10-membered heteroaryl, or 3-12-membered heterocyclyl is optionally substituted by one or more $R_{15}$;

$R_{11}$ is selected from $C_1$-$C_6$ alkyl and $C_3$-$C_{10}$ cycloalkyl;

$R_{12}$ and $R_{13}$ are each independently selected from H, $C_1$-$C_6$ alkyl, and $C_3$-$C_{10}$ cycloalkyl;

each $R_{14}$ is independently selected from H, halogen, cyano, oxo, $C_1$-$C_6$ alkyl, $C_1$-$C_6$ alkoxy, $-CONR_{12}R_{13}$, and $R_{11}C(O)$-; and

each $R_{15}$ is independently selected from H, halogen, cyano, oxo, $C_1$-$C_6$ alkyl, $C_3$-$C_{10}$ cycloalkyl, and 3-12-membered heterocyclyl, wherein each of the $C_1$-$C_6$ alkyl, $C_3$-$C_{10}$ cycloalkyl, or 3-12-membered heterocyclyl is optionally substituted by one or more substituents selected from $C_1$-$C_6$ alkyl and oxo;

with the proviso that:

(1) when $R_9$ is $C_3$-$C_{10}$ cycloalkyl, $C_6$-$C_{10}$ aryl, or 5-10-membered heteroaryl, then n is not 0;

(2) when L is $-(L_1)_m-(L_2)_{a-}(L_3)_p-$, $L_1$ is selected from $-N(R_{12})-$, m is 1, $L_2$ is selected from $C_1$-$C_6$ alkylene, and n is 1, then $L_3$ is not $-N(R_{12})C(O)-$;

(3) when L is $-(L_1)_m-(L_2)_{a-}(L_3)_p-$, $L_1$ is selected from $-N(R_{12})-$, m is 1, $L_2$ is selected from $C_1$-$C_6$ alkylene, n is 1, p is 0, and $R_9$ is 3-12-membered heterocyclyl, then the 3-12-membered heterocyclyl is substituted by oxo; and

(4) when L is $-(L_1)_m-(L_2)_n-(L_3)_p-$, $L_1$ is selected from $-N(R_{12})-$, m is 1, $L_2$ is selected from $C_1$-$C_6$ alkylene, n is 1, p is 0, and $R_9$ is 5-10-membered heteroaryl, then $R_2$ is not morpholinyl.

According to some embodiments of the present invention, at most one of $X_1$, $X_2$, $X_3$ is N.

According to some embodiments of the present invention, $R_1$ is selected from H, $C_1$-$C_6$ alkoxy, and $C_3$-$C_6$

cycloalkyl.

In some embodiments of the present invention, $R_1$ is selected from H, cyclopropyl, and methoxy.

According to some embodiments of the present invention, $R_5$ is H.

According to some embodiments of the present invention, $R_1$ and $R_5$ together with the groups that they are attached to, form 5-6-membered heterocycle.

In some embodiments of the present invention, $R_1$ and $R_5$ together with the groups that they are attached to, form 5-6-membered oxygen-containing heterocycle.

In some embodiments of the present invention,

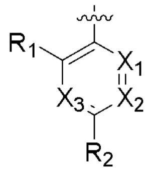

moiety is

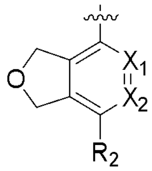

or

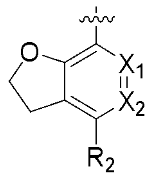

,

wherein the wavy line 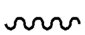 represents the point of attachment of the group to the rest of the molecule.

According to some embodiments of the present invention, $R_3$ is selected from H and $C_1$-$C_6$ haloalkyl.

In some embodiments of the present invention, $R_3$ is selected from $C_1$-$C_6$ haloalkyl.

In some embodiments of the present invention, $R_3$ is selected from H and trifluoromethyl.

In some embodiments of the present invention, $R_3$ is selected from trifluoromethyl.

According to some embodiments of the present invention, $R_2$ is selected from H, $C_2$-$C_6$ alkynyl, 3-10-membered monocyclic, spiro or fused heterocyclyl, and $R_7O$-, wherein each of the $C_2$-$C_6$ alkynyl or 3-10-membered monocyclic, spiro or fused heterocyclyl is optionally substituted by 1 or 2 $R_6$.

In some embodiments of the present invention, $R_2$ is selected from $C_2$-$C_6$ alkynyl, 3-10-membered monocyclic, spiro or fused heterocyclyl, and $R_7O$-, wherein each of the $C_2$-$C_6$ alkynyl or 3-10-membered monocyclic, spiro or fused heterocyclyl is optionally substituted by 1 or 2 $R_6$.

In some embodiments of the present invention, $R_2$ is selected from H, $C_2$-$C_4$ alkynyl, 3-6-membered monocyclic heterocyclyl, a 7-10-membered spiro or fused heterocyclyl, and $R_7O$-, wherein each of the $C_2$-$C_4$ alkynyl, 3-6-membered monocyclic heterocyclyl, 7-10-membered spiro or fused heterocyclyl is optionally substituted by 1 or 2 $R_6$.

In some embodiments of the present invention, each $R_6$ is independently selected from $C_1$-$C_6$ alkyl and 3-6-membered heterocyclyl, wherein the 3-6-membered heterocyclyl is optionally substituted by $C_1$-$C_6$ alkyl.

In some embodiments of the present invention, $R_7$ is selected from $C_1$-$C_6$ alkyl and a 5-6-membered heterocyclyl, wherein the $C_1$-$C_6$ alkyl is optionally substituted by 3-6-membered heterocyclyl, each of the 5-6-membered heterocyclyl, 3-6-membered heterocyclyl is optionally substituted by $C_1$-$C_6$ alkyl.

In some embodiments of the present invention, $R_7$ is selected from $C_1$-$C_6$ alkyl and 5-6-membered heterocyclyl, wherein the 5-6-membered heterocyclylis optionally substituted by $C_1$-$C_6$ alkyl.

According to some embodiments of the present invention, $R_2$ is selected from $C_2$-$C_6$ alkynyl, 3-10-membered monocyclic, spiro or fused heterocyclyl, and $R_7O$-, wherein each of the $C_2$-$C_6$ alkynyl or 3-10-membered monocyclic, spiro or fused heterocyclyl is optionally substituted by 1 or 2 $R_6$;

each $R_6$ is independently selected from $C_1$-$C_6$ alkyl and 3-6-membered heterocyclyl, wherein the 3-6-membered heterocyclyl is optionally substituted by $C_1$-$C_6$ alkyl; and

$R_7$ is selected from $C_1$-$C_6$ alkyl and 5-6-membered heterocyclyl, wherein the 5-6-membered heterocyclylis optionally substituted by $C_1$-$C_6$ alkyl.

In some embodiments of the present invention, $R_2$ is selected from H,

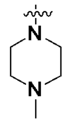, 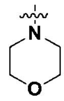,

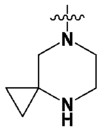, 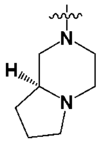, 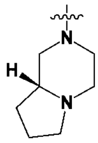, 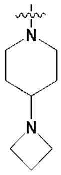, 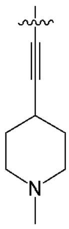, 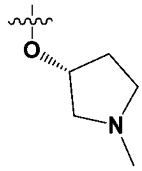, and 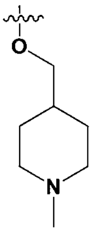,

wherein the wavy line represents the point of attachment of the group to the rest of the molecule.

In some embodiments of the present invention, $R_2$ is selected from

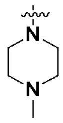, 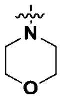, 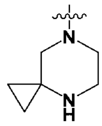,

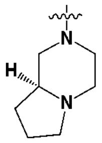, 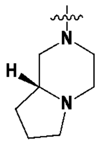, 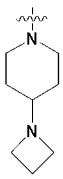, 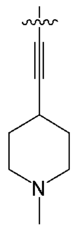, 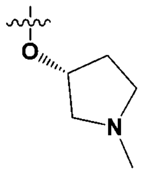, and 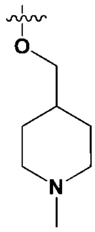,

wherein the wavy line represents the point of attachment of the group to the rest of the molecule.

According to some embodiments of the present invention, $R_4$ is selected from H, $C_1$-$C_6$ alkyl, $-NR_7R_8$, and $-NHCONR_7R_8$, wherein the $C_1$-$C_6$ alkyl is optionally substituted by 3-6-membered heterocyclyl, $R_7$ is selected from H, $R_8$ is selected from $C_6$-$C_{10}$ aryl, wherein the $C_6$-$C_{10}$ aryl is optionally substituted by amino, $-NHCO(C_1$-$C_6$ alkyl), or $R_7$ and $R_8$ together with the groups that they are attached to, form 5-6-membered heterocycle.

In some embodiments of the present invention, H,

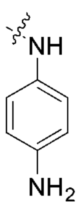, 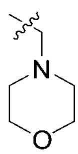, 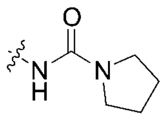,

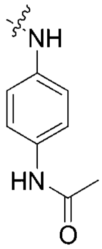
.

In some embodiments of the present invention, $R_4$ is H.

According to some embodiments of the present invention, $R_2$ and $R_4$ together with the groups that they are attached to, form 6-12-membered nitrogen-containing monocyclic heterocycle or nitrogen-containing spiro heterocycle, each of the 6-12-membered nitrogen-containing monocyclic heterocycle or nitrogen-containing spiro heterocycle is optionally substituted by one or more $R_6$.

In some embodiments of the present invention, $R_2$ and $R_4$ together with the groups that they are attached to, form 6-12-membered nitrogen-containing monocyclic heterocycle or nitrogen-containing spiro heterocycle, the 6-12-membered nitrogen-containing heterocycle is substituted by 1 or 2 $C_1$-$C_6$ alkyl.

In some embodiments of the present invention, $R_2$ and $R_4$ together with the groups that they are attached to, form 6-7-membered nitrogen-containing monocyclic heterocycle or a 7-12-membered nitrogen-containing spiro heterocycle, each of the 6-7-membered nitrogen-containing monocyclic heterocycle or 7-12-membered nitrogen-containing spiro heterocycle is substituted by 1 or 2 $C_1$-$C_6$ alkyl.

In some embodiments of the present invention,

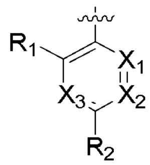

moiety is

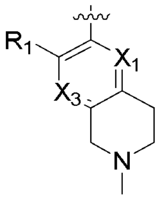
,

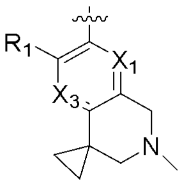
,
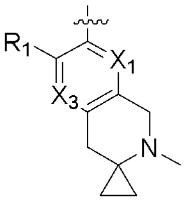
,
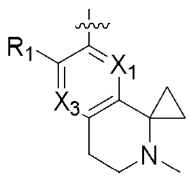
,
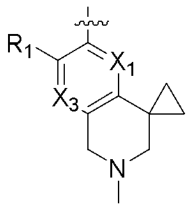
,
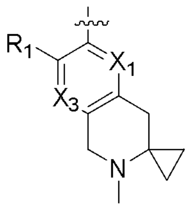
,

or

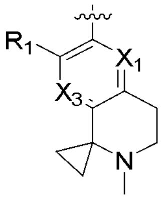
,

wherein the wavy line 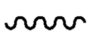 represents the point of attachment of the group to the rest of the molecule.

In some embodiments of the present invention,

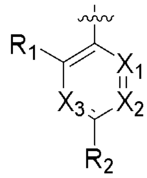

moiety is

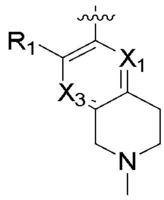

,

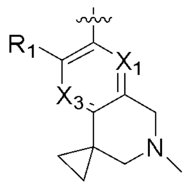

, or

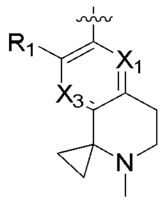

,

wherein the wavy line represents the point of attachment of the group to the rest of the molecule.

In some embodiments of the present invention,

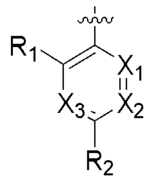

moiety is

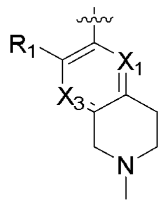

or

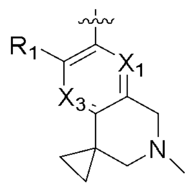

,

wherein the wavy line represents the point of attachment of the group to the rest of the molecule.

In some embodiments of the present invention,

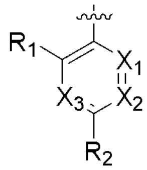

moiety is

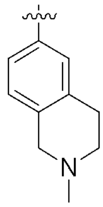
,

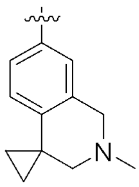
, 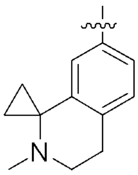
, 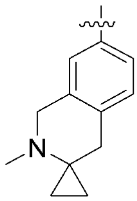
, 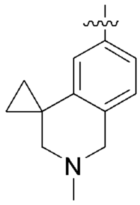
, 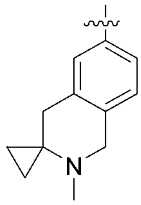
, or 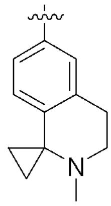
.

In some embodiments of the present invention,

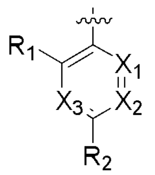

moiety is

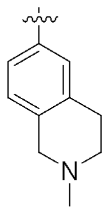

or

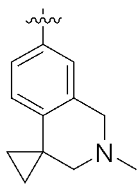
.

The present invention covers the compound of formula (1) obtained by any combination of the above preferred groups.

According to some embodiments of the present invention, the compound of the present invention is the compound of formula 1A:

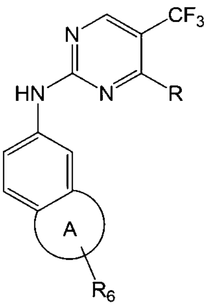

(1A)

wherein R is as defined hereinbefore for the compound of formula (1); ring A is 6-12-membered nitrogen-containing monocyclic heterocycle or nitrogen-containing spiro heterocycle; and $R_6$ is $C_1$-$C_6$ alkyl.

According to some embodiments of the present invention, in the compound of formula 1A,

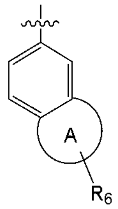

moiety is

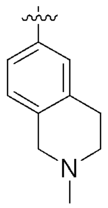 , 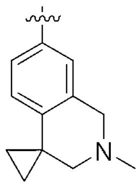 , 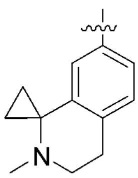 , 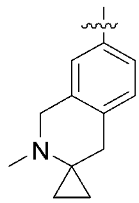 , 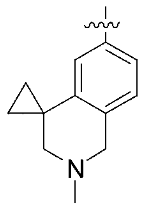 ,

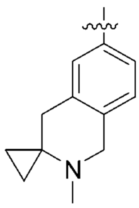 , or 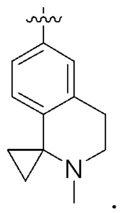 .

According to some embodiments of the present invention, in the compound of formula 1A,

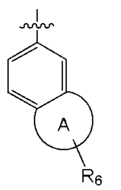

moiety is

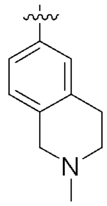, 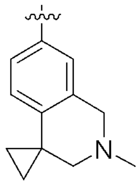, or 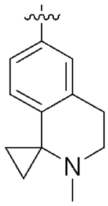,

wherein the wavy line represents the point of attachment of the group to the rest of the molecule.

According to some embodiments of the present invention, in the compound of formula 1A,

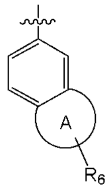

moiety is

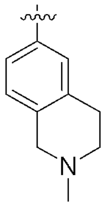 or 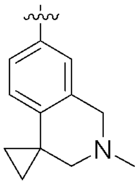.

According to some embodiments of the present invention, the compound of the present invention is the compound of formula 1B':

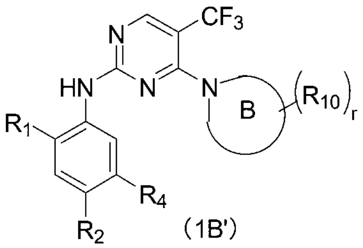

(1B')

wherein $R_1$, $R_2$, $R_4$, $R_{10}$ are as defined hereinbefore for the compound of formula (1); ring B is selected from 3-12-membered heterocyclyl and 5-10-membered heteroaryl; and r is selected from 0, 1, and 2.

In some embodiments of the present invention, in the compound of formula 1B' of the present invention, ring B is selected from 3-12-membered heterocyclyl.

According to some embodiments of the present invention, the compound of the present invention is the compound of formula 1B:

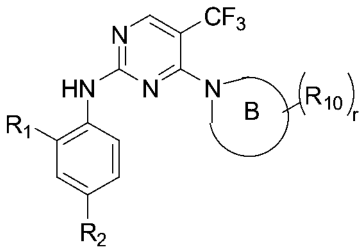

(1B)

wherein $R_1$, $R_2$, and $R_{10}$ are as defined hereinbefore for the compound of formula (1); ring B is selected from 3-12-membered heterocyclyl and 5-10-membered heteroaryl; and r is selected from 0, 1, and 2.

In some embodiments of the present invention, in the compound of formula 1B of the present invention, ring B is selected from 3-12-membered heterocyclyl.

According to some embodiments of the present invention, in the compound of formula 1B' or 1B, ring B is 5-12-membered spiro heterocyclyl or 5-12-membered bridged heterocyclyl, preferably 7-12-membered spiro heterocyclyl or 7-12-membered bridged heterocyclyl, preferably

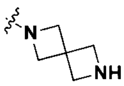 , 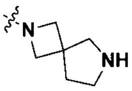 , 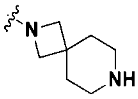 , 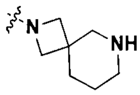 , 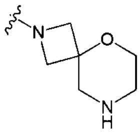 ,

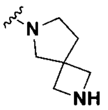 , 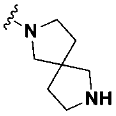 , 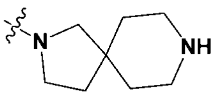 , 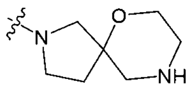 , 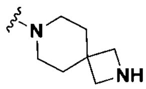 , 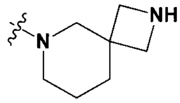 ,

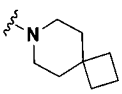 , 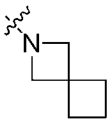 , 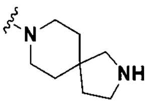 , 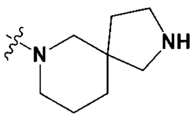 , 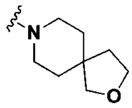 , 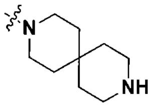 , 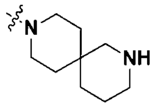 ,

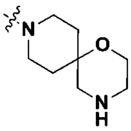 , 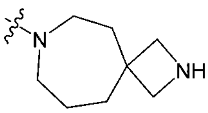 , 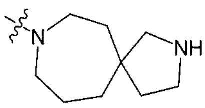 , 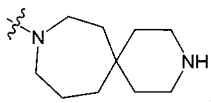 , 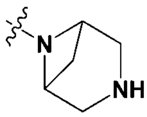 , 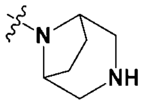 ,

or

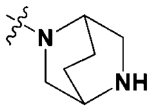 ,

more preferably

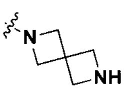 , 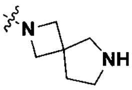 , 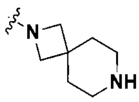 , 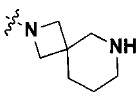 ,

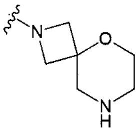 , 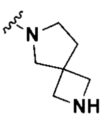 , 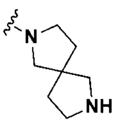 , 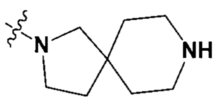 , 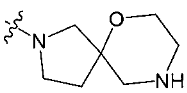 , 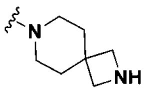 ,

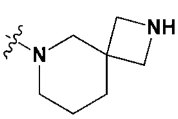 , 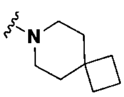 , 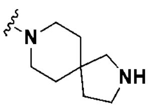 , 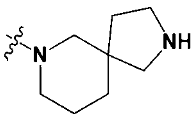 , 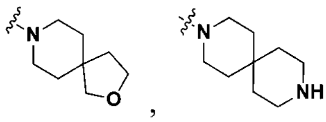 , ,

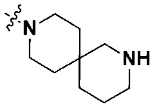, 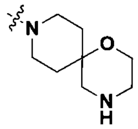, 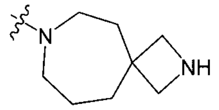, 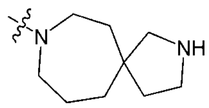, 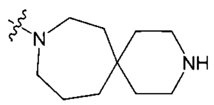,

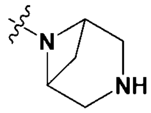, 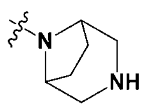, or 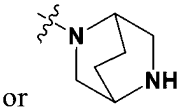;

and

each $R_{10}$ is independently selected from $C_1$-$C_6$ alkyl, oxo, 3-6-membered heterocyclyl, and -$NR_{12}R_{13}$, preferably, each $R_{10}$ is independently selected from methyl, oxo, amino, and morpholinyl.

According to some embodiments of the present invention, in the compound of formula 1B' or 1B, ring B is 3-10-membered monocyclic heterocyclyl or 5-10-membered heteroaryl, preferably 4-7-membered monocyclic heterocyclyl or 5-6-membered heteroaryl, more preferably azetidinyl, pyrrolidinyl, piperidinyl, piperazinyl, azepanyl, 1,4-diazepanyl or pyrazolyl;

each $R_{10}$ is independently selected from $C_1$-$C_6$ alkyl, oxo, 5-6-membered heteroaryl, 5-7-membered heterocyclyl, $R_{11}C(O)$-, and $R_{11}C(O)NH$-, wherein each of the $C_1$-$C_6$ alkyl, 5-6-membered heteroaryl, or 5-7-membered heterocyclyl is optionally substituted by 1-4 $R_{15}$;

$R_{11}$ is $C_4$-$C_6$ cycloalkyl; and

each $R_{15}$ is independently selected from halogen, oxo, $C_1$-$C_6$ alkyl, $C_3$-$C_6$ cycloalkyl, and 3-6-membered heterocyclyl, wherein each of the 3-6-membered heterocyclyl is optionally substituted by one or more substituents selected from $C_1$-$C_6$ alkyl and oxo,

preferably, ring B is 3-10-membered monocyclic heterocyclyl, preferably 4-7-membered monocyclic heterocyclyl, more preferably azetidinyl, pyrrolidinyl, piperidinyl, piperazinyl, azepanyl, 1,4-diazepanyl; preferably, each $R_{10}$ is independently selected from $C_1$-$C_6$ alkyl, oxo, 5-6-membered heteroaryl, 4-7-membered heterocyclyl, $R_{11}C(O)$-, and $R_{11}C(O)NH$-, wherein each of the $C_1$-$C_6$ alkyl, 5-6-membered heteroaryl, or 4-7-membered heterocyclyl is optionally substituted by 1-4 $R_{15}$.

preferably, each $R_{10}$ is independently selected from $C_1$-$C_6$ alkyl, oxo, pyrazolyl, piperidinyl, pyrrolidinyl, azetidinyl, $C_4$-$C_6$ cycloalkyl-C(O)-, and $C_4$-$C_6$ cycloalkyl-C(O)-NH-, wherein each of the azetidinyl, pyrrolidinyl, or piperidinyl is optionally substituted by 1-4 $C_1$-$C_6$ alkyl or oxo, the $C_1$-$C_6$ alkyl is optionally substituted by 1-4 substituent selected from fluorine and $C_3$-$C_6$ cycloalkyl, the pyrazolyl is optionally substituted by 1-4 $C_1$-$C_6$ alkyl;

preferably, each $R_{10}$ is independently selected from $C_1$-$C_6$ alkyl, oxo, pyrazolyl, piperidinyl, pyrrolidinyl, azetidinyl, $C_4$-$C_6$ cycloalkyl-C(O)-, and $C_4$-$C_6$ cycloalkyl-C(O)-NH-, wherein each of the azetidinyl, pyrrolidinyl, or piperidinyl is optionally substituted by 1-4 $C_1$-$C_6$ alkyl or oxo, the $C_1$-$C_6$ alkyl is optionally substituted by 1-4 substituent selected from fluorine and $C_3$-$C_6$ cycloalkyl;

more preferably, each $R_{10}$ is independently selected from methyl, oxo, pyrazolyl, piperidinyl, 3-methyl-1*H*-pyrazolyl, N-methylazetidinyl, N-methylpiperidinyl, cyclobutylformyl, cyclobutylformylamino, oxopyrrolidinyl, oxopiperidinyl,

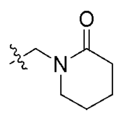,

and

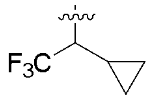,

wherein the wavy line represents the point of attachment of the group to the rest of the molecule;

further preferably, each $R_{10}$ is independently selected from methyl, oxo, pyrazolyl, piperidinyl, 3-methyl-1*H*-pyrazolyl, N-methylazetidinyl, N-methylpiperidinyl, cyclobutylformyl, cyclobutylformylamino, oxopyrrolidinyl, oxopiperidinyl and

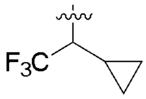,

wherein the wavy line represents the point of attachment of the group to the rest of the molecule.

According to some embodiments of the present invention, the compound of formula 1B is the compound of formula 1B-1, 1B-2, 1B-3, 1B-4, or 1B-5:

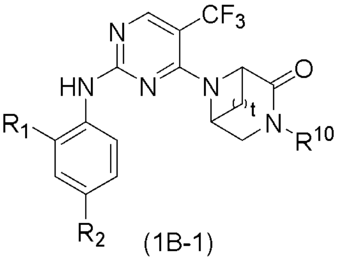
(1B-1)
,
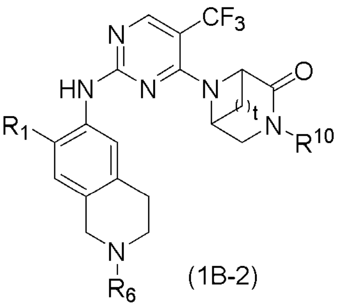
(1B-2)
,
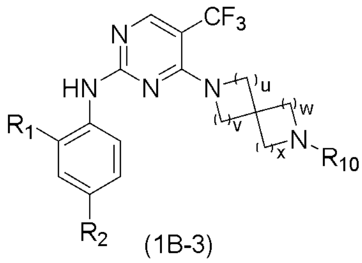
(1B-3)
,
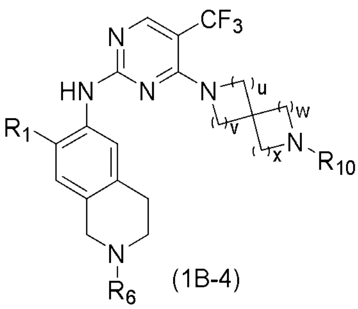
(1B-4)
,
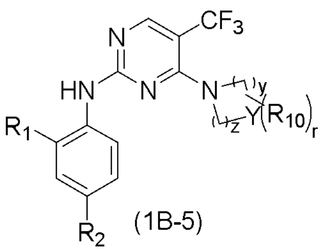
(1B-5)
;

wherein $R_1$, $R_2$ are as defined hereinbefore for the compound of formula (1); t is selected from 0, 1, 2, and 3; u, v, w, x, y, and z are each independently selected from 1, 2, and 3; Y is selected from -CH- and -N-; and r is selected from 1 and 2.

According to some embodiments of the present invention, the compound of the present invention is the compound of formula 1B-A, 1B-B, 1B-C, 1B-D, 1B-E, 1B-F, or 1B-G:

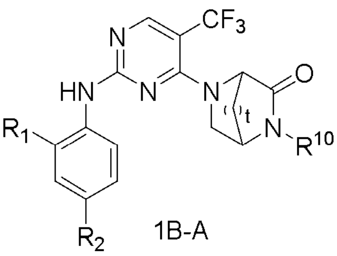
1B-A
,
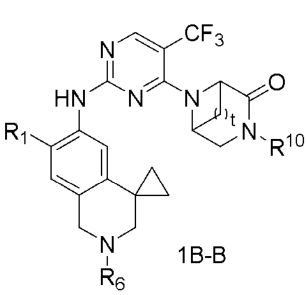
1B-B
,
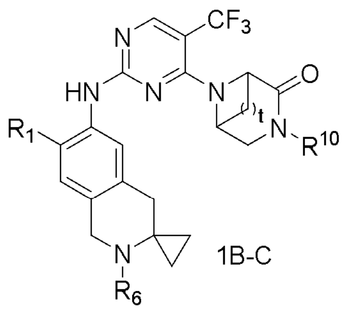
1B-C
,

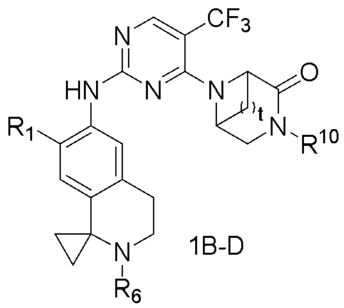

1B-D ,

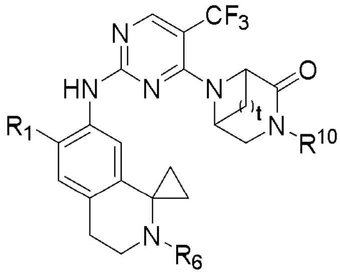

1B-E ,

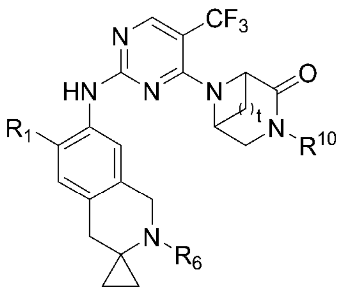

1B-F ,

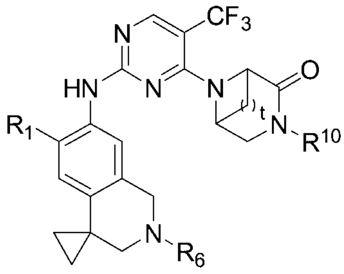

1B-G .

According to some embodiments of the present invention, the compound of the present invention is the compound of formula 1C:

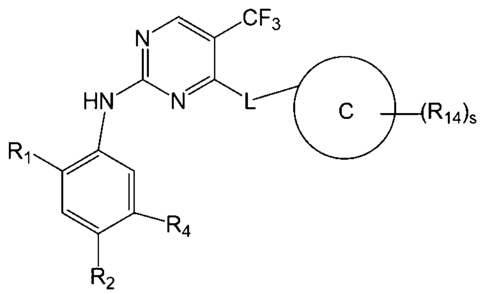

(1C)

wherein $R_1$, $R_2$, $R_4$ are as defined hereinbefore for the compound of formula (1);

L is $-(L_1)_m-(L_2)_n-(L_3)_p-$;

$L_1$ is selected from -O- and $-N(R_{12})-$, preferably selected from -O- and -NH-;

$L_2$ is selected from $C_1$-$C_6$ alkylene and $C_3$-$C_{10}$ cycloalkylene, preferably selected from $C_1$-$C_6$ alkylene and $C_3$-$C_6$ cycloalkylene, more preferably selected from ethylene, propylene, and cyclohexylidene;

$L_3$ is selected from $-N(R_{12})-$, $-N(R_{12})C(O)-$, and -C(O)-, preferably selected from -NH-, - NHC(O)-, and -C(O)-, preferably, when $L_3$ is -NHC(O)-, then the amino moiety of the - NHC(O)- is attached to $L_2$, and the carbonyl moiety is attached to ring C;

m, n, and p are each independently selected from 0 and 1, and m, n, and p are not simultaneously 0;

preferably, L is selected from -O-, -O-cyclohexylidene -, -NH-cyclohexylidene-, -O-propylene -, -NH-propylene -, -ethylene-, -ethylene-NHC(O)-, and -O-propylene-C(O)-;

ring C is selected from $C_3$-$C_{10}$ cycloalkyl, $C_6$-$C_{10}$ aryl, 5-10-membered heteroaryl, and 3-12-membered heterocyclyl, preferably selected from $C_3$-$C_6$ cycloalkyl, $C_6$-$C_{10}$ aryl, 5-6-membered heteroaryl, and 5-7-membered heterocyclyl, preferably selected from cyclobutyl, phenyl, pyrazolyl, piperidinyl, piperazinyl, pyrrolidinyl, imidazolidinyl, morpholinyl, 1,3-oxazinanyl, azepanyl, 1,4-oxazepanyl, and 5-azaspiro[2.5]octyl;

each $R_{14}$ is independently selected from H, halogen, cyano, oxo, $C_1$-$C_6$ alkyl, $C_1$-$C_6$ alkoxy, $-CONR_{12}R_{13}$, and $R_{11}C(O)-$, preferably, each $R_{14}$ is independently selected from H, fluorine, chlorine, cyano, oxo, methyl, methoxy, $-CONHCH_3$, and cyclobutyl-C(O)-; and s is selected from 0, 1, and 2.

According to some embodiments of the present invention, the compound of formula 1C is the compound of formula 1C-1, 1C-2, 1C-3, 1C-4, or IC-5:

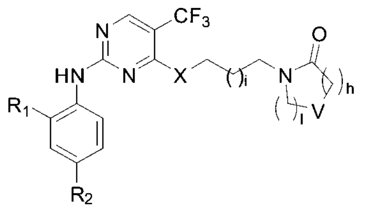 (1C-1) , 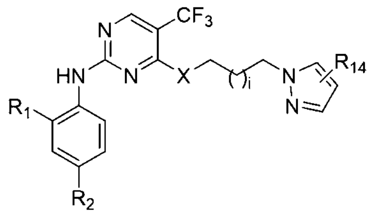 (1C-2) ,

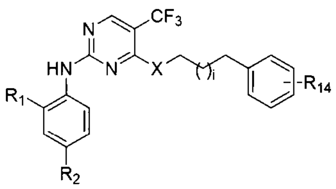 (1C-3) , 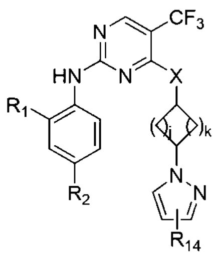 (1C-4) , 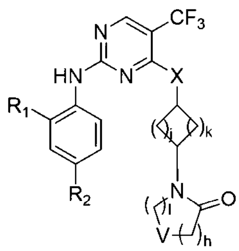 (1C-5);

wherein $R_1$, $R_2$ are as defined hereinbefore for the compound of formula (1);
X is selected from bond, -O-, -S-, -NH-, and $-CH_2-O-$, preferably, X is selected from -O-, -S-, -NH-, and $-CH_2-O-$;
V is selected from -O-, -S-, $-NR_{12}-$, and $-CHR_{12}-$;
$R_{12}$ is selected from H and $C_1-C_6$ alkyl;
h and l are each independently selected from 0, 1, 2, and 3;
i is selected from 0, 1, and 2; and
j and k are each independently selected from 1, 2, and 3.

According to some embodiments of the present invention, the compound of the present invention is the compound of formula 1D:

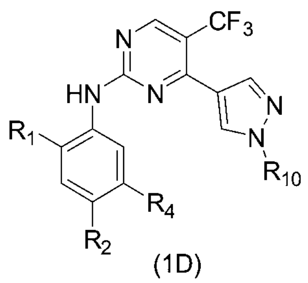
(1D)

wherein $R_1$, $R_2$, $R_4$, $R_{10}$ are as defined hereinbefore for the compound of formula (1);
preferably, $R_{10}$ is selected from 3-12-membered heterocyclyl, the 3-12-membered heterocyclyl is optionally substituted by one or more $R_{15}$; preferably, $R_{10}$ is selected from 4-7-membered heterocyclyl, the 4-7-membered heterocyclyl is optionally substituted by one or more $R_{15}$; more preferably, $R_{10}$ is selected from 4-7-membered nitrogen-containing heterocyclyl (for example piperidinyl), the 4-7-membered nitrogen-containing heterocyclyl is optionally substituted by one or more $R_{15}$;
preferably, $R_{15}$ is selected from $C_1-C_6$ alkyl, more preferably, $R_{15}$ is selected from methyl.

According to some embodiments of the present invention, in the compound of the present invention, L is $-(L_1)_m-(L_2)_n-(L_3)_p-*$, wherein the bond marked with "*" is attached to $R_9$;

$L_1$ is selected from -O-, $-N(R_{12})-$, $-C(R_{12})(R_{13})-O-$, and $-N(R_{12})CO-$;
$L_2$ is selected from $C_1-C_6$ alkylene, $C_1-C_6$ heteroalkylene, $C_3-C_{10}$ cycloalkylene, and 3-10-membered heterocyclylene;
$L_3$ is selected from $-N(R_{12})-$, $-N(R_{12})C(O)-$, and -C(O)-;
m, n, and p are each independently selected from 0, 1, and 2, and m, n, and p are not simultaneously 0;
preferably, $L_1$ is selected from -O-, $-N(R_{12})-$, $\#-C(R_{12})(R_{13})-O-$, and $\#-N(R_{12})CO-$, wherein the bond marked with "#" is attached to

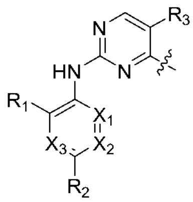

;

preferably, $L_1$ is selected from -O-, -N($R_{12}$)-;
preferably, $L_2$ is selected from $C_1$-$C_6$ alkylene, $C_1$-$C_6$heteroalkylene, $C_3$-$C_6$ cycloalkylene, and 3-6-membered heterocyclylene;
preferably, $L_3$ is selected from -N($R_{12}$)-, -N($R_{12}$)C(O)-$, and -C(O)-, wherein the bond marked with "$" is attached to $R_9$;
preferably, -$(L_1)_m$-$(L_2)_n$-$(L_3)_p$-* as a whole is selected from -$L_1$-$L_2$-*, -$L_1$-, -$L_2$-, -$L_1$-$L_2$-$L_3$-*, -$L_1$-$L_3$- -$L_2$-$L_3$-*, wherein the bond marked with "*" is attached to $R_9$;
preferably, -$(L_1)_m$-$(L_2)_n$-$(L_3)_p$-* as a whole is selected from -$L_1$-, -$L_2$-, -$L_1$-$L_2$-*, -$L_1$-$L_2$-$L_3$-*, -$L_2$-$L_3$-*;
preferably, -$(L_1)_m$-$(L_2)_n$-$(L_3)_p$-* as a whole is selected from -$L_1$-, -$L_1$-$L_2$-*, -$L_1$-$L_2$-$L_3$-*;
more preferably, -$(L_1)_m$-$(L_2)_n$-$(L_3)_p$-* as a whole is selected from -$L_1$-$L_2$-*, -$L_1$-$L_2$-$L_3$-*;
preferably, -$(L_1)_m$-$(L_2)_n$-$(L_3)_p$-* as a whole is selected from -N($R_{12}$)-$C_3$-$C_6$ cycloalkylene-*, -N($R_{12}$)-, -N($R_{12}$)-3-6-membered heterocyclylene-C(O)-*, -N($R_{12}$)C(O)-*, -N($R_{12}$)-$C_1$-$C_6$ alkylene-*, -O-$C_3$-$C_6$ cycloalkylene-*, -O-, -O-3-6-membered heterocyclylene-C(O)-*, -O-$C_1$-$C_6$ alkylene-*, -O-$C_1$-$C_6$ alkylene-C(O)-*, -O-$C_1$-$C_6$ alkylene-N($R_{12}$)-*, -$C_1$-$C_6$ alkylene-, - $C_1$-$C_6$ alkylene-N($R_{12}$)-C(O)-*, -C($R_{12}$)($R_{13}$)-O-$C_1$-$C_6$ alkylene-*, wherein the bond marked with "*" is attached to $R_9$;
preferably, -$(L_1)_m$-$(L_2)_n$-$(L_3)_p$-* as a whole is selected from -O-, -O-$C_1$-$C_6$ alkylene-*, - O-$C_1$-$C_6$ alkylene-C(O)-*, -N($R_{12}$)-$C_3$-$C_6$ cycloalkylene-*, -N($R_{12}$)-3-6-membered heterocyclylene-C(O)-*, -N($R_{12}$)-$C_1$-$C_6$ alkylene-*, -N($R_{12}$) C(O)-*, -N($R_{12}$)-, -$C_1$-$C_6$ alkylene-N($R_{12}$)-C(O)-*, -$C_1$-$C_6$ alkylene-, -O-3-6-membered heterocyclylene-C(O)-*;
preferably, -$(L_1)_m$-$(L_2)_n$-$(L_3)_p$-* as a whole is selected from -O-$C_1$-$C_6$ alkylene-*, - N($R_{12}$)-$C_1$-$C_6$ alkylene-*, -O-$C_1$-$C_6$ alkylene-C(O)-*, -N($R_{12}$)-$C_3$-$C_6$ cycloalkylene-*, - N($R_{12}$)-3-6-membered heterocyclylene-C(O)-*, -O-;
more preferably, -$(L_1)_m$-$(L_2)_n$-$(L_3)_p$-* as a whole is selected from -O-$C_1$-$C_6$ alkylene-*, - N($R_{12}$)-$C_1$-$C_6$ alkylene-*, -N($R_{12}$)-$C_3$-$C_6$ cycloalkylene-*, -N($R_{12}$)-3-6-membered heterocyclylene-C(O)-*.

According to some embodiments of the present invention, the compound of formula (1) of the present invention satisfies the conditions:

(1) when $R_9$ is $C_3$-$C_{10}$ cycloalkyl, $C_6$-$C_{10}$ aryl, or 5-10-membered heteroaryl optionally substituted by $R_{14}$, then n is not 0; and/or
(2) when L is -$(L_1)_m$-$(L_2)_n$-$(L_3)_p$-, $L_1$ is selected from -N($R_{12}$)-, m is 1, $L_2$ is selected from $C_1$-$C_6$ alkylene, and n is 1, then $L_3$ is not -N($R_{12}$)C(O)-; and/or
(3) when L is -$(L_1)_m$-$(L_2)_{a-}$$(L_3)_p$-, $L_1$ is selected from -N($R_{12}$)-, m is 1, $L_2$ is selected from $C_1$-$C_6$ alkylene, n is 1, p is 0, $R_9$ is 3-12-membered heterocyclyl substituted by $R_{14}$, and at least one $R_{14}$ is an oxo substituent, then $R_2$ is selected from $C_2$-$C_6$ alkynyl substituted by one or more $R_6$, or $R_2$ and $R_4$ together with the groups that they are attached to, form 7-12-membered spiro heterocycle substituted by one or more $R_6$; and/or
(4) when L is -$(L_1)_m$-$(L_2)_n$-$(L_3)_p$-, $L_1$ is selected from -N($R_{12}$)-, m is 1, $L_2$ is selected from $C_1$-$C_6$ alkylene, n is 1, p is 0, and $R_9$ is 5-10-membered heteroaryl, then $R_2$ is not morpholinyl; and/or
(5) when L is -$(L_1)_m$-$(L_2)_n$-$(L_3)_p$-, $L_1$ is selected from -N($R_{12}$)-, m is 1, n is 0, p is 0, and $R_9$ is a 3-12-membered heterocyclyl, then the 3-12-membered heterocyclyl is a monocyclic ring, and is at least substituted by one oxo group.

The definitions of the various groups in the compound of formula (1) of the present invention also apply to the compound of formula 1A, 1, 1B-1, 1B-2, 1B-3, IB-4, IB-5, 1B-A, 1B-B, 1B-C, 1B-D, 1B-E, 1B-F, 1B-G, 1C, 1C-1, 1C-2, 1C-3, 1C-4, 1C-5, or 1D.
According to some embodiments of the present invention, the compound of the present invention is selected from:

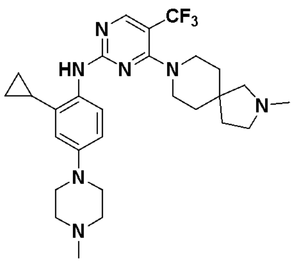
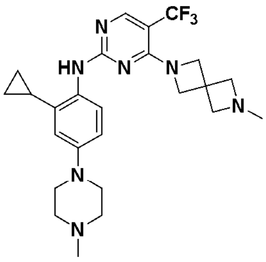
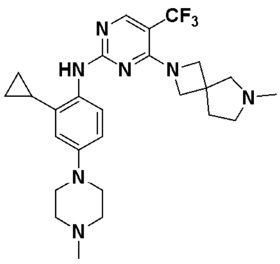
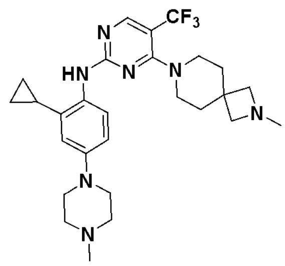
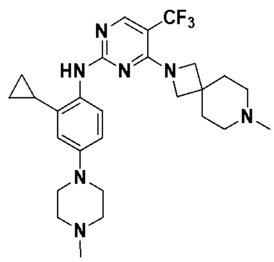
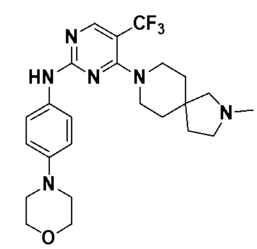
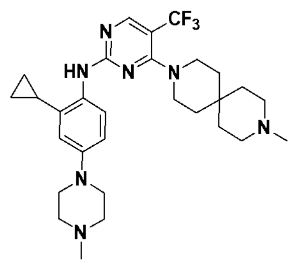
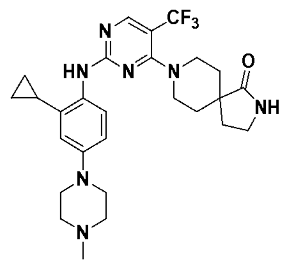
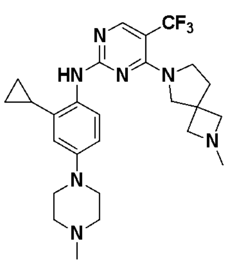
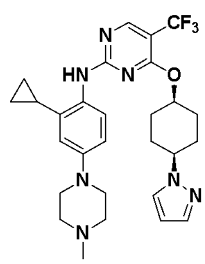
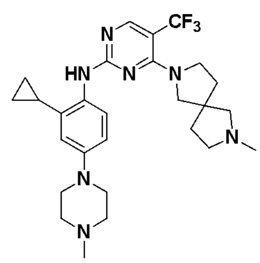
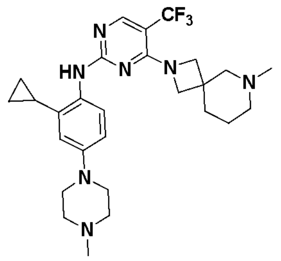
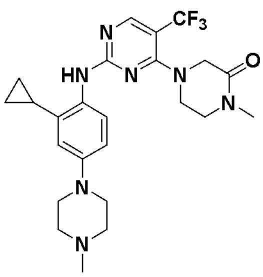
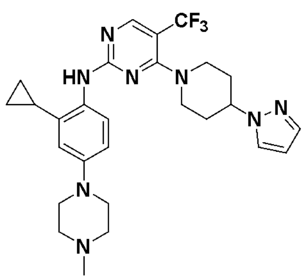
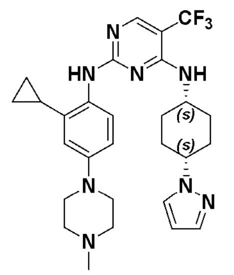
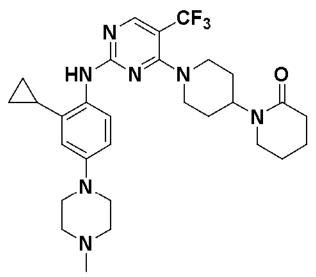
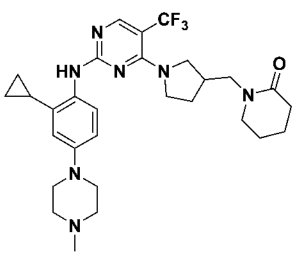
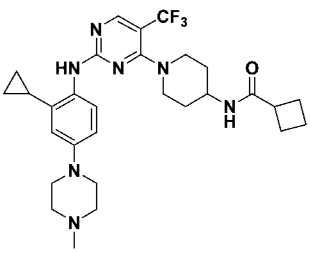
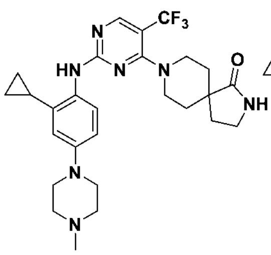
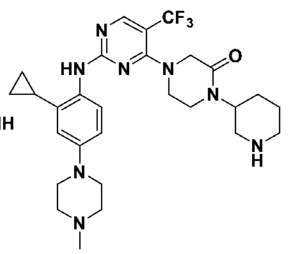

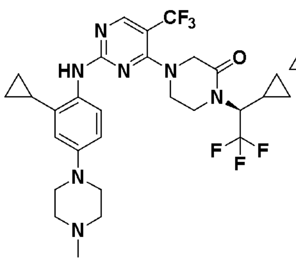
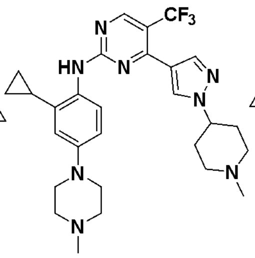
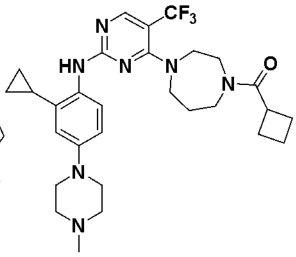
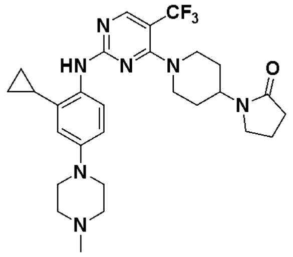

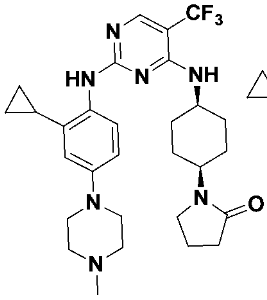
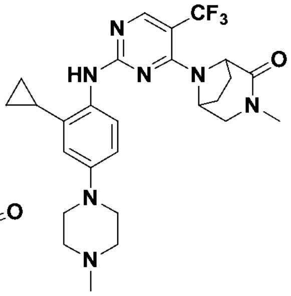
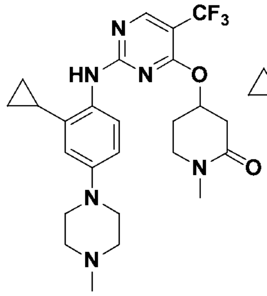
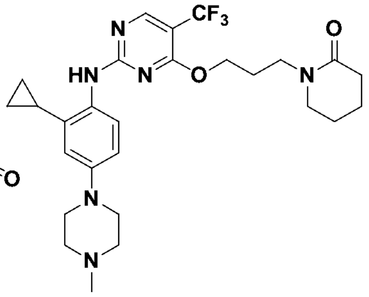

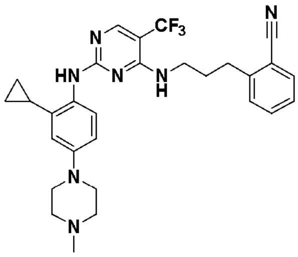
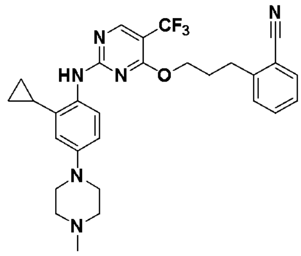
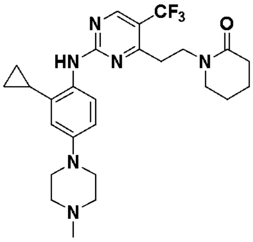
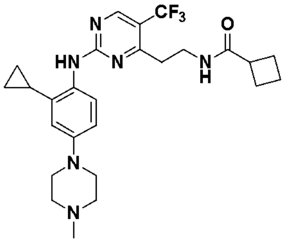

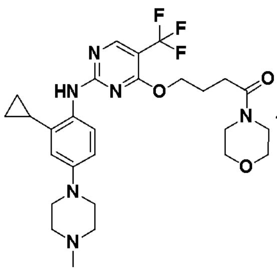
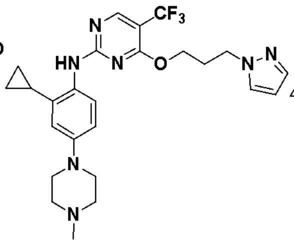
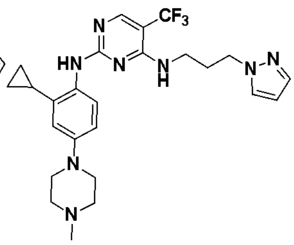
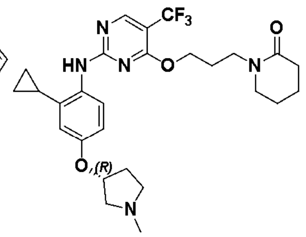

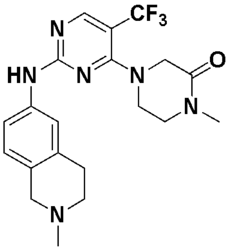
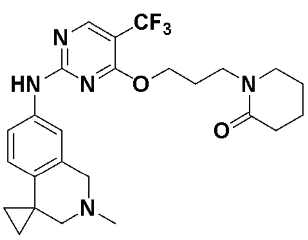
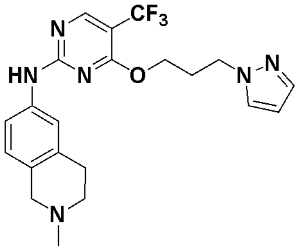
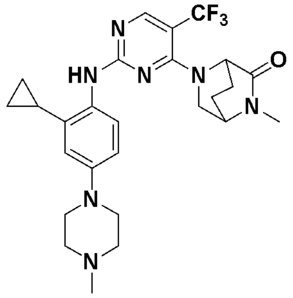

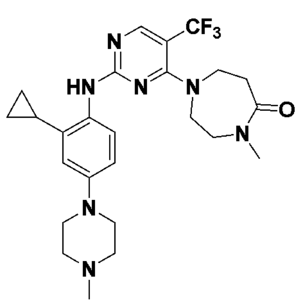

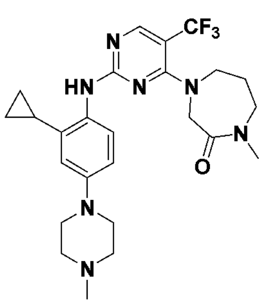

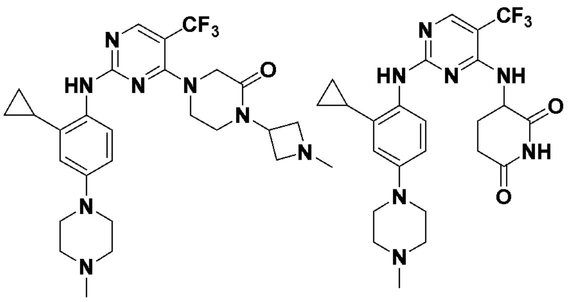

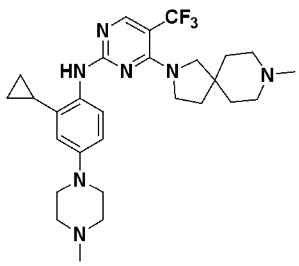

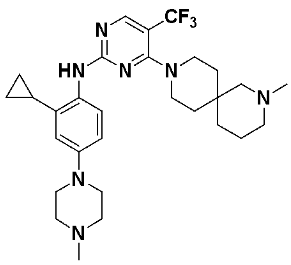

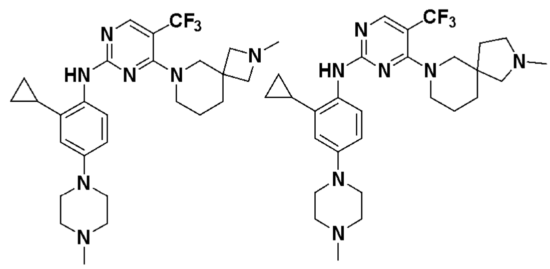

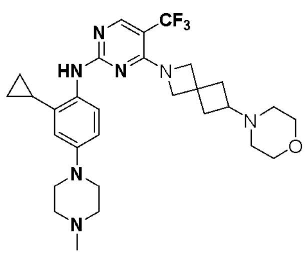

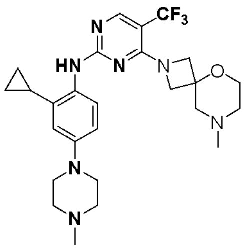

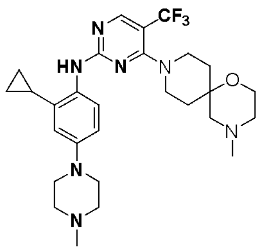

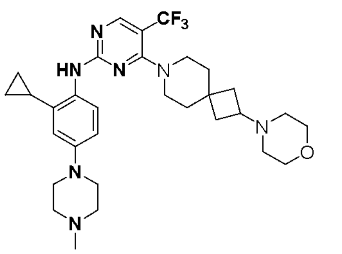

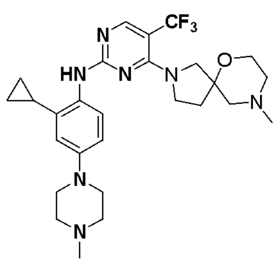

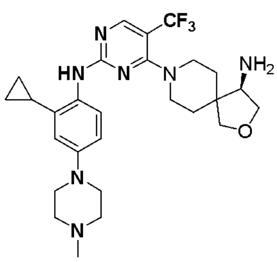

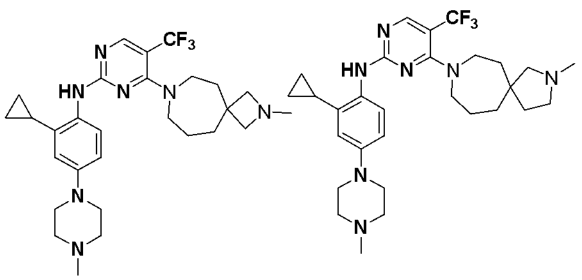

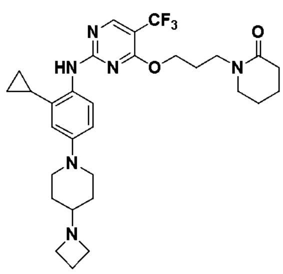

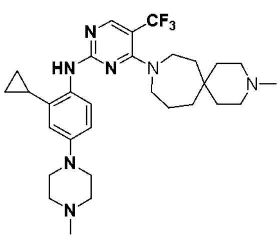

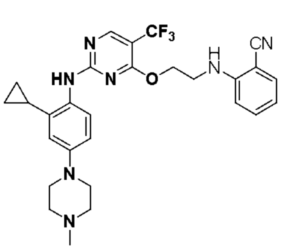

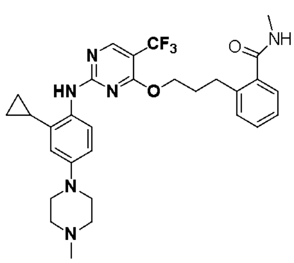

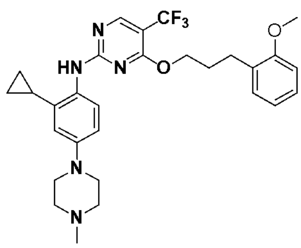

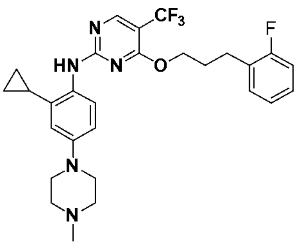

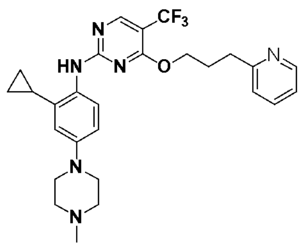

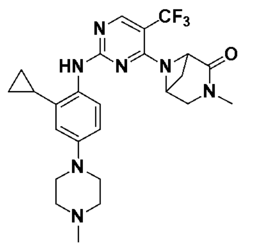

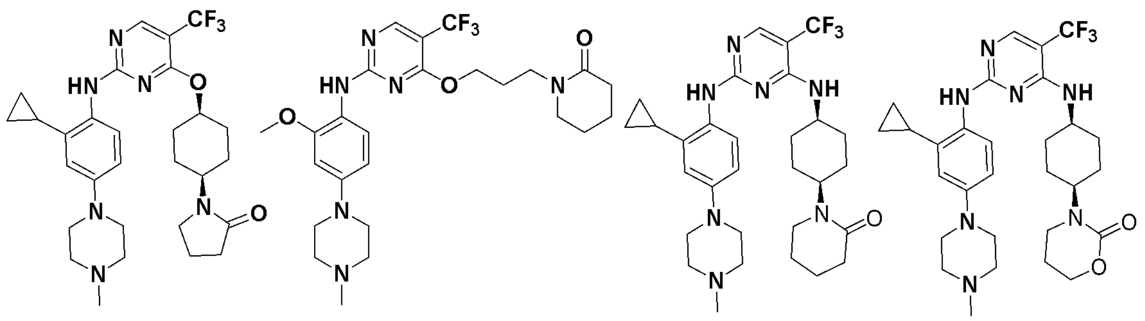

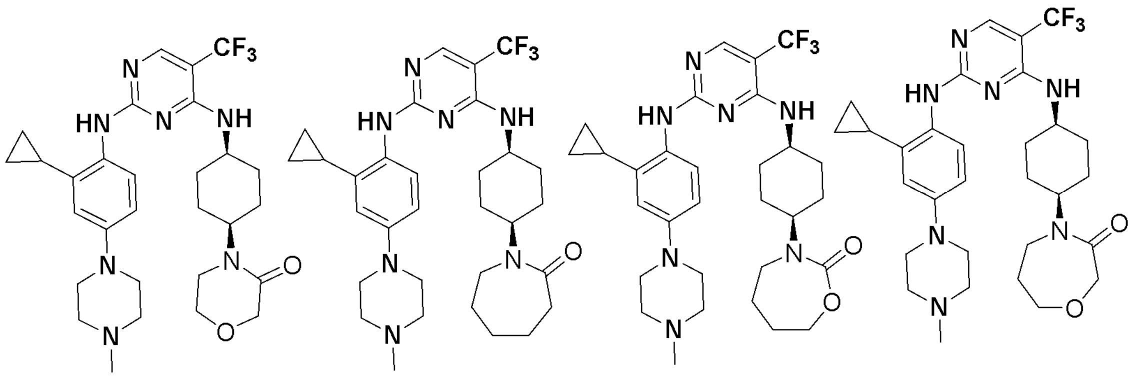

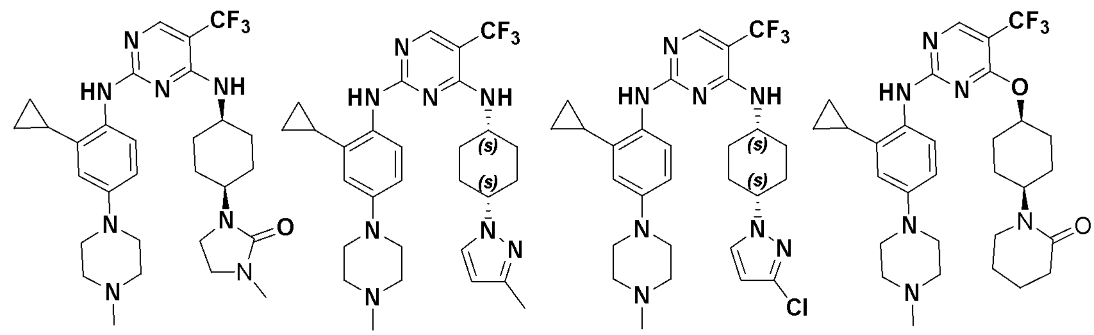

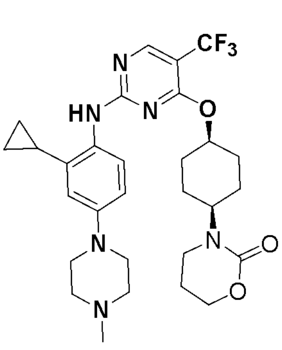

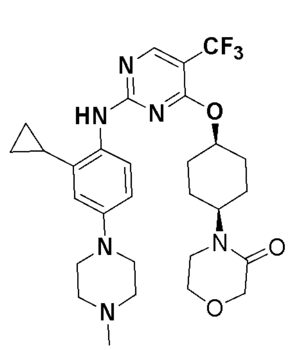

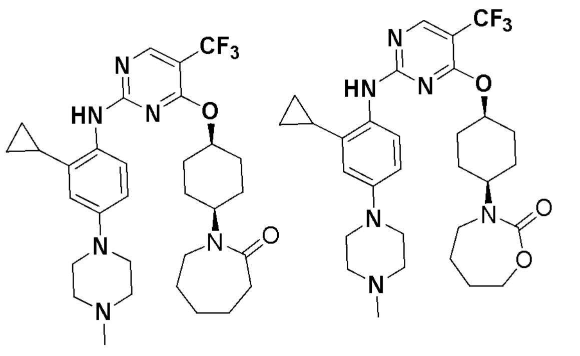

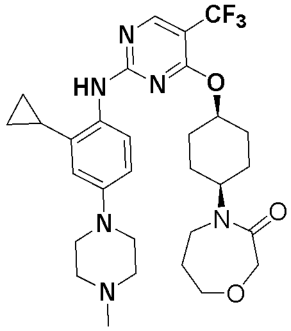

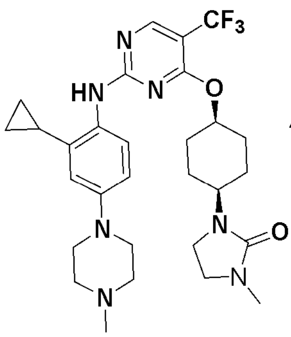

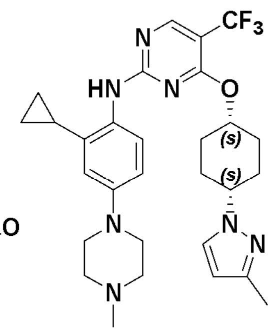

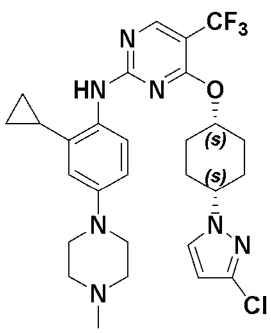

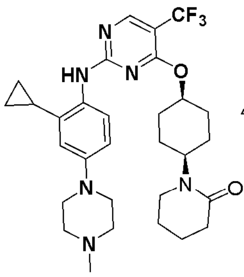

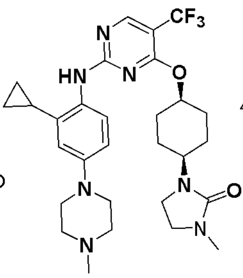

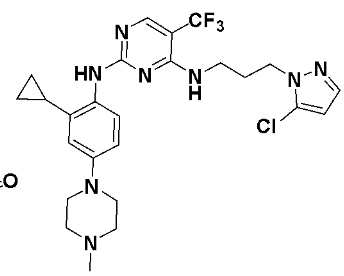

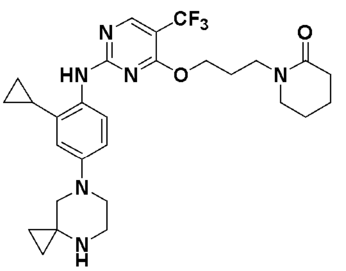

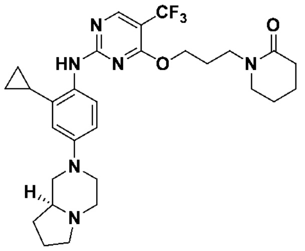

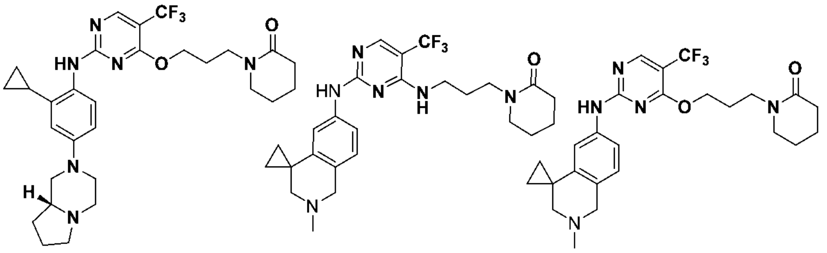

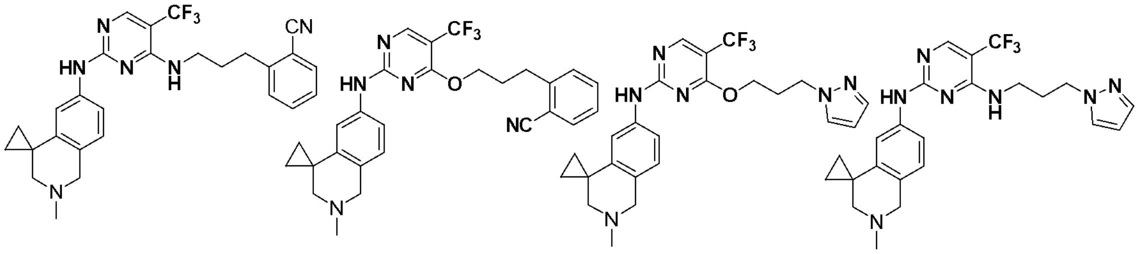

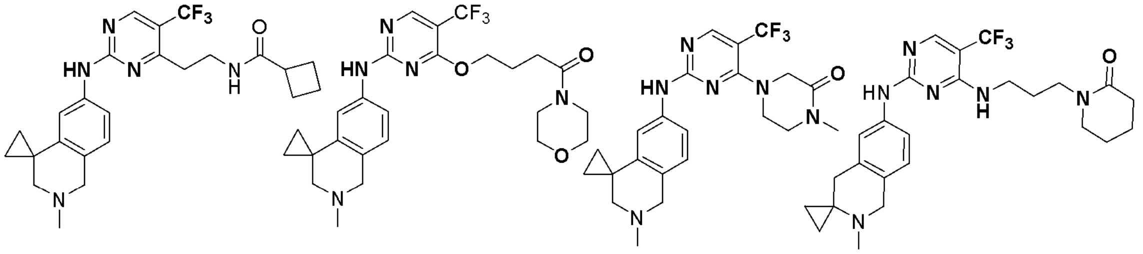

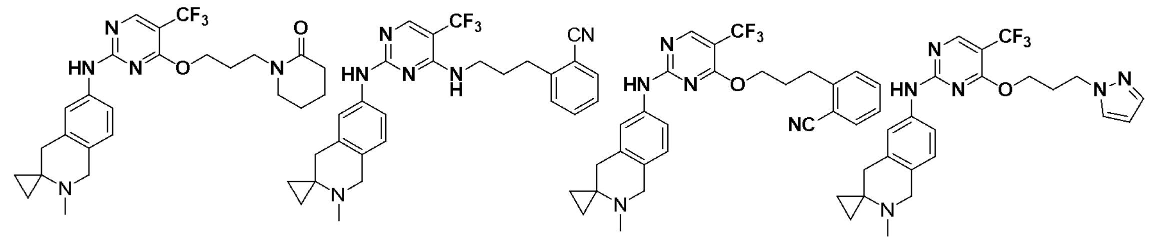

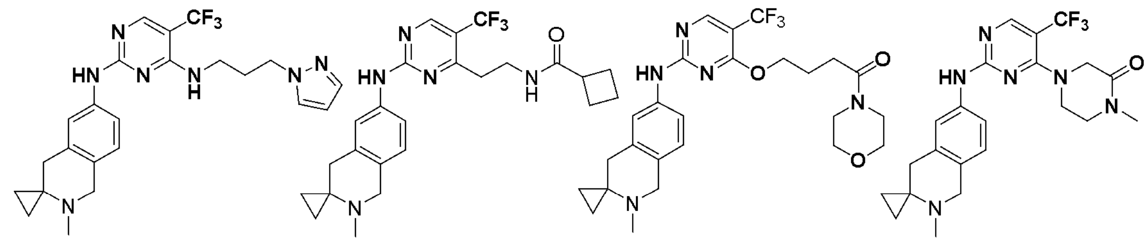

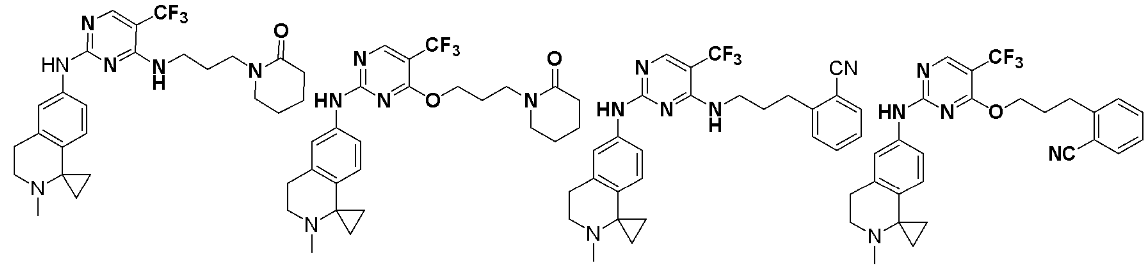

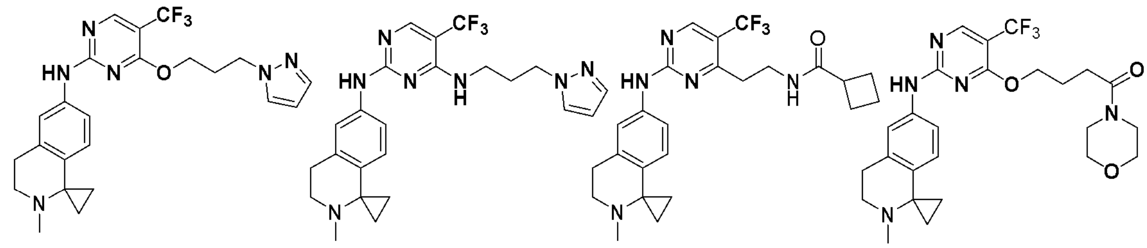

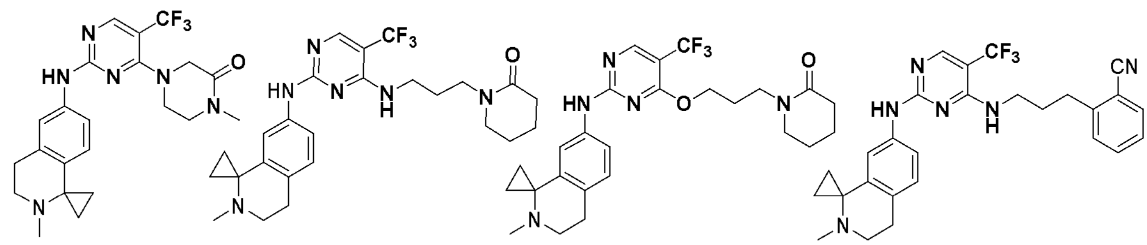

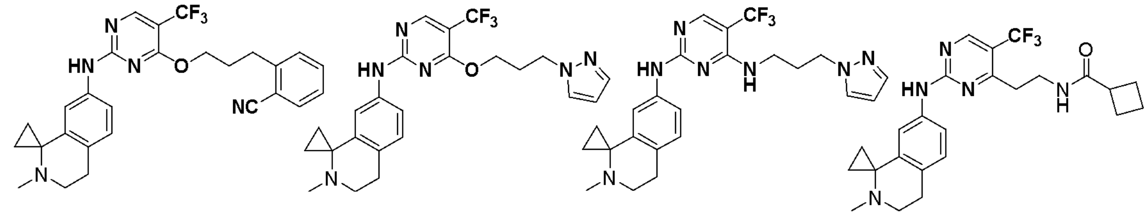

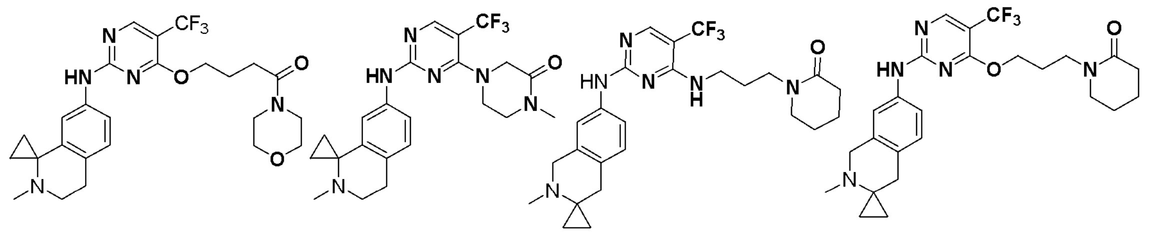

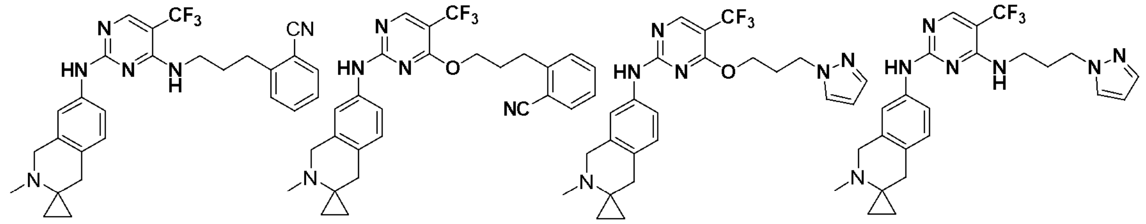

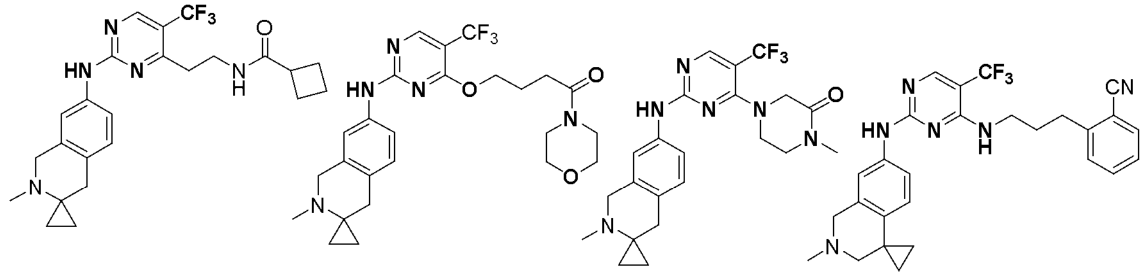

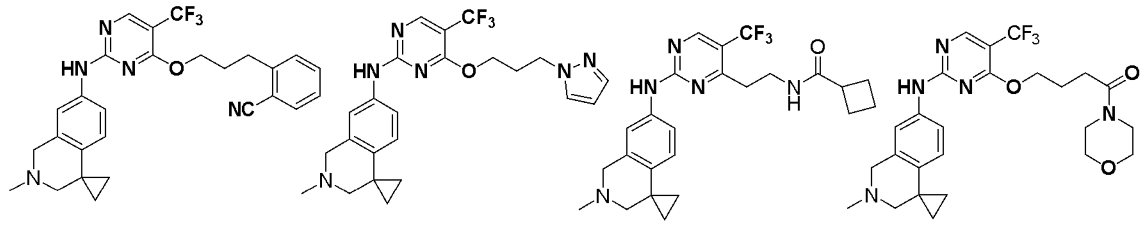

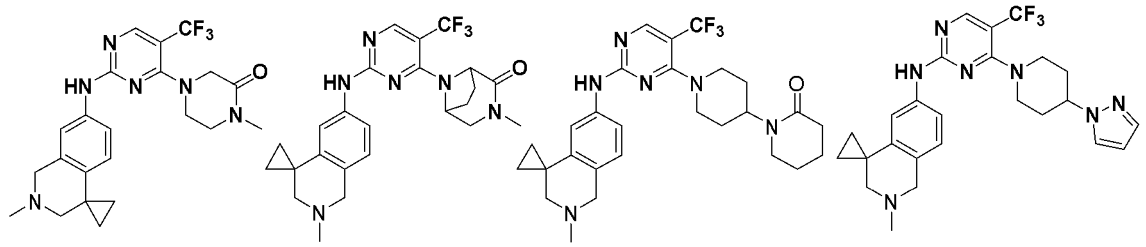

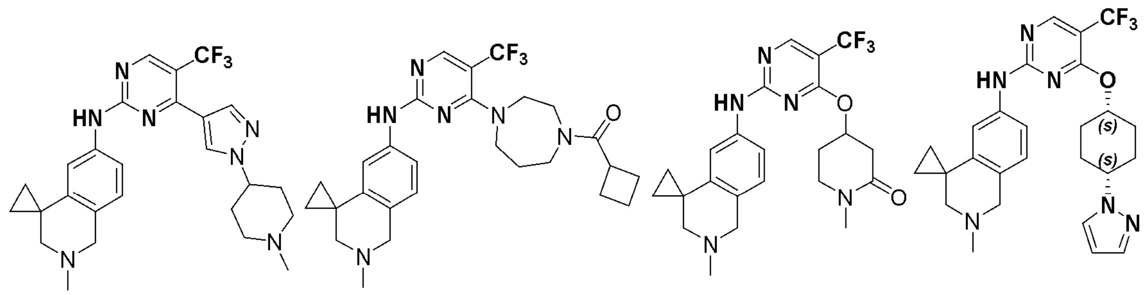

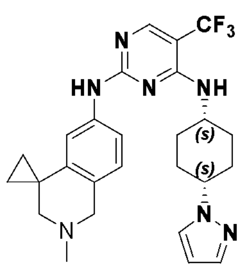

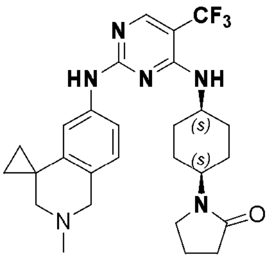

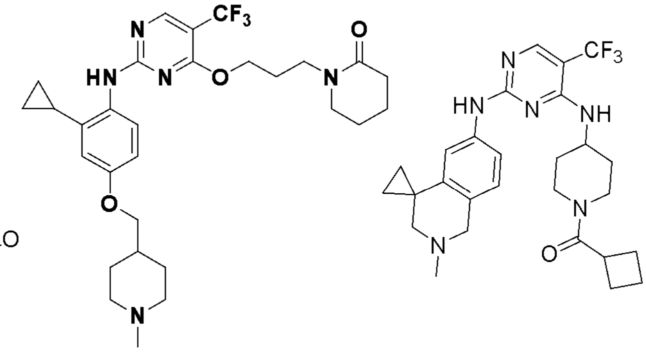

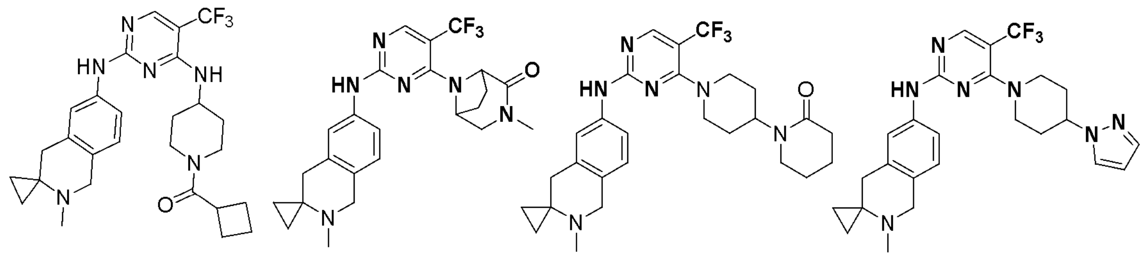

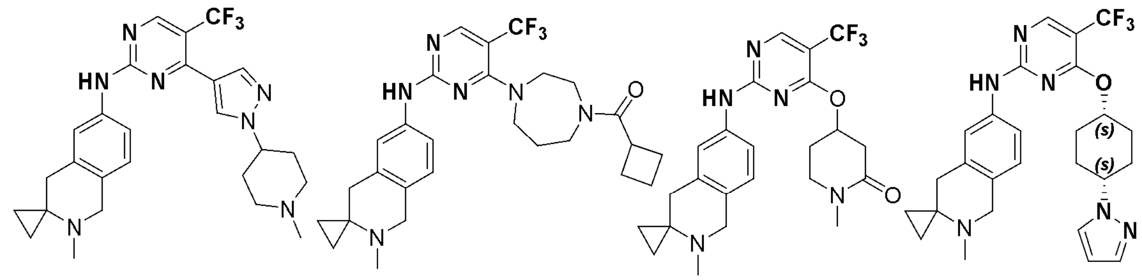

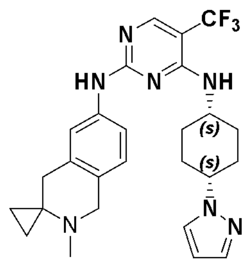

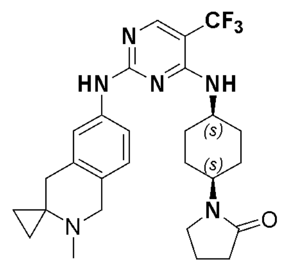

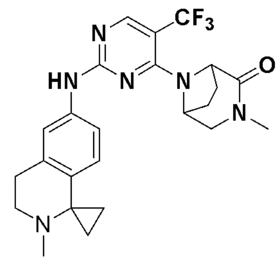

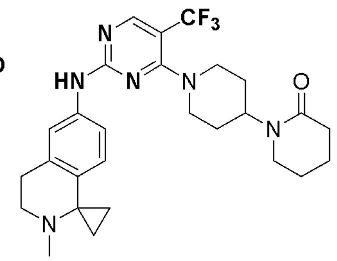

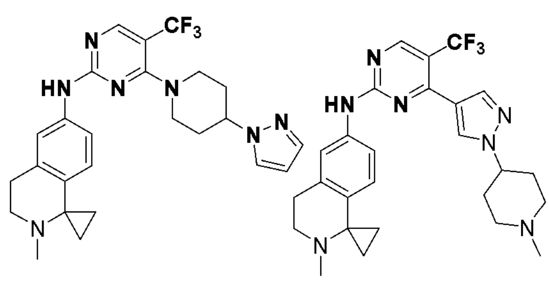

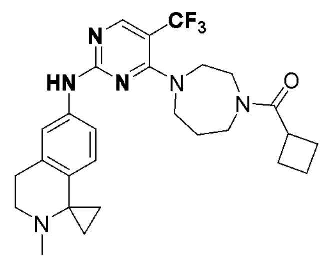

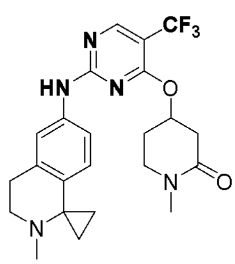

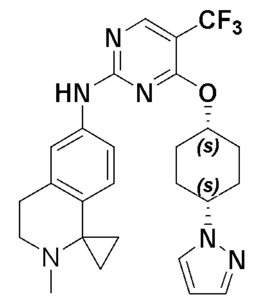
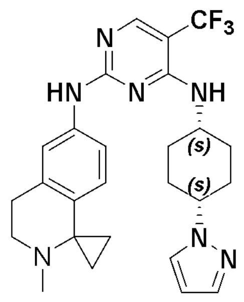
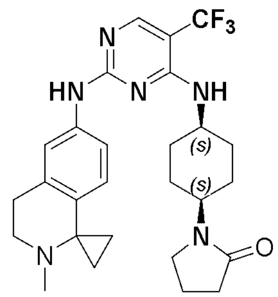
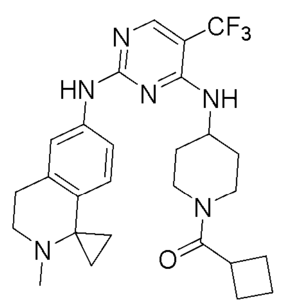

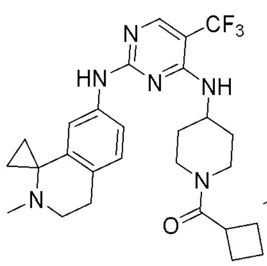
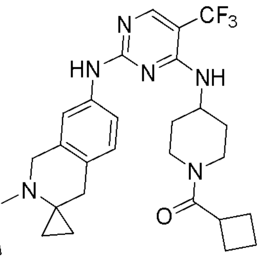
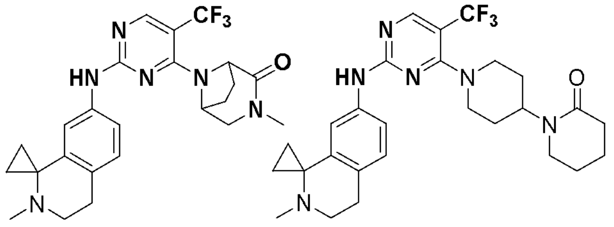

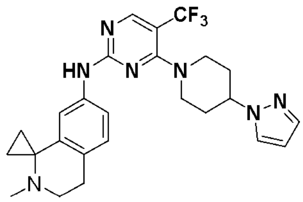
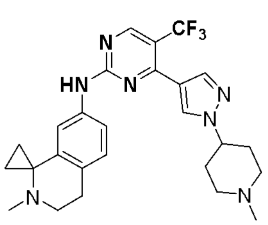
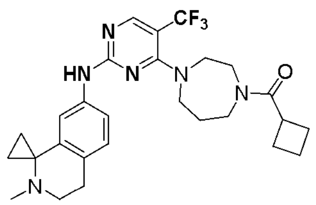
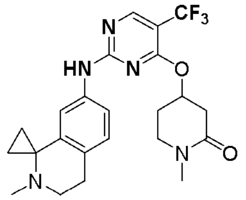

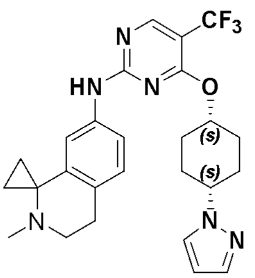
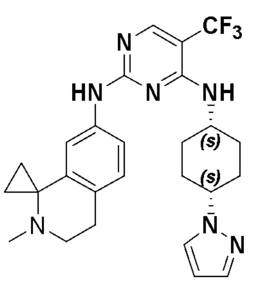
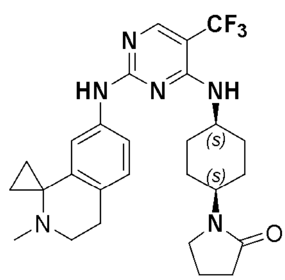
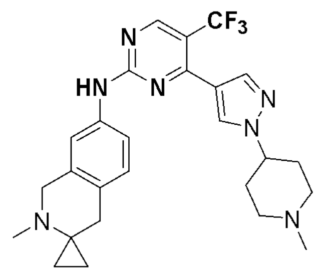

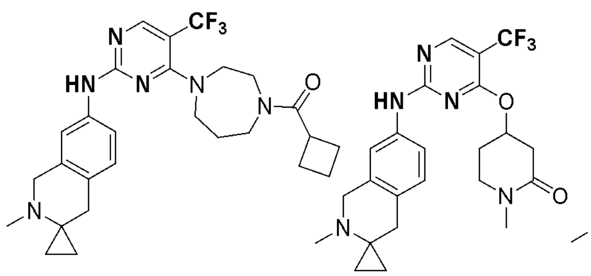
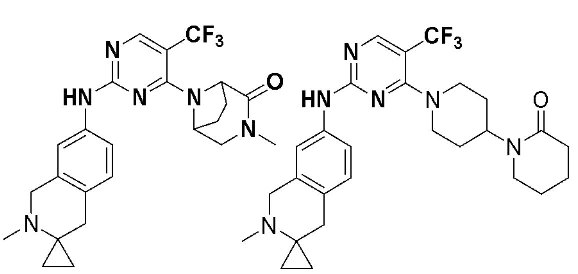

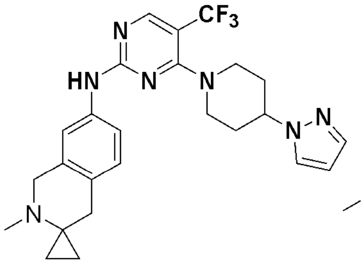
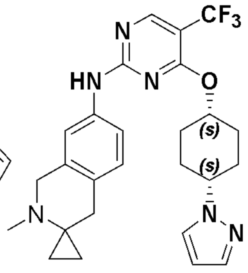
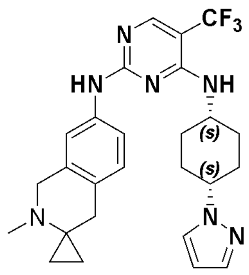
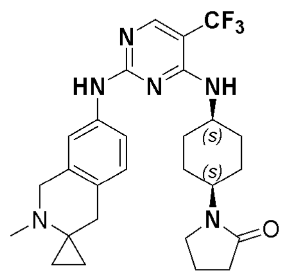

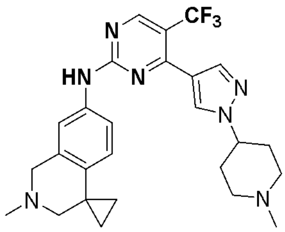
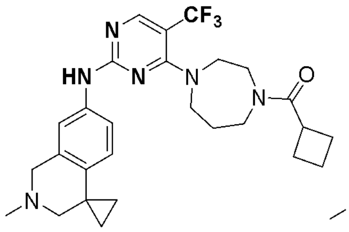
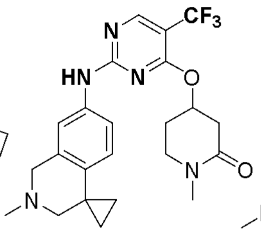
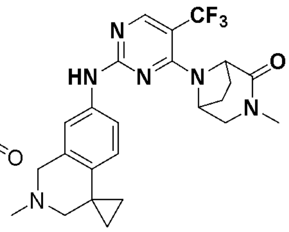

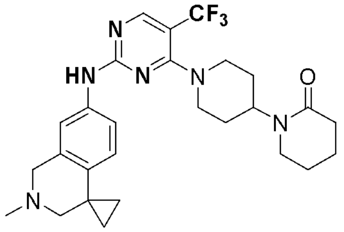
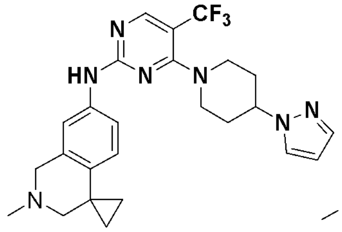
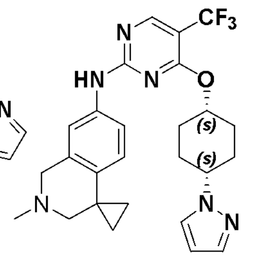
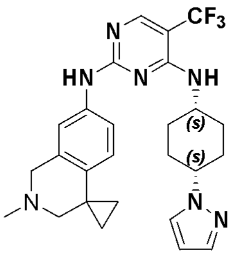

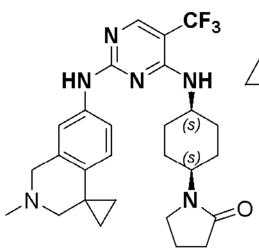
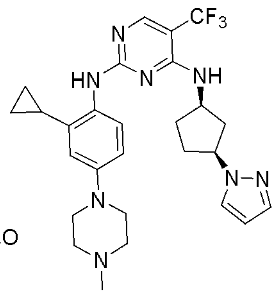
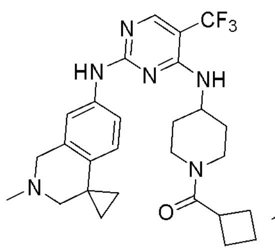
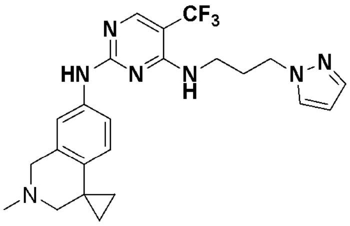

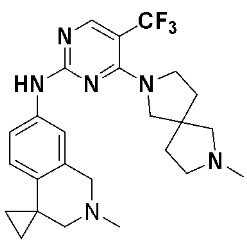
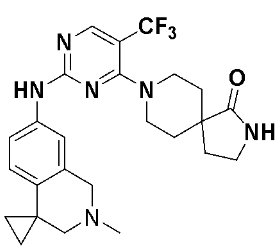
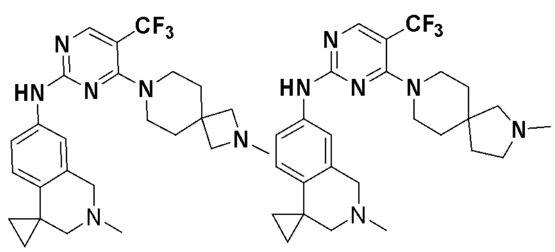

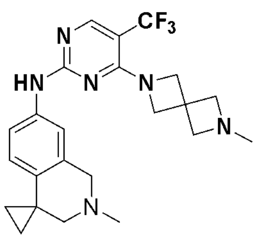
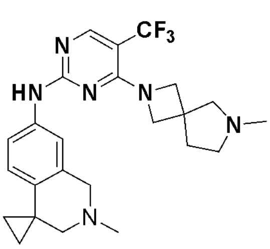
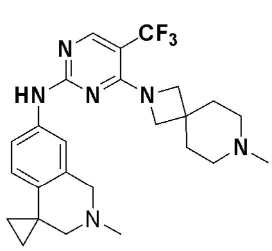
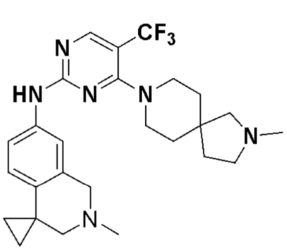

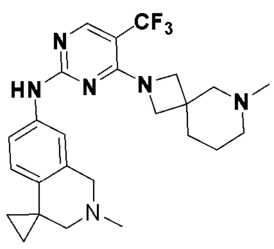
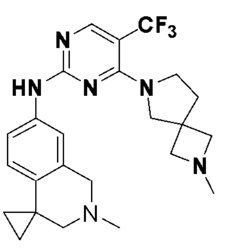
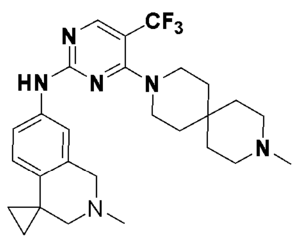
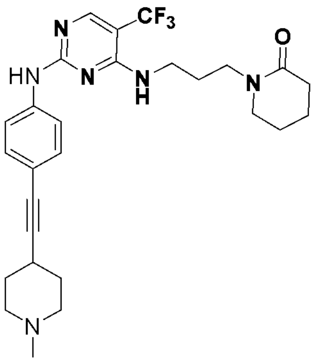

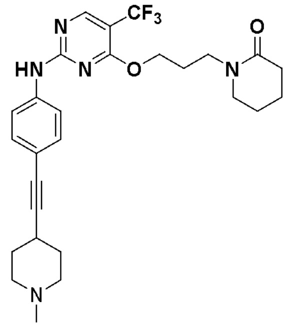
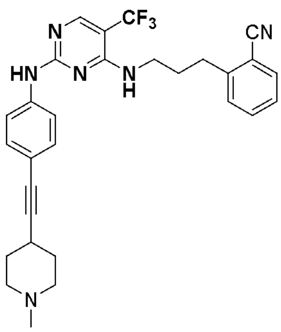
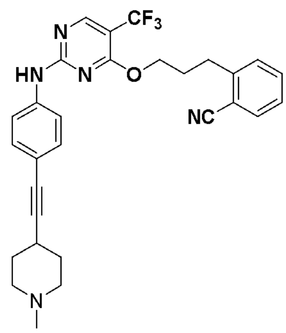
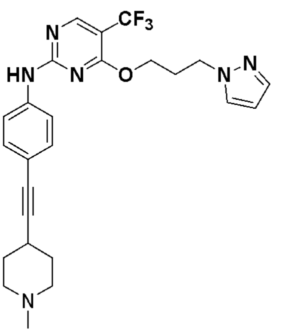

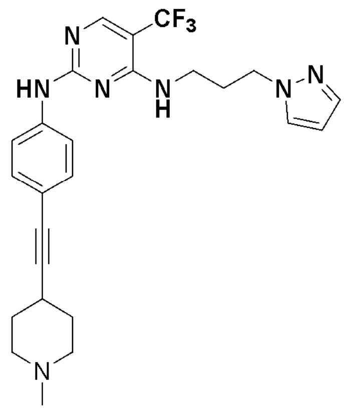
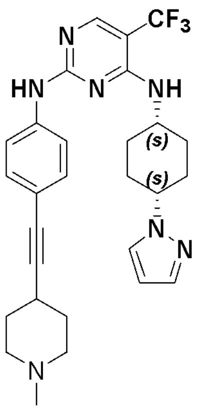
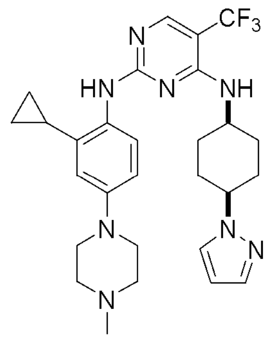
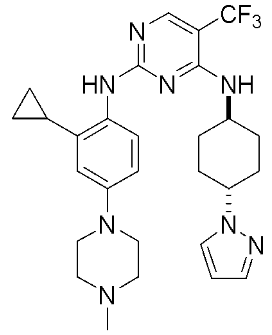

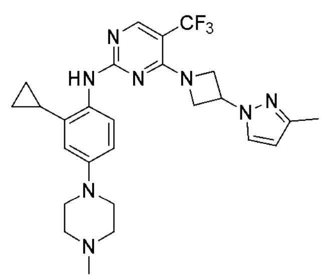
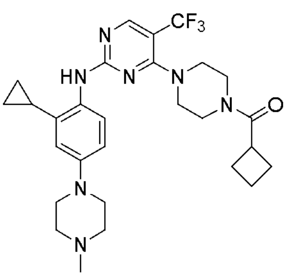
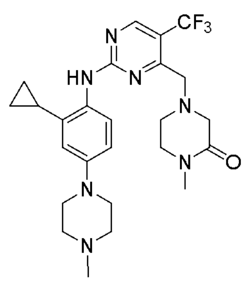
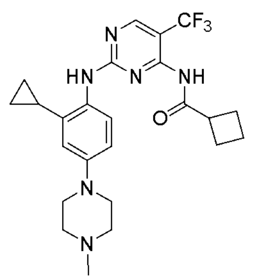

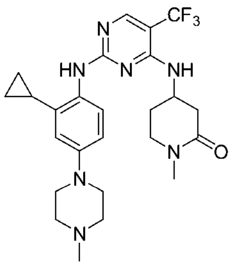
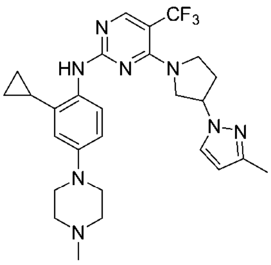
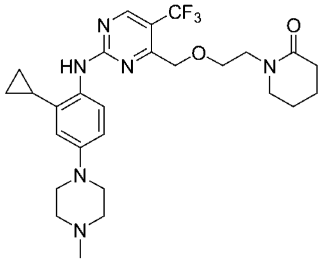
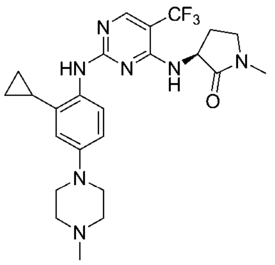
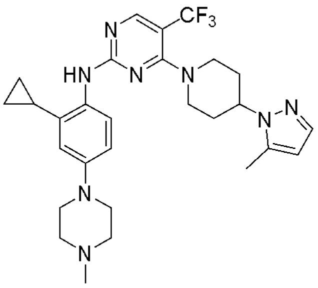
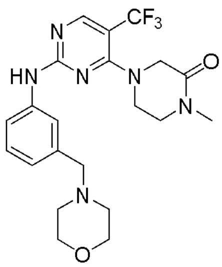
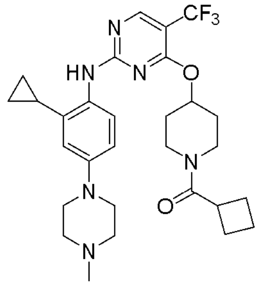
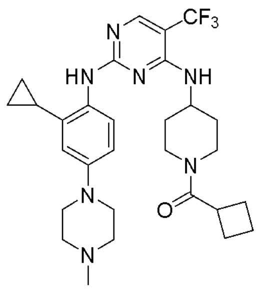
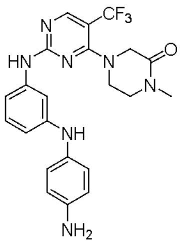
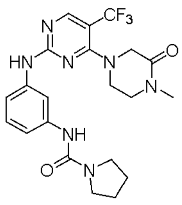
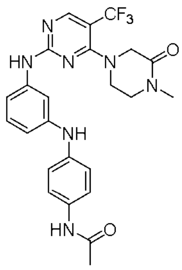
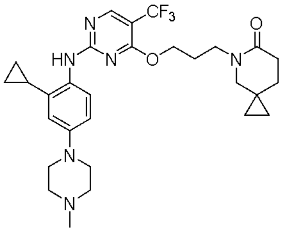
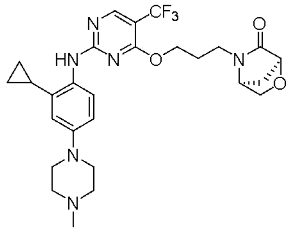
.

## Preparation methods

Another object of the present invention is to provide a method for preparing the compound of the present invention, comprising the steps shown in the following synthetic route A or B:

Synthetic route A

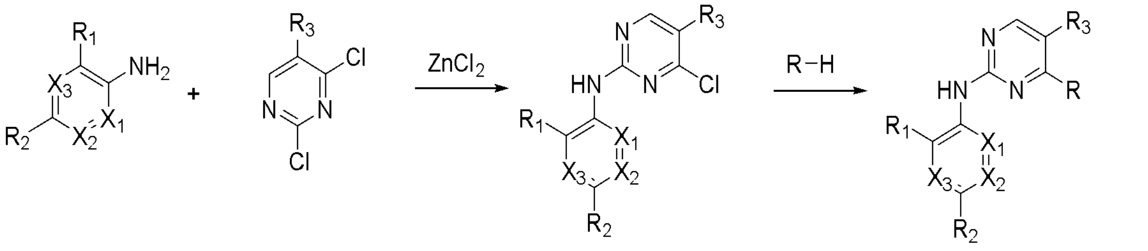


1-0 1-1 1-2 (1)

or

Synthetic route B

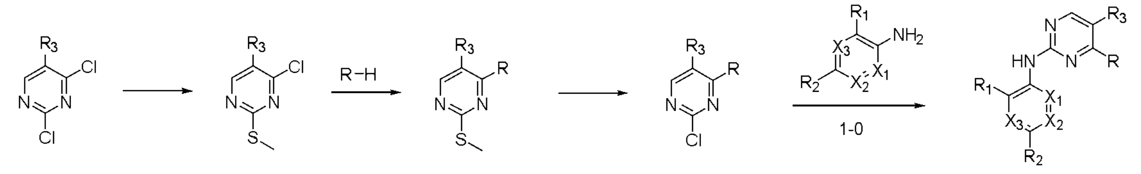

2-0 2-1 2-2 2-3 (1)

wherein: $R_1$, $R_2$, $R_3$, R, $X_1$, $X_2$, $X_3$ are as defined hereinbefore.

## Pharmaceutical compositions

Another object of the present invention is to provide a pharmaceutical composition, comprising an effective amount of the compound or pharmaceutically acceptable salt, stereoisomer, tautomer, polymorph, solvate, hydrate, N-oxide, isotope labeled compound, metabolite, ester, or prodrug thereof of the present invention, and one or more pharmaceutically acceptable carriers.

In the present invention, "pharmaceutically acceptable carrier" refers to a diluent, adjuvant, excipient or vehicle administered together with a therapeutic agent, which is suitable, to the extent of a reasonable medical judgment, for contacting the tissues of humans and/or other animals without excessive toxicity, irritation, allergic reactions, or other problems or complications commensurate with a reasonable benefit/risk ratio.

Pharmaceutically acceptable carriers that can be used in the pharmaceutical composition or pharmaceutical formulation of the present invention include, but are not limited to, sterile liquids, for example water and oils, including those of petroleum, animal, plant, or synthetic origin, for example peanut oil, soybean oil, mineral oil, sesame oil, etc.

The pharmaceutical composition may be in the form of, for example, solid formulations, semi-solid formulations, liquid formulations, or gaseous formulations. The solid formulations are, for example, tablets, capsules, powders, granules, or suppositories, etc.; the liquid formulations are, for example, solutions, suspensions, or injections. The composition may also be in dosage forms such as liposomes or microspheres. Particularly, the pharmaceutical composition is in a formulation form suitable for oral administration.

When the pharmaceutical composition is administered intravenously, water is an exemplary carrier. Physiological saline and glucose and glycerol aqueous solutions can also be used as liquid carriers, particularly for injections. Suitable pharmaceutical excipients include starch, glucose, lactose, sucrose, gelatin, maltose, chalk, silica gel, sodium stearate, glyceryl monostearate, talc, sodium chloride, skimmed milk powder, glycerol, propylene glycol, water, ethanol, etc. The composition may also contain small amounts of wetting agents, emulsifiers or pH buffers, as needed. Oral formulations may contain standard carriers such as a pharmaceutical-grade mannitol, lactose, starch, magnesium stearate, sodium saccharin, cellulose, magnesium carbonate, etc. Examples of suitable pharmaceutically acceptable carriers are as described in Remington's Pharmaceutical Sciences (1990).

The pharmaceutical composition of the present invention can act systemically and/or locally. For this purpose, they can be administered by suitable routes, for example, by injection (such as intravenous, intraarterial, subcutaneous, intraperitoneal, intramuscular injection, including infusion) or transdermal administration; or by oral, sublingual, transnasal, transmucosal, topical administration, in the form of ophthalmic formulations, or by inhalation.

For these administration routes, the pharmaceutical composition of the present invention can be administered in suitable dosage forms. The dosage forms include, but are not limited to, tablets, capsules, lozenges, hard candies, powders, sprays, creams, ointments, suppositories, gels, pastes, lotions, ointments, aqueous suspensions, injectable solutions, elixirs, syrups, etc.

The content or usage amount of the compound of the present invention in the pharmaceutical composition can be about 0.001 mg to about 1000 mg, suitably 0.001-100 mg, preferably 0.001-10 mg, more preferably 0.05-5 mg, particularly preferably 0.01-1 mg.

In some embodiments, the present invention provides a method for preparing the pharmaceutical composition of the present invention, the method comprising combining the compound or pharmaceutically acceptable salt, stereoisomer, tautomer, polymorph, solvate, hydrate, N-oxide, isotope labeled compound, metabolite, ester, or prodrug thereof of the present invention, with one or more pharmaceutically acceptable carriers.

## Treatment methods and uses

Another object of the present invention is to provide a method for treating a ULK1-mediated disease in a subject in need thereof, the method comprising administering to the subject an effective amount of the compound or pharmaceutically acceptable salt, stereoisomer, tautomer, polymorph, solvate, hydrate, N-oxide, isotope labeled compound, metabolite, ester, or prodrug thereof of the present invention, or the pharmaceutical composition of the present invention.

Another object of the present invention is to provide use of the compound or pharmaceutically acceptable salt, stereoisomer, tautomer, polymorph, solvate, hydrate, N-oxide, isotope labeled compound, metabolite, ester, or prodrug thereof of the present invention, or the pharmaceutical composition of the present invention in the preparation of a medicament for treating a ULK1-mediated disease in a subject in need thereof.

Another object of the present invention is to provide the compound or pharmaceutically acceptable salt, stereoisomer, tautomer, polymorph, solvate, hydrate, N-oxide, isotope labeled compound, metabolite, ester, or prodrug thereof of the present invention, or the pharmaceutical composition of the present invention, for use in treating a ULK1-mediated disease in a subject in need thereof.

Another object of the present invention is to provide a method for preventing or treating a ULK1-mediated related disease, comprising administering to an individual in need thereof a preventive or therapeutically effective amount of the compound or pharmaceutically acceptable salt, stereoisomer, tautomer, polymorph, solvate, N-oxide, isotope labeled compound, metabolite or prodrug thereof of the present invention, or the pharmaceutical composition of the present invention.

According to some embodiments of the present invention, the ULK1-mediated disease is characterized by an abnormal autophagy.

According to some embodiments of the present invention, the abnormal autophagy is induced by treatment.

According to some embodiments of the present invention, the ULK1-mediated disease is a cancer, for example lung cancer, breast cancer or pancreatic cancer, particularly, the lung cancer is non-small cell lung cancer, the breast cancer is triple negative breast cancer, or the pancreatic cancer is pancreatic ductal adenocarcinoma.

According to some embodiments of the present invention, the ULK1-mediated disease is tuberous sclerosis (TSC) or lymphangioleiomyomatosis (LAM).

According to some embodiments of the present invention, the compound is co-administered with an additional therapeutic agent.

According to some embodiments of the present invention, the additional therapeutic agent is a standard care therapy.

According to some embodiments of the present invention, the standard care therapy is an mTOR inhibitor, carboplatin, a MEK inhibitor or a PARP inhibitor.

According to some embodiments of the present invention, administration of the compound or pharmaceutical composition of the present invention degrades ATG13 in the subject.

As used herein, the term "effective amount" refers to an amount sufficient to achieve the desired preventive or therapeutic effect, for example, an amount that achieves the alleviation of one or more symptoms associated with the disease to be treated.

The dosing regimen can be adjusted to provide the optimal desired response. For example, a single bolus injection can be administered, several divided doses can be administered over time, or the dose can be proportionally reduced or increased as indicated by the urgency of the treatment situation. It should be noted that a dose value may vary depending on the type and severity of the condition to be alleviated, and may include a single or multiple doses. It is to be further understood that, for any specific individual, the particular dosing regimen should be adjusted over time according to the individual's needs and the professional judgment of the person administering the composition or supervising the administration of the composition.

The amount of the compound of the present invention administered will depend on the individual being treated, the severity of the disorder or condition, the rate of administration, the disposition of the compound, and the judgment of the prescribing physician. Generally, the effective dose is about 0.0001 to about 50 mg per kg of body weight per day, for example, about 0.01 to about 10 mg/kg/day (single or divided doses). For a 70 kg person, this would total about 0.007 mg/day to about 3500 mg/day, for example, about 0.7 mg/day to about 700 mg/day. In some cases, a dose level not exceeding the lower limit of the aforementioned range may be sufficient, while in other cases, a larger dose may still be used without causing any harmful side effects, with the proviso that the larger dose is first divided into several smaller doses for administration throughout the day.

Unless otherwise stated, as used herein, the term "treatment" means reversing, alleviating, inhibiting the disorder or condition to which such term applies or the progression of one or more symptoms of such disorder or condition, or preventing such disorder or condition or one or more symptoms of such disorder or condition.

As used herein, "individuals" include human beings or non-human animals. Exemplary human individuals include human individuals suffering from a disease (for example the diseases described herein) (referred to as patients) or normal individuals. In the present invention, "non-human animals" include all vertebrates, for example non-mammals (e.g., birds, amphibians, reptiles) and mammals, for example non-human primates, domestic animals and/or domesticated animals (e.g., sheep, dogs, cats, cows, pigs, etc.).

## Example

To make the objectives and technical solutions of the present invention clearer, the embodiments of the present invention are described in detail below with reference to examples. However, those skilled in the art will understand that the following examples are only used to illustrate the present invention and should not be regarded as limiting the scope of the present invention. If a particular condition is not specified in the examples, it is carried out according to a conventional condition or the condition suggested by the manufacturer. The used reagents or instruments whose manufacturers are not specified are all commercially available conventional products.

Example

The structure of a compound was determined by nuclear magnetic resonance (NMR) and/or mass spectrometry (MS). The NMR shift (δ) is given in unit of $10^{-6}$ (ppm). The determination of NMR was performed using a Bruker AVANCE NEO 500M nuclear magnetic instrument, the determination solvent was deuterated dimethyl sulfoxide (DMSO-$d_6$), deuterated chloroform ($CDCl_3$), deuterated methanol ($CD_3OD$), with tetramethylsilane (TMS) as the internal standard.
The determination of MS was performed using Agilent 1200/1290 DAD-6110/6120 Quadrupole MS liquid chromatograph-mass spectrometer (producer: Agilent, MS model: 6110/6120 Quadrupole MS), waters ACQuity UPLC-QD/SQD (producer: waters, MS model: waters ACQuity Qda Detector/waters SQ Detector), THERMO Ultimate 3000-Q Exactive (producer: THERMO, MS model: THERMO Q Exactive).
High performance liquid chromatography (HPLC) analysis was performed using Agilent HPLC 1200DAD, Agilent HPLC 1200VWD and Waters HPLC e2695-2489 liquid chromatograph.
Chiral HPLC analysis and determination were performed using Agilent 1260 DAD high performance liquid chromatograph.
Waters 2767, Waters 2767-SQ Detecor2, Shimadzu LC-20AP and Gilson-281 preparative chromatographs were used for high performance liquid preparation.
Shimadzu LC-20AP preparative chromatograph was used for chiral preparation.
Combiflash Rf200 (TELEDYNE ISCO) was used as the CombiFlash rapid preparation instrument.
Yantai Huanghai HSGF254 or Qingdao GF254 silica gel plate was used for a thin layer chromatography silica gel plate, and the specification of the silica gel plate employed for thin layer chromatography (TLC) was 0.15mm - 0.2mm.
Silica gel column chromatography typically used Yantai Huanghai silica gel with 200 - 300 mesh as the carrier.
The average inhibition ratio of a kinase and the IC50 value were determined using a NovoStar microplate reader (BMG company, Germany).
The known starting materials of the present disclosure can be synthesized using or according to methods known in the art, or can be purchased from ABCR GmbH&Co.KG, Acros Organics, Aldrich Chemical Company, Accela ChemBio Inc, Darui Chemicals Company, etc.
CEM Discover-S 908860 type microwave reactor was used for a microwave reaction.
Unless otherwise specified in the examples, "solution" refers to an aqueous solution.
Unless otherwise specified in the examples, the reaction temperature was room temperature, being 20°C - 30°C.
The reaction progress in the examples was monitored using thin layer chromatography (TLC), the developing agent used for the reaction, the eluant system for column chromatography used to purify a compound, and the developing agent system for thin layer chromatography included: A: dichloromethane/methanol system, B: n-hexane/ethyl acetate, the volume ratio of the solvents was adjusted according to the different polarities of the compounds, and a small amount of basic or acidic reagent such as triethylamine and acetic acid could also be added for adjustment.

Preparation of intermediate **Int-1**

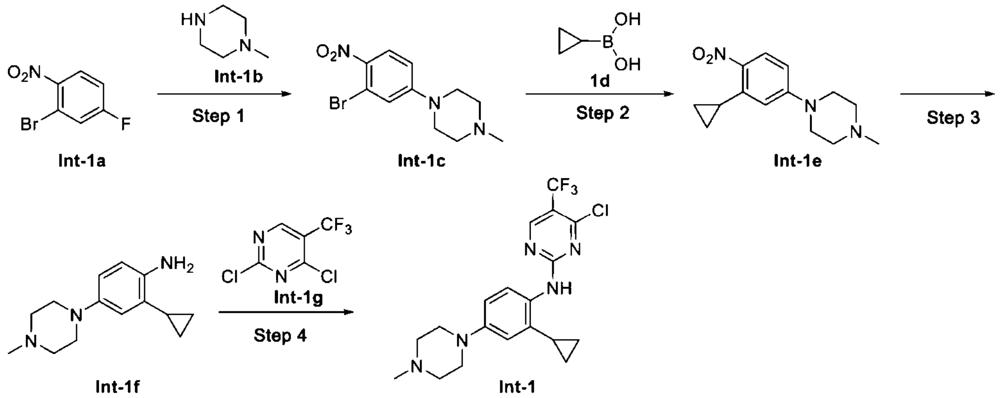


1) Step 1: Potassium carbonate (56.54 g, 409.09 mmol) was added to a solution of compound Int-1a (45 g, 204.55 mmol, Bide) and compound Int-1b (25 mL, 225.00 mmol, Bide) in N,N- dimethylformamide (400 mL) with stirring at 25°C under a nitrogen atmosphere, followed by heating to 60°C and stirring for 18 hours. The reactant was poured into water (500 mL), stirred for 10 minutes and filtered. The filter cake was washed with water (50 mL*4), and the filter cake was dissolved in dichloromethane (200 mL), dried over anhydrous sodium sulfate, filtered, and concentrated to dryness under reduced pressure to afford compound Int-1c. MS m/z (ESI): 300.1/302.1 [M+1].

2) Step 2: At 25°C, palladium acetate (3.44 g, 15.33 mmol), potassium phosphate (97.59 g, 459.75 mmol) and tricyclohexylphosphine (8.60 g, 30.65 mmol) were added to a mixed solution of compound Int-1c (46 g, 153.25 mmol), compound Int-1d (19.75 g, 229.88 mmol, Bide) in toluene (416 mL) and water (44 mL), respectively. The mixture was purged with nitrogen 3 times, heated to 100 °C, and stirred for 18 hours. The crude product was afforded by concentration under reduced pressure. Water (200 mL) was added to the mixture, and the mixture was extracted with dichloromethane (200 mL x3). The organic phases were combined, the organic phase was washed with saturated saline (100 mL), dried over anhydrous sodium sulfate, filtered, and then concentrated under reduced pressure to afford the crude product. The crude product was purified by silica gel chromatography (eluant: dichloromethane : methanol =0 to 10:1) to afford compound Int-1e. MS m/z (ESI): 262.1 [M+1].

3) Step 3: At room temperature, palladium/carbon (2 g, 18.79 mmol, 10% purity) was added to a solution of compound Int-1e (30 g, 114.80 mmol) in methanol (200 mL) and stirred. The mixture was purged with hydrogen 3 times, and stirred at 25°C for 18 hours. After filtration, the filter cake was washed with methanol (50 mL x3), and the filtrate was concentrated under reduced pressure to afford compound Int-1f. MS m/z (ESI): 232.2 [M+1].

4) Step 4: At 0°C under a nitrogen atmosphere, zinc chloride (102 mL, 101.86 mmol) was added to a mixed solution of compound Int-1g (10 g, 46.30 mmol) in 1,2-dichloroethane (400 mL) and t-butyl alcohol(50mL). After stirring at 0°C for 1 hour, triethylamine (7.1 mL, 50.93 mmol) and compound Int-1f (10.71 g, 46.30 mmol) were added, respectively. The resulting mixture was stirred at 0°C for 3 hours. The crude product was afforded by concentration to dryness under reduced pressure. The crude product was purified by silica gel chromatography (elution solution: methanol : dichloromethane = 0 to 10%) to afford compound Int-1. MS m/z (ESI): 412.2 [M+1]. $^1$H NMR (400 MHz, DMSO-$d_6$): δ 10.01 (s, 1H), 8.65 - 8.50 (m, 1H), 7.12 (t, *J* = 9.0 Hz, 1H), 6.81 (dd, *J* = 8.6, 2.4 Hz, 1H), 6.51 (d, *J* = 2.6 Hz, 1H), 3.14 - 3.06 (m, 4H), 2.69 (s, 3H), 1.93 - 1.64 (m, 4H), 1.27 (dd, *J*= 17.4, 8.4 Hz, 1H), 0.88 - 0.75 (m, 2H), 0.63 (q, *J* = 5.6 Hz, 2H).

**Example 1. Preparation of compound 1**

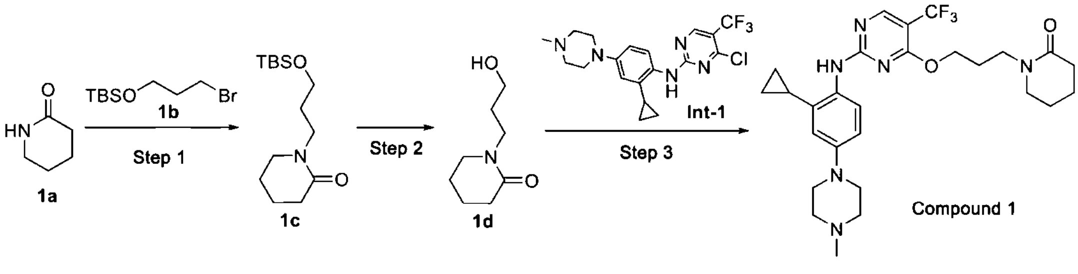


Step 1: At 0°C under a nitrogen atmosphere, sodium hydride (2.42 g, 60.53 mmol, 60% purity) was added to a

solution of compound 1a (5 g, 50.44 mmol, Bide) in *N,N*-dimethylformamide (50 mL). After stirring for 20 minutes, compound 1b (14 mL, 60.53 mmol, Bide) was added. The resulting mixture was allowed to warm to room temperature naturally and stirred for 18 hours. The mixture was poured into water (100 mL), followed by extraction with ethyl acetate (100 mL x3). The organic phases were combined, washed with saturated saline (100 mL×4), dried over anhydrous sodium sulfate, filtered, and concentrated under reduced pressure to afford the crude product. The crude product was purified by silica gel chromatography (eluant: MeOH : DCM = 5%-10%) to afford compound 1c. $^1$H NMR (400 MHz, CDCl3): δ 3.60 (t, J = 6.2 Hz, 2H), 3.40-3.32 (m, 2H), 3.25 (t, J = 5.2 Hz, 2H), 2.31 (t, J = 6.0 Hz, 2H), 1.79-1.68 (m, 6H), 0.84 (s, 9H), 0.00 (s, 6H).

Step 2: At 25°C, TBAF (8.1 mL, 27.63 mmol) was added to a solution of compound 1c *(5* g, 18.42 mmol) in tetrahydrofuran (50 mL), followed by stirring for 18 hours. The crude product was afforded by concentration under reduced pressure. The crude product was purified by silica gel chromatography (elution solution: MeOH : DCM = 3%-10%) to afford compound 1d. MS m/z (ESI): 158.1 [M+1].

Step 3: At 0°C under a nitrogen atmosphere, sodium hydride (58.27 mg, 1.46 mmol, 60% purity) was added to a solution of compound 1d (214.71 mg, 1.09 mmol) in tetrahydrofuran (5 mL). After stirring for 10 minutes, compound Int-1 (300 mg, 0.73 mmol) was added. The resulting mixture was heated to 60°C, and stirred for 5 hours. Water (3 mL) was added to the mixture, concentrated under reduced pressure, to afford the crude product. The crude product was purified by preparative HPLC (chromatographic column: Waters-Xbridge-C18-10μm-19*250mm; mobile phase: A: 10 mM ammonium bicarbonate/water; B: acetonitrile, gradient ratio: acetonitrile 95%, flow rate: 20 mL/min), to afford compound 1. MS m/z (ESI): 533.3 [M+1]. $^1$H NMR (400 MHz, DMSO-d6): δ 9.35-9.25 (m, 1H), 8.39-8.28 (m, 1H), 7.19-7.09 (m, 1H), 6.77-6.71 (m, 1H), 6.43 (s, 1H), 4.45-4.11 (m, 2H), 3.23-3.14(m, 2H), 3.09 (s, 4H), 2.51 (s, 2H), 2.44 (s, 4H), 2.22 (s, 3H), 2.16 (s, 2H), 1.95-1.80 (m, 3H), 1.67 (s, 4H), 0.81 (s, 2H), 0.60 (d, J = 3.7 Hz, 2H).

**Example 2. Preparation of compound 2**

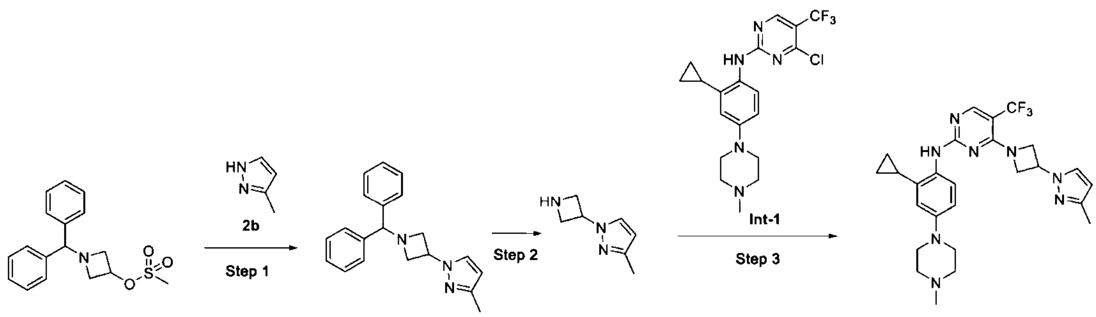

2a 2c 2d Compound 2

Step 1: Compound 2a (1 g, 3.15 mmol, Bide) and 2b (0.51 mL, 6.30 mmol, Bide) were dissolved in acetonitrile (30 mL). N,N-diisopropylethylamine (1.04 mL, 6.30 mmol) was added, and the reaction solution was stirred at 70 °C for 18 hours. The reaction solution was concentrated to dryness, and the crude was purified by silica gel column chromatography (eluant system: ethyl acetate : petroleum ether = 10:1 to 3:1) to afford compound 2c. MS m/z (ESI): 304.2 [M+1].

Step 2: Compound 2c (100 mg, 0.33 mmol) was dissolved in 5 mL methanol. Palladium on carbon (35.07 mg, 0.03 mmol) was added, followed by stirring at 50°C for 4 hours. The reaction solution was filtered. The filter cake was washed with methanol (5 mL × 3), and the filtrate was concentrated to dryness to afford compound 2d. MS m/z (ESI): 138.2 [M+1].

Step 3: Compound 2d (45 mg, 0.33 mmol) and compound Int-1 (122.81 mg, 0.30 mmol) were dissolved in 1 mL *N,N*- dimethylformamide. N,N-diisopropylethylamine (0.15 mL, 0.89 mmol) was added, followed by stirring at 90°C for 1 hour. The reaction solution was purified by high performance liquid chromatography (Gilson_306_1741, chromatographic column: Shimadzu-Shim-pack-C18-5um-20*250mm; mobile phase: water (containing 0.05% HCl) and acetonitrile, gradient ratio: acetonitrile 23%-33%, flow rate: 20 mL/min) to afford compound 2. MS m/z (ESI): 513.4 [M+1]. $^1$H NMR (400 MHz, DMSO-d6): δ 11.34 (s, 1H), 9.85 (s, 1H), 8.42 (s, 1H), 7.43 (s, 1H), 6.88 -6.74 (m, 1H), 6.57 (d, J = 3.2 Hz, 1H), 6.06 (dd, J = 4.4, 2.4 Hz, 1H), 5.29 (s, 1H), 4.68 (d, J =10.2 Hz, 2H), 4.47 (s, 2H), 3.74 (s, 2H), 3.42 (d, J = 11.4 Hz, 2H), 3.15 (d, J = 21.2 Hz, 4H),2.76 (d, J = 4.6 Hz, 3H), 2.22 (dq, J = 28.2, 2.2 Hz, 3H), 1.96 (s, 1H), 0.92 (d, J = 8.0 Hz, 2H),0.74 - 0.58 (m, 2H).

**Example 3. Preparation of compound 3**

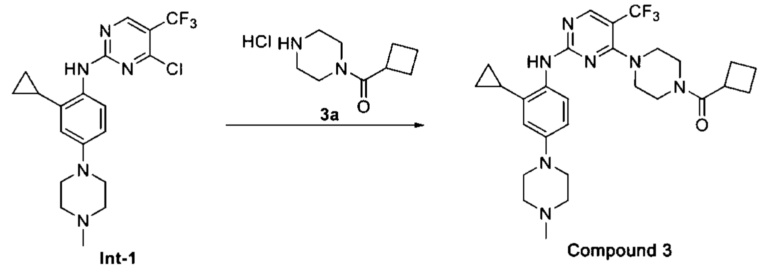


At room temperature, compound 3a (89.87 mg, 0.53 mmol, Bide) was added to a solution of compound Int-1 (200 mg, 0.49 mmol) in N,N-dimethylformamide (3 mL), followed by heating to 100°C and stirring for 2 h. After cooling to room temperature, the crude product was purified by preparative HPLC (chromatographic column: Waters-Xbridge-C18-10μm-19*250mm; mobile phase: A: 10 mM ammonium bicarbonate/water; B: acetonitrile, gradient ratio: acetonitrile 95%, flow rate: 25 mL/min) to afford compound **3.** MS m/z (ESI): 544.3 [M+1]. $^1$H NMR(400 MHz, DMSO-$d_6$): δ 8.89 (s, 1H), 8.29 (s, 1H), 7.24-7.12 (m, 1H), 6.76-6.68 (m, 1H), 6.42 (s, 1H), 3.41 (s, 8H), 3.08 (m, 4H), 2.43 (m, 4H), 2.21 (s, 3H), 2.07 (m, 5H), 1.96-1.83 (m, 2H), 1.80-1.66 (m, 1H), 0.80 (m, 2H), 0.59 (d, *J* = 3.6 Hz, 2H).

**Example 4. Preparation of compound 4**

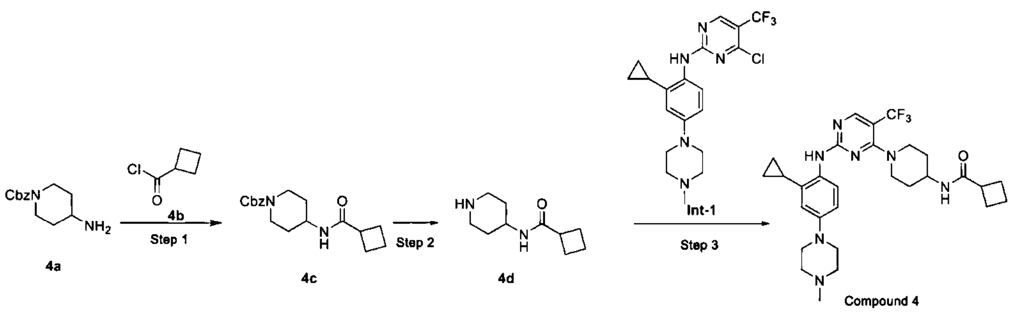


Step 1: At room temperature, compound 4b (535.73 μL, 4.69 mmol, Bide) was added to a solution of compound 4a (1 g, 4.27 mmol, Bide) and triethylamine (1.8 mL, 12.80 mmol) in N,N-dimethylformamide (10 mL), followed by stirring at room temperature for 18 hours. The mixture was poured into water (20 mL), followed by extraction with ethyl acetate (20 mL x 3). The organic phases were combined, washed with saturated saline (20 mL × 4), dried over anhydrous sodium sulfate, filtered and concentrated under reduced pressure, to afford the crude product. The crude was purified by silica gel chromatography (eluant: petroleum ether : ethyl acetate =10:1 to 1:2) to afford compound 4c. MS m/z (ESI): 317.1 [M+1].

Step 2: At room temperature, palladium on carbon (0.13 g, 4.11 mmol) was added to a solution of compound 4c (1.8 g, 5.69 mmol) and acetic acid (2 mL, 34.97 mmol) in methanol (20 mL). The mixture was purged with hydrogen 3 times, heated to 50 °C, reacted under stirring for 18 h, and filtered. The filter cake was washed with methanol (20 mL x3), and the filtrate was concentrated under reduced pressure to afford compound 4d. MS m/z (ESI): 183.1 [M+1].

Step 3: At 25°C, triethylamine (336.56 μL, 2.43 mmol) and compound 4d (139.09 mg, 0.53 mmol) were added to compound Int-1 (200 mg, 0.49 mmol) in *N,N*-dimethylformamide (3 mL), respectively, followed by heating to 100 °C and stirring for 2 hours. The crude product was afforded by concentration under reduced pressure. The crude product was purified by preparative HPLC (chromatographic column: Waters-Xbridge-C18-10μm-19*250mm; mobile phase: A: 10 mM ammonium bicarbonate/water; B: acetonitrile, gradient ratio: acetonitrile 95%, flow rate: 25 mL/min) to afford compound 4. MS m/z (ESI): 558.3 [M+1]. $^1$H NMR(400 MHz, DMSO-d6): δ 8.79 (s, 1H), 8.26 (s, 1H), 7.61-7.56 (m, 1H), 7.25-7.17 (m, 1H), 6.74-6.68 (m, 1H), 6.42 (s, 1H), 3.96-3.87 (m, 2H), 3.85-3.74 (m, 1H), 3.07 (m, 6H), 2.97-2.90 (m, 1H), 2.43 (m, 4H), 2.21 (s, 3H), 2.13-2.06 (m, 2H), 2.00-1.83 (m, 4H), 1.78-1.68 (m, 3H), 1.41-1.31 (m, 2H), 0.85-0.80 (m, 2H), 0.59 (d, J= 4.0 Hz, 2H).

**Example 5. Preparation of compound 5**

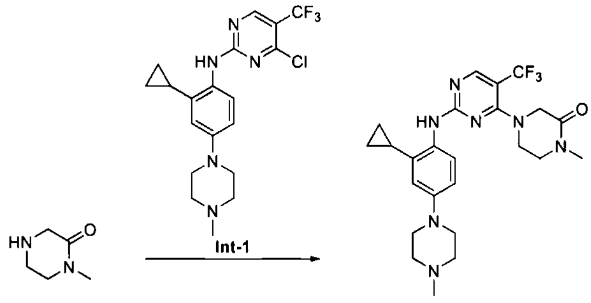


5a

Compound 5

Compound 5a (60.97 mg, 0.53 mmol, Bide) and compound Int-1 (200 mg, 0.49 mmol) were dissolved in *N,N*-dimethylformamide (2 mL). *N, N*-diisopropylethylamine (0.24 mL, 1.46 mmol) was added, followed by stirring at 90°C for 1 hour. The reaction solution was purified by high performance liquid chromatography (Gilson_306_1741, chromatographic column: Waters-Xbridge-C18-10μm-19*250mm; mobile phase: water (containing 10 mM sodium bicarbonate) and acetonitrile, gradient ratio: acetonitrile 38%-68%, flow rate: 25 mL/min) to afford compound 5. MS m/z (ESI): 490.2 [M+1]. $^1$H NMR (400 MHz, DMSO-d6): δ 8.96 (s, 1H), 8.31 (s, 1H), 7.17 (d, J = 8.6 Hz, 1H), 6.72 (dd, J = 8.8, 2.8 Hz, 1H), 6.41 (d, J = 2.8 Hz, 1H), 4.00 (s, 2H), 3.72 (s, 2H), 3.35 (s, 2H), 3.08 (t, J =5.0 Hz, 4H), 2.85 (s, 3H), 2.43 (t, J = 5.0 Hz, 4H), 2.21 (s, 3H), 1.92 (ddd, J = 8.4, 5.2, 3.2 Hz,1H), 0.86 - 0.78 (m, 2H), 0.63 - 0.56 (m, 2H).

**Example 6. Preparation of compound 6**

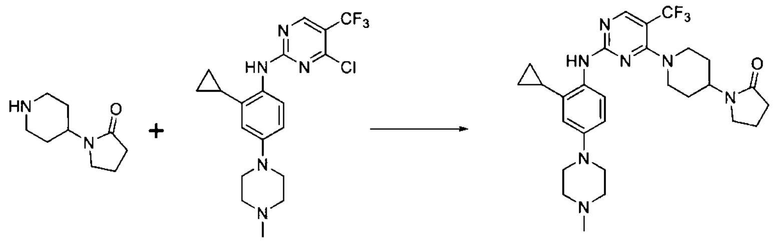

6a Int-1 Compound 6

Compound 6a (122.55 mg, 0.73 mmol, Bide) and compound Int-1 (300 mg, 0.73 mmol) were dissolved in *N,N*-dimethylformamide (3 mL). *N,N*-diisopropylethylamine (0.60 mL, 3.64 mmol) was added, followed by stirring at 90°C for 1 hour. The reaction solution was purified by high performance liquid chromatography (Gilson_306_1741, chromatographic column: Puningtech-Pntulips-C18-5um-21.2*150mm; mobile phase: water (containing 10 mM sodium bicarbonate) and acetonitrile, gradient ratio: acetonitrile 50%-80%, flow rate: 20 mL/min) to afford compound 6. MS m/z (ESI): 544.5 [M+1]. $^1$H NMR (400 MHz, DMSO-d6): δ 8.82 (s, 1H), 8.27 (s, 1H), 7.20 (d, J = 8.0 Hz,1H), 6.71 (dd, J = 8.8, 2.8 Hz, 1H), 6.42 (d, J = 4.0 Hz, 1H), 4.12 - 3.95 (m, 3H),3.25 (t, J = 7.0 Hz, 2H), 3.07 (t, J = 5.0 Hz, 4H), 2.96 (s, 2H), 2.43 (t, J = 5.0 Hz,4H), 2.20 (d, J = 7.2 Hz, 5H), 1.94 - 1.84 (m, 3H), 1.58 (s, 4H), 0.85 - 0.78 (m, 2H),0.59 (dd, J = 5.2, 1.9 Hz, 2H).

**Example 7. Preparation of compound 7**

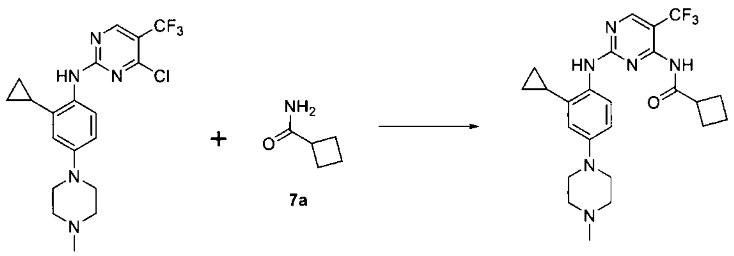

7a

Int-1 Compound 7

Compound Int-1 (300 mg, 0.73 mmol) and compound 7a (79.43 mg, 0.80 mmol, Bide) were dissolved in 1,4-dioxane (6 mL). Tris(dibezylideneacetone)dipalladium (66.70 mg, 0.07 mmol), 4,5-bis(diphenylphosphino)-9,9-dimethylxanthene (66.70 mg, 0.07 mmol) and cesium carbonate (711.98 mg, 2.19 mmol) were added, followed by stirring at 110°C for 18 hours under a nitrogen atmosphere. The reaction solution was purified by high performance liquid chromatography (Gilson_306_1741, chromatographic column: Puningtech-Pntulips-C18-5um-21.2*150mm; mobile phase: water (containing 0.225% formic acid) and acetonitrile, gradient ratio: acetonitrile 25%-35%, flow rate: 15 mL/min) to afford compound 7. MS m/z (ESI): 475.1 [M+1]. $^1$H NMR (400 MHz, DMSO-d6): δ 9.41 (s, 1H), 8.53 (s, 1H), 8.15 (s, 1H), 7.11 (d, J = 8.6 Hz, 1H),6.76 (dd, J = 8.6, 2.8 Hz, 1H), 6.47 (d, J = 2.8 Hz, 1H), 3.11 (t, J = 5.0 Hz, 4H), 2.68(t, J = 2.0 Hz, 1H), 2.47 (d, J = 4.0 Hz, 4H), 2.25 (s, 3H), 2.08 (s, 3H), 1.90 (s, 2H),1.73 (s, 2H), 0.82 - 0.76 (m, 2H), 0.60 (dd, J = 5.4, 2.0 Hz, 2H).

**Example 8. Preparation of compound 8**

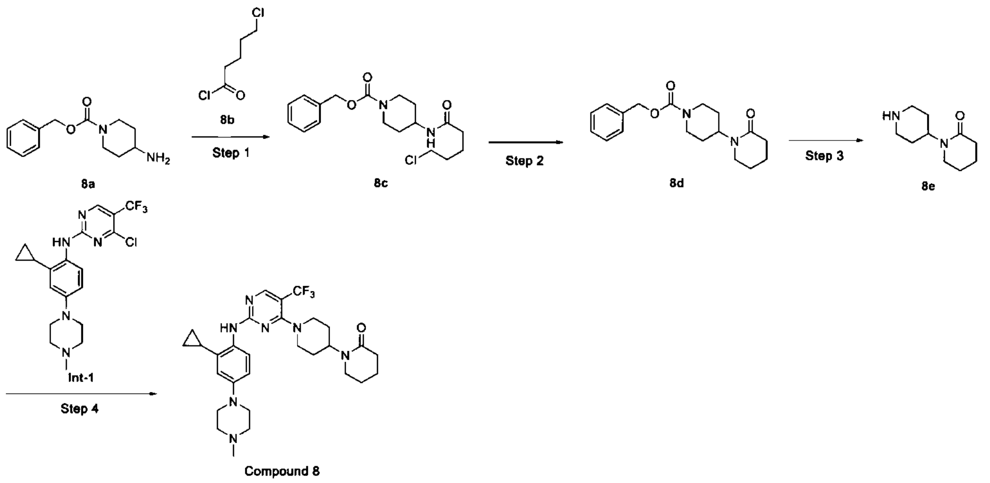

8a 8c 8d 8e Int-1 Compound 8

Step 1: Compound 8a (1 g, 4.27 mmol, Bide) and triethylamine (0.89 mL, 6.40 mmol) were dissolved in dichloromethane (20 mL). 8b (0.55 mL, 4.27 mmol) was added dropwise at 0°C, followed by stirring at room temperature for 18 hours. The reaction solution was concentrated to afford compound 8c. MS m/z (ESI): 353.0 [M+1].

Step 2: Compound 8c (1.3 g, 3.68 mmol) was dissolved in *N,N*- dimethylformamide (15 mL). Potassium tert-butoxide (7.37 mL, 7.37 mmol) was added, followed by stirring at room temperature for 3 hours. The reaction solution was diluted by adding water (30 mL), followed by extraction with ethyl acetate (30 mL × 3). The organic phases were combined, washed with saturated saline, dried over anhydrous sodium sulfate, filtered, and concentrated. The residue was purified by silica gel column chromatography (eluant system: methanol : dichloromethane = 10: 1) to afford compound 8d. MS m/z (ESI): 317.3 [M+1].

Step 3: Compound 8d (460 mg, 1.45 mmol) was dissolved in methanol (15 mL) and formic acid (1 mL). Palladium on carbon (154.72 mg, 0.15 mmol) was added, followed by stirring at room temperature for 18 hours under a hydrogen atmosphere. The reaction solution was filtered. The filter cake was washed with methanol (10 mL × 3), and the filtrate was concentrated to afford compound 8e. MS m/z (ESI): 183.2 [M+1].

Step 4: Compound 8e (265 mg, 1.45 mmol) and compound Int-1 (598.80 mg, 1.45 mmol) were dissolved in *N,N*-dimethylformamide (6 mL). *N,N*-diisopropylethylamine (0.96 mL, 5.82 mmol) was added, followed by stirring at 90°C for 1 hour. The reaction solution was purified by high performance liquid chromatography (Gilson_306_1741, chromatographic column: Waters-Xbridge-C18-10μm-19*250mm; mobile phase: water (containing 10 mM ammonium bicarbonate) and acetonitrile, gradient ratio: acetonitrile 39%-69%, flow rate: 25 mL/min) to afford compound 8. MS m/z (ESI): 558.5 [M+1]. $^1$H NMR (400 MHz, DMSO-d6): δ 8.82 (s, 1H), 8.26 (s, 1H), 7.20 (d, J = 8.6 Hz, 1H), 6.72 (dd, J = 8.6, 2.8 Hz, 1H), 6.42 (d, J = 2.8 Hz, 1H), 4.57 - 4.47 (m, 1H), 4.07 (d, J = 13.2 Hz, 2H), 3.09 (d, J = 6.4 Hz, 6H), 2.94 (t, J = 12.6 Hz, 2H), 2.51-2.49 (m, 4H), 2.27 - 2.18 (m, 5H), 1.93 (s, 1H), 1.70 - 1.61 (m, 6H), 1.50 (d, J = 12.0 Hz, 2H), 0.81 (dt, J = 8.4, 3.0 Hz, 2H), 0.59 (dt, J = 5.4, 2.8 Hz, 2H).

**Example 9. Preparation of compound 9**

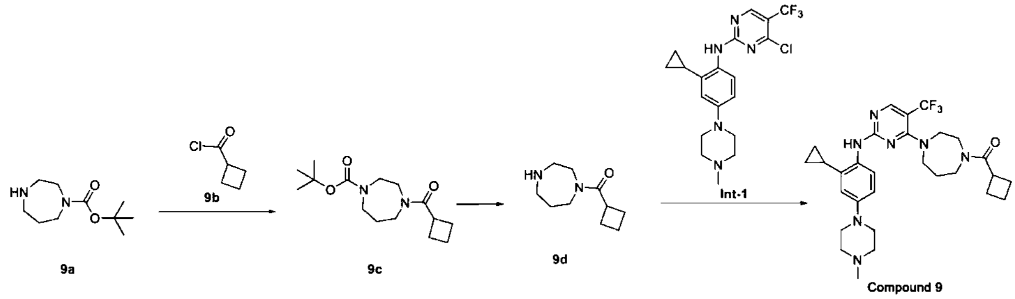


Step 1: At room temperature, compound 9b (313.06 μ L, 2.75 mmol, Bide) was added to a solution of compound 9a (485.44 μL, 2.50 mmol, Bide) and triethylamine (1.1 mL, 7.49 mmol) in dichloromethane (5 mL), followed by stirring at room temperature for 18 h. The mixture was poured into water (20 mL), followed by extraction with ethyl acetate (20 mL x3). The organic phases were combined, washed with saturated saline (30 mL), dried over anhydrous sodium sulfate, filtered, and concentrated under reduced pressure to afford compound 9c. MS m/z (ESI): 227.1 [M-56].

Step 2: At room temperature, hydrochloric acid/1,4-dioxane (5 mL, 4M) was added to a solution of compound 9c (970 mg, 2.40 mmol) in 1,4-dioxane (10 mL), followed by stirring at room temperature for 4 h. Compound 9d was afforded by concentration under reduced pressure. MS m/z (ESI): 183.2 [M+1].

Step 3: Triethylamine (504.84 μL, 3.64 mmol) and compound 9d were added to a solution of compound Int-1 (300 mg, 0.73 mmol) in N,N-dimethylformamide (3 mL). The resulting mixture was heated to 100 °C and reacted under stirring for 2 h. The crude product was afforded by concentration under reduced pressure. The crude product was purified by preparative HPLC (chromatographic column: Waters-Xbridge-C18-10μm-19*250 mm; mobile phase: A: 10 mM ammonium bicarbonate/water B: acetonitrile, gradient ratio: acetonitrile 95%, flow rate: 25 mL/min) to afford compound 9. MS m/z (ESI): 558.3 [M+1]. $^1$H NMR(400 MHz, DMSO-$d_6$): δ 8.78 (d, *J* = 43.8 Hz, 1H), 8.24 (s, 1H), 7.16 (d, *J* = 8.3 Hz, 1H), 6.77- 6.65 (m, 1H), 6.43 (dd, *J* = 8.1, 2.4 Hz, 1H), 3.66 (s, 1H),3.54 (s, 3H), 3.36 (s, 1H), 3.32-3.22 (m, 4H), 3.08 (s, 4H), 2.45-2.36 (m, 4H), 2.21 (s, 3H), 2.07-1.76 (m, 6H), 1.63 (d, *J* = 9.8 Hz, 3H), 0.87-0.76 (m, 2H),0.64-0.54 (m, 2H).

**Example 10. Preparation of compound 10**

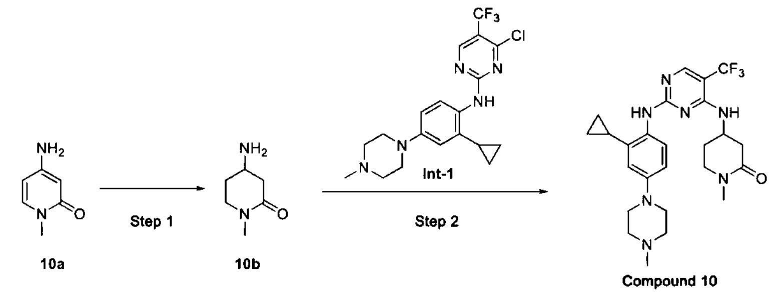


Step 1: Compound 10a (175 mg, 1.41 mol, Bide) was dissolved in methanol (6 mL), and platinum dioxide (50 mg, 0.22 mmol, Bide) was added. After reacting under stirring at room temperature for 18 hours under a hydrogen atmosphere, the reaction solution was filtered. The filter cake was washed with methanol (5mL ×3), and the filtrate was concentrated under reduced pressure to afford compound 10b. MS m/z (ESI): 129.2 [M+1].

Step 2: Compound 10b (180 mg, 0.7 mol), compound Int-1 (289 mg, 0.7 mol) were dissolved in 5 mL *N,N*-dimethylformamide, and *N,N*-diisopropylethylamine (0.35 mL, 2.11 mmol) was added. After reacting under stirring at 100°C for 1 hour, the reaction solution was separated and purified by high performance liquid chromatography (Waters-2545, chromatographic column: Gemini-NX 80*40mm, 3 μm; mobile phase: water (containing 0.01 mol/L ammonium bicarbonate) and acetonitrile, gradient ratio: acetonitrile 35%-45%, flow rate: 30 mL/min) to afford compound 10. MS m/z (ESI): 504.4 [M+1]. $^1$H NMR (400 MHz, DMSO-d6): δ 8.74 (s, 1H), 8.09 (s, 1H), 7.15 (d, J = 8.6 Hz, 1H), 6.70 (dd, J = 8.7, 2.8 Hz, 1H), 6.59 (d, J = 7.3 Hz, 1H), 6.40 (d, J = 2.8 Hz, 1H), 3.19 (s, 1H), 3.07(t, J = 4.9 Hz, 4H), 2.78 (s, 3H), 2.52 (s, 2H), 2.50 (s, 2H), 2.46 (s, 1H), 2.44 (t, J = 5.0 Hz,4H), 2.21 (s, 3H), 1.97 - 1.88 (m, 2H), 0.85 - 0.78 (m, 2H), 0.62 - 0.54 (m, 2H).

**Example 11. Preparation of compound 11**

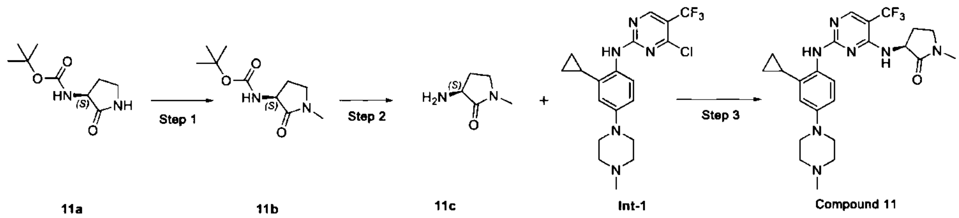


11a 11b 11c Int-1 Compound 11

Step 1: Compound 11a (500 mg, 2.50 mmol, Bide) was dissolved in *N,N*-dimethylformamide (10 mL). Sodium hydride (109.87 mg, 2.75 mmol) was added at 0°C, followed by stirring for 30 minutes under a nitrogen atmosphere. Iodomethane (0.16 mL, 2.62 mmol) after being dissolved in *N,N*-dimethylformamide (1 mL), was then added dropwise to the reaction solution, followed by stirring at room temperature for 18 hours under a nitrogen atmosphere. The reaction solution was diluted by adding water (30 mL), followed by extraction with ethyl acetate (20 mL×3). The organic phases were combined, washed with saturated saline, dried over anhydrous sodium sulfate, filtered, and concentrated to afford compound 11b. MS m/z (ESI): 215.2 [M+1].

Step 2: Compound 11b (535 mg, 2.50 mmol) was dissolved in dichloromethane (10 mL). Hydrochloric acid (10 mL, 40.00 mmol) was added, followed by stirring at room temperature for 18 hours. The reaction solution was concentrated to afford compound 11c. MS m/z (ESI): 115.1 [M+1].

Step 3: Compound 11c (316 mg, 2.30 mmol) and compound Int-1 (505.13 mg, 1.23 mmol) were dissolved in *N,N*-dimethylformamide (5 mL). N,N-diisopropylethylamine (1.02 mL, 6.15 mmol) was added, followed by stirring at 90°C for 1 hour. The reaction solution was purified by high performance liquid chromatography (Gilson_306_1741, chromatographic column: Waters-CORTECS-C18-2.7μm-4.6*30mm; mobile phase: water (containing 10mmol/L ammonium bicarbonate) and acetonitrile, gradient ratio: acetonitrile 39%-59%, flow rate: 20 mL/min) to afford compound 11. MS m/z (ESI): 490.4 [M+1]. $^1$H NMR (400 MHz, DMSO-d6): δ 8.64 (s, 1H), 8.09 (s, 1H), 7.11 (d, J = 8.6 Hz, 1H), 6.89(d, J = 7.2 Hz, 1H), 6.72 (dd, J = 8.6, 2.8 Hz, 1H), 6.44 (d, J = 2.8 Hz, 1H), 4.44 (s, 1H), 3.33-3.30(m, 1H), 3.08(t, J = 5.0 Hz, 6H), 2.60 (s, 2H), 2.43 (t, J = 5.0 Hz, 4H), 2.21 (s, 3H), 2.08-2.03 (m, 2H), 1.88 (td, J = 8.4, 4.2 Hz, 1H), 0.79 (d, J = 8.2 Hz, 2H), 0.62 - 0.54 (m, 2H).

**Example 12. Preparation of compound 12**

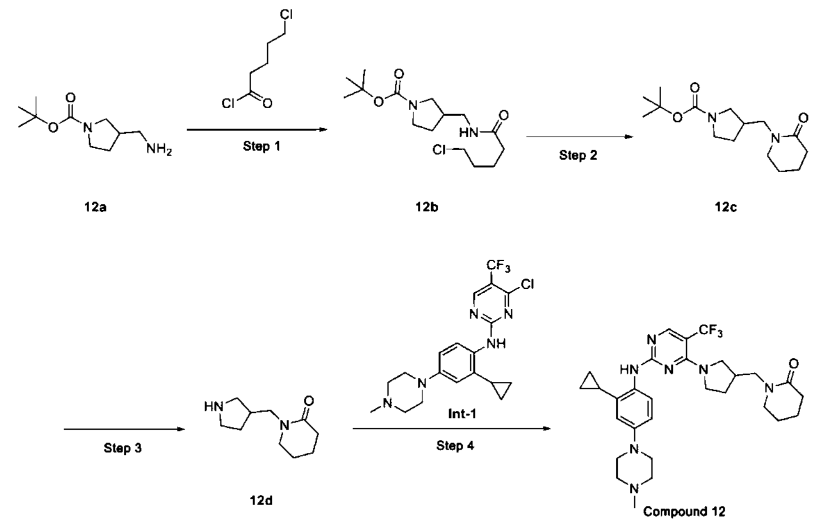


12a 12b 12c 12d Compound 12

Step 1: At 25°C, 5-chlorovaleryl chloride (154.80 μL, 1.20 mmol, Bide) was added to a solution of compound 12a (190.48 μL, 1.00 mmol, Bide) and TEA (415.27 μL, 3.00 mmol) in N,N-dimethylformamide (2 mL), followed by stirring for 18 hours. The mixture was poured into water (10 mL), followed by extraction with ethyl acetate (20 mL x3). The organic phases were combined, washed with saturated saline (20 mL x 4), dried over anhydrous sodium sulfate, filtered, and concentrated under reduced pressure to afford compound 12b. MS m/z (ESI): 319.2 [M+1].

Step 2: At 25°C, potassium tert-butoxide (2.3 mL, 2.32 mmol, 1M) was added to a solution of compound 12b (370 mg, 1.16 mmol) in *N,N*- dimethylformamide (4 mL), followed by stirring for 4 h. The mixture was poured into water (20 mL), followed by extraction with ethyl acetate (20 mL x 3). The organic phases were combined, washed with saturated saline(20 mL x 4), dried over anhydrous sodium sulfate, filtered, and concentrated under reduced pressure to afford compound 12c. MS m/z (ESI): 283.2 [M+1].

Step 3: At 25°C, hydrochloric acid-1,4-dioxane (2 mL, 4 M) was added to a solution of compound 12c (340 mg, 0.60 mmol) in 1,4-dioxane (4 mL). The resulting mixture was stirred for 18 h and concentrated under reduced pressure to afford compound 12d. MS m/z (ESI): 183.2 [M+1].

Step 4: At room temperature, triethylamine (588.98 μL, 4.25 mmol) was added to a solution of compound Int-1 (350 mg, 0.85 mmol) and compound 12d (340.77 mg, 0.93 mmol) in *N,N*- dimethylformamide (3 mL), followed by heating to 100°C and stirring for 2 h. The crude product was afforded by concentration under reduced pressure. The crude product was purified by preparative HPLC (chromatographic column: Waters-Xbridge-C18-10μm-19*250mm; mobile phase: A: 10 mM ammonium bicarbonate/water: acetonitrile, gradient ratio: acetonitrile 95%, flow rate: 20 mL/min) to afford compound 12. MS m/z (ESI): 558.3 [M+1]. $^1$H NMR(400 MHz, DMSO-$d_6$): δ 8.53 (s, 1H), 8.20(s, 1H), 7.34 (s, 1H), 6.72 (dd, *J* = 8.6, 2.8 Hz,1H), 6.44 (d, *J*= 2.8 Hz, 1H), 3.57 (dd, *J* = 11.6, 7.0 Hz, 2H), 3.49-3.35 (m, 2H), 3.23 (td, *J* = 12.4,6.6 Hz, 4H), 3.06 (t, *J* =5.0 Hz, 4H), 2.43 (t, *J* = 4.8 Hz, 4H), 2.20 (d, *J* = 4.6 Hz, 4H), 1.98-1.90(m, 2H), 1.69 (d, *J*= 5.0 Hz, 4H), 1.61 (dd, *J* = 12.0, 7.6 Hz, 1H), 1.23 (s, 2H), 0.86-0.79 (m, 2H),0.58 (q, *J*= 5.0, 4.6 Hz, 2H).

**Example 13. Preparation of compound 13**

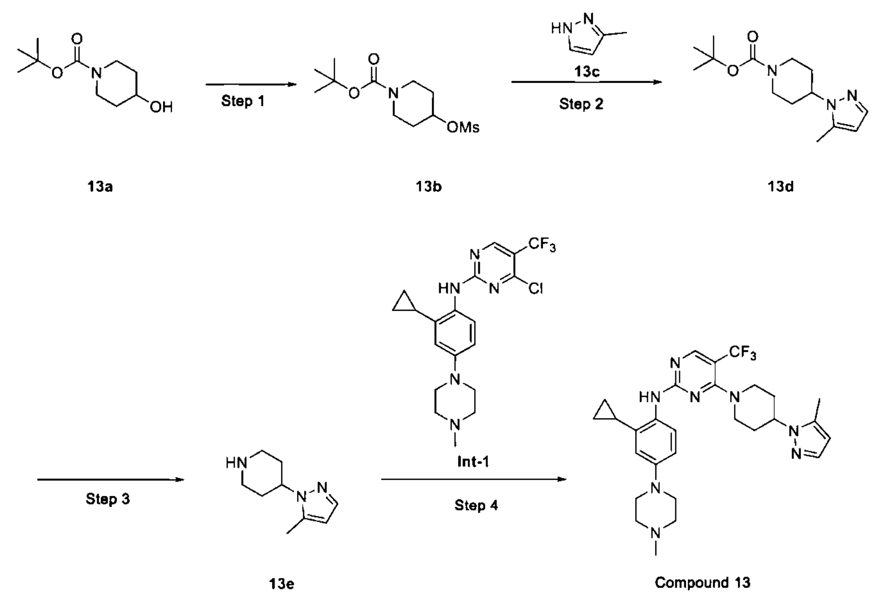


Step 1: At 0°C, methylsulfonyl chloride (211.48 μL, 2.73 mmol) was added to a solution of compound 13a (500 mg, 2.48 mmol, Bide) and triethylamine (378.79 μL, 2.73 mmol) in dichloromethane (5 mL), followed by stirring for 2 hours. The mixture was poured into water (10 mL), followed by extraction with dichloromethane (10 mL x 3). The organic phases were combined, washed with saturated saline (20 mL), dried over anhydrous sodium sulfate, filtered, and concentrated under reduced pressure to afford compound 13b. MS m/z (ESI): 280.1 [M+1].

Step 2: At 0°C, sodium hydride (154.33 mg, 3.86 mmol, 60%) was added to a solution of compound 13c (215.69 μL, 2.68 mmol, Bide) in *N,N*- dimethylformamide (5 mL). After stirring for 10 minutes, compound 13b (598.78 mg, 2.14 mmol) was added, followed by heating to 50 °C and stirring for 18 hours. The mixture was poured into water (20 mL), followed by extraction with ethyl acetate (20 mL x 3). The organic phases were combined, washed with saturated saline (20 mL x 4), dried over anhydrous sodium sulfate, filtered, and concentrated under reduced pressure to afford compound 13d. MS m/z (ESI): 266.2 [M+1].

Step 3: At 25°C, hydrochloric acid -1,4-dioxane (2 mL, 4 M) was added to a solution of compound 13d (731 mg, 2.48 mmol) in 1,4-dioxane (5 mL), followed by stirring for 3h. Compound 13e was afforded by concentration under reduced pressure. MS m/z (ESI): 166.2 [M+1].

Step 4: At room temperature, triethylamine (841.41 μL, 6.07 mmol) was added to a solution of compound Int-1 (500 mg, 1.21 mmol) and compound 13e (219.93 mg, 1.33 mmol) in N,N- dimethylformamide (5 mL), followed by heating to

100 °C and stirring for 2 h. The crude product was afforded by concentration under reduced pressure. The crude product was purified by preparative HPLC (chromatographic column: Waters-Xbridge-C18-10μm-19*250mm; mobile phase: A: 10 mM ammonium bicarbonate/water; B: acetonitrile, gradient ratio: acetonitrile 95%, flow rate: 25 mL/min) to afford compound 13. MS m/z (ESI): 541.3 [M+1]. $^1$H NMR(400 MHz, DMSO-$d_6$): δ 8.84 (s, 1H), 8.30 (s, 1H), 7.30 (m, 2H), 6.74-6.65 (m, 1H), 6.41 (s, 1H), 5.99 (s, 1H), 4.45-4.34 (m, 1H), 4.17-4.02 (m, 2H), 3.07 (s, 4H), 2.43 (m, 4H), 2.29 (s, 3H), 2.21 (s, 3H), 1.90 (d, $J$ = 27.8 Hz, 7H), 0.84 (m, 2H), 0.60 (m, 2H).

**Example 14. Preparation of compound 14**

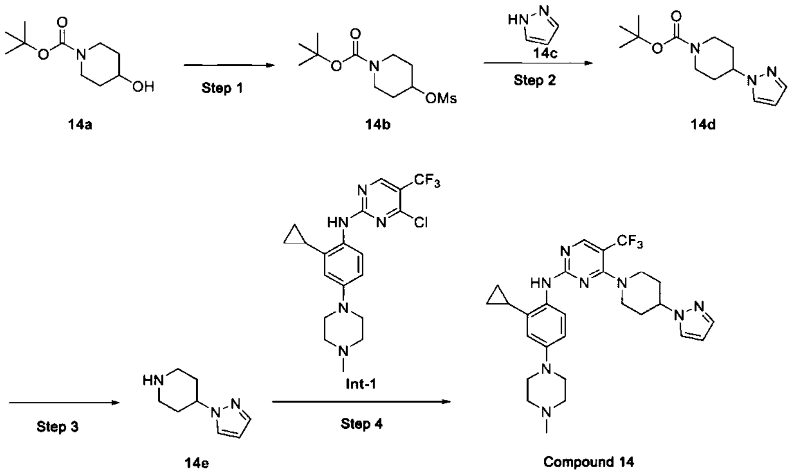


Step 1: At 0°C, methylsulfonyl chloride (212 μL, 2.73 mmol) was added to a solution of compound 14a (500 mg, 2.48 mmol, Bide) and triethylamine (379 mL, 2.73 mmol) in dichloromethane (5 mL), followed by stirring for 2 hours. The mixture was poured into water (10 mL), followed by extraction with dichloromethane (10 mL x3). The organic phases were combined, washed with saturated saline (20 mL), dried over anhydrous sodium sulfate, filtered, and concentrated under reduced pressure to afford compound 14b. MS m/z (ESI): 280.1 [M+1].

Step 2: At 0°C, sodium hydride (169.21 mg, 4.23 mmol, 60% purity) was added to a solution of compound 14c (141.84 μL, 2.94 mmol, Bide) in *N,N*- dimethylformamide (5mL). After stirring for 10 minutes, compound 14b (820 mg, 2.35 mmol) was added, followed by heating to 50°C and stirring for 18 hours. The mixture was poured into water (20 mL), followed by extraction with ethyl acetate (20 mL x 3). The organic phases were combined, washed with saturated saline (20 mL x 4), dried over anhydrous sodium sulfate, filtered, and concentrated under reduced pressure to afford the crude compound, which was purified by silica gel chromatography (eluant : MeOH : DCM = 5%-10%) to afford compound 14d. MS m/z (ESI): 252.2 [M+1].

Step 3: At 25°C, hydrochloric acid-1,4-dioxane (6 mL, 4 M) was added to a solution of compound 14d (304 mg, 1.21 mmol) in 1,4-dioxane (3 mL), followed by stirring for 3 h. Compound 14e was afforded by concentration under reduced pressure.

MS m/z (ESI): 152.2 [M+1].

Step 4: At room temperature, triethylamine (674 μL, 4.86 mmol) was added to a solution of compound Int-1 (400 mg, 0.97 mmol) and compound 14e (200 mg, 1.06 mmol) in *N,N*-dimethylformamide (5 mL), followed by heating to 100°C and stirring for 2 h. The crude product was afforded by concentration under reduced pressure. The crude product was purified by preparative HPLC (chromatographic column: Waters-Xbridge-C18-10μm-19*250mm; mobile phase: A: 10 mM ammonium bicarbonate/water; B: acetonitrile, gradient ratio: acetonitrile 95%, flow rate: 20 mL/min) to afford compound 14. MS m/z (ESI): 527.5 [M+1]. $^1$H NMR(400 MHz, DMSO-$d_6$): δ 8.85 (s, 1H), 8.29 (s, 1H), 7.77 (d, $J$ = 1.9 Hz, 1H), 7.43(s, 1H), 7.25-7.17 (m, 1H), 6.71 (dd, $J$ = 8.7, 2.7 Hz, 1H), 6.41 (d, J=2.6 Hz, 1H), 6.23 (d, $J$ = 4.0 Hz, 1H), 4.50-4.39 (m, 1H), 4.07 (dd, $J$ = 12.2,3.0 Hz, 2H), 3.14-3.08 (m, 2H), 3.06 (d, J = 9.6 Hz, 4H), 2.42 (d, J = 9.7 Hz, 4H),2.20 (s, 3H), 2.06-1.87 (m, 5H), 0.87-0.77 (m, 2H), 0.63-0.56 (m, 2H).

**Example 15. Preparation of compound 15**

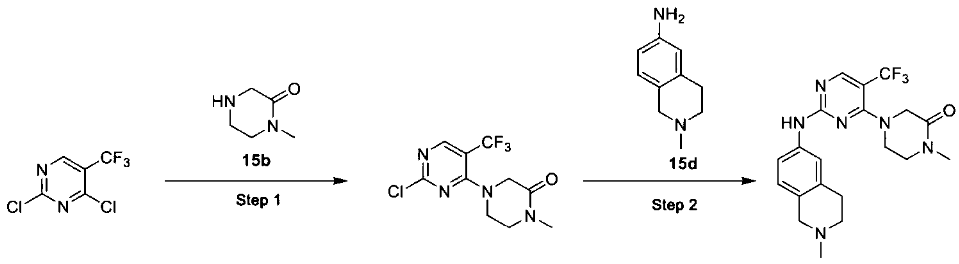


15a 15c Compound 15

Step 1: Compound 15a (600 mg, 2.77 mmol, Bide) was dissolved in 10 mL of *N,N*-dimethylformamide, and compound 15b (0.30 mL, 2.77 mmol, Bide) and triethylamine (0.42 mL, 3.04 mmol) were added at 0°C. The resulting mixture was reacted under stirring at 0°C for 10 minutes, followed by heating to 25°C and continuing the reaction for 5 hours. After the reaction was completed, 10 mL of ice water was added to the reaction solution, which was then stirred at 0°C for 30 minutes, followed by suction filtration. The filter cake was washed with ethyl acetate (20 mL) and extracted (20 mL×3). The organic phases were combined, dried over anhydrous sodium sulfate, filtered, and concentrated. The residue was purified by high performance liquid chromatography (chromatographic column: Phenomenex Gemini C18, 25*150mm,10μm; mobile phase: water (containing 10 mmol/L ammonium bicarbonate) and acetonitrile, gradient ratio: acetonitrile 30%-40%, flow rate: 30 mL/min) to afford compound 15c. MS m/z (ESI): 295.0 [M+1]. $^1$H NMR (400 MHz, DMSO-d6): δ 8.67 (s, 1H), 4.15 (s, 2H), 3.93 (t, J = 5.4 Hz, 2H), 3.44 (t, J = 5.4 Hz, 2H), 2.88 (s, 3H).

Step 2: Compound 15c (132 mg, 0.45 mmol) was dissolved in N,N-dimethylformamide (2 mL), and compound 15d (79.95 mg, 0.49 mmol) and a dioxane solution of hydrochloric acid (223.99 μL, 0.90 mmol, 4 M) were added. After reacting under stirring at 100°C for 4 hours, the reaction solution was concentrated, and the residue was purified by high performance liquid chromatography (chromatographic column: Waters-SunFire-C18-10μm-19*250mm; mobile phase: water (containing 0.1% formic acid) and acetonitrile, gradient ratio: acetonitrile 3%-33%, flow rate: 25 mL/min) to afford compound 15-. MS m/z (ESI): 421.2 [M+1]. $^1$H NMR (400 MHz, DMSO-d6): δ 9.75 (s, 1H), 8.43 (s, 1H), 8.17 (s, 1H), 7.53 - 7.51 (m, 1H), 7.36 - 7.33 (m, 1H), 6.98 - 6.95 (m, 1H), 4.12 (s, 2H), 3.85 -3.83 (m, 2H), 3.47 - 3.44 (m, 4H), 2.88 (s, 3H), 2.83 - 2.80 (m, 2H), 2.63 - 2.59 (m, 2H), 2.35 (s, 3H).

### Example 16. Preparation of compound 16

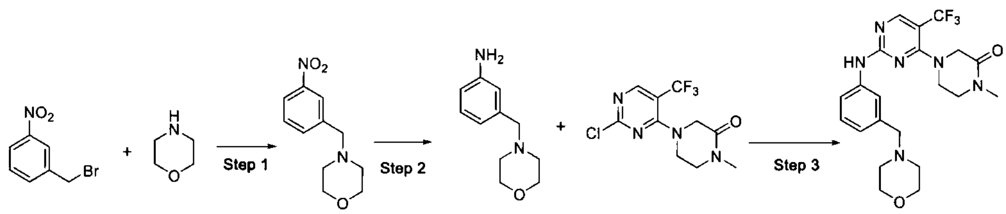


16a 16b 16c 16d 15c Compound 16

Step 1: Compound 16a (1.0 g, 4.63 mmol, Bide) and compound 16b (0.81 mL, 9.26 mmol, Bide) were dissolved in tetrahydrofuran (20 mL). Triethylamine (0.64 mL, 4.63 mmol, Bide) was added, followed by stirring at 60°C for 2 hours. The reaction solution was diluted by adding water (40 mL), followed by extraction with ethyl acetate (20 mL×3). The organic phases were combined, washed with saturated saline, dried over anhydrous sodium sulfate, filtered, and concentrated to afford compound 16c. MS m/z (ESI): 223.2 [M+1].

Step 2: Compound 16c (1.05 g, 4.72 mmol) was dissolved in methanol (40 mL). 10% palladium on carbon (503 mg, 0.47 mmol) was added, followed by stirring at room temperature for 2 hours under a hydrogen atmosphere. The reaction solution was filtered, and the filtrate was concentrated to afford compound 16d. MS m/z (ESI): 193.1 [M+1].

Step 3: Compound 16d (146.81 mg, 0.76 mmol) and compound 15c (150 mg, 0.51 mmol) were dissolved in *N,N*-dimethylformamide (1 mL). 4 M hydrochloric acid (0.38 mL, 1.53 mmol) was added, followed by stirring at 100°C for 1 hour. The reaction solution was purified by high performance liquid chromatography (Waters-2545, chromatographic column: Waters-SunFire-C18-10μm-19*250mm; mobile phase: water (containing 0.1%FA) and acetonitrile, gradient ratio: acetonitrile 7%-37%, flow rate: 25 mL/min) to afford compound 16. MS m/z (ESI): 451.2 [M+1]. $^1$H NMR (400 MHz, DMSO-d6): δ 9.85 (s, 1H), 8.45 (s, 1H), 7.79 (s, 1H),7.50 (d, J = 8.2 Hz, 1H), 7.24 (t, J = 7.8 Hz, 1H), 6.95 (d, J = 7.4 Hz, 1H),4.14 (s, 2H), 3.87 (s, 2H), 3.57 (t, J = 4.6 Hz, 4H), 3.46 (d, J = 6.0 Hz, 4H),2.88 (s, 3H), 2.37 (s, 4H).

### Example 17. Preparation of compound 17

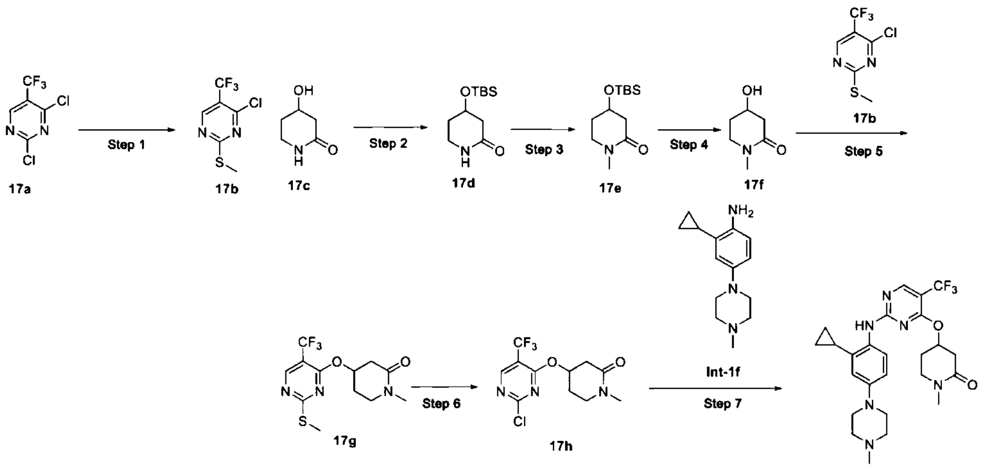


17a 17b 17c 17d 17e 17f 17b 17g 17h Compound 17

Step 1: At 0°C under a nitrogen atmosphere, a zinc chloride solution (265 mL, 265.01 mmol, c=1M/L) was added to a solution of compound 17a (25 g, 115.22 mmol, Bide) in tetrahydrofuran (250 mL). After stirring at 0°C for 90 minutes, sodium methyl mercaptide (10.50 g, 149.79 mmol) was added. The resulting mixture was allowed to warm to room temperature naturally and stirred for 18 hours. The reaction solution was concentrated to afford the crude product. The crude was purified by silica gel chromatography (eluant: ethyl acetate: petroleum ether = 2%-5%) to afford compound 17b. MS m/z (ESI): 228.97 [M+1]. $^1$H NMR (400 MHz, $CDCl_3$): δ 8.67 (s, 1H), 2.61 (s, 3H).

Step 2: 4-(dimethylamino)pyridine (742.80 mg, 6.08 mmol) and chlorodimethyl(2-methylpropan-2-yl)silane (15.8 mL, 91.20 mmol) were added to a solution of compound 17c (7 g, 60.80 mmol, Bide) and imidazole (8278.64 mg, 121.60 mmol) in *N,N*-dimethylformamide (80 mL), respectively, followed by heating to 90 °C and stirring for 2 hours. The mixture was poured into water (100 mL), followed by extraction with ethyl acetate (70 mL x 3). The organic phases were combined, dried over anhydrous sodium sulfate, filtered and concentrated under reduced pressure, to afford compound 17d. MS m/z (ESI): 230.15 [M+1].

Step 3: At 0°C under a nitrogen atmosphere, sodium hydride (1.83 g, 60% purity, 45.77 mmol) was added to a solution of compound 17d (10 g, 30.51 mmol) in *N,N*-dimethylformamide (100 mL). After stirring at 0°C for 10 minutes, iodomethane (2.85 mL, 45.77 mmol) was added. The resulting mixture was allowed to warm to room temperature naturally and stirred for 18 hours. The mixture was poured into water (100 mL), extracted with ethyl acetate (80 mL x 3). The organic phases were combined, dried over anhydrous sodium sulfate, filtered and concentrated under reduced pressure, to afford the crude product. The crude was purified by silica gel chromatography (elution solution: dichloromethane - methanol = 4-6%) to afford compound 17e. MS m/z (ESI): 244.17 [M+1].

Step 4: At 25 °C under a nitrogen atmosphere, tetrabutylammonium fluoride (5.7 mL, 19.47 mmol) was added to a solution of compound 17e (3.16 g, 12.98 mmol) in tetrahydrofuran (30 mL), followed by stirring for 5 hours. The reaction solution was concentrated under reduced pressure to afford the crude product. The crude was purified by silica gel chromatography (elution solution: dichloromethane - methanol = 6-10 %) to afford compound 17f. MS m/z (ESI): 130.08 [M+1].

Step 5: At 0°C under a nitrogen atmosphere, sodium hydride (325.18 mg, 60% purity, 8.13 mmol) was added to a solution of compound 17f (1 g, 5.42 mmol) in *N,N*-dimethylformamide (20 mL). After stirring at 0°C for 10 minutes, compound 17b (2.85 mL, 45.77 mmol) was added. The resulting mixture was allowed to warm to room temperature naturally and stirred for 18 hours. The mixture was poured into water (20 mL), extracted with ethyl acetate (30 mL x 3). The organic phases were combined, dried over anhydrous sodium sulfate, filtered and concentrated under reduced pressure, to afford the crude product. The crude was purified by silica gel chromatography (elution solution: dichloromethane - methanol = 7-9%) to afford compound 17g. MS m/z (ESI): 322.08 [M+1].

Step 6: At 0°C, sulfonyl chloride (1.02 mL, 12.70 mmol) was added to a mixed solution of compound 17g (408 mg, 1.27 mmol) in dichloromethane (5 mL) and acetonitrile (5 mL), followed by stirring at 0°C for 2 hours. The mixture was poured into water (10 mL). The reaction mixture was adjusted to pH = 8 with saturated sodium bicarbonate solution,followed by extraction with dichloromethane (10 mL x 3). The organic phases were combined, dried over anhydrous sodium sulfate, filtered and concentrated under reduced pressure, to afford compound 17h. MS m/z (ESI): 310.05 [M+1].

Step 7: At 25°C, trifluoroacetic acid (292.39 μL, 3.92 mmol) was added to a solution of compound 17h (270 mg,

0.78 mmol) and compound Int-1f (199.69 mg, 0.86 mmol) in isopropanol (5 mL), followed by heating to 40°C and stirring for 18 hours. The reaction solution was concentrated under reduced pressure to afford the crude product. The crude product was purified by preparative HPLC (chromatographic column: Waters-SunFire-C18-10μm-19*250mm; mobile phase: A: 0.1% formic acid/water B: acetonitrile, gradient ratio: acetonitrile 54%:46%, flow rate: 25 mL/min) to afford compound 17. MS m/z (ESI): 505.25 [M+1]. $^1$H NMR (400 MHz, DMSO-$d_6$): δ 9.35 (s, 1H), 8.35 (s, 1H), 7.14 (s, 1H), 6.74 (s, 1H), 6.43 (s, 1H), 5.64-4.98 (m, 1H), 3.25 (d, *J* = 23.0 Hz, 3H), 3.13 (s, 4H),2.81 (s, 3H), 2.55 (s, 4H), 2.38 (d, *J* = 19.5Hz, 1H), 2.30 (s, 3H), 2.17-1.98 (m,2H), 1.91 (t, *J* = 6.3 Hz, 1H), 0.86-0.78 (m, 2H), 0.64-0.57 (m, 2H).

**Example 18. Preparation of compound 18**

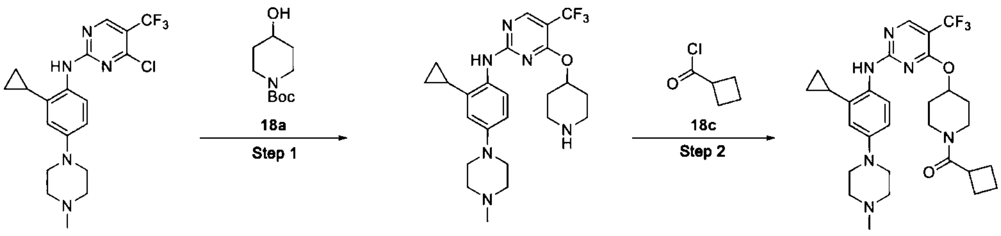


Int-1 18b Compound 18

Step 1: At room temperature, compound Int-1 (600 mg, 1.45 mmol), compound 18a (586 mg, 2.91 mmol), N-methylpyrrolidone (5 mL) and potassium tert-butoxide (327 mg, 2.91 mmol) were added to 20 mL of microwave tube, respectively. The temperature was raised to 180 °C, followed by stirring for 1 hour. After the reaction was completed, the reaction solution was poured into water (10 mL), followed by extraction with ethyl acetate (10 mL x 3). The organic phases were combined, washed with saturated saline (20 mL), dried over anhydrous sodium sulfate, filtered, and concentrated under reduced pressure to afford the crude, which was purified by silica gel chromatography (eluant: MeOH:DCM = 5%-10%) to afford compound 18b. MS m/z (ESI): 477.2 [M+1].

Step 2: At room temperature, N,N-diisopropylethylamine (54 mg, 0.42mmol) was added to a solution of compound 18b (100 mg, 0.21 mmol) in dichloromethane (2 mL). After reducing the temperature to 0°C, compound 18c (32 mg, 0.27 mmol) was added, and the reaction was completed after stirring at 0°C for 3 hours. The crude product was purified by preparative HPLC (chromatographic column: Bonnasil-BR-C18-10um-20*250mm; mobile phase: A: 10 mM ammonium bicarbonate/water; B: acetonitrile, gradient ratio: acetonitrile 75%-95%, flow rate: 20 mL/min) to afford compound 18. MS m/z (ESI): 559.3 [M+1]. $^1$H NMR (400 MHz, DMSO-$d_6$): δ 8.82 (s, 1H), 8.27 (s, 1H), 7.20 (s, 1H), 6.72 (s, 1H), 6.44 - 6.36 (m, 1H), 4.90 (d, *J* = 4.8 Hz, 1H), 3.77 - 3.54 (m, 2H), 3.18 - 3.11 (m, 1H), 3.10 - 3.03 (m, 4H), 2.69 - 2.65 (m, 1H), 2.43 - 2.40 (m, 2H), 2.34 - 2.31 (m, 1H), 2.21 (s, 3H), 2.13 (s, 4H), 2.01 - 1.75 (m, 6H), 1.62 - 1.46 (m, 2H), 1.30 - 1.16 (m, 1H), 0.87 - 0.76 (m, 2H), 0.62 - 0.54 (m, 2H).

**Example 19. Preparation of compound 19**

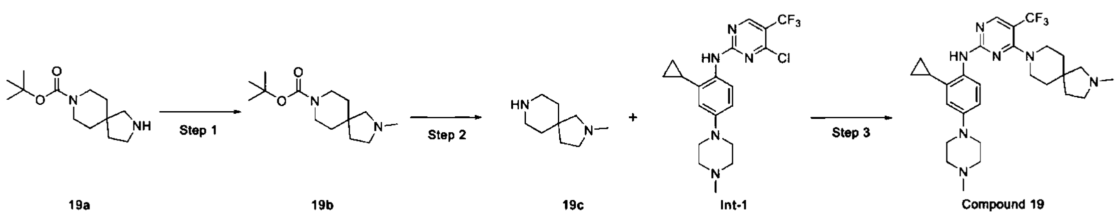


19a 19b 19c Int-1 Compound 19

Step 1: Compound 19a (500 mg, 1.81 mmol, Bide) was dissolved in *N,N*-dimethylformamide (10 mL), and sodium hydride (108.38 mg, 2.71 mmol) was added at 0°C. After stirring for 30 minutes under a nitrogen atmosphere, iodomethane (0.12 mL, 1.99 mmol) was dissolved in N,N-dimethylformamide (1 mL), and then was added dropwise to the reaction solution, which was stirred at room temperature for 18 hours under a nitrogen atmosphere. The reaction solution was diluted by adding water (30 mL), followed by extraction with dichloromethane/methanol = 5:1 (20 mL×3). The organic phases were combined, washed with a saturated saline solution, dried over anhydrous sodium sulfate, filtered, and concentrated to afford compound 19b. MS m/z (ESI): 255.4 [M+1].

Step 2: Compound 19b (460 mg, 1.81 mmol) was dissolved in 1,4-dioxane (6 mL). Hydrochloric acid (6 mL, 24.00

mmol) was added, followed by stirring at room temperature for 1 hour. The reaction solution was concentrated to afford compound 19c. MS m/z (ESI): 155.3 [M+1].

Step 3: Compound 19c (220 mg, 1.43 mmol), compound Int-1 (533.98 mg, 1.30 mmol) were dissolved in *N,N*-dimethylformamide (5 mL). N,N-diisopropylethylamine (1.07 mL, 6.48 mmol) was added, followed by reacting under stirring at 100°C for 1 hour. The reaction solution was purified by high performance liquid chromatography (Gilson_306_1741, chromatographic column: Waters-CORTECS-C18-2.7μm-4.6*30mm; mobile phase: water (containing 0.225% formic acid) and acetonitrile, gradient ratio: acetonitrile 10%-20%, flow rate: 20 mL/min) to afford compound 19. MS m/z (ESI): 530.2 [M+1]. $^1$H NMR (400 MHz, DMSO-d6): δ 8.78 (s, 1H), 8.25 (s, 1H), 7.22 (s, 1H), 6.72 (dd, J= 8.6, 2.8 Hz, 1H), 6.42 (d, J = 2.8 Hz, 1H), 3.46-3.09 (m, 4 H), 3.08 (t, J = 4.8 Hz, 4H), 2.56 (d, J = 6.9 Hz, 2H), 2.46-2.43 (m, 4H), 2.40 (s, 2H), 2.27 (s, 3H), 2.22 (s, 3H), 2.08 (s, 1H), 1.63 (t, J = 6.9Hz, 2H), 1.53 (s, 4H), 0.82 (dd, J = 8.2, 2.2 Hz, 2H), 0.59 (dd, J = 5.6, 2.0 Hz, 2H).

**Example 20. Preparation of compound 20**

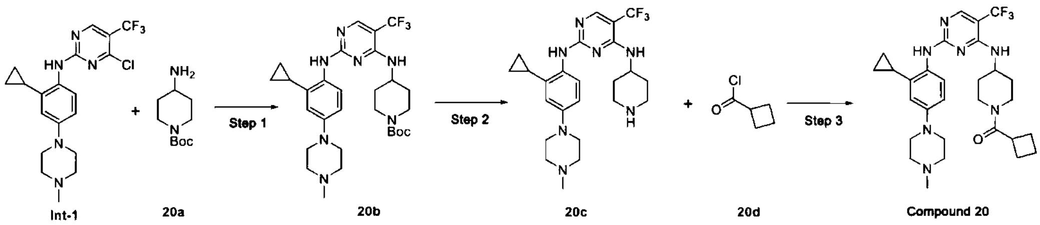


Int-1 20a 20b 20c 20d Compound 20

Step 1: Compound Int-1 (300 mg, 0.73 mmol) and 20a (218.83 mg, 1.09 mmol, Bide) were dissolved in N,N-dimethylformamide (1 mL). *N*,*N*-diisopropylethylamine (0.36 mL, 2.19 mmol) was added, followed by stirring at 100°C for 1 hour. The reaction solution was diluted by adding water (30 mL), followed by extraction with ethyl acetate (20 mL×3). The organic phases were combined, washed with saturated saline, dried over anhydrous sodium sulfate, filtered, and concentrated. The crude was purified by silica gel column chromatography (eluant system: dichloromethane: methanol = 20: 1) to afford compound 20b. MS m/z (ESI): 576.4 [M+1].

Step 2: Compound 20b (430 mg, 0.75 mmol) was dissolved in a solution of hydrochloric acid/1,4-dioxane (8 mL, 4 M), followed by stirring at room temperature for 1 hour. The reaction solution was concentrated to afford compound 20c. MS m/z (ESI): 476.3 [M+1].

Step 3: Compound 20c (360 mg, 0.76 mmol) and *N,N*-diisopropylethylamine (0.63 mL, 3.79 mmol) were dissolved in *N*,*N*-dimethylformamide (6 mL). Compound 20d (62.83 mg, 0.53 mmol) was added at 0°C, followed by stirring at 0°C for 30 minutes. The reaction solution was purified by high performance liquid chromatography (Gilson_306_1741, chromatographic column: Waters-SunFire-C18-10μm-19*250mm; mobile phase: water (containing 0.1% formic acid) and acetonitrile, gradient ratio: acetonitrile 10%-40%, flow rate: 25 mL/min) to afford compound 20. MS m/z (ESI): 558.4 [M+1]. $^1$H NMR (400 MHz, DMSO-d6): δ 8.66 (s, 1H), 8.07 (s, 1H), 7.23 (d, J = 8.4 Hz, 1H), 6.72 (dd, J = 8.8, 2.8Hz, 1H), 6.42 (d, J = 2.8 Hz, 1H), 6.37 (d, J = 7.6 Hz, 1H), 4.35 (d, J = 13.0 Hz, 1H), 3.66 (d, J = 13.6 Hz, 1H), 3.32 (p, J = 8.6 Hz, 4H), 3.07 (t, J = 5.0 Hz, 4H), 2.44 (t, J = 5.0 Hz, 4H), 2.21(s, 3H), 2.16 (td, J = 9.4, 8.8, 4.0 Hz, 2H), 2.11 - 2.03 (m, 2H), 1.96 - 1.85 (m, 2H), 1.78 - 1.68 (m, 3H),1.40 (t, J = 19.8 Hz, 2H), 0.82 (dt, J = 8.6, 3.0 Hz, 2H), 0.59 (td, J = 5.8, 3.8 Hz, 2H).

**Example 21. Preparation of compound 21**

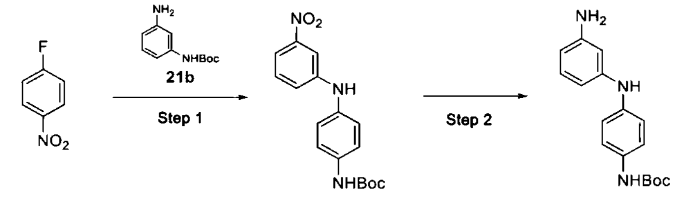


21a 21c 21d

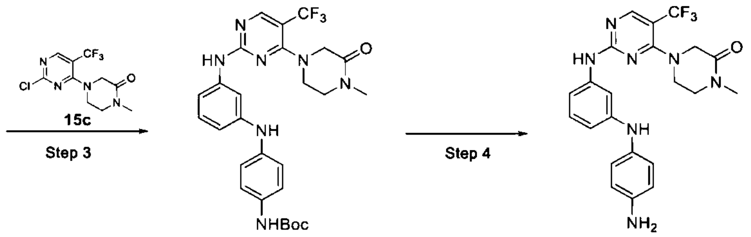


21f Compound 21

Step 1: At room temperature, compound 21b (4.43 g, 21.26 mmol, Bide) was added to a solution of compound 21a (3.00 g, 21.26 mmol, Bide) and triethylamine (8.84 mL, 63.78 mmol) in dimethyl sulfoxide (30 mL), and the reaction was carried out at 160°C for 8 hours. The mixture was poured into water (20 mL), followed by extraction with ethyl acetate (30 mL x2). The organic phases were combined, washed with saturated saline (100 mL), dried over anhydrous sodium sulfate, filtered, and concentrated under reduced pressure to afford the crude. The crude was purified by silica gel chromatography (eluant: EtOAc: PE = 5%-35%) to afford compound 21c. MS m/z (ESI): 330.3 [M+1].

Step 2: At room temperature, palladium on carbon (40 mg, 0.33 mmol) and ammonia water (1.38 mL, 10.02 mmol) were added to a solution of compound 21c (1.10 g, 3.34 mmol) in methanol (15 mL). The reaction solution was stirred at room temperature under a hydrogen balloon for 18 hours after purging with hydrogen three times. The reaction solution was filtered and concentrated under reduced pressure to afford the crude. The crude was purified by silica gel chromatography (eluant: EtOAc: PE= 5%-60%) to afford compound 21d. MS m/z (ESI): 300.2 [M+1].

Step 3: At room temperature, compound 21d (200 mg, 0.67 mmol) was dissolved in dioxane (3 mL). Cesium carbonate (653 mg, 2.00 mmol), compound 15c (197 mg, 0.67 mmol), 4,5-bis(diphenylphosphino)-9,9-dimethylxanthene (77 mg, 0.13 mmol, Bide) and tris(dibezylideneacetone)dipalladium (61 mg, 0.07 mol, Bide) were added to the above reaction solution, respectively, followed by stirring at 100 °C for 18 hours under nitrogen protection. The reaction solution was filtered, and the filtrate was concentrated to afford the crude. The crude was purified by silica gel chromatography (eluant: MeOH: DCM= 0%-10%) to afford compound 21f. MS m/z (ESI): 558.2 [M+1].

Step 4: At room temperature, trifluoroacetic acid (1 mL) was added to a solution of compound 21f (240 mg, 1.21 mmol) in dichloromethane (4 mL), followed by stirring at room temperature for 2 h. The crude product was afforded by concentration under reduced pressure. The crude product was purified by high performance liquid chromatography (chromatographic column: Waters-Xbridge-C18-10μm-19*250mm; mobile phase: A: 10 mM ammonium bicarbonate/-water; B: acetonitrile, gradient ratio: acetonitrile 95%, flow rate: 20 mL/min) to afford compound 21. MS m/z (ESI): 458.2 [M+1]. $^1$H NMR (400 MHz, DMSO-$d_6$): δ 9.64 (s, 1H), 8.40 (s, 1H), 7.72 (s, 1H), 7.48 (d, $J$ = 8.9 Hz, 2H), 7.00 (d, $J$ = 8.9 Hz, 2H), 6.84 (t, $J$ = 7.9 Hz, 1H), 6.30 (s, 1H), 6.20 (d, $J$ = 7.8 Hz, 1H), 6.03 (d, $J$ = 9.1 Hz, 1H), 5.02 (brs, 2H), 4.10 (s, 2H), 3.82 (t, $J$ = 5.2 Hz, 2H), 3.44 (t, $J$ = 5.2 Hz, 2H), 2.87 (s, 3H).

## Example 22. Preparation of compound 22

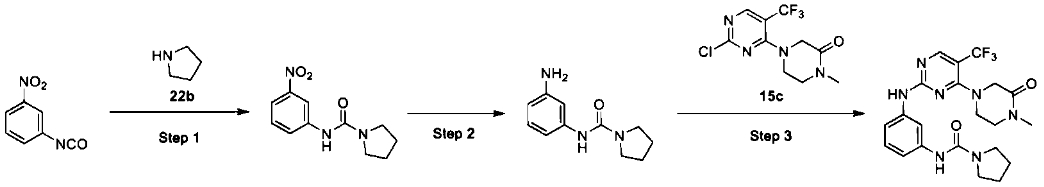


22a 22c 22d Compound 22

Step 1: At room temperature, compound 22a (0.33 mL, 3.05 mmol, Bide) was dissolved in 3 mL tetrahydrofuran.

Then, compound 22b (0.30 mL, 3.66 mmol, Bide) was added, followed by reacting under stirring for 1 hour while maintaining the temperature. After the reaction was completed, the reaction solution was concentrated and dried to afford compound 22c. MS m/z (ESI): 236.1 [M+1].

Step 2: Under nitrogen protection, compound 22c (700 mg, 2.68 mmol, purity: 90%) was dissolved in 50% ethanol (8 mL) solution. Then, iron powder (598.18 mg, 10.71 mmol, Bide) and ammonium chloride (859.51 mg, 16.07 mmol, Bide) were added, followed by reacting under stirring at 80°C for 3 hours. After the reaction was completed, the reaction solution was filtered, washed with dichloromethane (30 mL), and the filtrate was concentrated and dried. The residue was purified by silica gel column chromatography with methanol : dichloromethane = 2% - 3% gradient to afford compound 22d. MS m/z (ESI): 206.1 [M+1].

Step 3: Compound 22d (100 mg, 0.49 mmol) was dissolved in N,N-dimethylformamide (2 mL). Compound 15c (143.55 mg, 0.49 mmol) and a dioxane solution of hydrochloric acid (243.59 μL, 0.97 mmol, 4 M) were added, followed by reacting under stirring at 100°C for 3 hours. After being concentrated under reduced pressure, the reaction solution was purified by high performance liquid chromatography (chromatographic column: Waters-Xbridge-C18-10μm-19*250mm; mobile phase: water (containing 10 mmol $NH_4HCO_3$) and acetonitrile, gradient ratio: acetonitrile 27%-57%, flow rate: 25 mL/min) to afford compound 22. MS m/z (ESI): 464.2 [M+1]. $^1$H NMR (400 MHz, DMSO-d6): δ 9.82 (s, 1H), 8.44 (s, 1H), 8.12 (s, 1H), 7.92 (s, 1H), 7.21 - 7.11 (m, 3H), 4.13 (s, 2H), 3.87 (t, J = 5.4 Hz, 2H), 3.45 - 3.36 (m, 6H), 2.86 (s, 3H), 1.89 - 1.81 (m, 4H).

**Example 23. Preparation of compound 23**

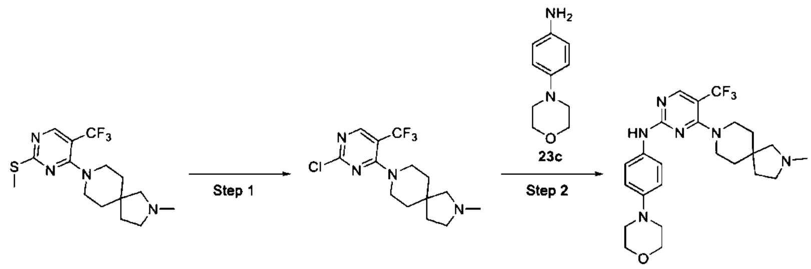


23a 23b Compound 23

Step 1: At 0°C, sulfonyl chloride (2332.84 μL, 28.87 mmol) was added to a solution of compound 23a (1000 mg, 2.89 mmol) in acetonitrile (5 mL) and dichloromethane (5 mL), followed by stirring at 0°C for 3 hours. The mixture was concentrated under reduced pressure to afford the crude product. The crude product was separated and purified by reversed-phase HPLC (mobile phase: A: 0.1% formic acid/water; B: acetonitrile, gradient ratio: acetonitrile 30-50%, flow rate: 25 mL/min) to afford compound 23b. MS m/z (ESI): 335.1 [M+1].

Step 2: Compound 23b (160 mg, 0.48 mmol, Bide) and 23c (170 mg, 0.96 mmol, Bide) were dissolved in N,N-dimethylformamide (2 mL). Then, hydrochloric acid (238.97 μL, 0.96 mmol, 4 M) was added, followed by stirring at 100°C for 1 hour. After the reaction was completed, pH was adjusted 8 - 9 using an ammonia methanol solution. Then, the mixture was purified by high performance liquid chromatography (chromatographic column: Waters-Xbridge-C18-10μm-19*250mm; mobile phase: water (containing 10 mmol/L ammonium bicarbonate) and acetonitrile, gradient ratio: acetonitrile 29%-59%, flow rate: 25 mL/min) to afford compound 23. MS m/z (ESI): 477.3 [M+1]. $^1$H NMR (400 MHz, DMSO-$d_6$): δ 9.49 (s, 1H), 8.32 (s, 1H), 7.61 - 7.49 (m, 2H), 6.89 (d, *J* = 9.2 Hz, 2H), 3.76 - 3.70 (m, 4H), 3.58 - 3.44 (m, 4H), 3.09 - 3.00 (m, 4H), 2.46 (t, *J* = 5.6 Hz, 2H), 2.33 (s, 2H), 2.21 (s, 3H), 1.66 - 1.54 (m, 6H).

**Example 24. Preparation of compound 24**

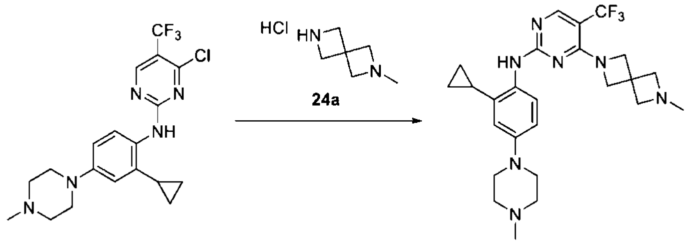


Int-1 Compound 24

Compound Int-1 (100 mg, 0.24 mmol) was dissolved in *N,N*- dimethylformamide (1 mL). Then, compound 24a (54.13 mg, 0.36 mmol, Bide) and N,N-diisopropylethylamine (201.17 μL, 1.21 mmol) were added, respectively. The reaction solution was stirred at 80°C for 1 hour. After the reaction was completed, the mixed solution was purified by high performance liquid chromatography (chromatographic column: Waters-SunFire-C18-10μm-19*250mm; mobile phase: water (containing 0.1% formic acid) and acetonitrile, gradient ratio: acetonitrile 2%-32%, flow rate: 25 mL/min) to afford compound 24. MS m/z (ESI): 488.4 [M+1]. $^{1}H$ NMR (400 MHz, DMSO-$d_6$): δ 8.63 (s, 1H), 8.30 (d, *J* = 3.6 Hz, 1H), 8.13 (s, 1H), 6.73-6.70 (m, 1H), 6.43 (d, *J* = 2.8 Hz, 1H), 4.15 (s, 4H), 3.33 - 3.27 (m, 4H), 3.07 (t, *J* = 5.2 Hz, 4H), 2.43 (t, *J* = 5.2 Hz, 4H), 2.23 - 2.16 (m, 6H), 1.98 - 1.84 (m, 1H), 0.85 - 0.78 (m, 2H), 0.60 - 0.55 (m, 2H).

**Example 25. Preparation of compound 25**

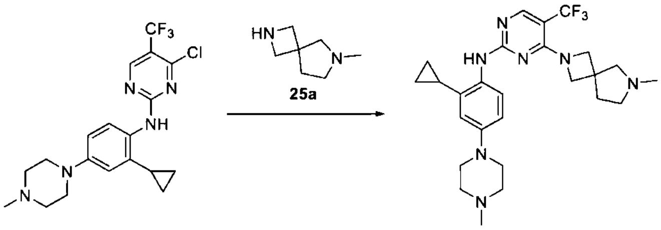


Int-1 Compound 25

Compound Int-1 (100 mg, 0.24 mmol) was dissolved in *N,N*-dimethylformamide (1 mL). Then, compound 25a (36.77 μL, 0.29 mmol, Bide) and *N,N*-diisopropylethylamine (80.47 μL, 0.49 mmol) were added, respectively, followed by stirring at 80°C for 2 hours. After the reaction was completed, the reaction solution was purified by high performance liquid chromatography (chromatographic column: Waters-Xbridge-C18-10μm-19*250mm; mobile phase: water (containing 10 mmol/L ammonium bicarbonate) and acetonitrile, gradient ratio: acetonitrile 44%-74%, flow rate: 25 mL/min) to afford compound 25. MS m/z (ESI): 502.3 [M+1]. $^{1}H$ NMR (400 MHz, DMSO-$d_6$): δ 8.61 (s, 1H), 8.13 (s, 1H), 7.29 (s, 1H), 6.73-6.70 (m, 1H), 6.43 (d, *J* = 2.8 Hz, 1H), 4.10 - 3.90 (m, 4H), 3.09 -3.02 (t, *J* = 5.2 Hz, 4H), 2.62 (s, 2H), 2.45 - 2.37 (m, 6H), 2.23 - 2.17 (m, 6H), 2.07 - 2.00 (m, 2H), 1.95 - 1.88 (m, 1H), 0.86 - 0.77 (m, 2H), 0.61 - 0.53 (m, 2H).

**Example 26. Preparation of compound 26**

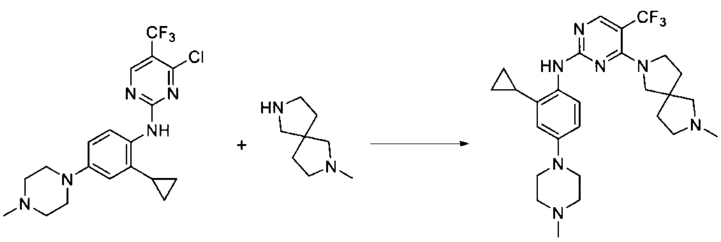

Int-1 26a Compound 26

Compound Int-1 (200 mg, 0.49 mmol), compound 26a (74.91 mg, 0.53 mmol, Bide) were dissolved in *N,N*-dimethylformamide (1 mL). *N,N*-diisopropylethylamine (0.24 mL, 1.46 mmol) was added, followed by reacting under

stirring at 100°C for 1 hour. The reaction solution was purified by high performance liquid chromatography (Waters-2545, chromatographic column: Waters-Xbridge-C18-10μm-19*250mm; mobile phase: water (containing 10 mM ammonium bicarbonate) and acetonitrile, gradient ratio: acetonitrile 49%-79%, flow rate: 25 mL/min) to afford compound 26. MS m/z (ESI): 516.4 [M+1]. $^1$H NMR (400 MHz, DMSO-d6): δ 8.58 (s, 1H), 8.20 (s, 1H), 7.29 (s, 1H), 6.72 (dd, J = 8.8, 2.8 Hz, 1H), 6.43 (d, J = 2.8 Hz, 1H), 3.59 - 3.35 (m, 4H), 3.07 (t, J = 5.0 Hz, 4H), 2.55 (dd, J = 8.4, 6.0 Hz, 2H), 2.46 - 2.38 (m, 6H), 2.21 (s, 6H), 1.88 (ddt, J = 26.4, 13.8, 6.4 Hz, 3H), 1.75 - 1.64 (m, 2H), 0.86 - 0.79 (m, 2H), 0.62 - 0.56 (m, 2H).

**Example 27. Preparation of compound 27**

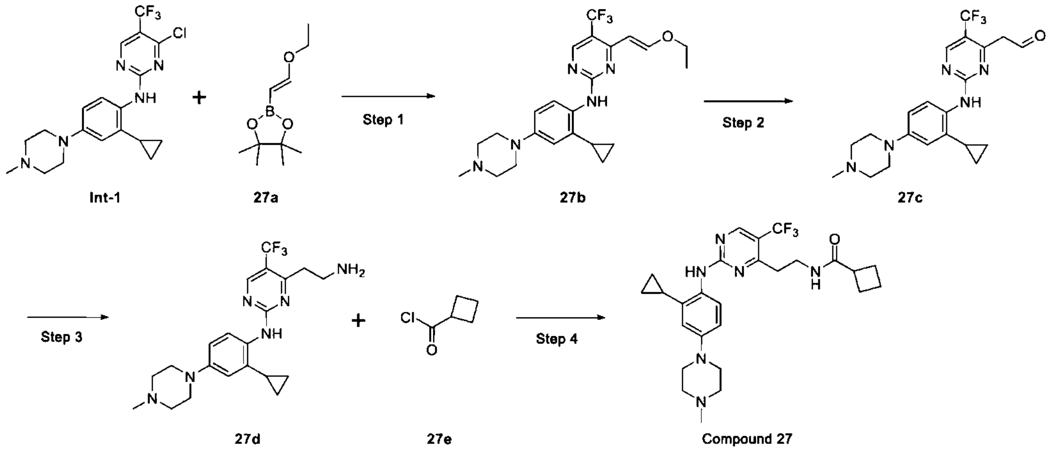

Int-1 27a 27b 27c
27d 27e Compound 27

Step 1: Compound Int-1 (2.00 g, 4.86 mmol) and 27a (2.05 mL, 9.71 mmol, Bide) were dissolved in 1,4-dioxane (20 mL) and water (4 mL). [1,1'-bis (diphenylphosphino) ferrocene] dichloropalladium (0.36 g, 0.49 mmol) and cesium carbonate (4.75 g, 14.57 mmol) were added, respectively. The reaction solution was stirred at 100 °C for 18 hours under a nitrogen atmosphere. The reaction solution was diluted by adding water (50 mL), followed by extraction with ethyl acetate (40 mL×3). The organic phases were combined, dried over anhydrous sodium sulfate, filtered, and concentrated to dryness. The residue was purified by silica gel column chromatography (eluant system: dichloromethane: methanol = 50:1 to 20:1) to afford compound 27b. MS m/z (ESI): 448.4 [M+1].

Step 2: Compound 27b (2.06 g, 4.60 mmol) was dissolved in tetrahydrofuran (20 mL). Hydrochloric acid (40 mL, 1 M) was added, followed by stirring at room temperature for 18 hours. A saturated sodium bicarbonate solution was added to the reaction solution to adjust pH to 8, followed by extracting with ethyl acetate (50 mL×3). The organic phases were combined, dried over anhydrous sodium sulfate, filtered, and concentrated to dryness. The residue was purified by silica gel column chromatography (eluant system: dichloromethane: methanol = 20: 1 to 10: 1) to afford compound 27c. MS m/z (ESI): 420.4 [M+1].

Step 3: Compound 27c (330 mg, 0.79 mmol) and ammonium acetate (485.14 mg, 6.29 mmol) were dissolved in acetic acid (6 mL). Sodium cyanoborohydride (98.88 mg, 1.57 mmol) was added, followed by reacting under stirring at room temperature for 1 hour. The reaction solution was purified by reversed-phase silica gel column chromatography (eluant system: 30% acetonitrile and water) to afford compound 27d. MS m/z (ESI): 421.4 [M+1].

Step 4: Compound 27d (60 mg, 0.14 mmol) and N,N-diisopropylethylamine (0.12 mL, 0.71 mmol) were dissolved in N,N-dimethylformamide (1 mL). 27e (15.23 mg, 0.13 mmol, Bide) was added at 0°C, followed by stirring for 30 minutes. The reaction solution was purified by high performance liquid chromatography (Gilson_306_1741, chromatographic column: Waters-Xbridge-C18-10μm-19*250mm; mobile phase: water (containing 10 mM $NH_4HCO_3$) and acetonitrile, gradient ratio: acetonitrile 38%-68%, flow rate: 25 mL/min) to afford compound 27 (3.09 mg). MS m/z (ESI): 503.3 [M+1]. $^1$H NMR (400 MHz, DMSO-d6): δ 9.36 (s, 1H), 8.48 (s, 1H), 7.60 (s, 1H), 7.15 (s, 1H), 6.75-6.72 (m, 1H), 6.44 (d, J = 2.8 Hz, 1H), 3.33-3.20 (m, 2H), 3.10 (t, J = 4.8 Hz, 4H), 2.90-2.78 (m, 3H), 2.44 (t, J = 5.0 Hz, 4H), 2.21 (s, 3H), 2.06 (d, J = 9.8 Hz, 2H), 1.98 - 1.83 (m, 4H), 1.72 (s, 1H), 0.82 - 0.77 (m,2H), 0.60 (dd, J = 5.2, 1.8 Hz, 2H).

**Example 28. Preparation of compound 28**

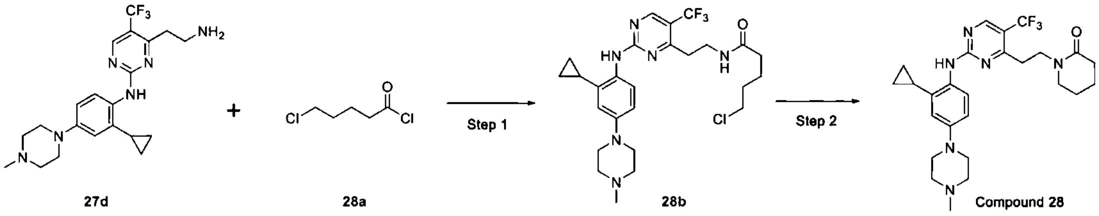


27d 28a 28b Compound 28

Step 1: Compound 27d (165 mg, 0.39 mmol) and triethylamine (109.09 μL, 0.78 mmol) were dissolved in dichloromethane (3 mL). Compound 28a (50.69 μL, 0.39 mmol, Bide) was added, followed by stirring at room temperature for 1 hour. The reaction solution was concentrated to dryness to afford compound 28b. MS m/z (ESI): 539.4 [M+1].

Step 2: Compound 28b (60 mg, 0.14 mmol) was dissolved in N,N-dimethylformamide (3 mL). Potassium tert-butoxide (131.77 mg, 1.17 mmol) was added, followed by stirring at room temperature for 3 hours. The reaction solution was purified by high performance liquid chromatography (Gilson_306_1741, chromatographic column: Waters-Xbridge-C18-10 μm-19*250 mm; mobile phase: water (containing 10 mM $NH_4HCO_3$) and acetonitrile, gradient ratio: acetonitrile 34%-64%, flow rate: 25 mL/min) to afford compound 28. MS m/z (ESI): 503.4 [M+1]. $^1$H NMR (400 MHz, DMSO-d6): δ 9.39 (s, 1H), 8.48 (s, 1H), 7.12 (d, J = 8.4Hz, 1H), 6.74 (d, J = 8.6 Hz, 1H), 6.49 - 6.40 (m, 1H), 3.58 (s, 2H), 3.09 (d, J = 5.4 Hz, 6H), 2.87 (s, 2H), 2.44 (s, 4H), 2.22 (s, 3H), 2.18 - 2.09 (m, 2H),1.90 (s, 1H), 1.62 (s, 4H), 0.79 (d, J = 8.0 Hz, 2H), 0.60 (d, J = 5.0 Hz, 2H).

**Example 29. Preparation of compound 29**

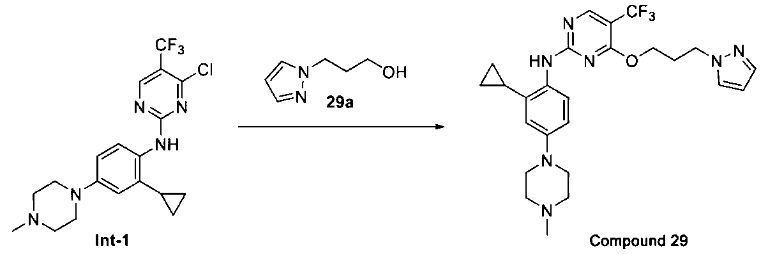


Int-1 Compound 29

At 0°C, sodium hydride (38.85 mg, 0.97 mmol, 60% purity) was added to a solution of compound 29a (91.90 mg, 0.73 mmol, Bide) in tetrahydrofuran (5 mL). After stirring for 10 minutes, compound Int-1 (200 mg, 0.49 mmol) was added. The resulting mixture was heated to 60°C, and stirred for 18 hours. The mixture was poured into water (10 mL), followed by extraction with ethyl acetate (10 mL x3). The organic phases were combined and washed with saturated saline (10 mL), dried over anhydrous sodium sulfate, filtered, and concentrated under reduced pressure, to afford the crude product. The crude product was purified by preparative HPLC (chromatographic column: Waters-Xbridge-C18-10μm-19*250mm; mobile phase: A: 10 mM ammonium bicarbonate/water; B: acetonitrile, gradient ratio: acetonitrile 95%, flow rate: 25 mL/min) to afford compound 29. MS m/z (ESI): 502.3 [M+1]. $^1$H NMR(400 MHz, DMSO-$d_6$): δ 9.29 (s, 1H), 8.35 (s, 1H), 7.63 (s, 1H), 7.43 (s, 1H), 7.10 (d, *J* = 8.5 Hz, 1H), 6.71 (dd, *J* = 8.7, 2.7 Hz, 1H), 6.41 (d, *J* = 2.8 Hz, 1H), 6.22 (s, 1H), 4.18 (s, 4H),3.08 (t, *J* = 5.0 Hz, 4H), 2.43 (t, *J* = 5.0 Hz, 4H), 2.21 (s, 5H), 1.88 (t, *J* = 5.2 Hz, 1H), 0.82-0.74(m, 2H), 0.58 (dt, *J* = 5.4, 3.0 Hz, 2H).

**Example 30. Preparation of compound 30**

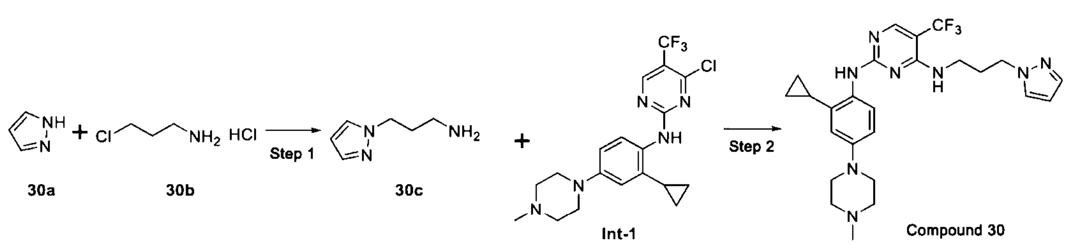


30a 30b 30c Int-1 Compound 30

Step 1: Compound 30a (2.00 g, 29.38 mmol, Bide) and tetrabutylammonium sulfate (0.68 mL, 1.18 mmol, Bide) were dissolved in acetonitrile (60 mL). Sodium hydroxide (4.47 g, 111.64 mmol) was added, followed by stirring at room temperature for 30 minutes. Compound 30b (4.58 g, 35.26 mmol, Bide) was added to the reaction solution, followed by stirring at room temperature for 3 days. The reaction solution was filtered, the filter cake was washed with acetonitrile (20 mL×3), and the filtrate was concentrated to dryness to afford compound 30c. MS m/z (ESI): 126.2 [M+1].

Step 2: Compound 30c (72.94 mg, 0.58 mmol) and Int-1 (200 mg, 0.49 mmol) were dissolved in *N*,*N*-dimethylformamide (2 mL). *N*,*N*-diisopropylethylamine (0.24 mL, 1.46 mmol) was added, followed by stirring at room temperature for 1 hour. The reaction solution was purified by high performance liquid chromatography (Gilson_306_1741, chromatographic column: Bonnasil-HS-AK-C18-10um-20*250mm; mobile phase: water (containing 10 mmol $NaHCO_3$) and acetonitrile, gradient ratio: acetonitrile 50%-80%, flow rate: 20 mL/min) to afford compound 30. MS m/z (ESI): 501.4 [M+1]. $^1$H NMR (400 MHz, DMSO-d6): δ 8.58 (s, 1H), 8.06 (s, 1H), 7.61 (s, 1H), 7.40 (d, J = 1.8 Hz, 1H), 7.24 (d, J = 8.8 Hz, 1H), 6.97-6.96 (m, 1H), 6.72-6.69 (m, 1H), 6.43 (d, J = 2.8 Hz, 1H), 6.20-6.19 (m, 1H), 3.97 (s, 2H), 3.26 (s, 2H), 3.06 (t, J = 5.0 Hz, 4H), 2.42 (t, J = 5.0 Hz, 4H), 2.20 (s, 3H), 2.02 - 1.86 (m, 3H), 0.81 (dd, J = 8.4, 2.0 Hz, 2H), 0.57 (dd, J = 5.4, 1.8 Hz, 2H).

### Example 31. Preparation of compound 31

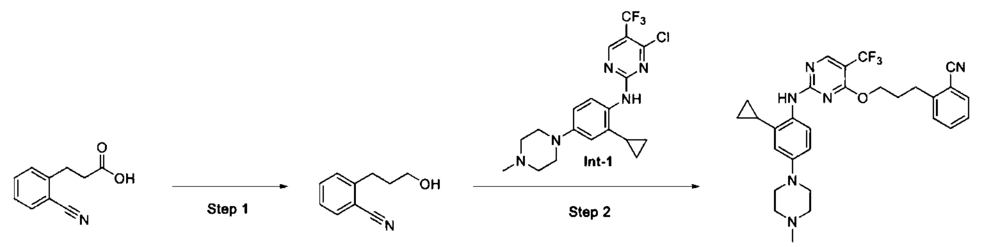


31a 31b Compound 31

Step 1: At 0°C under nitrogen protection, oxalyl chloride (489.51 μL, 5.71 mmol, Bide) was added to a solution of compound 31a (400 mg, 2.28 mmol) in dichloromethane (4 mL), followed by stirring at 25°C for 1h. The reaction solution was concentrated under reduced pressure. The solid was dissolved in tetrahydrofuran (4 mL), and sodium borohydride (863.7 mg, 22.83 mmol) was added. The mixture was stirred at 20°C for 16 h. The mixture was poured into water (20 mL), followed by extraction with ethyl acetate (30 mL x 3). The organic phases were combined, washed with brine (10 mL), dried over anhydrous sodium sulfate, filtered, and concentrated to afford compound 31b. MS m/z (ESI): 162.1 [M+1].

Step 2: At 0°C, under nitrogen protection, compound 31b (70 mg, 0.43 mmol) was added to tetrahydrofuran (2 mL) solution, and then sodium hydride (34.74 mg, 0.87 mmol, 60%) was added. The reaction was carried out at an condition of 0°C for 0.5 h. Then, compound Int-1 (178.85 mg, 0.43 mmol) was added, followed by stirring at 60°C for 16 h. The mixture was poured into water (20 mL), followed by extraction with ethyl acetate (10 mL × 3). The organic phases were combined, washed with brine (10 mL), dried over anhydrous sodium sulfate, filtered, and concentrated to afford the crude. The crude was purified by high performance liquid chromatography (chromatographic column: Waters-Xbridge-C18-10μm-19*250mm; mobile phase: A: 10 mM ammonium bicarbonate/water; B: acetonitrile, gradient ratio: acetonitrile 95%, flow rate: 20 mL/min) to afford compound 31. MS m/z (ESI): 537.4 [M+1]. $^1$H NMR (400 MHz, DMSO-$d_6$): δ 9.27 (s, 1H), 8.42 - 8.28 (m, 1H), 7.78 (d, *J*= 7.6 Hz, 1H), 7.63 (t, *J* = 7.6 Hz, 1H), 7.41 (t, *J* = 7.4 Hz, 2H), 7.12 (d, *J* = 8.2 Hz, 1H), 6.69 (d, *J* = 8.6 Hz, 1H), 6.42 (d, *J* = 2.6 Hz, 1H), 4.31 (s, 2H), 3.12 - 3.05 (m, 4H), 2.95 - 2.80 (m, 2H), 2.46 - 2.40 (m, 4H), 2.21 (s, 3H), 2.12 - 1.95 (m, 2H), 1.89 (ddd, *J* = 13.6, 8.4, 5.4 Hz, 1H), 0.82 - 0.76 (m, 2H), 0.58 (q, *J* = 5.8 Hz, 2H).

### Example 32. Preparation of compound 32

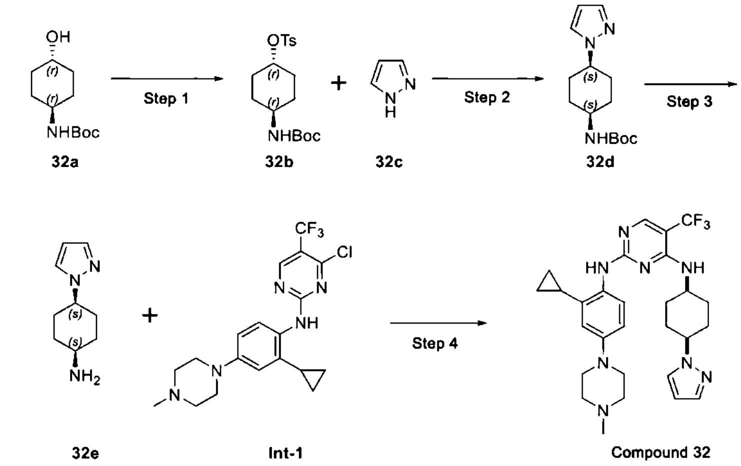


Step 1: Compound 32a (3.00 g, 13.93 mmol, Bide) and p-toluenesulfonyl chloride (3.19 mL, 16.72 mmol) were dissolved in dichloromethane (80 mL). 4-dimethylaminopyridine (0.85 g, 6.97 mmol, Bide) and triethylamine (3.86 mL, 27.87 mmol) were added, respectively, followed by stirring at room temperature for 18 hours. The reaction solution was concentrated, and the crude was purified by silica gel column chromatography (eluant system: dichloromethane: methanol = 20:1 to 5:1) to afford compound 32b. MS m/z (ESI): 370.5 [M+1].

Step 2: Compound 32c (245.68 mg, 3.61 mmol, Bide) and compound 32b (2.0 g, 5.41 mmol) were dissolved in *N*, *N*-dimethylformamide (20 mL). Tetrabutylammonium bromide (116.34 mg, 0.36 mmol) and cesium carbonate (2.35 g, 7.22 mmol) were added, respectively, followed by stirring at 80°C for 18 hours. The reaction solution was diluted by adding water (40 mL), followed by extraction with ethyl acetate (30 mL×3). The organic phases were combined, dried over anhydrous sodium sulfate, filtered, and concentrated to dryness. The residue was purified by silica gel column chromatography (eluant system: petroleum ether : tetrahydrofuran = 10:1 to 3:1) to afford compound 32d. MS m/z (ESI): 266.2 [M+1].

Step 3: Compound 32d (200 mg, 0.75 mmol) was dissolved in hydrochloride-ethyl acetate (4 mL, 4 M) solution, followed by stirring at room temperature for 1 hour. The reaction solution was concentrated to dryness to afford compound 32e. MS m/z (ESI): 166.0 [M+1].

Step 4: Compound 32e (124 mg, 0.75 mmol) and Int-1 (206.05 mg, 0.50 mmol) were dissolved in *N*,*N*-dimethylformamide (2 mL). N,N-diisopropylethylamine (0.41 mL, 2.50 mmol) was added, followed by stirring at 100°C for 1 hour. The reaction solution was purified by high performance liquid chromatography (Gilson_306_1741, chromatographic column: Waters-Xbridge-C18-10μm-19*250mm; mobile phase: water (containing 10 mmol $NaHCO_3$) and acetonitrile, gradient ratio: acetonitrile 37%-67%, flow rate: 25 mL/min) to afford compound 32. MS m/z (ESI): 541.4 [M+1]. $^1$H NMR (400 MHz, DMSO-d6): δ 8.63 (s, 1H), 8.08 (s, 1H), 7.80 (d, J = 2.2 Hz, 1H), 7.45 (d, J = 1.8 Hz, 1H), 7.25 (s, 1H), 6.74-6.71 (m, 1H), 6.44 (d, J = 2.8 Hz, 1H), 6.26-6.25 (m, 1H), 5.99 (d, J =7.0 Hz, 1H), 4.26 (s, 1H), 4.09 (s, 1H), 3.08 (t, J = 5.0 Hz, 4H), 2.44 (t, J = 5.0 Hz, 4H), 2.21 (s, 3H), 2.20 -2.10 (m, 2H), 1.97 - 1.91 (m, 1H), 1.83 - 1.70 (m, 4H), 1.66 (s, 2H), 0.86 - 0.80 (m, 2H), 0.62 - 0.57 (m, 2H).

## Example 33. Preparation of compound 33

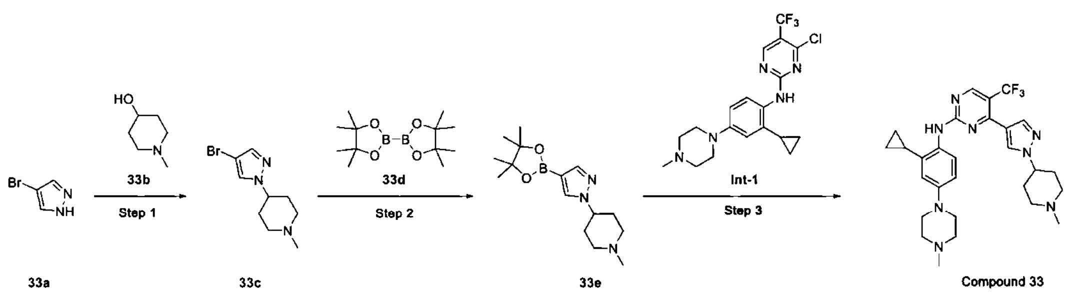


Step 1: Under nitrogen protection, compound 33a (1.5 g, 10.21 mmol, Bide) was dissolved in tetrahydrofuran (20 mL). Then, under an ice bath, compound 33b (0.65 g, 9.66 mmol, Bide), triphenylphosphine (2.68 g, 10.21 mmol), and diisopropyl azodicarboxylate (2.02 mL, 10.21 mmol) were added, respectively. The reaction solution was slowly warmed to room temperature and then stirred continuously for 18 hours. After the reaction was completed, the reaction solution was

concentrated under reduced pressure to dryness. The residue was purified by silica gel column chromatography (methanol: dichloromethane = 2% - 5%) to afford compound 33c. MS m/z (ESI): 244.0/246.0 [M+1].

Step 2: Under nitrogen protection, compound 33c (650 mg, 2.66 mmol) was dissolved in 1,4-dioxane (10 mL). Then, compound 33d (1352.18 mg, 5.32 mmol, Bide), potassium acetate (522.58 mg, 5.32 mmol), and DPPFdichloropalladium (38.96 mg, 0.05 mmol) were added, respectively. The resulting mixture was purged with nitrogen 3 times, and stirred at 90°C for 18 hours. After the reaction was completed, the reaction solution was filtered, washed with ethyl acetate (30 mL). The filtrate was concentrated to dryness, and the residue was purified by silica gel column chromatography (methanol: dichloromethane =10% - 17%) gradient to afford compound 33e. MS m/z (ESI): 292.2 [M+1].

Step 3: Under nitrogen protection, compound Int-1 (141.44 mg, 0.34 mmol) was dissolved in a mixed solvent of 1,4 -dioxane (3 mL) and water (0.5 mL). Then, compound 33e (5100 mg, 0.34 mmol), potassium carbonate (335.67 mg, 1.03 mmol), and DPPFdichloropalladium (25.13 mg, 0.03 mmol) were added, respectively. The reaction solution was purged with nitrogen 3 times, and stirred at 100 °C for 2 hours. After the reaction was completed, the reaction solution was poured into water (15 mL), followed by extraction with ethyl acetate (30 mL × 3). The organic phases were combined, dried over anhydrous sodium sulfate, filtered, and concentrated. The residue was purified by high performance liquid chromatography (chromatographic column: Waters-CORTECS-C18-2.7μm-4.6*30mm; mobile phase: water (containing 10 mmol $NH_4HCO_3$) and acetonitrile, gradient ratio: acetonitrile 40%-70%, flow rate: 25 mL/min) to afford compound 33. MS m/z (ESI): 541.3 [M+1]. $^1H$ NMR (400 MHz, DMSO-$d_6$): δ 9.28 (s, 1H), 8.56 (s, 1H), 8.15 (s, 1H), 7.88 (s, 1H), 7.18 (d, $J$ = 7.6 Hz, 1H), 6.77 (d, $J$ = 2.4 Hz, 1H), 6.46 (d, $J$ = 2.4 Hz, 1H), 4.28-4.22 (m, 1H), 3.13 - 3.00 (m, 4H), 2.84 (d, $J$ = 10.8 Hz, 2H), 2.47 - 2.41 (m, 4H), 2.21 (d, $J$ = 8.4 Hz, 6H), 2.07 - 1.87 (m, 7H), 0.82-0.78 (m, 2H), 0.62-0.59 (m, 2H).

**Example 34. Preparation of compound 34**

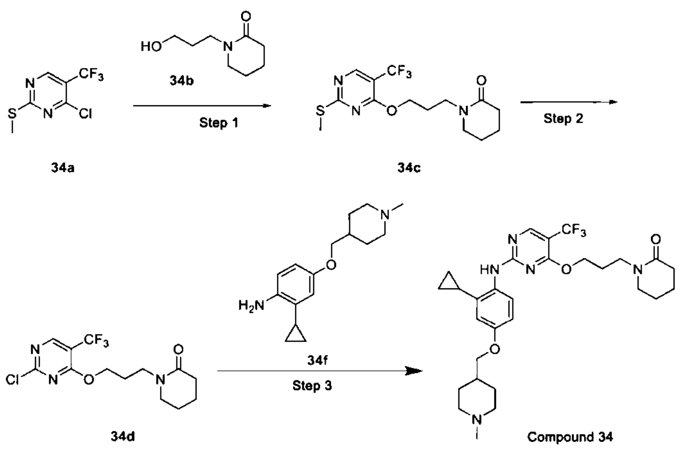


Step 1: At 0°C, sodium hydride (95.9 mg, 2.40 mmol, 60% purity) was added to a solution of compound 34b (377 mg, 2.40 mmol, Bide) in tetrahydrofuran (8 mL). After stirring for 30 minutes, compound 34a (457 mg, 2.00 mmol) was added. The resulting mixture was heated to 60°C and stirred for 18 hours. The mixture was poured into water (20 mL), followed by extraction with ethyl acetate (10 mL x 3). The organic phases were combined, washed with saturated saline (20 mL), dried over anhydrous sodium sulfate, filtered, and concentrated under reduced pressure to afford the crude compound, which was purified by silica gel chromatography (eluant: petroleum ether: ethyl acetate = 5:1-1:2) to afford compound 34c. MS m/z (ESI): 350.1 [M+1].

Step 2: At 0°C, sulfonyl chloride (888 mg, 6.58 mmol) was added to a solution of compound 34c (230 mg, 0.66 mmol) in acetonitrile (2 mL) and dichloromethane (2 mL). The reaction solution was stirred at 0°C for 2 hours. The mixture was poured into water (10 mL), adjusted to pH=7 using a saturated sodium bicarbonate solution, followed by extraction with dichloromethane (5 mL x 3). The organic phases were combined, washed with saturated saline (10 mL), dried over anhydrous sodium sulfate, filtered, and concentrated under reduced pressure to afford compound 34d. MS m/z (ESI): 338.2 [M+1].

Step 3: Compound 34d (140 mg, 0.41 mmol) was dissolved in isopropanol (2 mL). Then, compound 34f (107.94 mg, 0.41 mmol) and trifluoroacetic acid (157.46 μL, 2.07 mmol) were added, respectively, followed by stirring at 40 °C for 18 hours. After the reaction was completed, the reaction solution was concentrated under reduced pressure to dryness. The residue was purified by high performance liquid chromatography (chromatographic column: Waters-CORTECS-C18-2.7μm-4.6*30mm; mobile phase: water (containing 10 mmol $NH_4HCO_3$) and acetonitrile, gradient ratio: acetonitrile 46%-76%, flow rate: 25 mL/min) to afford compound 34. MS m/z (ESI): 562.3 [M+1]. $^1H$ NMR (400 MHz, DMSO-$d_6$): δ 9.37

(s, 1H), 8.35 (s, 1H), 7.17 (d, *J* = 8.6 Hz, 1H), 6.73-6.70 (m, 1H), 6.41 (d, *J* = 2.8 Hz, 1H), 4.27 (s, 2H), 3.78 (d, *J* = 6.3 Hz, 2H), 3.31 (s, 1H), 3.17 (s, 2H), 2.76 (dt, *J* = 11.6, 3.2 Hz, 2H), 2.19 - 2.10 (m, 5H), 2.01 - 1.56 (m, 13H), 1.28 (dt, *J* = 12.0, 6.0 Hz, 2H), 0.87 - 0.79 (m, 2H), 0.64 - 0.56 (m, 2H).

**Example 35. Preparation of compound 35**

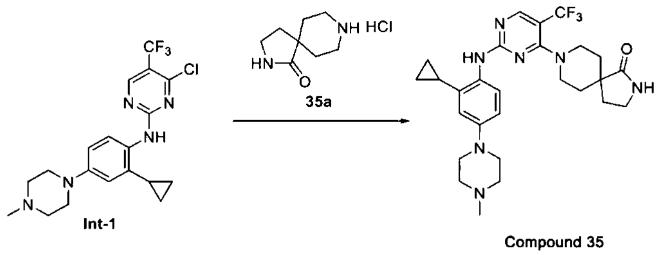

Int-1 35a Compound 35

At 25°C, triethylamine (252.42 μL, 1.82 mmol) was added to a solution of compound Int-1 (150 mg, 0.36 mmol) and compound 35a (73.01 mg, 0.47 mmol, Bide) in *N,N*-dimethylformamide (3 mL). The resulting mixture was heated to 100 °C and stirred for 2 hours. The mixture was concentrated under reduced pressure to afford the crude product. The crude product was purified by preparative HPLC (chromatographic column: Waters-Xbridge-C18-10μm-19*250mm; mobile phase: A: 0.1% formic acid/water; B: acetonitrile, gradient ratio: acetonitrile 40-60%, flow rate: 25 mL/min) to afford compound 35. MS m/z (ESI): 530.28 [M+1]. $^1$H NMR(400 MHz, DMSO-$d_6$): δ 8.79-8.69 (m, 1H), 8.32-8.21 (m, 1H), 7.64 -7.55 (m, 1H), 7.23 (d, *J* = 4.7 Hz, 1H), 6.73-6.70 (m, 1H), 6.46 -6.36 (m, 1H), 3.90 (s, 2H), 3.49 (dd, *J* = 23.1, 6.1 Hz, 1H), 3.20-3.18 (m, 1H), 3.13-3.02 (m, 6H), 2.44 (d, *J* = 4.9 Hz, 4H), 2.22 (s, 3H), 2.00 (t, *J* = 6.7 Hz, 2H), 1.95-1.87 (m, 1H), 1.67 (dd, *J* = 18.8, 7.8 Hz, 2H), 1.44-1.34 (m, 2H), 0.86-0.79 (m, 2H), 0.63-0.56 (m, 2H).

**Example 36. Preparation of compound 36**

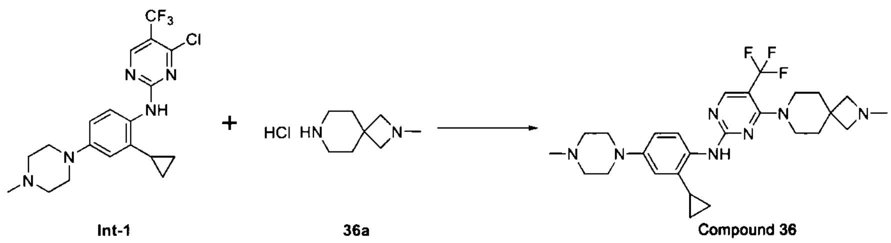

Int-1 36a Compound 36

Compound Int-1 (200 mg, 0.49 mmol) and compound 36a (102.96 mg, 0.58 mmol, Bide) were dissolved in *N,N*-dimethylformamide (2 mL). *N,N*-diisopropylethylamine (0.40 mL, 2.43 mmol) was added, followed by stirring at 100°C for 1 hour. The reaction solution was purified by high performance liquid chromatography (Gilson_306_1741, chromatographic column: Waters-Xbridge-C18-10μm-19*250mm; mobile phase: water (containing 10 mM $NH_4HCO_3$) and acetonitrile, gradient ratio: acetonitrile 40%-70%, flow rate: 25 mL/min) to afford compound 36. MS m/z (ESI): 516.4 [M+1]. $^1$H NMR (400 MHz, DMSO-d6): δ 8.76 (s, 1H), 8.24 (s, 1H), 7.08 (d, J = 8.8 Hz, 1H), 6.73-6.71 (m, 1H), 6.41 (d, J = 2.8 Hz, 1H), 3.87 (s, 2H), 3.06 (d, J = 5.4 Hz, 7H), 2.53-2.49 (m, 2H), 2.42 (t, J = 5.0 Hz, 4H), 2.36 - 2.27 (m, 2H), 2.20 (s, 3H), 1.96 - 1.90 (m, 1H), 1.80(s, 2H), 1.24 (s, 2H), 0.93 (s, 2H), 0.83 - 0.77 (m, 2H), 0.61 - 0.56 (m, 2H).

**Example 37. Preparation of compound 37**

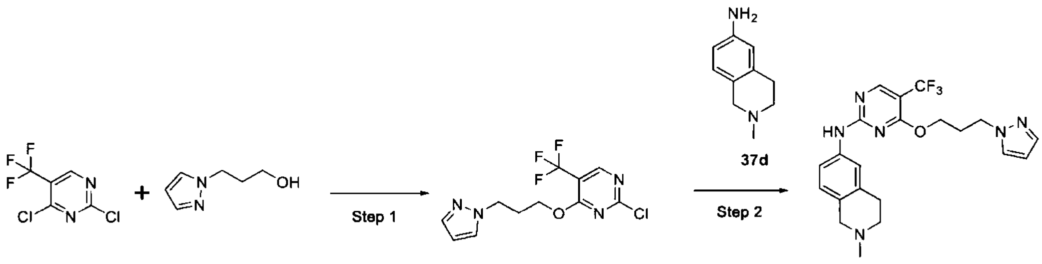


37a 37b 37c Compound 37

Step 1: Compound 37b (174.44 mg, 1.38 mmol, Bide) was dissolved in *N,N*-dimethylformamide (3 mL), and sodium hydride (50.69 μL, 60% purity, 0.39 mmol) was added at 0°C. After stirring at 0°C for 20 minutes, compound 37a (0.19 mL, 1.38 mmol, Bide) was added. The resulting mixture was stirred at room temperature for 18 hours. The reaction solution was purified by high performance liquid chromatography (Gilson_306_1741, chromatographic column: Waters-Xbridge-C18-10μm-19*250 mm; mobile phase: water (containing 10 mM $NH_4HCO_3$) and acetonitrile, gradient ratio: acetonitrile 40%-70%, flow rate: 25 mL/min) to afford compound 37c. MS m/z (ESI): 307.0 [M+1].

Step 2: Compound 37c (100 mg, 0.33 mmol) and compound 37d (58.19 mg, 0.36 mmol, Bide) were dissolved in isopropanol (2 mL). Trifluoroacetic acid (0.12 mL, 1.63 mmol) was added, followed by stirring at 40°C for 18 hours. The reaction solution was purified by high performance liquid chromatography (Gilson_306_1741, chromatographic column: Waters-Xbridge-C18-10 μm-19*250 mm; mobile phase: water (containing 10 mM $NH_4HCO_3$) and acetonitrile, gradient ratio: acetonitrile 33%-63%, flow rate: 25 mL/min) to afford compound 37. MS m/z (ESI): 433.2 [M+1]. $^1$H NMR (400 MHz, DMSO-d6): δ 10.05 (s, 1H), 8.51 (s, 1H), 7.69 (d, J = 2.2 Hz, 1H), 7.45-7.44 (m, 2H), 7.39 (d, J = 8.4 Hz, 1H), 6.97 (d, J = 8.2 Hz, 1H), 6.24-6.23 (m, 1H), 4.37 (t, J = 6.2 Hz,2H), 4.25 (t, J = 6.8 Hz, 2H), 3.42 (s, 2H), 2.77 (t, J = 5.8 Hz, 2H), 2.56 (t, J = 5.8 Hz, 2H), 2.32 (s, 3H),2.27 (q, J = 6.4 Hz, 2H).

**Example 38. Preparation of compound 38**

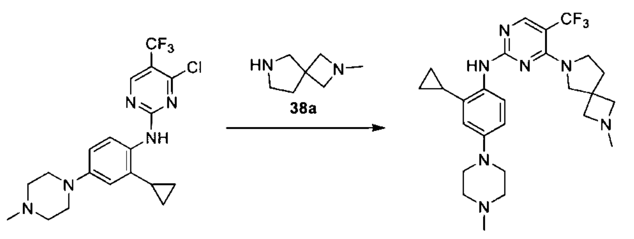


Int-1 Compound 38

Compound Int-1 (100 mg, 0.24 mmol) was dissolved in *N*,*N*-dimethylformamide (2 mL). Then, compound 38a (39.83 mg, 0.32 mmol, Bide) and *N*,*N*-diisopropylethylamine (80.47 μL, 0.49 mmol) were added, respectively, followed by stirring at 80°C for 1 hour. After the reaction was completed, the reaction solution was purified by high performance liquid chromatography (chromatographic column: Waters-Xbridge-C18-10μm-19*250mm; mobile phase: water (containing 10 mmol/L ammonium bicarbonate) and acetonitrile, gradient ratio: acetonitrile 45%-75%, flow rate: 25 mL/min) to afford compound 38. MS m/z (ESI): 502.4 [M+1]. $^1$H NMR (400 MHz, DMSO-$d_6$): δ 8.58 (s, 1H), 8.20 (s, 1H), 7.29 (s, 1H), 6.73-6.70 (m, 1H), 6.43 (d, *J* = 2.8 Hz, 1H), 3.57 (s, 2H), 3.52 - 3.43 (m, 2H), 3.11 - 3.01 (m, 8H), 2.43 (t, *J* = 4.8 Hz, 4H), 2.21 (s, 6H), 1.99 (t, *J* = 6.8 Hz, 2H), 1.95 - 1.89 (m, 1H), 0.85- 0.78 (m, 2H), 0.61 - 0.56 (m, 2H).

**Example 39. Preparation of compound 39**

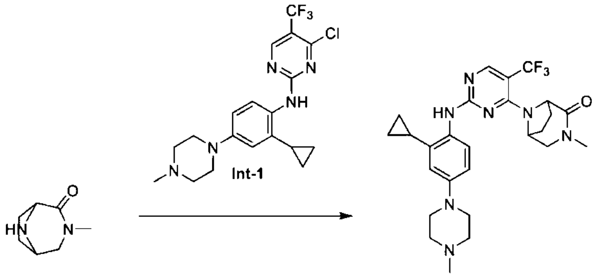

Int-1

39a

Compound 39

At 25°C, triethylamine (100.97 μL, 0.73 mmol) was added to a solution of compound Int-1 (100 mg, 0.24 mmol) and compound 39a (40.85 mg, 0.29 mmol, ACON) in acetonitrile (5 mL). The resulting mixture was heated to 100°C and stirred for 18 hours. The mixture was concentrated under reduced pressure to afford the crude product. The crude product was purified by preparative HPLC (chromatographic column: Waters-Xbridge-C18-10μm-19*250mm; mobile phase: A: 0.1% formic acid/water; B: acetonitrile, gradient ratio: acetonitrile 40-60 %, flow rate: 25 mL/min) to afford compound 39. MS m/z (ESI): 516.36 [M+1]. $^1$H NMR(400 MHz, DMSO-$d_6$): δ 8.92 (s, 1H), 8.30 (s, 1H), 7.22 (s, 1H), 6.74-6.70 (m, 1H), 6.43 (d, $J$ = 2.2 Hz, 1H), 4.66-4.53 (m, 2H), 3.53 (d, $J$ = 6.0 Hz, 1H), 3.17 (s, 1H), 3.08 (d, $J$ = 9.6 Hz, 4H), 2.71 (s, 3H), 2.46-2.42(m, 4H), 2.21 (s, 3H), 2.09-1.75 (m, 5H), 0.84-0.78 (m,2H), 0.61-0.56 (m, 2H).

## Example 40. Preparation of compound 40

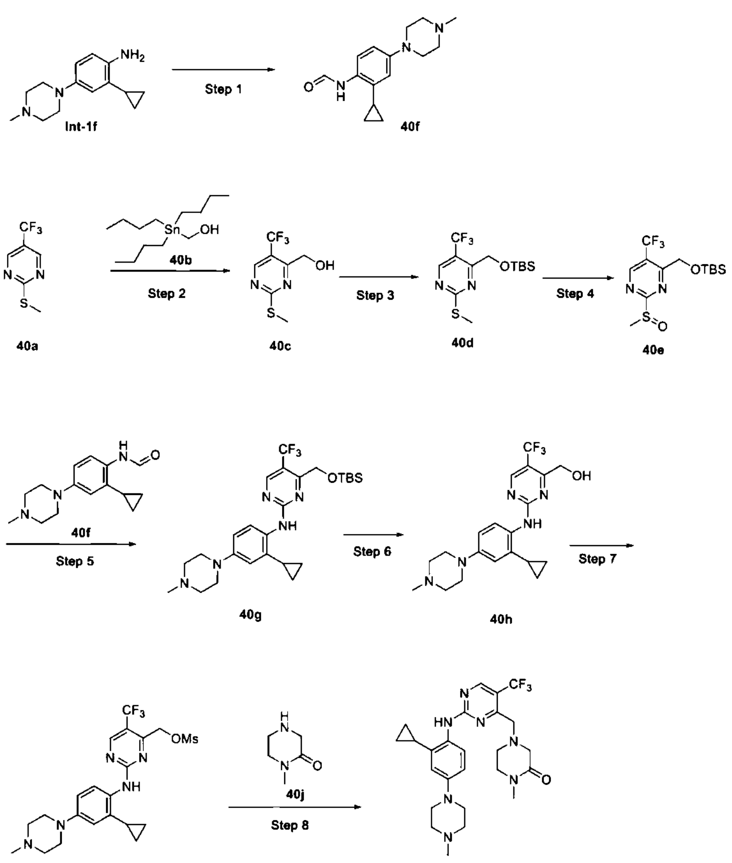


Int-1f 40f

40b 40a 40c 40d 40e

40f 40g 40h

40j 40i Compound 40

Step 1: At 25°C, a solution of compound Int-1f (6 g, 25.94 mmol) in formic acid (60 mL) was heated to 100°C, followed by reacting under stirring for 18 hours. After cooling to room temperature, the reaction mixture was concentrated under reduced pressure to afford the crude product. The crude product was purified by silica gel chromatography (elution solution: dichloromethane - methanol =10 % to 15%) to afford compound 40f, MS m/z (ESI): 260.2 [M+1].

Step 2: At 0°C, tetrakis(triphenylphosphine)palladium (2.98 g, 2.58 mmol) was added to a solution of compound

40a (5.90 g, 25.8 mmol) and compound 40b (9.94 g, 30.9 mmol, Bide) in toluene (50 mL). The reaction solution was stirred at 100°C for 18 hours. The mixture was concentrated under reduced pressure. The crude compound was purified by silica gel chromatography (eluant: petroleum ether: ethyl acetate = 50:1-10:1) to afford compound 40c. MS m/z (ESI): 225.0 [M+1].

Step 3: At 0°C, tert-butyldimethylsilyl chloride (2.54 g, 16.86 mmol) and imidazole (1.91g, 28.10 mmol) were added to a solution of compound 40c (3.15 g, 14.05 mmol) in N,N-dimethylformamide (5 mL). The reaction solution was stirred at room temperature for 18 hours. The mixture was concentrated under reduced pressure to afford the crude. The crude was purified by silica gel chromatography (eluant: petroleum ether: ethyl acetate = 50:1-10:1) to afford compound 40d. MS m/z (ESI): 339.1 [M+1].

Step 4: At 0°C, m-chloroperoxybenzoic acid (610 mg, 3.55 mmol) was added to a solution of compound 40d (1.0 g, 2.95 mmol) in dichloromethane (10 mL). The reaction solution was stirred at room temperature for 18 hours. The mixture was concentrated under reduced pressure to afford the crude. The crude was purified by silica gel chromatography (eluant: petroleum ether: ethyl acetate = 20:1-1:1) to afford compound 40e. MS m/z (ESI): 355.1 [M+1].

Step 5: At 0°C, potassium bis(trimethylsilyl)amide (8.2 mL, 8.2 mmol, 1 M) was added to a solution of compound 40e (1.0 g, 4.1 mmol) in tetrahydrofuran (10 mL). The reaction solution was stirred at room temperature for 1 hour. Then, compound 40f (1.45 g, 4.1 mmol) was added, and the reaction solution was stirred at room temperature for another 2 hours. Then, the mixture was concentrated under reduced pressure to afford the crude. The crude was purified by silica gel chromatography (eluant: ethyl acetate / petroleum ether = 5%-100%) to afford compound 40g. MS m/z (ESI): 522.4 [M+1].

Step 6: At 0°C, 4 M hydrochloric acid/ethyl acetate (1 mL, 4.0 mmol) was added to a solution of compound 40g (150 mg, 0.29 mmol) in ethyl acetate (2 mL). The reaction solution was stirred at room temperature for 3 hours. The mixture was adjusted to pH=7 using a sodium bicarbonate solution, followed by extraction with dichloromethane (5 mL*3). The organic phases were combined, washed with saturated saline, dried, filtered, and concentrated to afford compound 40h. MS m/z (ESI): 408.2 [M+1].

Step 7: At 0°C, methanesulfonyl chloride (34 mg, 0.29 mmol) and triethylamine (60 mg, 0.59 mmol) were added to a solution of compound 40h (80 mg, 0.20 mmol) in dichloromethane (2 mL). The reaction solution was stirred at room temperature for 3 hours. The mixture was poured into water solution (5 mL), followed by extraction with dichloromethane (5 mL*3). The organic phases were combined, washed with saturated saline, dried, filtered, and concentrated under reduced pressure to afford compound 40i. MS m/z (ESI): 486.2 [M+1].

Step 8: At room temperature, N,N-diisopropylethylamine (85 mg, 0.66 mmol) was added to a solution of compound 40i (80 mg, 0.16 mmol) and compound 40j (38 mg, 0.33 mmol, Bide) in tetrahydrofuran (2 mL). The resulting mixture was heated to 60 °C and stirred for 3 hours. The crude product was afforded by concentration under reduced pressure. The crude product was purified by preparative HPLC (chromatographic column: Waters-SunFire-C18-10μm-19*250mm; mobile phase: A: 0.1% formic acid/water; B: acetonitrile, gradient ratio: acetonitrile 10-40%, flow rate: 25 mL/min) to afford compound 40. MS m/z (ESI): 504.2 [M+1]. $^1$H NMR (400 MHz, MeOD): δ 8.48 (s, 1H), 8.44 (s, 1H), 7.41 (d, *J* = 8.6 Hz, 1H), 6.85 (dd, *J* = 8.6, 2.6 Hz, 1H), 6.66 (d, *J* = 2.6 Hz, 1H), 3.76 (s, 2H), 3.34 - 3.30 (m, 8H), 3.14 - 3.09 (m, 4H), 2.93 (s, 3H), 2.90 - 2.85 (m, 2H), 2.72 (s, 3H), 1.99 - 1.89 (m, 1H), 0.97 - 0.83 (m, 2H), 0.70 - 0.58 (m, 2H).

### Example 41. Preparation of compound 41

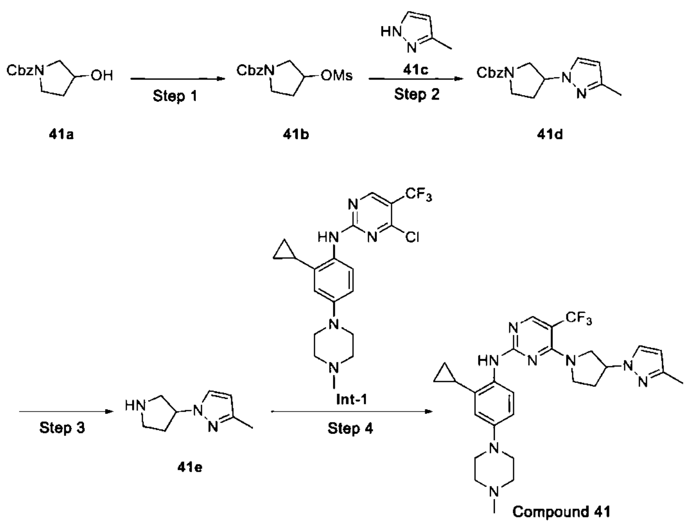

Step 1: Compound 41a (2 g, 9.04 mmol, Bide) and triethylamine (2.51 mL, 18.08 mmol, Bide) were dissolved in dichloromethane (20 mL), and methylsulfonyl chloride (1.04 mL, 6.30 mmol) was added. The reaction solution was stirred at 0°C for 1 hour. The reaction solution was poured into water (100 mL), extracted with dichloromethane (30 mLx3). The organic phases were combined, washed with saturated saline (20 mL), dried over anhydrous sodium sulfate, filtered, and concentrated under reduced pressure to afford compound 41b.

Step 2: Compound 41c (820 mg, 10.02 mmol, Bide) was dissolved in N,N-dimethylformamide (20 mL). Under an ice bath, sodium hydride (500 mg, 60% purity, 12.53 mmol) was added, followed by stirring at 0°C for 0.5 hours. Compound 41b (2.5 g, 8.35 mmol) was added to reaction solution, and the reaction solution was stirred at 60°C for 18 hours. The reaction solution was quenched with a saturated ammonium chloride solution, extracted with ethyl acetate (30 mL*3). The organic phases were combined, washed with saturated saline (20 mL), dried over anhydrous sodium sulfate, filtered, and concentrated under reduced pressure to afford the crude. The crude was purified by silica gel chromatographic column, and then was purified by high performance liquid chromatography (Gilson_306_1741, chromatographic column: SW-8201-220g; mobile phase: water (containing 0.05% TFA) and acetonitrile, gradient ratio: acetonitrile 23%-33%, flow rate: 60 mL/min) to afford compound 41d. MS m/z (ESI): 286.2 [M+1].

Step 3: Compound 41d (400 mg, 1.40 mmol) was dissolved in tetrahydrofuran (10 mL) and hydrochloric acid (0.5 mL). Palladium on carbon (149.0 mg, 1.40 mmol) was added, followed by stirring at 25°C for 18 hours under a hydrogen atmosphere. The reaction solution was filtered and then concentrated under reduced pressure to afford compound 41e. MS m/z (ESI): 152.2 [M+1].

Step 4: Compound 41e (55.07 mg, 0.36 mmol) and compound Int-1 (150 mg, 0.36 mmol) were dissolved in N,N-dimethylformamide (2 mL). N,N-diisopropylethylamine (0.05 mL, 0.36 mmol) was added, followed by stirring at 100°C for 18 hours. The reaction solution was purified by high performance liquid chromatography (Gilson_306_1741, chromatographic column: Shimadzu-Shim-pack-C18-5um-20*250mm; mobile phase: water (containing 0.05% HCl) and acetonitrile, gradient ratio: acetonitrile 23%-33%, flow rate: 20 mL/min) to afford compound 41. MS m/z (ESI): 527.4 [M+1]. $^1H$ NMR (400 MHz, DMSO-d6): δ 8.64 (s, 1H), 8.23 (s, 1H), 7.64 (d, J = 2.4 Hz, 1H), 7.27 (s, 1H), 6.71 (dd, J = 8.8, 2.8 Hz, 1H), 6.42 (d, J = 2.8 Hz, 1H), 6.02 (d, J = 2.4 Hz, 1H), 5.00 - 4.86 (m, 1H), 3.97 - 3.85 (m, 1H), 3.86 - 3.75 (m, 1H), 3.76 - 3.58 (m, 2H), 3.07 (t, J = 4.8 Hz, 4H), 2.47 - 2.41 (m, 4H), 2.37 - 2.30 (m, 2H), 2.22 (s, 3H), 2.14 (s, 3H), 1.97 - 1.89 (m, 1H), 0.86 - 0.77 (m, 2H), 0.64 - 0.55 (m, 2H).

**Example 42. Preparation of compound 42**

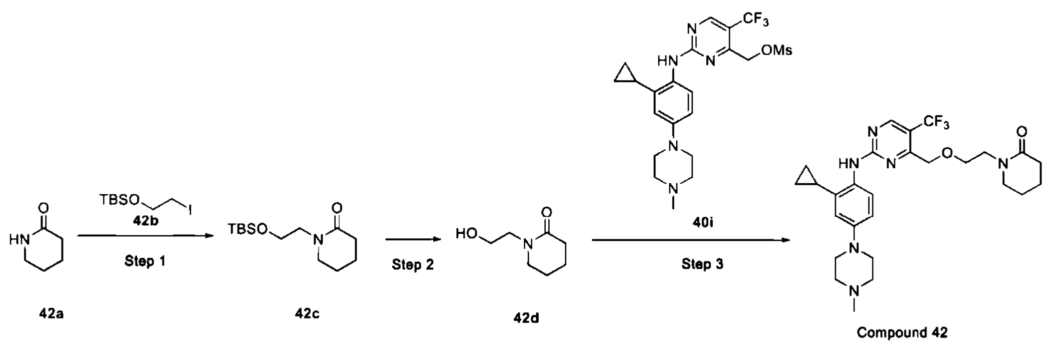


42a 42c 42d Compound 42

Step 1: At 0°C, sodium hydride (1.05 g, 17.8 mmol, 60% purity) was added to a solution of compound 42a (1.39 g, 14.0 mmol, Bide) in N,N-dimethylformamide (40 mL). After stirring for 30 minutes, compound 42b (5.00 g, 17.8 mmol, Bide) was added, and the reaction solution was stirred at room temperature for 3 hours. The mixture was poured into water (40 mL), followed by extraction with ethyl acetate (40 mL x 3). The organic phases were combined, washed with saturated saline (50 mL), dried over anhydrous sodium sulfate, filtered, and concentrated under reduced pressure to afford the crude. The crude was purified by silica gel chromatography (eluant: petroleum ether: ethyl acetate = 5:1-1:1) to afford compound 42c. MS m/z (ESI): 258.1 [M+1].

Step 2: At 0°C, 4M of hydrochloric acid/ethyl acetate (1 mL, 4.0 mmol) was added to a solution of compound 42c (200 mg, 0.78 mmol) in ethyl acetate (4 mL). The reaction solution was stirred at room temperature for 2 hours. The mixture was adjusted to pH=7 using a sodium bicarbonate solution, followed by extraction with dichloromethane (8 mL*3). The organic phases were combined, washed with saturated saline, dried, filtered, and concentrated under reduced pressure to afford compound 42d. MS m/z (ESI): 144.1 [M+1].

Step 3: At 0°C, sodium hydride (25 mg, 0.62 mmol, 60% purity) was added to a solution of compound 42d (200 mg, 0.41 mmol) in tetrahydrofuran (5 mL). After stirring for 30 minutes, compound 40i (88 mg, 0.62 mmol) was added. The

resulting mixture was heated to 60°C and stirred for 18 hours. The crude product was afforded by concentration under reduced pressure. The crude product was purified by preparative HPLC (chromatographic column: Waters-Xbridge-C18-10pum-19*250mm; mobile phase: A: 10 mM ammonium bicarbonate/water; B: acetonitrile, gradient ratio: acetonitrile 40-70%, flow rate: 25 mL/min) to afford compound 42. MS m/z (ESI): 533.4 [M+1]. $^1$H NMR (400 MHz, MeOD): δ 8.49 (s, 1H), 7.42 (d, *J* = 8.6 Hz, 1H), 6.84 (dd, *J* = 8.6, 2.8 Hz, 1H), 6.65 (d, *J* = 2.8 Hz, 1H), 4.57 (s, 2H), 3.80 - 3.63 (m, 2H), 3.59 - 3.44 (m, 2H), 3.42 - 3.33 (m, 2H), 3.22 - 3.12 (m, 4H), 2.69 - 2.54 (m, 4H), 2.35 (s, 3H), 2.33 - 2.26 (m, 2H), 1.98 - 1.87 (m, 1H), 1.80 - 1.69 (m, 4H), 0.94 - 0.84 (m, 2H), 0.67 - 0.58 (m, 2H).

**Example 43. Preparation of compound 43**

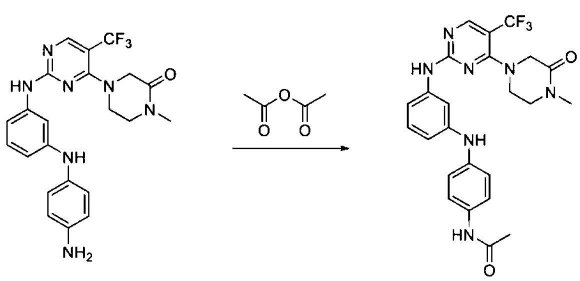

Compound 21 Compound 43

At room temperature, triethylamine (40 mg, 0.39 mmol) was added to a solution of compound 21 (60 mg, 0.13 mmol) in dichloromethane (2 mL). Under an ice bath, acetic anhydride (0.01 mL, 0.13 mmol) was added dropwise, and the reaction was carried out at room temperature for 2 hours. The crude product was afforded by concentration under reduced pressure. The crude product was purified by preparative HPLC (chromatographic column: Waters-Xbridge-C18-10μm-19*250mm; mobile phase: A: 10 mM ammonium bicarbonate/water; B: acetonitrile, gradient ratio: acetonitrile 95%, flow rate: 20 mL/min) to afford compound 43. MS m/z (ESI): 500.2 [M+1]. $^1$H NMR(400 MHz, DMSO-$d_6$): δ 9.77 (s, 1H), 9.69 (s, 1H), 8.41 (s, 1H), 8.04 (s, 1H), 7.52 (d, *J* = 8.8 Hz, 2H), 7.44 - 7.38 (m, 1H), 7.08 (t, *J* = 8.0 Hz, 1H), 7.04 (d, *J* = 8.8 Hz, 2H), 6.94 (d, *J* = 8.2 Hz, 1H), 6.66 (d, *J* = 6.0 Hz, 1H), 4.10 (s, 2H), 3.83 (t, *J* = 5.0 Hz, 2H), 3.44 (t, *J* = 5.0 Hz, 2H), 2.87 (s, 3H), 2.01 (s, 3H).

**Example 44. Preparation of compound 44**

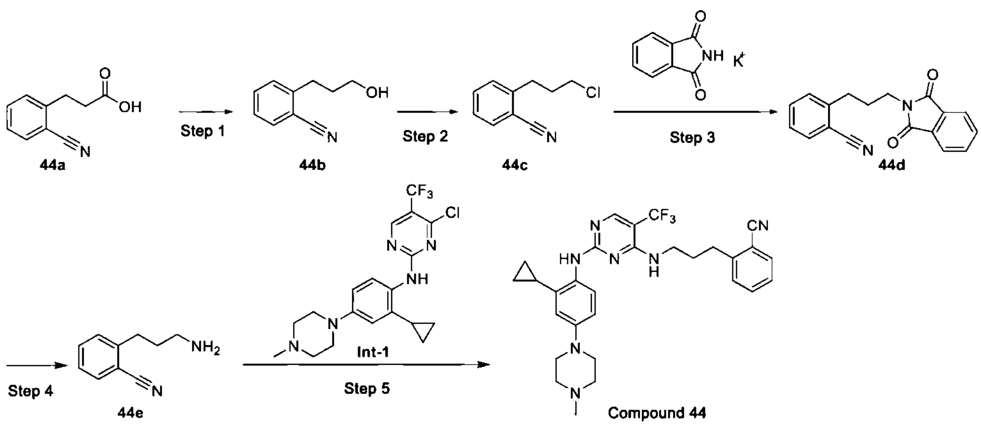


Step 1: At 0°C, under nitrogen protection, compound 44a (500 mg, 2.85 mmol, Bide) was added to an anhydrous dichloromethane (5 mL), and then 2-chloro-2-oxoacetyl chloride (611.88 μL, 7.14 mmol) was added. The resulting mixture was stirred at 25°C for 1 hour. The mixture was concentrated to dryness. The solid was dissolved in tetrahydrofuran (5 mL), and sodium borohydride (1.08 g, 28.54 mmol) was added. The mixture was stirred at 20°C for 16 hours. The mixture was poured into water (20 mL), followed by extraction with ethyl acetate (30 mLx3). The combined organic phase was washed with brine (10 mL), dried over anhydrous sodium sulfate, filtered, and concentrated under reduced pressure to dryness, to afford compound 44b. MS m/z (ESI): 162.2 [M+1].

Step 2: At 0°C, compound 44b (170 mg, 1.0 mmol) was added to DCM (2 mL) solution, then sulfoxide chloride (382.48 μL, 5.27 mmol) and N,N- dimethylformamide (8.20 μL, 0.11 mmol) were added, respectively. The resulting mixture was stirred at 55°C for 3 hours. Compound 44c was afforded by concentration to dryness. MS m/z (ESI): 180.5 [M+1].

Step 3: Compound 44c (80 mg, 0.45 mmol) was added to N,N-dimethylformamide (4 mL), and then potassium 1,3-isoindolinedione(164.96 mg, 0.89 mmol, Bide) was added. The resulting mixture was stirred at 60°C for 16 hours. The mixture was poured into water (20 mL), followed by extraction with ethyl acetate (20 mL x3). The combined organic phase was washed with brine (10 mL), dried over anhydrous sodium sulfate, filtered, and concentrated under reduced pressure to dryness, to afford compound 44d. MS m/z (ESI): 291.3 [M+1].

Step 4: 44d (90 mg, 0.31 mmol) was added to ethanol (2 mL), and then hydrazine hydrate (0.18 mL, 3.10 mmol, 85%) was added. The resulting mixture was stirred at 25°C for 2 hours. The reaction solution was concentrated to dryness to afford compound 44e. MS m/z (ESI): 161.1 [M+1].

Step 5: N,N-dimethylformamide (1 mL) was added to a mixture of compound Int-1 (77.12 mg, 0.19 mmol), 44e (30 mg, 0.19 mmol) and N,N-diisopropylethylamine (94.45 μL, 0.57 mmol), followed by stirring at 100% for 1 hour. The reaction solution was purified by preparative HPLC (Phenomenex Gemini 150 mm*25 mm*25 mm*10_; mcolume (elution solution: 30% to 60% (v/v) $CH_3CN$ and $H_2O$ with 0.025% $NH_4HCO_3$) to afford compound 44. MS m/z (ESI): 536.4 [M+1]. $^1$H NMR (400 MHz, DMSO-$d_6$): δ 8.56 (s, 1H), 8.06 (s, 1H), 7.80 - 7.73 (m, 1H), 7.64 - 7.55 (m, 1H), 7.42 - 7.35 (m, 1H), 7.36 - 7.21 (m, 2H), 7.02 - 6.95 (m, 1H), 6.71 - 6.65 (m, 1H), 6.47 - 6.39 (m, 1H), 3.41 - 3.33 (m, 2H), 3.09 - 2.99 (m, 4H), 2.69 - 2.62 (m, 2H), 2.41 (s, 4H), 2.19 (s, 3H), 1.96 - 1.89 (m, 1H), 1.85 - 1.71 (m, 2H), 0.85 - 0.78 (m, 2H), 0.60 - 0.54 (m, 2H).

## Example 45. Preparation of compound 45

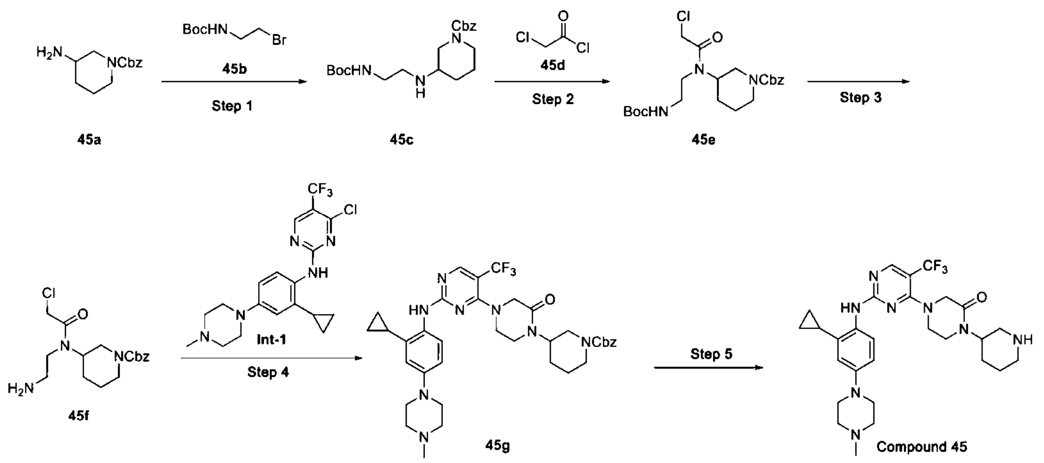

45a 45c 45e 45f 45g Compound 45

Step 1: At about 25°C, potassium carbonate (8.85 g, 64.02 mmol) was added to a solution of compound 45b (1.6 g, 18.37 mmol, Leyan) and compound 45a (5.74 g, 25.61 mmol, Bide) in N,N-dimethylformamide (50 mL). The resulting mixture was heated to 60 °C and stirred for 48 hours. The mixture was poured into water (50 mL), followed by extraction with EA (50 mL x3). The combined organic phase was washed with brine (50 mL), dried over anhydrous sodium sulfate, filtered, and concentrated under reduced pressure, to afford the crude product. The crude product was purified by silica gel chromatography (eluant: dichloromethane - methanol = 4%-10 %) to afford compound 45c. MS m/z (ESI): 378.2 [M+1].

Step 2: At about 0 °C, triethylamine (3855.66 μL, 27.82 mmol) and chloroacetyl chloride 45d (884.92 μL, 11.13 mmol) were added to a solution of compound 45c (3.5 g, 9.27 mmol) in dichloromethane (60 mL), respectively. The resulting mixture was stirred at 25°C for 18 hours. The mixture was poured into water (20 mL), followed by extraction with dichloromethane (30 mL×3). The combined organic phase was washed with saturated saline (20 mL), dried over anhydrous sodium sulfate, filtered, and concentrated under reduced pressure, to afford compound 45e. MS m/z (ESI): 454.2 [M+1].

Step 3: At about 25°C, hydrochloric acid/1,4-dioxane (10 mL, 40.00 mmol, 4 mmol/L) was added to a solution of compound 45e (2.5 g, 5.51 mmol) in dichloromethane (20 mL). The resulting mixture was stirred at 25°C for 3 hours. The mixture was concentrated under reduced pressure to afford compound 45f. MS m/z (ESI): 354.1 [M+1].

Step 4: At about 25°C, triethylamine (336.56 μL, 2.43 mmol) was added to a solution of compound Int-1 (200 mg, 0.49 mmol) and compound 45f (368.21 mg, 0.73 mmol) in acetonitrile (5 mL). The resulting mixture was heated to 90°C and stirred for 18 hours. The mixture was concentrated under reduced pressure to afford the crude product. The crude product was purified by preparative HPLC (0.1% aqueous ammonium bicarbonate solution- acetonitrile = 40-60%) to afford

compound 45g. MS m/z (ESI): 693.3 [M+1].

Step 5: At about 25°C, palladium on carbon (3.07 mg, 0.00 mmol, 10%) and acetic acid (0.3 mL, 5.25 mmol) were added to a solution of compound 45g (20 mg, 0.03 mmol) in methanol (3 mL). The mixture was purged with hydrogen 3 times, and stirred at 25°C for 6 hours under a hydrogen atmosphere. The mixture was concentrated under reduced pressure to dryness, to afford the crude product. The crude was purified by preparative HPLC (chromatographic column: Waters-Xbridge-C18-10μm-19*250mm; mobile phase: A: 10mM ammonium bicarbonate/water; B: acetonitrile, gradient ratio: acetonitrile 40-60%, flow rate: 25 mL/min) to afford compound 45. MS m/z (ESI): 559.3 [M+1]. $^1$H NMR(400 MHz, DMSO-$d_6$): δ 8.35 (s, 1H), 7.80 (d, *J* = 8.6 Hz, 1H), 7.33-7.27 (m, 1H), 6.84 (dd, *J* = 8.8, 2.6 Hz, 1H), 6.72 (d, *J* = 2.4 Hz, 1H), 4.44-4.34 (m, 1H), 4.30 - 4.20 (m, 2H), 3.89-3.72 (m, 2H), 3.49-3.31 (m, 2H), 3.25-3.07 (m, 6H), 2.82-2.72 (m, 1H), 2.63 (d, *J* = 9.4 Hz, 4H), 2.60-2.52 (m, 1H), 2.39 (s, 3H), 1.69 (td, *J* = 11.6, 6.0 Hz, 5H), 1.03-0.94 (m, 2H), 0.70-0.64 (m, 2H).

**Example 46. Preparation of compound 46**

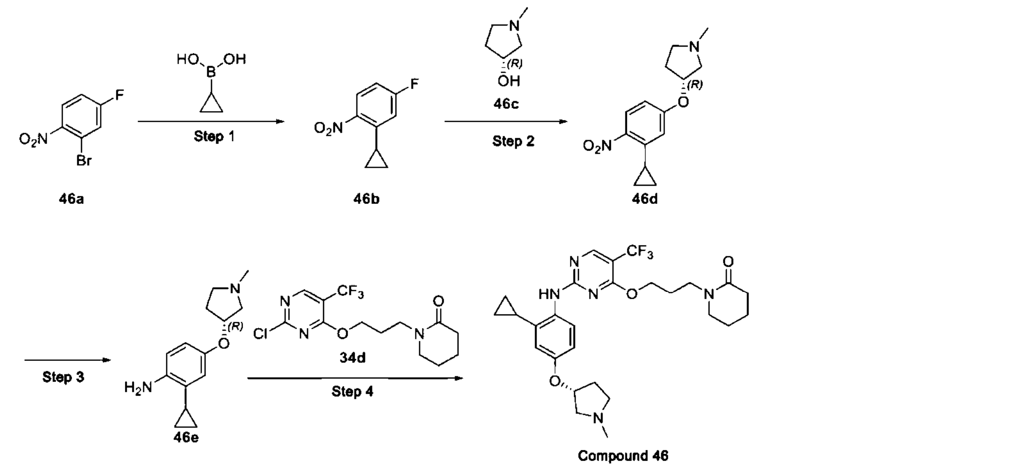


Step 1: At about 25°C, palladium acetate (0.51g, 2.27 mmol) and tricyclohexylphosphine (0.64 g, 2.27 mmol) were added to a mixed solution of compound 46a (5.00 g, 22.73 mmol, Bide), cyclopropylboronic acid (2.93 g, 34.09 mmol, Bide), and potassium phosphate (14.47 g, 68.18 mmol) in toluene (80 mL) and $H_2O$ (16 mL), respectively. The mixture was purged with nitrogen 3 times, and then heated to 90°C and stirred for 18 hours. After cooling to room temperature, the mixture was concentrated to afford a crude product. The crude product was purified by silica gel chromatography (elution solution: ethyl acetate: petroleum ether =15-25%) to afford compound 46b. MS m/z (ESI): 182.0 [M+1].

Step 2: At 0°C, sodium hydride (0.66 g, 16.56 mmol, 60% purity) was added to a solution of compound 46b (2 g, 11.04 mmol) in N,N-dimethylformamide (20 mL). After stirring at 0°C for 15 minutes, compound 46c (1213.73 μL, 11.04 mmol, Bide) was added, and the resulting mixture was stirred at 25°C for 18 hours. The mixture was poured into water (30 mL), followed by extraction with ethyl acetate (50 mL×3). The organic phases were combined, washed with saturated saline (30 mL), dried over anhydrous sodium sulfate, filtered, and concentrated under reduced pressure, to afford the crude product. The crude product was purified by silica gel chromatography (elution solution: methanol: dichloromethane =1%-8%) to afford compound 46d. MS m/z (ESI): 263.1 [M+1].

Step 3: At 25°C, palladium on carbon (0.1 g, 0.94 mmol, 10%) was added to a solution of compound 46d (1 g, 3.81 mmol) in methanol (20 mL). The mixture was purged with hydrogen 3 times, and stirred at 25°C for 18 hours under a hydrogen atmosphere. The mixture was filtered. The filter cake was rinsed with methanol (20 mL×3), and the filtrate was concentrated under reduced pressure to afford compound 46e. MS m/z (ESI): 233.2 [M+1].

Step 4: At 25°C, trifluoroacetic acid (165.49 μL, 2.22 mmol) was added to a solution of compound 46e (123.82 mg, 0.53 mmol) and compound 34d (150 mg, 0.44 mmol) in isopropanol (5 mL). The resulting mixture was heated to 40°C and stirred for 18 hours. After cooling to room temperature, the mixture was concentrated under reduced pressure to afford the crude product. The crude was purified by preparative HPLC (chromatographic column: Waters-Xbridge-C18-10μm-19*250mm; mobile phase: A: 0.1% formic acid/water; B: acetonitrile, gradient ratio: acetonitrile 40-60%, flow rate: 25 mL/min) to afford compound 46. MS m/z (ESI): 534.4 [M+1]. $^1$H NMR(400 MHz, DMSO-$d_6$): δ 9.41 (s, 1H), 8.35 (s, 1H), 7.19 (s, 1H), 6.68 (dd, *J* = 8.4, 2.8 Hz, 1H), 6.35 (d, *J* = 2.6 Hz, 1H), 4.90-4.84 (m, 1H), 4.48-4.10 (m, 2H), 3.17 (dd, *J* = 18.8, 8.2 Hz, 2H), 2.87- 2.80 (m, 1H), 2.76-2.65 (m, 2H), 2.45 - 2.39 (m, 1H), 2.34-2.11 (m, 7H), 1.94-1.75 (m, 4H), 1.74-1.59 (m, 5H), 0.87-0.80 (m, 2H), 0.62-0.56 (m, 2H).

**Example 47. Preparation of compound 47**

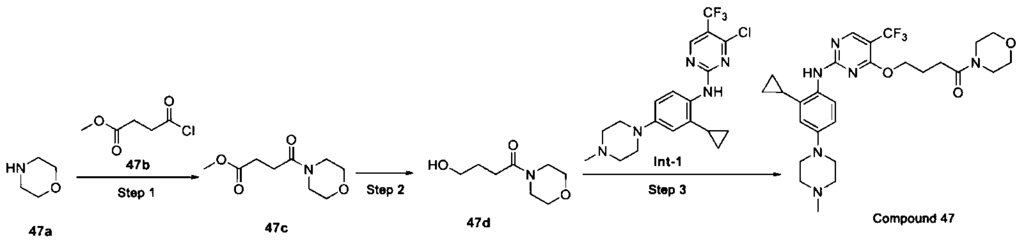

47a 47c 47d Compound 47

Step 1: At about 0°C, compound 47b (4.15 g, 27.55 mmol, Bide) was added to a solution of compound 47a (1.6 g, 18.37 mmol, Bide) and triethylamine (7.64 mL, 55.10 mmol) in dichloromethane (30 mL). The resulting mixture was stirred at 25°C for 2 hours. The mixture was poured into water (30 mL), followed by extraction with dichloromethane (30 mL x2). The organic phases were combined, washed with saturated saline (30 mL), dried over anhydrous sodium sulfate, filtered, and concentrated under reduced pressure, to afford the crude product. The crude product was purified by silica gel chromatography (eluant: petroleum ether: tetrahydrofuran =5-30%) to afford compound 47c. MS m/z (ESI): 202.1 [M+1].

Step 2: At about 25°C, lithium borohydride (19.88 mL, 19.88 mmol, 1 mmol/L) was added to a solution of compound 47c (2 g, 9.94 mmol) in THF (20 mL). The resulting mixture was stirred at 25°C for 18 hours. Water (20 mL) was added to the mixture, followed by extraction with ethyl acetate (30 mL×3). The organic phases were combined, washed with saturated saline (30 mL), dried over anhydrous sodium sulfate, filtered, and concentrated under reduced pressure, to afford the crude product. The crude product was purified by silica gel chromatography (eluant: petroleum ether - tetrahydrofuran = 5%-80%) to afford compound 47d. MS m/z (ESI): 173.1 [M+1].

Step 3: At about 0°C, sodium hydride (19.42 mg, 0.49 mmol, 60% purity) was added to a solution of compound 47d (54.67 mg, 0.32 mmol) in tetrahydrofuran (5 mL). After stirring at 0°C for 15 minutes, compound Int-1 (100 mg, 0.24 mmol) was added. The resulting mixture was heated to 60°C and stirred for 18 hours. After cooling to room temperature, water (3 mL) was poured into the mixture for quenching, and the mixture was concentrated under reduced pressure to afford the crude. The crude was purified by preparative HPLC (chromatographic column: Waters-Xbridge-C18-10μm-19*250mm; mobile phase: A: 10 mM ammonium bicarbonate/water; B: acetonitrile, gradient ratio: acetonitrile 40-60%, flow rate: 25 mL/min) to afford compound 47. MS m/z (ESI): 549.3 [M+1]. $^1H$ NMR(400 MHz, DMSO-$d_6$): δ 9.31 (s, 1H), 8.33 (s, 1H), 7.12 (s, 1H), 6.73 (dd, *J* = 8.6, 2.6 Hz, 1H), 6.42 (d, *J* = 2.6 Hz, 1H), 4.52-4.10 (m, 2H), 3.53 (d, *J* = 4.8 Hz, 4H), 3.36 (s, 4H), 3.13-3.04 (m, 4H), 2.43 (t, *J* = 5.0 Hz, 6H), 2.21 (s, 3H), 2.00-1.78 (m, 3H), 0.80 (d, *J* = 8.8 Hz, 2H), 0.63-0.56 (m, 2H).

**Example 48. Preparation of compound 48**

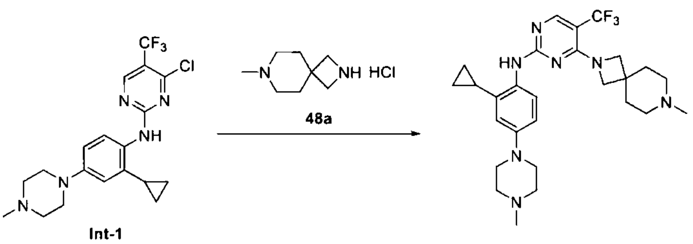

Int-1 Compound 48

At about 25°C, triethylamine (336.56 μL, 2.43 mmol) was added to a solution of compound Int-1 (200 mg, 0.49 mmol) and compound 48a (81.72 mg, 0.58 mmol, Bide) in N,N-dimethylformamide (3 mL). The resulting mixture was heated to 100°C and stirred for 2 hours. After cooling to room temperature, the reaction solution was purified by preparative HPLC (chromatographic column: Waters-Xbridge-C18-10μm-19*250mm; mobile phase: A: 10mM ammonium bicarbonate/water; B: acetonitrile, gradient ratio: acetonitrile 40-60%, flow rate: 25 mL/min) to afford compound 48. MS m/z (ESI): 516.3 [M+1]. $^1H$ NMR(400 MHz, DMSO-$d_6$): δ 8.59 (s, 1H), 8.13 (s, 1H), 7.27 (s, 1H), 6.71 (dd, *J* = 8.8, 2.6 Hz, 1H), 6.43 (s, 1H), 3.78 (s, 4H), 3.07 (d, *J* = 9.6 Hz, 4H), 2.43 (d, *J* = 9.6 Hz, 4H), 2.34-2.01 (m, 10H), 1.98-1.92 (m, 1H), 1.70 (t, *J* = 10.2 Hz, 4H), 0.85-0.77 (m, 2H), 0.62-0.55 (m, 2H).

**Example 49. Preparation of compound 49**

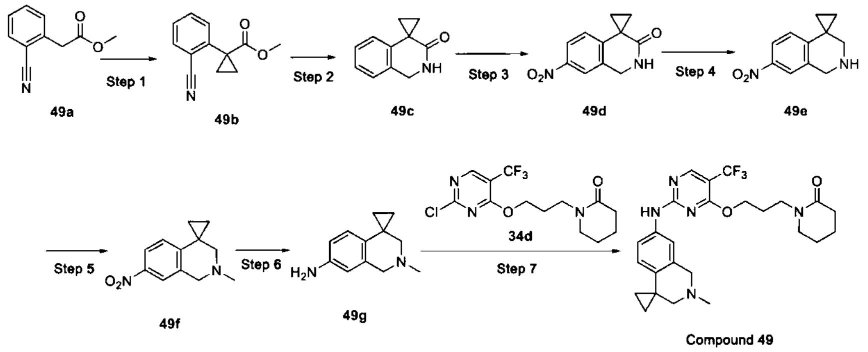


49a 49b 49c 49d 49e 49f 49g 34d Compound 49

Step 1: Compound 49a (10 g, 57.08 mmol, Bide) and 1,2-dibromoethane (7.41 mL, 85.62 mmol) were dissolved in an aqueous sodium hydroxide solution (40 mL, 50%) and toluene (80 mL). Tetrabutylammonium bromide (3.68 g, 11.42 mmol) was added, and the reaction solution was stirred at room temperature for 1 hour. The reaction solution was poured into water (100 mL) for dilution, followed by extraction with ethyl acetate (50 mL × 3). The organic phases were combined, washed with saturated saline (10 mL), dried over anhydrous sodium sulfate, filtered, and concentrated under reduced pressure to afford the crude. The residue was purified by silica gel column chromatography (eluant system: petroleum ether : ethyl acetate = 10:1 to 4:1) to afford compound 49b. MS m/z (ESI): 202.0 [M+1].

Step 2: Compound 49b (8.94 g, 44.43 mmol) was dissolved in methanol (80 mL). Raney nickel (1.8 g, 30.67 mmol) was added, and the reaction solution was purged with hydrogen 5 times, followed by reacting under stirring at room temperature for two days under a hydrogen atmosphere. The reaction solution was filtered. The filter cake was washed with methanol (30 mL×3), and the filtrate was dried by spin to afford compound 49c. MS m/z (ESI): 174.0 [M+1].

Step 3: Compound 49c (6.95 g, 40.12 mmol) was dissolved in a concentrated sulfuric acid (120 mL), and potassium nitrate (4.46 g, 44.13 mmol) was added. The reaction solution was stirred at 0°C for 5 minutes, and then warmed to room temperature and stirred for 15 minutes. The reaction solution was poured into 1000 mL water for dilution, and then filtered. The filter cake was washed with water (200 mL), and the filter cake was dried to afford compound 49d. MS m/z (ESI): 219.0 [M+1].

Step 4: Boron trifluoride ethyl etherate (4.52 mL, 36.66 mmol) was dissolved in tetrahydrofuran (20 mL). Sodium borohydride (1.04 g, 27.50 mmol) was added to the reaction solution at 0°C, followed by warming to room temperature and stirring for 1 hour. Then, compound 49d (2 g, 9.17 mmol) was dissolved in tetrahydrofuran (20 mL). The resulting mixture was added to the reaction solution, followed by stirring at 70°C for 2 hours. The reaction solution was poured into a saturated ammonium chloride solution (100 mL) for quenching. Then, the solution pH was adjusted to 7 using a saturated sodium bicarbonate solution, followed by extraction with ethyl acetate (50 mL × 3). The organic phases were combined, washed with saturated saline (10 mL), dried over anhydrous sodium sulfate, filtered, and concentrated under reduced pressure to afford compound 49e. MS m/z (ESI): 205.2 [M+1].

Step 5: Compound 49e (0.5 g, 2.45 mmol) was dissolved in methanol (10 mL). Formaldehyde (1.82 mL, 24.48 mmol) and sodium triacetoxyborohydride (2.58 g, 12.24 mmol) were added at room temperature. The reaction was carried out at room temperature for 4 hours. pH was adjusted to 7 using a saturated sodium bicarbonate solution, followed by extraction with ethyl acetate (30 mL × 3). The organic phases were combined, washed with saturated saline (10 mL), dried over anhydrous sodium sulfate, filtered, and concentrated under reduced pressure to afford compound 49f. MS m/z (ESI): 219.2 [M+1].

Step 6: Compound 49f (0.5 g, 2.29 mmol) was dissolved in methanol (10 mL). Ammonium formate (1.44 g, 22.91 mmol) and 10% palladium on carbon (0.10 g, 0.09 mmol) were added at room temperature. The reaction was carried out for 18 hours under a hydrogen atmosphere. The reaction solution was filtered, and water (100 mL) was poured into the filtrate for dilution, followed by extraction with ethyl acetate (30 mL × 3). The organic phases were combined, washed with saturated saline (10 mL), dried over anhydrous sodium sulfate, filtered, and concentrated under reduced pressure to afford compound 49g. MS m/z (ESI): 189.3 [M+1].

Step 7: Compound 49g (100 mg, 0.30 mmol) and compound 34d (61.32 mg, 0.33 mmol) were dissolved in isopropanol (5 mL). Trifluoroacetic acid (110.33 μL, 1.48 mmol) was added at room temperature, and the reaction was carried out at 40°C for 18 hours. The reaction solution was purified by high performance liquid chromatography Gilson_306_1741, chromatographic column: Waters-SunFire-C18-10μm-19*250mm; mobile phase: water (containing 0.1% formic acid) and acetonitrile, gradient ratio: acetonitrile 0%-95%, flow rate: 20 mL/min) to afford compound 49. MS m/z (ESI): 490.5 [M+1]. $^1$H NMR (400 MHz, DMSO-d6): δ 10.04 (s, 1H), 8.49 (s, 1H), 7.44 (dd, J = 8.4, 2.2 Hz, 1H), 7.39 (s,

1H), 6.69 (d, J = 8.4 Hz, 1H), 4.43 (t, J = 6.4 Hz, 2H), 3.59 (s, 2H), 3.39 (d, J = 7.0 Hz, 4H), 3.24 (t, J = 5.6 Hz, 3H), 2.46 (d, J = 2.0 Hz, 2H), 2.18 (t, J = 6.2 Hz, 2H), 1.95 (p, J = 6.6 Hz, 2H), 1.72 - 1.63 (m, 4H), 0.92 - 0.82 (m, 4H).

**Example 50. Preparation of compound 50**

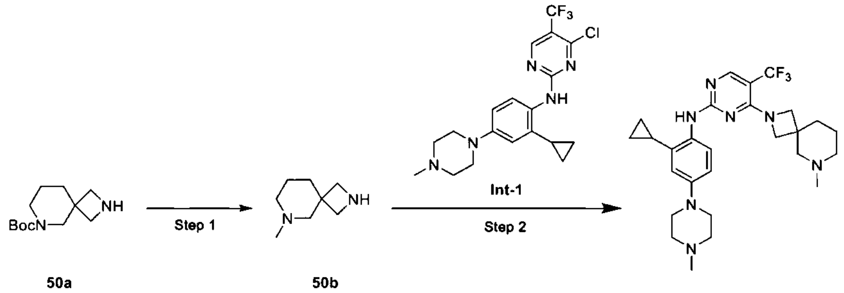


50a 50b Compound 50

Step 1: Under nitrogen protection, compound 50a (200 mg, 0.88 mmol, Bide) was dissolved in tetrahydrofuran (4 mL). Then, lithium aluminium hydride (1.41 mL, 3.53 mmol) was added under an ice bath. The reaction solution was slowly warmed to room temperature, followed by reacting under stirring for 16 hours. After the reaction was completed, a sufficient amount of sodium sulfate decahydrate was added to the reaction solution for quenching, followed by filtration and washing with tetrahydrofuran (50 mL). The organic layer was dried over anhydrous sodium sulfate, filtered, and concentrated under reduced pressure to dryness, to afford compound 50b. MS m/z (ESI): 141.1 [M+1].

Step 2: Compound 50b (137.43 mg, 0.49 mmol, purity: 50%) was dissolved in N,N-dimethylformamide (2 mL). Then, compound Int-1 (100 mg, 0.24 mmol) and N,N-diisopropylethylamine (120.70 μL, 0.73 mmol) were added, respectively, followed by stirring at 80°C for 1 hour. The reaction solution was purified by high performance liquid chromatography (chromatographic column: Waters-CORTECS-C18-2.7μm-4.6*30mm; mobile phase: water (containing 10 mmol $NH_4HCO_3$) and acetonitrile , gradient ratio: acetonitrile 46%-76%, flow rate: 25 mL/min) to afford compound 50. MS m/z (ESI): 516.3 [M+1]. $^1H$ NMR (400 MHz, DMSO-$d_6$): δ 8.58 (s, 1H), 8.13 (s, 1H), 7.28 (s, 1H), 6.71 (dd, *J* = 8.8, 2.8 Hz, 1H), 6.43 (d, *J* = 2.8 Hz, 1H), 3.85 - 3.65 (m, 4H), 3.07 (t, *J* = 5.0 Hz, 4H), 2.45 - 2.34 (m, 6H), 2.21 - 2.14 (m, 7H), 1.95 - 1.87 (m, 1H), 1.63 - 1.41 (m, 4H), 1.24 (s, 1H), 0.84 - 0.76 (m, 2H), 0.63 - 0.55 (m, 2H).

**Example 51. Preparation of compound 51**

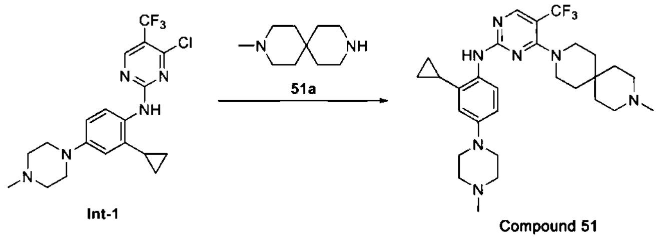


Int-1 Compound 51

Compound Int-1(100 mg, 0.24 mmol) and 51a (40.86 mg, 0.24 mmol, Bide) were dissolved in N,N- dimethylformamide (1 mL). N,N-diisopropylethylamine (62.64 mg, 0.49 mmol) was added to the reaction solution, and the reaction was carried out at room temperature for 1 hour. The reaction solution was purified by high performance liquid chromatography Gilson_306_1741, chromatographic column: Waters-SunFire-C18-10 μm-19*250mm; mobile phase: water (containing 10mM ammonium bicarbonate) and acetonitrile , gradient ratio: acetonitrile 5%-60%, flow rate: 20 mL/min) to afford compound 51. MS m/z (ESI): 544.4 [M+1]. $^1H$ NMR (400 MHz, DMSO-d6): δ 8.76 (s, 1H), 8.24 (s, 1H), 7.21 (s, 1H), 6.71 (dd, J = 8.8, 2.8 Hz, 1H), 6.41 (d, J = 2.8 Hz, 1H), 3.43 (s, 4H), 3.08 (t, J = 4.8 Hz, 4H), 2.44 (t, J = 5.0 Hz, 6H), 2.25 (d, J = 26.4 Hz, 7H), 1.94-1.90 (m, 1H), 1.47 (d, J = 27.2 Hz, 9H), 0.86 - 0.78 (m, 2H), 0.59 (dt, J = 5.4, 2.8 Hz, 2H).

**Example 52. Preparation of compound 52**

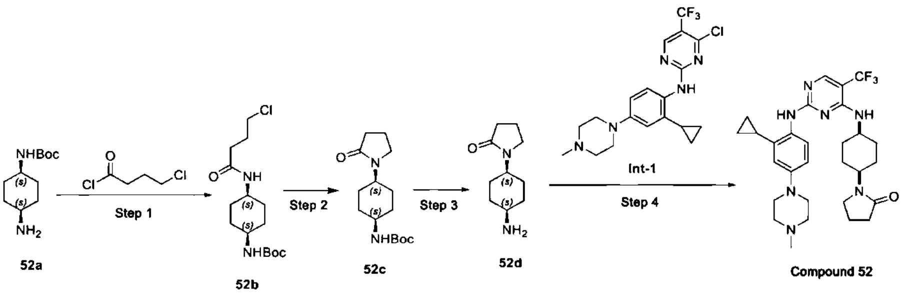


Step 1: At 0°C, 4-chlorobutyryl chloride (0.63 mL, 5.60 mmol) was added to a solution of compound 52a (1 g, 4.67 mmol, Bide) in dichloromethane (8 mL). Then, the mixed solution was warmed to 25°C and stirred for 2 hours. The mixture was poured into water (30 mL) , followed by extraction with ethyl acetate (20 mL×3). The combined organic phase was washed with brine (50 mL), dried over anhydrous sodium sulfate, filtered and concentrated under reduced pressure to dryness, to afford compound 52b. MS m/z (ESI): 319.8 [M+1].

Step 2: At 25°C, compound 52b (500 mg, 1.57 mmol) and potassium tert-butoxide solution (4.71 mL,4.71 mmol, C=1M) were dissolved in tetrahydrofuran (5 mL) solution. The mixed solution was purged with $N_2$ for 3 times, and stirred at 25°C for 2 hours under $N_2$. The mixed solution was poured into water (30 mL) , followed by extraction with ethyl acetate (15 mL×3). The combined organic phase was washed with brine (50 mL), dried over anhydrous sodium sulfate, filtered and concentrated under reduced pressure to dryness, to afford the crude product. The crude product was purified by silica gel chromatography (elution solution: PE:THF=1:1-1:5) to afford compound 52c. MS m/z (ESI): 283.3 [M+1].

Step 3: At 25°C, compound 52c (300 mg, 1.06 mmol) and HCl (3 mL, 12.00 mmol, c=4M) were dissolved in tetrahydrofuran (5 mL) solution, followed by stirring at 25°C for 2 hours under $N_2$. The mixed solution was filtered and the filter cake was washed with ethyl acetate (20 mL x3). The filtrate was concentrated under reduced pressure to dryness to afford compound 52d. MS m/z (ESI): 183.2 [M+1].

Step 4: Compound Int-1 (300 mg, 0.73 mmol) was dissolved in acetonitrile (6 mL) solution. Then, compound 52d (455.20 mg,0.87 mmol) and triethylamine (504.84μL, 3.64 mmol) were added, and the mixed solution was stirred at 60°C for 18 hours. The mixture was concentrated under reduced pressure to afford the crude product. The crude was purified by preparative HPLC (chromatographic column: Waters-Xbridge-C18-10μm-19*250mm; mobile phase: A: 0.1% ammonium bicarbonate/water; B: acetonitrile, gradient ratio: acetonitrile 40-60%, flow rate: 25 mL/min) to afford compound 52. MS m/z (ESI): 558.3 [M+1]. $^1$H NMR (400 MHz, DMSO-$d_6$): δ 8.73 (s, 1H), 8.11 (s, 1H), 7.21 (s, 1H), 6.71 (dd, *J* = 8.8, 2.6 Hz, 1H), 6.42 (d, *J* = 2.6 Hz, 1H), 5.56 (d, *J* = 6.4 Hz, 1H), 4.21 - 3.96 (m, 1H), 3.80 (s, 1H), 3.36 (s, 2H), 3.07 (t, *J* = 5.0 Hz, 4H), 2.43 (t, *J* = 5.0 Hz, 4H), 2.20 (d, *J* = 3.8 Hz, 5H), 1.91 (q, *J* = 7.4, 7.0 Hz, 5H), 1.67 - 1.51 (m, 4H), 1.45 (t, *J* = 11.8 Hz, 2H), 0.82 - 0.79 (m, 2H), 0.66 - 0.53 (m, 2H).

**Example 53. Preparation of compound 53**

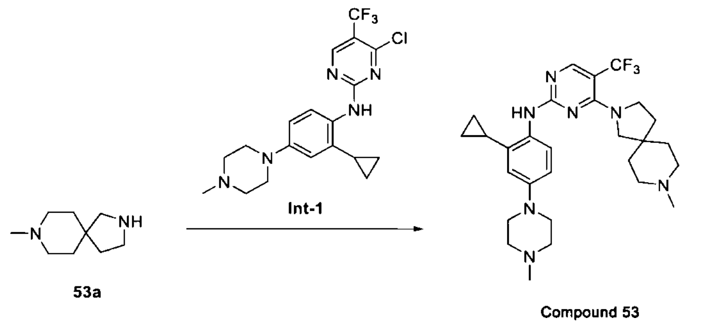


Triethylamine (201.94 μl, 1.46 mmol) was added to a solution of compound 53a (82.40 mg, 0.53 mmol, Bide) in acetonitrile (4 mL). Then, Int-1 (200 mg, 0.49 mmol) was added, and the mixture was stirred at 90°C for 1 hour under $N_2$ atmosphere. The mixture was concentrated under reduced pressure to afford the crude product. The crude product was purified by preparative HPLC (chromatographic column: Waters-Xbridge-C18-10 μm-19*250 mm; mobile phase: A: 10 mM ammonium bicarbonate/water; B: acetonitrile, gradient ratio: acetonitrile 40-60%, flow rate: 25 mL/min) to afford compound 53. MS m/z (ESI): 530.3 [M+1]. $^1$H NMR (400 MHz, DMSO-$d_6$): δ 8.59 (s, 1H), 8.20 (s, 1H), 7.28 (s, 1H), 6.71 (dd, *J* = 8.8, 2.8 Hz, 1H), 6.43 (d, *J* = 2.8 Hz, 1H), 3.58 - 3.50 (m, 2H), 3.07 (t, *J* = 5.0 Hz, 4H), 2.47 - 2.32 (m, 7H), 2.25 - 2.11

(m, 8H), 1.96 - 1.90 (m, 1H), 1.78 - 1.72 (m, 2H), 1.53 - 1.46 (m, 4H), 1.26-1.23 (m, 1H), 0.85 - 0.79 (m, 2H), 0.61 - 0.56 (m, 2H).

**Example 54. Preparation of compound 54**

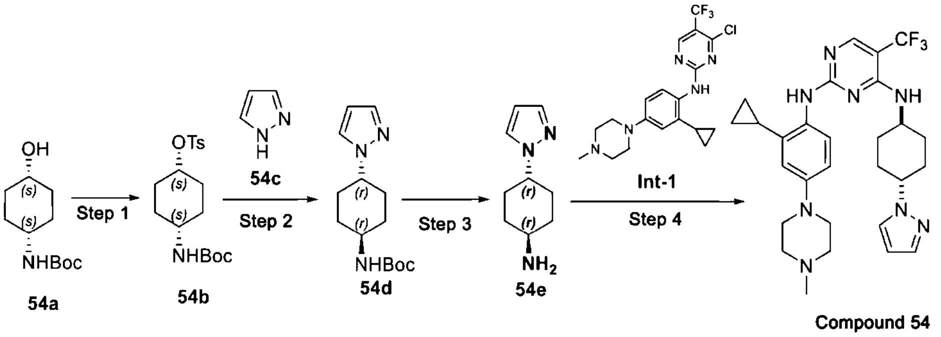


54a 54b 54d 54e Compound 54

Step 1: Compound 54a (1 g, 4.64 mmol, Bide), triethylamine (1.29 mL, 9.29 mmol) and 4-dimethylaminopyridine (0.28 g, 2.32 mmol) were dissolved in dichloromethane (10 mL). P-toluenesulfonyl chloride (1.06 mL, 5.57 mmol) was added, followed by stirring at room temperature for 18 hours. The reaction solution was concentrated to dryness to afford a crude. The crude was purified by silica gel column chromatography (eluant system: dichloromethane : methanol = 100:1 to 5:1) to afford compound 54b. MS m/z (ESI): 370.5 [M+1].

Step 2: Compound 54b (1 g, 2.71 mmol) and compound 54c (0.18 g, 2.71 mmol, Bide) were dissolved in N,N-dimethylformamide (1 mL). Tetrabutylammonium bromide (0.09 g, 0.27 mmol) and cesium carbonate (1.76 g, 5.41 mmol) were added, followed by stirring at 80°C for 18 hours under a nitrogen atmosphere. The reaction solution was poured into water (20 mL), followed by extraction with ethyl acetate (10 mL×3). The organic phases were combined, washed with saturated saline (20 mL), dried over anhydrous sodium sulfate, filtered, and concentrated to dryness to afford the crude. The crude was purified by silica gel column chromatography (eluant system: petroleum ether : dichloromethane = 10:1 to 3:1) to afford compound 54d. MS m/z (ESI): 266.3 [M+1].

Step 3: Compound 54d (212 mg, 0.80 mmol) was dissolved in hydrochloric acid/ethyl acetate (3 mL), followed by stirring at room temperature for 2 hours. Compound 54e was afforded by concentration to dryness. MS m/z (ESI): 166.2 [M+1].

Step 4: Compound Int-1 (250 mg, 0.61 mmol) and compound 54e (200 mg, 1.21 mmol) were dissolved in N,N-dimethylformamide (5 mL), and N,N-diisopropylethylamine (0.40 mL, 2.43 mmol) was added at room temperature. The reaction was carried out at 90°C under a nitrogen atmosphere for 1 hour. The reaction solution was purified by high performance liquid chromatography Gilson_306_1741, chromatographic column: Waters-SunFire-C18-10μm-19*250mm; mobile phase: water (containing 0.1% formic acid) and acetonitrile, gradient ratio: acetonitrile 1%-95%, flow rate: 20 mL/min) to afford compound 54. MS m/z (ESI): 541.4 [M+1]. $^1H$ NMR (400 MHz, DMSO-d6): δ 8.69 (s, 1H), 8.07 (s, 1H), 7.71 (d, J = 2.2 Hz, 1H), 7.41 (d, J = 1.8 Hz, 1H), 7.20 (d, J = 8.6 Hz, 1H), 6.71 (dd, J = 8.6, 2.6 Hz, 1H), 6.41 (d, J = 2.6 Hz, 2H), 6.21 (t, J = 2.0 Hz, 1H), 4.06 (s, 1H), 3.04 (t, J = 5.0 Hz, 4H), 2.41 (t, J = 5.0 Hz, 4H), 2.21 (s, 3H), 1.97 (dd, J = 13.0, 4.2 Hz, 3H), 1.84 (s, 2H), 1.62 (d, J = 10.2 Hz, 4H), 1.24 (s, 1H), 0.87 - 0.80 (m, 2H), 0.60 (dd, J = 5.4, 1.8 Hz, 2H).

**Example 55. Preparation of compound 55**

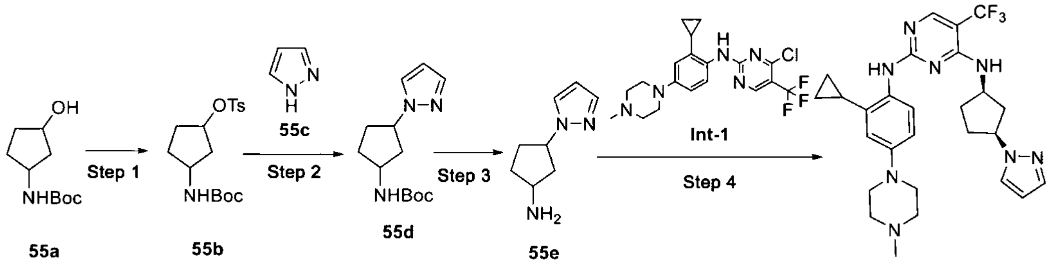


55a 55b 55d 55e Compound 55

Step 1: Compound 55a (500 mg, 2.48 mmol, Bide), triethylamine (0.69 mL, 4.97 mmol) and 4-dimethylaminopyridine (151.75 mg, 1.24 mmol) were dissolved in dichloromethane (5 mL). P-toluenesulfonyl chloride (0.57 mL, 2.98 mmol) was added, followed by stirring at room temperature for 18 hours. The reaction solution was concentrated to dryness to afford a crude. The crude was purified by silica gel column chromatography (eluant system: petroleum ether : ethyl acetate = 20:1 to 5:1) to afford compound 55b. MS m/z (ESI): 356.4 [M+1].

Step 2: Compound 55b (280 mg, 0.79 mmol) and compound 55c (53.63 mg, 0.79 mmol, Bide) were dissolved in N,N-dimethylformamide (5 mL). Tetrabutylammonium bromide (24.42 μL, 0.08 mmol) and cesium carbonate (513.32 mg, 1.58 mmol) were added, followed by stirring at 80°C for 3.5 hours under a nitrogen atmosphere. The reaction solution was poured into water (20 mL), followed by extraction with ethyl acetate (10 mL×3). The organic phases were combined, washed with saturated saline (20 mL), dried over anhydrous sodium sulfate, filtered, and concentrated to dryness to afford the crude. The crude was purified by silica gel column chromatography (eluant system : petroleum ether : ethyl acetate = 10:1 to 3:1) to afford compound 55d. MS m/z (ESI): 252.3 [M+1].

Step 3: Compound 55d (100 mg, 0.40 mmol) was dissolved in hydrochloric acid/ethyl acetate (2 mL), followed by stirring at room temperature for 2 hours. The reaction solution was concentrated to dryness to afford the title compound 55e. MS m/z (ESI): 152.2 [M+1].

Step 4: Compound Int-1 (80 mg, 0.19 mmol) and 55e (58.74 mg, 0.39 mmol) were dissolved in N,N-dimethylformamide (5 mL). N,N-diisopropylethylamine (0.12 mL, 0.73 mmol) was added at room temperature, followed by stirring at 90°C under a nitrogen atmosphere for 1 hour. The reaction solution was purified by high performance liquid chromatography Gilson_306_1741, chromatographic column: Waters-SunFire-C18-10μm-19*250mm; mobile phase: water (containing 0.1% formic acid) and acetonitrile, gradient ratio: acetonitrile 1%-95%, flow rate: 20 mL/min) to afford compound 55. MS m/z (ESI): 527.2 [M+1]. $^1$H NMR (400 MHz, Chloroform-d): δ 8.27 (d, J = 7.4 Hz, 1H), 8.11 (s, 1H), 8.00 (d, J = 8.8 Hz, 1H), 7.66 (s, 1H), 7.56 (d, J = 1.8 Hz, 1H), 7.42 (d, J = 2.3 Hz, 1H), 6.80 (dd, J = 8.8, 2.8 Hz, 1H), 6.72 (d, J = 2.8 Hz, 1H), 6.23 (t, J = 2.0 Hz, 1H), 4.86 - 4.77 (m, 2H), 3.29 (t, J = 5.0 Hz, 4H), 2.91 (t, J = 5.0 Hz, 4H), 2.55 (s, 3H), 2.48 - 2.39 (m, 1H), 2.29 (d, J = 8.0 Hz, 1H), 2.21 (d, J = 14.6 Hz, 1H), 2.07-2.00 (m, 3H), 1.88 (d, J = 5.4 Hz, 1H), 1.02-0.98 (m, 2H), 0.70 - 0.60 (m, 2H).

## Biological evaluation

### Test example 1: Experiment on the inhibition of the compound on ULK1 protein activity

1. Test purpose

In this experiment, the inhibitory effect of a compound on the phosphorylation function of ULK1 was detected by ADP-Glo method. The inhibitory effect of the compound of the present disclosure on the ULK1 target was evaluated based on $IC_{50}$.

2. Experimental method

Compound formulation: Using an ECHO (Beckman, ECHO650), the compound was serially diluted 3-fold in DMSO in a 384-well plate (PE, 6007290). A total of 10 concentration points were set, with the maximum concentration of the compound being 10 μM, and 50 nL per well. Positive control wells and negative control wells were added with 50 nL DMSO, respectively.

Reaction process: A 32 nM of ULK1 (Promega, V3521) solution was formulated, and 2.5 μL/well was transferred to the compound test wells and the negative control wells (NC). 2.5 μL/well of 1× Assay buffer was added to the positive control wells (PC). A mixed solution of 100 μM of ATP and 1.86 μM of MBP was formulated, and 2.5 μL/well was

transferred to the reaction plate. After centrifugation, the reaction was initiated and incubated at room temperature for 60 min. After the reaction was completed, 5 μL/well of ADP-Glo Reagent (Promega, V9102) was added to the reaction plate. After centrifugation, the reaction was incubated at room temperature for 40 minutes. Finally, 10 μL/well of Kinase Detection Buffer was added, followed by centrifugation and incubation at room temperature for 30 min. The chemiluminescence value was read using Envision (PE, Envision2105).

Assay buffer: a reaction buffer; and Kinase Detection buffer: a detection buffer, both were taken from the ADP-Glodetection kit, purchased from Promega, model V9102. 3. Data analysis

The inhibition ratio of a compound on ULK1 activity was calculated using the following formula: Inhibition ratio = (sample signal value - negative control signal value)/(positive control signal value - negative control signal value) x 100%. The dose-effect curve was fitted using the log(inhibitor) vs. response -Variable slope of the analysis software GraphPad Prism 8, thereby affording the $IC_{50}$ value of each compound for enzyme activity.

The experimental results of this test example are shown in Table 1 below:

Table 1: $IC_{50}$ of the compounds of the present invention for inhibiting ULK1

| Example | ULK1 $IC_{50}$ (nM) | Example | ULK1 $IC_{50}$ (nM) | Example | ULK1 $IC_{50}$ (nM) |
|---|---|---|---|---|---|
| 1 | A | 16 | C | 32 | A |
| 2 | B | 17 | A | 45 | B |
| 3 | B | 18 | B | 33 | A |
| 4 | B | 19 | A | 46 | A |
| 40 | C | 20 | A | 34 | B |
| 5 | A | 21 | B | 35 | A |
| 6 | C | 22 | C | 47 | A |
| 7 | B | 43 | C | 36 | C |
| 8 | C | 23 | A | 48 | A |
| 9 | B | 24 | A | 37 | A |
| 10 | B | 25 | A | 49 | A |
| 41 | A | 26 | A | 50 | B |
| 42 | C | 27 | B | 38 | A |
| 11 | B | 28 | B | 51 | A |
| 12 | B | 29 | A | 52 | A |
| 13 | B | 30 | A | 39 | A |
| 14 | A | 44 | A | 53 | A |
| 15 | A | 31 | A | | |

Upon testing, the compounds of the present invention have a better inhibitory activity on ULK1. The activities of some compounds of the present invention, measured using the above method, are as shown in Table 1, wherein A represents IC50 < 100 nM; B represents 100 nM < IC50 < 1 μM; and C represents IC50 > 1 μM.

**Test example 2: Experiment on the inhibition of the compound on ATG13 phosphorylation level in MIA PaCa-2 cells**

1. Test purpose

The inhibitory effect of the compound of the present disclosure on ULK1 was evaluated by testing the inhibitory effect of the compound of the present disclosure on ATG13 phosphorylation level in MIA PaCa-2 cells.

2. Experimental method

MIA PaCa-2 cells (ATCC, CRM-CRL-1420) were cultured in a complete medium, namely, a DMEM medium

(Gibco, 11965-092) containing 10% fetal bovine serum (Coming, 35-081-CV), 2.5% horse serum (Gibco, 16050-122) and 1% double antibody (Gibco, 15140-122). The experiments were performed according to the requirements of the kit (Cell Signaling Technology, 17208C).

Cell plating: MIA PaCa-2 at 80% growth confluence were digested, diluted with the complete medium, and added to a 96-well cell culture plate (Costar, 3596) at 20,000 cells per well, with a volume of 90 μL.

Compound incubation: 5 μL of Torin1 and 5 μL of a serially diluted compound were added to each well so that the highest concentration of the compound in the reaction system was 10 μM. 5-fold serial dilution with 8 concentrations was performed, and the final concentration of Torin1 was 100 nM. The DMSO content in the system was 0.1%. The reaction plate was placed in an incubator at 37°C in 5% $CO_2$, and was incubated for 24 h. 10 μL of Torin1 was added to the negative control wells (NC) to a final concentration of 100 nM, with a DMSO content of 0.1%.

Reaction detection: After the incubation was completed, the cell plate was washed with a pre-cooled DPBS (Corning, 21-031-CVC) to wash away the compound and medium. 50 μL of pre-cooled cell lysis solution was added to each well, incubated on ice for 10 min. The cell plate was centrifuged, and the supernatant was transfered to an ELISA reaction plate. Simultaneously, 50 μL of Phospho-Atg13 (ser355) rabbit detection antibody was added, followed by incubation at room temperature under shaking for 60 min. After the incubation was completed, the reaction plate was washed with a washing solution for 4 times. 100 μL of TMB substrate was added for color development reaction, followed by incubation under shaded conditions with shaking for 15 min. Finally, 100 μL of stop solution was added to each well. The reaction was terminated by shaking for 10 s, and the absorbance value at 450 nm and 560 nm were collected within 10 min using Envision (Perkin Elmer, EnVision 2105).

3. Data analysis

The inhibition ratio was calculated by subtracting the absorbance value at 560 nM from the absorbance value at 450 nm for each well. The inhibition ratio formula is as follows: inhibition ratio = (sample signal value - negative control signal value)/(positive control signal value - negative control signal value) x 100%.

The dose-effect curve was fitted using the log (inhibitor) vs. response -Variable slope of Graphpad Prism8, thereby affording the $IC_{50}$ value of each compound for inhibiting ATG13 phosphorylation level.

The experimental results of this test example are shown in Table 2 below:

Table 2: $IC_{50}$ of the compounds of the present invention for inhibiting ATG13 phosphorylation level

| Example | pATG13 ELISA IC50 (nM) | Example | pATG13 ELISA IC50 (nM) | Example | pATG13 ELISA IC50 (nM) |
|---|---|---|---|---|---|
| 1 | B | 20 | C | 30 | C |
| 5 | C | 24 | B | 32 | B |
| 14 | A | 25 | B | 49 | A |
| 15 | B | 26 | B | 51 | C |
| 19 | C | 29 | B | 53 | B |

Upon testing, the compounds of the present invention have a better inhibitory activity on ULK1. The activities of some compounds of the present invention, measured using the above method, are as shown in Table 1, wherein A represents IC50 < 100 nM; B represents 100 nM < IC50 < 1 μM; and C represents IC50 > 1 μM.

**Test example 3: Experiment on the inhibition of the compound on autophagy level in HEK293 GFP-LC3 stably transfected cells**

1. Test purpose

The inhibitory effect of the compound of the present disclosure on intracellular autophagy was evaluated by testing the impact of the compound of the present disclosure on the fluorescence intensity of HEK293 GFP-LC3 stably transfected cells.

2. Experimental method

HEK293 GFP-LC3 stably transfected cells (HEK293, ATCC, CRL-1573) were cultured in a complete medium, namely, a DMEM medium (Gibco, 11965-092) containing 10% fetal bovine serum (Coming, 35-081-CV), 1% double

antibody (Gibco, 15140-122) and 1 μg/mL of Puromycin (Invitrogen- A1113803).

Cell plating: HEK293 GFP-LC3 stably transfected cells at 80% growth confluence were digested, diluted with a medium free of Puromycin, and added to a 96-well cell culture plate (Geriner, 655090) at 12,000 cells per well, with a volume of 90 μL.

Compound incubation: 5 μL of Torin1 and 5 μL of a serially diluted compound were added to each well so that the highest concentration of the compound in the reaction system was 10 μM. 3-fold serial dilution with 8 concentrations was performed, and the final concentration of Torin1 was 100 nM. The DMSO content in the system was 0.1%. The positive control wells (PC) contain DMSO at a final concentration of 0.1%, and the negative control wells (NC) contain 100 nM of Torin1 with 0.1% of DMSO. The reaction plate was placed in an incubator at 37°C in 5% $CO_2$, and was incubated for 24 h.

Cell staining and fixation: The cell plate was taken out, and 10 μL of Hochest33342 nuclear staining reagent (Thermo, H1399) was added to each well, followed by incubation at 37°C in 5% $CO_2$ for 30 min. After the incubation was completed, the cell supernatant was discarded, and 100 μL of pre-cooled 4% paraformaldehyde was added to each well to fix the cells for 15 min. Then, the fixative was discarded, and 100 μL of PBS was added to each well. Images were captured for analysis using a high-content cell imaging analyzer (PE, Operetta CLS), and GFP fluorescence signals were collected for data processing.

3. Data analysis

The average GFP fluorescence signal intensity in the reaction wells was collected for the calculation of the inhibition ratio:

Inhibition ratio = (sample signal value - negative control signal value)/(positive control signal value - negative control signal value) x 100%.

The dose-effect curve was fitted using the log(inhibitor) vs. response -Variable slope of Graphpad Prism8, thereby affording the $IC_{50}$ value of each compound for inhibiting autophagy level in HEK293 GFP-LC3 stably transfected cells.

The experimental results of this test example are shown in Table 3 below:

Table 3: $IC_{50}$ of the compounds of the present invention for inhibiting autophagy level in HEK293 GFP-LC3 stably transfected cells

| Example | Autophagic Flux IC50 (nM) | Example | Autophagic Flux IC50 (nM) | Example | Autophagic Flux IC50 (nM) |
|---|---|---|---|---|---|
| 1 | B | 20 | C | 32 | C |
| 5 | C | 24 | B | 49 | A |
| 14 | C | 26 | C | 51 | B |
| 15 | B | 29 | B | 53 | C |
| 19 | B | 30 | C | | |

Upon testing, the compounds of the present invention have a better inhibitory activity on ULK1. The activities of some compounds of the present invention, measured using the above method, are as shown in Table 1, wherein A represents IC50 < 100 nM; B represents 100 nM < IC50 < 1 μM; and C represents IC50 > 1 μM.

It should be noted that the present application is not limited to the above embodiments. The above embodiments are merely exemplary. Embodiments with the same composition as the essence of the technical concept and exerting the same effect within the scope of the technical solution of the present application are all included in the technical scope of the present application. Furthermore, within the scope of not departing from the gist of the present application, various modifications that can be conceived by those skilled in the art to the embodiments, and other modes constructed by combining some of the constituent elements in the embodiments, are also included within the scope of the present application.

## Claims

1. A compound of formula 1 or a pharmaceutically acceptable salt, a stereoisomer, a tautomer, a polymorph, a solvate, a

hydrate, an N-oxide, an isotope labeled compound, a metabolite, an ester, or a prodrug thereof:

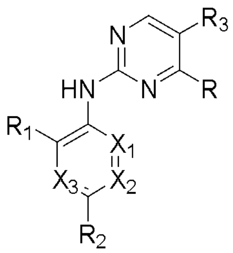

(1)

wherein $X_1$ is CH or N; $X_2$ is N or $CR_4$; $X_3$ is N or $CR_5$;

$R_1$ is selected from H, halogen, cyano, nitro, $C_1$-$C_6$ alkyl, $C_2$-$C_6$ alkenyl, $C_2$-$C_6$ alkynyl, $C_1$-$C_6$ alkoxy, $C_3$-$C_{10}$ cycloalkyl, 3-12-membered heterocyclyl, $C_6$-$C_{10}$ aryl, and 5-10-membered heteroaryl, wherein each of the $C_1$-$C_6$ alkyl, $C_2$-$C_6$ alkenyl, $C_2$-$C_6$ alkynyl, $C_1$-$C_6$ alkoxy, $C_3$-$C_{10}$ cycloalkyl, 3-12-membered heterocyclyl, $C_6$-$C_{10}$ aryl, or 5-10-membered heteroaryl is optionally substituted by one or more $R_6$;

$R_2$ is selected from H, halogen, cyano, nitro, $C_2$-$C_6$ alkenyl, $C_2$-$C_6$ alkynyl, $C_3$-$C_{10}$ cycloalkyl, 3-12-membered heterocyclyl, $C_6$-$C_{10}$ aryl, 5-10-membered heteroaryl, $R_7O$-, $-SR_7$, $-NR_7R_8$, $-NHCONR_7R_8$, $-NHCOR_7$, $-CONR_7R_8$, and $-SO_2R_7$, wherein each of the $C_2$-$C_6$ alkenyl, $C_2$-$C_6$ alkynyl, $C_3$-$C_{10}$ cycloalkyl, 3-12-membered heterocyclyl, $C_6$-$C_{10}$ aryl, or 5-10-membered heteroaryl is optionally substituted by one or more $R_6$;

$R_3$ is selected from H, cyano, halogen, $C_1$-$C_6$ alkyl, $C_1$-$C_6$ hydroxyalkyl, $C_1$-$C_6$ aminoalkyl, $C_1$-$C_6$ haloalkyl, $C_1$-$C_6$ heteroalkyl, $C_2$-$C_6$ alkynyl, and $C_3$-$C_{10}$cycloalkyl;

$R_4$ is selected from H, halogen, cyano, nitro, $C_1$-$C_6$ alkyl, $C_2$-$C_6$ alkenyl, $C_2$-$C_6$ alkynyl, $C_3$-$C_{10}$ cycloalkyl, 3-12-membered heterocyclyl, $C_6$-$C_{10}$ aryl, 5-10-membered heteroaryl, $R_7O$-, $-NR_7R_8$, and $-NHCONR_7R_8$, wherein each of the $C_1$-$C_6$ alkyl, $C_2$-$C_6$ alkenyl, $C_2$-$C_6$ alkynyl, $C_3$-$C_{10}$ cycloalkyl, 3-12-membered heterocyclyl, $C_6$-$C_{10}$ aryl, or 5-10-membered heteroaryl is optionally substituted by one or more $R_6$;

or, $R_2$ and $R_4$ together with the groups that they are attached to, form 3-12-membered monocyclic or spiro heterocycle, $C_6$-$C_{10}$ aromatic ring, or 5-10-membered heteroaromatic ring, wherein each of the 3-12-membered monocyclic or spiro heterocycle, $C_6$-$C_{10}$ aromatic ring, or 5-10-membered heteroaromatic ring is substituted by one or more $R_6$;

$R_5$ is selected from H, $C_1$-$C_6$ alkyl, $C_2$-$C_6$ alkenyl, $C_2$-$C_6$ alkynyl, $C_3$-$C_{10}$ cycloalkyl, 3-12-membered heterocyclyl, $C_6$-$C_{10}$ aryl, and 5-10-membered heteroaryl;

or, $R_1$ and $R_5$ together with the groups that they are attached to, form 5-6-membered heterocycle, benzene ring, or 5-6-membered heteroaromatic ring;

each $R_6$ is independently selected from halogen, cyano, nitro, amino, $-NHCO(C_1$-$C_6$ alkyl), $C_1$-$C_6$ alkyl, $C_2$-$C_6$ alkenyl, $C_2$-$C_6$ alkynyl, $C_3$-$C_{10}$ cycloalkyl, 3-12-membered heterocyclyl, $C_6$-$C_{10}$ aryl, and 5-10-membered heteroaryl, wherein each of the $C_3$-$C_{10}$ cycloalkyl, 3-12-membered heterocyclyl, $C_6$-$C_{10}$ aryl, or 5-10-membered heteroaryl is optionally substituted by one or more halogen, hydroxyl, cyano, $C_1$-$C_6$ alkyl, $C_1$-$C_6$ alkoxy;

$R_7$, $R_8$ are each independently selected from H, $C_1$-$C_6$ alkyl, $C_3$-$C_{10}$ cycloalkyl, 3-12-membered heterocyclyl, and $C_6$-$C_{10}$ aryl, wherein each of the $C_1$-$C_6$ alkyl, $C_3$-$C_{10}$ cycloalkyl, 3-12-membered heterocyclyl, or $C_6$-$C_{10}$ aryl is optionally substituted by one or more $R_6$;

or $R_7$ and $R_8$ together with the groups that they are attached to, form 5-6-membered heterocycle or 5-6-membered heteroaromatic ring;

R is selected from 3-12-membered heterocyclyl, 5-10-membered heteroaryl, and $-L-R_9$, wherein each of the 3-12-membered heterocyclyl or 5-10-membered heteroaryl is optionally substituted by one or more $R_{10}$;

L is $-(L_1)_m-(L_2)_n-(L_3)_p-$;

$L_1$ is selected from -O-, $-N(R_{12})$-, $-C(R_{12})(R_{13})-O$-, $-N(R_{12})CO$-, and -S-;

$L_2$ is selected from $C_1$-$C_6$ alkylene, $C_1$-$C_6$ heteroalkylene, $C_3$-$C_{10}$ cycloalkylene, and 3-10-membered heterocyclylene;

$L_3$ is selected from $-N(R_{12})$-, $-N(R_{12})C(O)$-, and -C(O)-;

m, n, and p are each independently selected from 0, 1, and 2, and m, n, and p are not simultaneously 0;

$R_9$ is selected from $C_3$-$C_{10}$ cycloalkyl, $C_6$-$C_{10}$ aryl, 5-10-membered heteroaryl, and 3-12-membered heterocyclyl, each of the $C_3$-$C_{10}$ cycloalkyl, $C_6$-$C_{10}$ aryl, 5-10-membered heteroaryl, or 3-12-membered heterocyclyl is optionally substituted by one or more $R_{14}$;

each $R_{10}$ is independently selected from $C_1$-$C_6$ alkyl, oxo, 5-10-membered heteroaryl, 3-12-membered heterocyclyl, $R_{11}C(O)$-, $R_{11}C(O)N(R_{12})$-, and $-NR_{12}R_{13}$, wherein each of the $C_1$-$C_6$ alkyl, 5-10-membered heteroaryl, or 3-12-membered heterocyclyl is optionally substituted by one or more $R_{15}$;

$R_{11}$ is selected from $C_1$-$C_6$ alkyl and $C_3$-$C_{10}$ cycloalkyl;

$R_{12}$ and $R_{13}$ are each independently selected from H, $C_1$-$C_6$ alkyl, and $C_3$-$C_{10}$ cycloalkyl; each $R_{14}$ is independently selected from H, halogen, cyano, oxo, $C_1$-$C_6$ alkyl, $C_1$-$C_6$ alkoxy, -$CONR_{12}R_{13}$, and $R_{11}C(O)$-; and each $R_{15}$ is independently selected from H, halogen, cyano, oxo, $C_1$-$C_6$ alkyl, $C_3$-$C_{10}$ cycloalkyl, and 3-12-membered heterocyclyl, wherein each of the $C_1$-$C_6$ alkyl, $C_3$-$C_{10}$ cycloalkyl, or 3-12-membered heterocyclyl is optionally substituted by one or more substituents selected from $C_1$-$C_6$ alkyl and oxo;

with the proviso that:

(1) when $R_9$ is $C_3$-$C_{10}$ cycloalkyl, $C_6$-$C_{10}$ aryl, or 5-10-membered heteroaryl, then n is not 0;

(2) when L is -$(L_1)_m$-$(L_2)_n$-$(L_3)_p$-, $L_1$ is selected from -$N(R_{12})$-, m is 1, $L_2$ is selected from $C_1$-$C_6$ alkylene, and n is 1, then $L_3$ is not -$N(R_{12})C(O)$-;

(3) when L is -$(L_1)_m$-$(L_2)_n$-$(L_3)_p$-, $L_1$ is selected from -$N(R_{12})$-, m is 1, $L_2$ is selected from $C_1$-$C_6$ alkylene, n is 1, p is 0, and $R_9$ is 3-12-membered heterocyclyl, then the 3-12-membered heterocyclyl is substituted by oxo; and

(4) when L is -$(L_1)_m$-$(L_2)_n$-$(L_3)_p$-, $L_1$ is selected from -$N(R_{12})$-, m is 1, $L_2$ is selected from $C_1$-$C_6$ alkylene, n is 1, p is 0, and $R_9$ is 5-10-membered heteroaryl, then $R_2$ is not morpholinyl.

**2.** The compound or pharmaceutically acceptable salt, stereoisomer, tautomer, polymorph, solvate, hydrate, *N*-oxide, isotope labeled compound, metabolite, ester, or prodrug thereof of claim 1, wherein,
at most one of $X_1$, $X_2$, and $X_3$ is N.

**3.** The compound or pharmaceutically acceptable salt, stereoisomer, tautomer, polymorph, solvate, hydrate, N-oxide, isotope labeled compound, metabolite, ester, or prodrug thereof of claim 1 or 2, wherein,
$R_1$ is selected from H, $C_1$-$C_6$ alkoxy, and $C_3$-$C_6$ cycloalkyl, preferably selected from H, cyclopropyl, and methoxy.

**4.** The compound or pharmaceutically acceptable salt, stereoisomer, tautomer, polymorph, solvate, hydrate, *N*-oxide, isotope labeled compound, metabolite, ester, or prodrug thereof of anyone of claims 1 - 3, wherein,
$R_5$ is H.

**5.** The compound or pharmaceutically acceptable salt, stereoisomer, tautomer, polymorph, solvate, hydrate, *N*-oxide, isotope labeled compound, metabolite, ester, or prodrug thereof of claim 1 or 2, wherein,

$R_1$ and $R_5$ together with the groups that they are attached to, form 5-6-membered heterocycle, preferably 5-6-membered oxygen-containing heterocycle;

preferably,

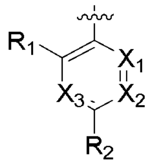

moiety is

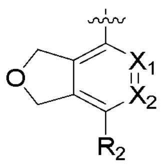 or 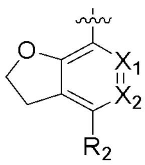,

wherein the wavy line 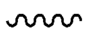 represents the point of attachment of the group to the rest of the molecule.

**6.** The compound or pharmaceutically acceptable salt, stereoisomer, tautomer, polymorph, solvate, hydrate, *N*-oxide, isotope labeled compound, metabolite, ester, or prodrug thereof of anyone of claims 1 - 5, wherein,
$R_3$ is selected from H and $C_1$-$C_6$ haloalkyl, preferably, $R_3$ is selected from $C_1$-$C_6$ haloalkyl, preferably, $R_3$ is selected from H and trifluoromethyl, more preferably, $R_3$ is selected from trifluoromethyl.

**7.** The compound or pharmaceutically acceptable salt, stereoisomer, tautomer, polymorph, solvate, hydrate, *N*-oxide, isotope labeled compound, metabolite, ester, or prodrug thereof of anyone of claims 1 - 6, wherein,

$R_2$ is selected from H, $C_2$-$C_6$ alkynyl, 3-10-membered monocyclic, spiro or fused heterocyclyl, and $R_7O$-, wherein each of the $C_2$-$C_6$ alkynyl or 3-10-membered monocyclic, spiro or fused heterocyclyl is optionally substituted by 1 or 2 $R_6$;
preferably, $R_2$ is selected from H, $C_2$-$C_4$ alkynyl, 3-6-membered monocyclic heterocyclyl, 7-10-membered spiro or fused heterocyclyl, and $R_7O$-, wherein each of the $C_2$-$C_4$ alkynyl, 3-6-membered monocyclic heterocyclyl, 7-10-membered spiro or fused heterocyclyl is optionally substituted by 1 or 2 $R_6$; each $R_6$ is independently selected from $C_1$-$C_6$ alkyl and 3-6-membered heterocyclyl, wherein the 3-6-membered heterocyclyl is optionally substituted by $C_1$-$C_6$ alkyl;
$R_7$ is selected from $C_1$-$C_6$ alkyl and 5-6-membered heterocyclyl, wherein the $C_1$-$C_6$ alkyl is optionally substituted by a 3-6-membered heterocyclyl, each of the 5-6-membered heterocyclyl, 3-6-membered heterocyclyl is optionally substituted by $C_1$-$C_6$ alkyl;
preferably, $R_2$ is selected from H,

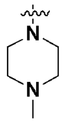, 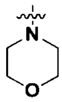, 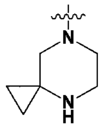, 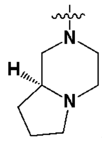, 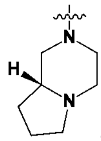, 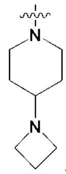, 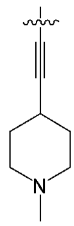,

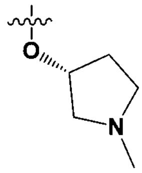, and 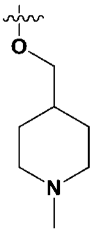,

wherein the wavy line 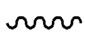 represents the point of attachment of the group to the rest of the molecule.

**8.** The compound or pharmaceutically acceptable salt, stereoisomer, tautomer, polymorph, solvate, hydrate, *N*-oxide, isotope labeled compound, metabolite, ester, or prodrug thereof of anyone of claims 1 - 7, wherein,

$R_4$ is selected from H, $C_1$-$C_6$ alkyl, -$NR_7R_8$, and -$NHCONR_7R_8$, wherein the $C_1$-$C_6$ alkyl is optionally substituted by a 3-6-membered heterocyclyl;
$R_7$ is selected from H, $R_8$ is selected from $C_6$-$C_{10}$ aryl, wherein the $C_6$-$C_{10}$ aryl is optionally substituted by amino, -NHCO($C_1$-$C_6$ alkyl);
or $R_7$ and $R_8$ together with the groups that they are attached to, form 5-6-membered heterocycle;
preferably, $R_4$ is H,

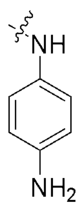, 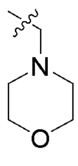, 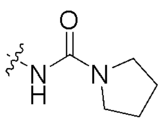, 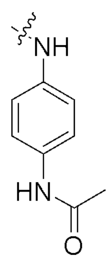;

preferably, $R_4$ is H.

**9.** The compound or pharmaceutically acceptable salt, stereoisomer, tautomer, polymorph, solvate, hydrate, *N*-oxide,

isotope labeled compound, metabolite, ester, or prodrug thereof of anyone of claims 1 - 7, wherein,

$R_2$ and $R_4$ together with the groups that they are attached to, form 6-12-membered nitrogen-containing monocyclic heterocycle or nitrogen-containing spiro heterocycle, each of the 6-12-membered nitrogen-containing monocyclic heterocycle or nitrogen-containing spiro heterocycle is optionally substituted by one or more $R_6$;
preferably, $R_2$ and $R_4$ together with the groups that they are attached to, form 6-12-membered nitrogen-containing monocyclic heterocycle or nitrogen-containing spiro heterocycle, the 6-12-membered nitrogen-containing heterocycle is substituted by 1 or 2 $C_1$-$C_6$ alkyl;
preferably, $R_2$ and $R_4$ together with the groups that they are attached to, form 6-7-membered nitrogen-containing monocyclic heterocycle or 7-12-membered nitrogen-containing spiro heterocycle, each of the 6-7-membered nitrogen-containing monocyclic heterocycle or 7-12-membered nitrogen-containing spiro heterocycle is substituted by 1 or 2 $C_1$-$C_6$ alkyl;
preferably,

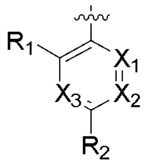

moiety is

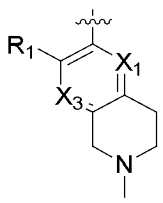
,
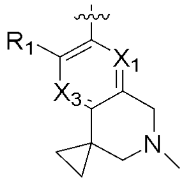
,
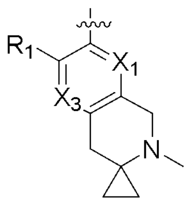
,

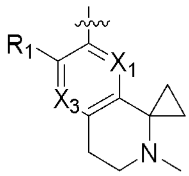
,
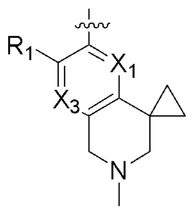
,
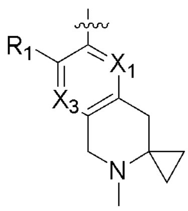
, or
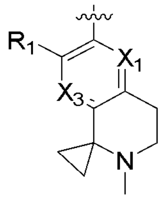
,

preferably

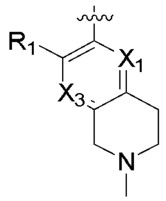
,

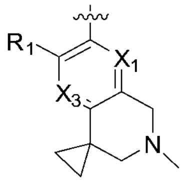
, or
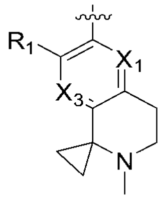
,

more preferably

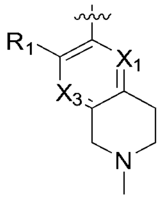 or 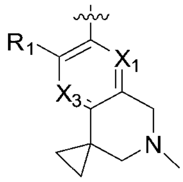 ;

preferably,

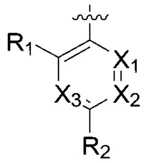

moiety is

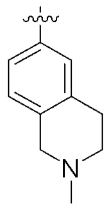 , 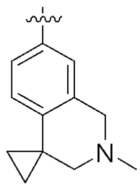 , 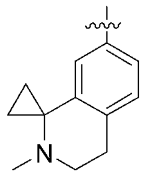 , 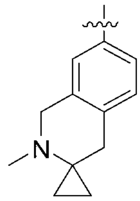 ,

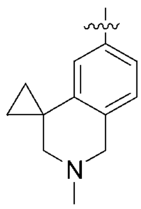 , 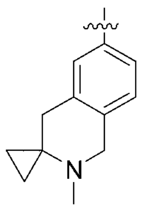 , or 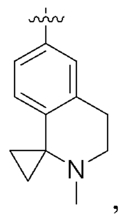 ,

preferably

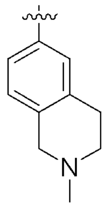 or 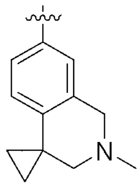 ;

wherein the wavy line 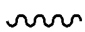 represents the point of attachment of the group to the rest of the molecule.

**10.** The compound or pharmaceutically acceptable salt, stereoisomer, tautomer, polymorph, solvate, hydrate, *N*-oxide, isotope labeled compound, metabolite, ester, or prodrug thereof of anyone of claims 1 - 3, wherein the compound is the compound of formula 1A:

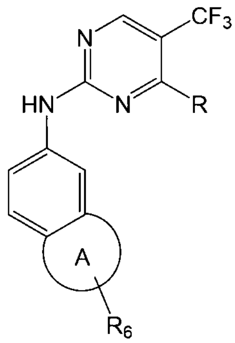

(1A)

wherein, ring A is 6-12-membered nitrogen-containing monocyclic heterocycle or nitrogen-containing spiro heterocycle; and $R_6$ is $C_1$-$C_6$ alkyl,
preferably,

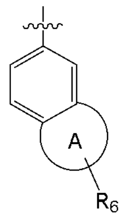

moiety is

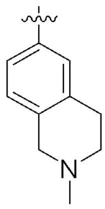 , 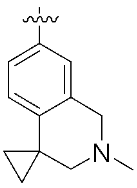 , 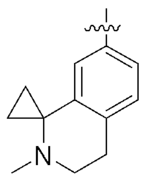 , 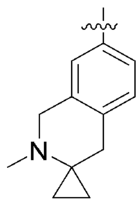 ,

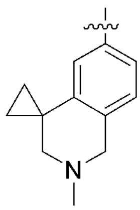 , 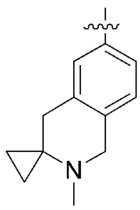 , or 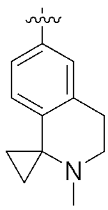 ,

preferably,

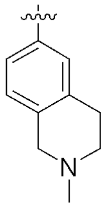 , 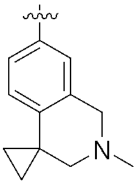 , or 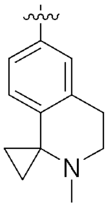 ,

more preferably

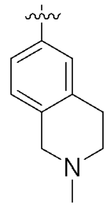 or 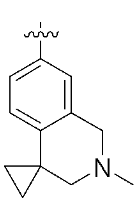 ;

wherein the wavy line 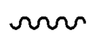 represents the point of attachment of the group to the rest of the molecule.

**11.** The compound or pharmaceutically acceptable salt, stereoisomer, tautomer, polymorph, solvate, hydrate, *N*-oxide, isotope labeled compound, metabolite, ester, or prodrug thereof of anyone of claims 1 - 10, wherein the compound is the compound of formula 1B':

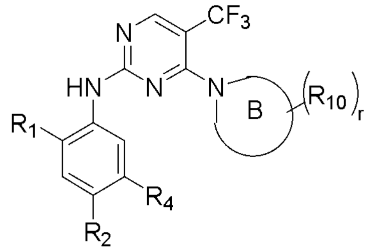

(1B')

wherein, ring B is selected from 3-12-membered heterocyclyl and 5-10-membered heteroaryl, preferably, ring B is selected from 3-12-membered heterocyclyl; and r is selected from 0, 1, and 2.

**12.** The compound or pharmaceutically acceptable salt, stereoisomer, tautomer, polymorph, solvate, hydrate, *N*-oxide, isotope labeled compound, metabolite, ester, or prodrug thereof of claim 11, wherein,
ring B is 5-12-membered spiro heterocyclyl or 5-12-membered bridged heterocyclyl, preferably 7-12-membered spiro heterocyclyl or 7-12-membered bridged heterocyclyl, more preferably

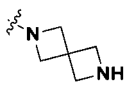 , 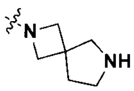 , 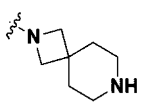 , 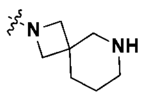 , 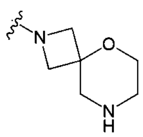 , 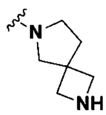 ,

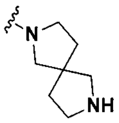 , 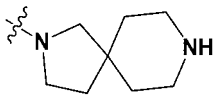 , 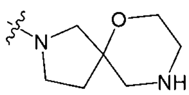 , 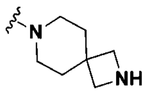 , 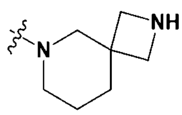 , 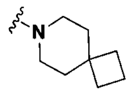 ,

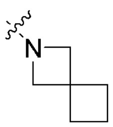 , 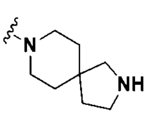 , 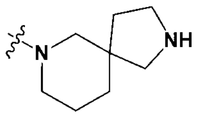 , 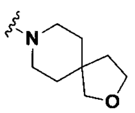 , 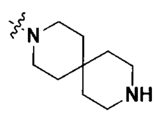 , 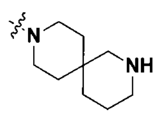 ,

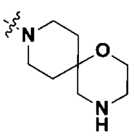 , 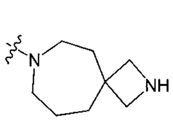 , 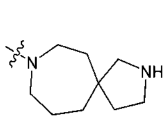 , 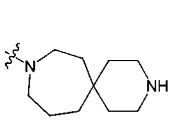 , 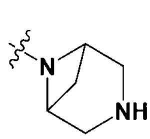 , 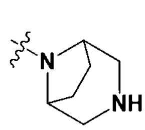 ,

or

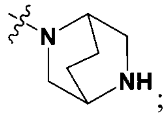

and each $R_{10}$ is independently selected from $C_1$-$C_6$ alkyl, oxo, 3-6-membered heterocyclyl, and $-NR_{12}R_{13}$, preferably, each $R_{10}$ is independently selected from methyl, oxo, amino, and morpholinyl.

**13.** The compound or pharmaceutically acceptable salt, stereoisomer, tautomer, polymorph, solvate, hydrate, N-oxide, isotope labeled compound, metabolite, ester, or prodrug thereof of claim 11, wherein,

ring B is 3-10-membered monocyclic heterocyclyl or 5-10-membered heteroaryl, preferably 4-7-membered monocyclic heterocyclyl or 5-6-membered heteroaryl, more preferably azetidinyl, pyrrolidinyl, piperidinyl, piperazinyl, azepanyl, 1,4-diazepanyl, or pyrazolyl;
preferably, ring B is 3-10-membered monocyclic heterocyclyl, preferably 4-7-membered monocyclic heterocyclyl, more preferably azetidinyl, pyrrolidinyl, piperidinyl, piperazinyl, azepanyl, 1,4-diazepanyl;
each $R_{10}$ is independently selected from $C_1$-$C_6$ alkyl, oxo, 5-6-membered heteroaryl, 4-7-membered heterocyclyl, $R_{11}C(O)$-, and $R_{11}C(O)NH$-, wherein each of the $C_1$-$C_6$ alkyl, 5-6-membered heteroaryl, or 4-7-membered heterocyclyl is optionally substituted by 1-4 $R_{15}$;
$R_{11}$ is $C_4$-$C_6$ cycloalkyl; and
each $R_{15}$ is independently selected from halogen, oxo, $C_1$-$C_6$ alkyl, $C_3$-$C_6$ cycloalkyl, and 3-6-membered heterocyclyl, wherein each of the 3-6-membered heterocyclyl is optionally substituted by one or more substituents selected from $C_1$-$C_6$ alkyl and oxo,
preferably, each $R_{10}$ is independently selected from $C_1$-$C_6$ alkyl, oxo, pyrazolyl, piperidinyl, pyrrolidinyl, azetidinyl, $C_4$-$C_6$ cycloalkyl-C(O)-, and $C_4$-$C_6$ cycloalkyl-C(O)-NH-, wherein each of the azetidinyl, pyrrolidinyl, or piperidinyl is optionally substituted by 1-4 $C_1$-$C_6$ alkyl or oxo, the $C_1$-$C_6$ alkyl is optionally substituted by 1-4 substituent selected from fluorine and $C_3$-$C_6$ cycloalkyl, the pyrazolyl is optionally substituted by 1-4 $C_1$-$C_6$ alkyl;
more preferably, each $R_{10}$ is independently selected from methyl, oxo, pyrazolyl, piperidinyl, 3-methyl-1*H*-pyrazolyl, N-methylazetidinyl, N-methylpiperidinyl, cyclobutylformyl, cyclobutylformylamino, oxopyrrolidinyl, oxopiperidinyl,

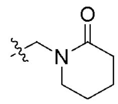

and

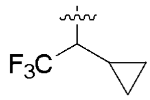

wherein the wavy line 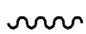 represents the point of attachment of the group to the rest of the molecule.

**14.** The compound or pharmaceutically acceptable salt, stereoisomer, tautomer, polymorph, solvate, hydrate, *N*-oxide, isotope labeled compound, metabolite, ester, or prodrug thereof of anyone of claims 11 - 13, wherein the compound is the compound of formula 1B-1, 1B-2, 1B-3, 1B-4, 1B-5, 1B-A, 1B-B, 1B-C, 1B-D, 1B-E, 1B-F, or 1B-G:

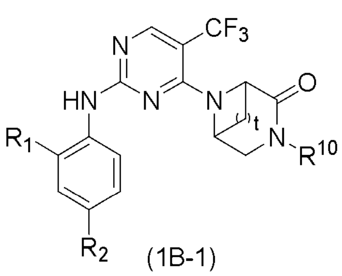

(1B-1) ,

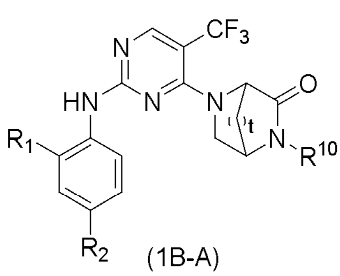

(1B-A) ,

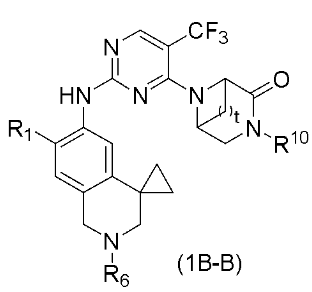

(1B-B) ,

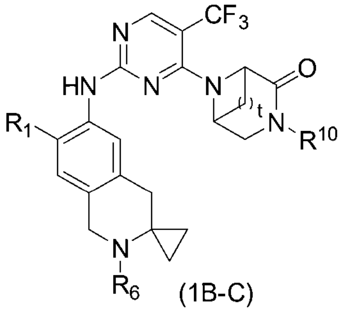
(1B-C) ,
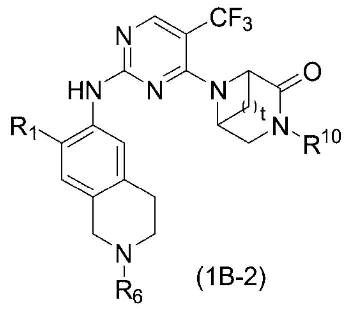
(1B-2) ,
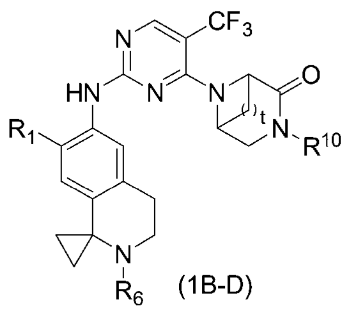
(1B-D) ,
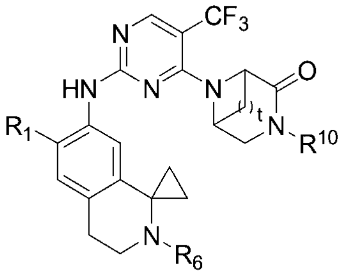
(1B-E) ,
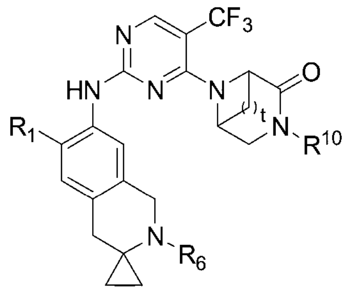
(1B-F) ,
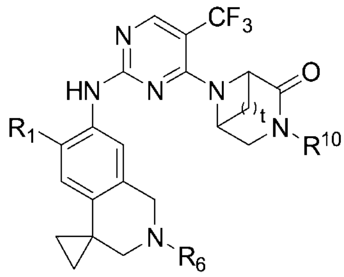
(1B-G) ,
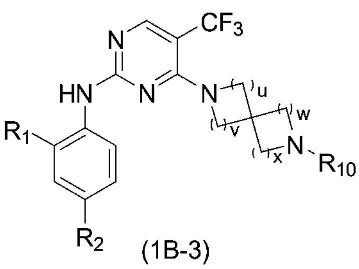
(1B-3) ,
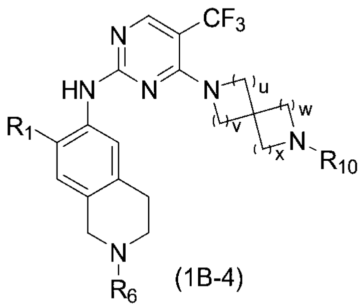
(1B-4) ,
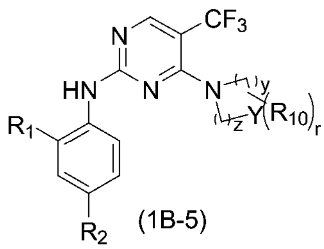
(1B-5) ;

wherein, t is selected from 0, 1, 2, and 3; u, v, w, x, y, and z are each independently selected from 1, 2, and 3; Y is selected from -CH- and -N-; and r is selected from 1 and 2.

**15.** The compound or pharmaceutically acceptable salt, stereoisomer, tautomer, polymorph, solvate, hydrate, *N*-oxide, isotope labeled compound, metabolite, ester, or prodrug thereof of anyone of claims 1 - 10, wherein the compound is the compound of formula 1C:

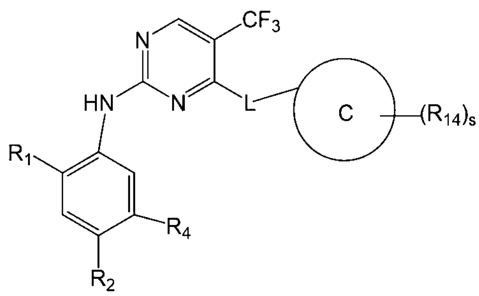
(1C)

wherein, L is $-(L_1)_m-(L_2)_n-(L_3)_p-$;
$L_1$ is selected from -O- and $-N(R_{12})-$, preferably selected from -O- and -NH-;
$L_2$ is selected from $C_1$-$C_6$ alkylene and $C_3$-$C_{10}$ cycloalkylene, preferably selected from $C_1$-$C_6$ alkylene and $C_3$-$C_6$ cycloalkylene, more preferably selected from ethylene, propylene, and cyclohexylidene;

$L_3$ is selected from $-N(R_{12})-$, $-N(R_{12})C(O)-$, and -C(O)-, preferably selected from -NH-, - NHC(O)-, and -C(O)-, preferably, when $L_3$ is -NHC(O)-, then the amino moiety of the - NHC(O)- is attached to $L_2$, and the carbonyl moiety is attached to ring C;

m, n, and p are each independently selected from 0 and 1, and m, n, and p are not simultaneously 0;

preferably, L is selected from -O-, -O-cyclohexylidene -, -NH-cyclohexylidene-, -O-propylene -, -NH-propylene -, -ethylene-, -ethylene-NHC(O)-, and -O-propylene-C(O)-;

ring C is selected from $C_3$-$C_{10}$ cycloalkyl, $C_6$-$C_{10}$ aryl, 5-10-membered heteroaryl, and 3-12-membered heterocyclyl, preferably selected from $C_3$-$C_6$ cycloalkyl, $C_6$-$C_{10}$ aryl, 5-6-membered heteroaryl, and 5-7-membered heterocyclyl, preferably selected from cyclobutyl, phenyl, pyrazolyl, piperidinyl, piperazinyl, pyrrolidinyl, imidazolidinyl, morpholinyl, 1,3-oxazinanyl, azepanyl, 1,4-oxazepanyl, and 5-azaspiro[2.5]octyl;

each $R_{14}$ is independently selected from H, halogen, cyano, oxo, $C_1$-$C_6$ alkyl, $C_1$-$C_6$ alkoxy, $-CONR_{12}R_{13}$, and $R_{11}C(O)-$, preferably, each $R_{14}$ is independently selected from H, fluorine, chlorine, cyano, oxo, methyl, methoxy, $-CONHCH_3$, and cyclobutyl-C(O)-; and

s is selected from 0, 1, and 2.

**16.** The compound or pharmaceutically acceptable salt, stereoisomer, tautomer, polymorph, solvate, hydrate, N-oxide, isotope labeled compound, metabolite, ester, or prodrug thereof of claims 15, wherein the compound is the compound of formula 1C-1, 1C-2, 1C-3, 1C-4, or IC-5:

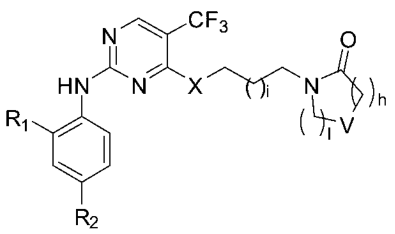

(1C-1),

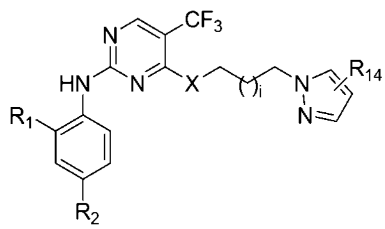

(1C-2),

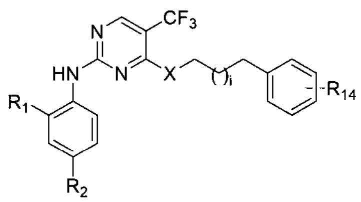

(1C-3),

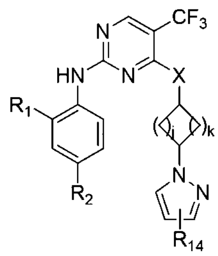

(1C-4),

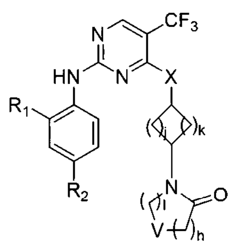

(1C-5);

wherein, X is selected from bond, -O-, -S-, -NH-, and $-CH_2-O-$;

V is selected from -O-, -S-, $-NR_{12}-$, and $-CHR_{12}-$;

$R_{12}$ is selected from H and $C_1$-$C_6$ alkyl;

h and 1 are each independently selected from 0, 1, 2, and 3;

i is selected from 0, 1, and 2; and

j and k are each independently selected from 1, 2, and 3.

**17.** The compound or pharmaceutically acceptable salt, stereoisomer, tautomer, polymorph, solvate, hydrate, N-oxide, isotope labeled compound, metabolite, ester, or prodrug thereof of anyone of claims 1 - 10, wherein the compound is the compound of formula (1D):

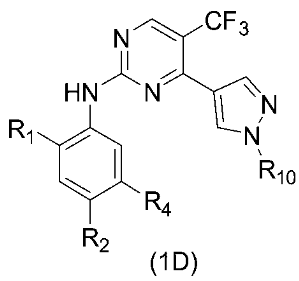

(1D) ,

wherein, $R_{10}$ is as defined in claim 1; preferably, $R_{10}$ is selected from 3-12-membered heterocyclyl, the 3-12-membered heterocyclyl is optionally substituted by one or more $R_{15}$; preferably, $R_{10}$ is selected from 4-7-membered heterocyclyl, the 4-7-membered heterocyclyl is optionally substituted by one or more $R_{15}$; more preferably, $R_{10}$ is selected from 4-7-membered nitrogen-containing heterocyclyl (for example piperidinyl), the 4-7-membered nitrogen-containing heterocyclyl is optionally substituted by one or more $R_{15}$;
$R_{15}$ is as defined in claim 1; preferably, $R_{15}$ is selected from $C_1$-$C_6$ alkyl, more preferably, $R_{15}$ is selected from methyl.

**18.** The compound or pharmaceutically acceptable salt, stereoisomer, tautomer, polymorph, solvate, hydrate, *N*-oxide, isotope labeled compound, metabolite, ester, or prodrug thereof of anyone of claims 1 - 17, wherein:

L is $-(L_1)_m-(L_2)_n-(L_3)_p-*$, wherein the bond marked with "*" is attached to $R_9$;
$L_1$ is selected from -O-, $-N(R_{12})-$, $-C(R_{12})(R_{13})-O-$, and $-N(R_{12})CO-$;
$L_2$ is selected from $C_1$-$C_6$ alkylene, $C_1$-$C_6$ heteroalkylene, $C_3$-$C_{10}$ cycloalkylene, and 3-10-membered heterocyclylene;
$L_3$ is selected from $-N(R_{12})-$, $-N(R_{12})C(O)-$, and -C(O)-;
m, n, and p are each independently selected from 0, 1, and 2, and m, n, and p are not simultaneously 0;
preferably, $L_1$ is selected from -O-, $-N(R_{12})-$, $\#-C(R_{12})(R_{13})-O-$, and $\#-N(R_{12})CO-$, wherein the bond marked with "#" is attached to

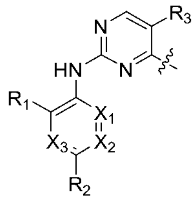
;

preferably, $L_1$ is selected from -O-, $-N(R_{12})-$;
preferably, $L_2$ is selected from $C_1$-$C_6$ alkylene, $C_1$-$C_6$heteroalkylene, $C_3$-$C_6$ cycloalkylene, and 3-6-membered heterocyclylene;
preferably, $L_3$ is selected from $-N(R_{12})-$, $-N(R_{12})C(O)-\$$, and -C(O)-, wherein the bond marked with "$" is attached to $R_9$;
preferably, $-(L_1)_m-(L_2)_n-(L_3)_p-*$ as a whole is selected from $-L_1-L_2-*$, $-L_1-$, $-L_2-$, $-L_1-L_2-L_3-*$, $-L_1-L_3-*$, $-L_2-L_3-*$, wherein the bond marked with "*" is attached to $R_9$;
preferably, $-(L_1)_m-(L_2)_n-(L_3)_p-*$ as a whole is selected from $-L_1-$, $-L_2-$, $-L_1-L_2-*$, $-L_1-L_2-L_3-*$, $-L_2-L_3-*$;
preferably, $-(L_1)_m-(L_2)_n-(L_3)_p-*$ as a whole is selected from $-L_1-$, $-L_1-L_2-*$, $-L_1-L_2-L_3-*$;
more preferably, $-(L_1)_m-(L_2)_n-(L_3)_p-*$ as a whole is selected from $-L_1-L_2-*$, $-L_1-L_2-L_3-*$;
preferably, $-(L_1)_m-(L_2)_n-(L_3)_p-*$ as a whole is selected from $-N(R_{12})-C_3-C_6$ cycloalkylene-*, $-N(R_{12})-$, $-N(R_{12})$-3-6-membered heterocyclylene-C(O)-*, $-N(R_{12})C(O)-*$, $-N(R_{12})-C_1-C_6$ alkylene-*, $-O-C_3-C_6$ cycloalkylene-*, -O-, -O-3-6-membered heterocyclylene-C(O)-*, $-O-C_1-C_6$ alkylene-*, $-O-C_1-C_6$ alkylene-C(O)-*, $-O-C_1-C_6$ alkylene-$N(R_{12})$-*, $-C_1-C_6$ alkylene-, - $C_1-C_6$ alkylene-$N(R_{12})$-C(O)-*, $-C(R_{12})(R_{13})-O-C_1-C_6$ alkylene-*, wherein the bond marked with "*" is attached to $R_9$;
preferably, $-(L_1)_m-(L_2)_n-(L_3)_p-*$ as a whole is selected from -O-, $-O-C_1-C_6$ alkylene-*, - $O-C_1-C_6$ alkylene-C(O)-*, $-N(R_{12})-C_3-C_6$ cycloalkylene-*, $-N(R_{12})$-3-6-membered heterocyclylene-C(O)-*, $-N(R_{12})-C_1-C_6$ alkylene-*, $-N(R_{12})C(O)-*$, $-N(R_{12})-$, $-C_1-C_6$ alkylene-$N(R_{12})$-C(O)-*, $-C_1-C_6$ alkylene-, -O-3-6-membered heterocyclylene-C(O)-*;
preferably, $-(L_1)_m-(L_2)_n-(L_3)_p-*$ as a whole is selected from $-O-C_1-C_6$ alkylene-*, - $N(R_{12})-C_1-C_6$ alkylene-*, $-O-C_1-C_6$ alkylene-C(O)-*, $-N(R_{12})-C_3-C_6$ cycloalkylene-*, - $N(R_{12})$-3-6-membered heterocyclylene-C(O)-*, -O-;
more preferably, $-(L_1)_m-(L_2)_n-(L_3)_p-*$ as a whole is selected from $-O-C_1-C_6$ alkylene-*, - $N(R_{12})-C_1-C_6$ alkylene-*, $-N(R_{12})-C_3-C_6$ cycloalkylene-*, $-N(R_{12})$-3-6-membered heterocyclylene-C(O)-*.

**19.** The compound or pharmaceutically acceptable salt, stereoisomer, tautomer, polymorph, solvate, hydrate, *N*-oxide, isotope labeled compound, metabolite, ester, or prodrug thereof of anyone of claims 1 - 18, with the proviso that:

(1) when $R_9$ is $C_3$-$C_{10}$ cycloalkyl, $C_6$-$C_{10}$ aryl, or 5-10-membered heteroaryl optionally substituted by $R_{14}$, then n is not 0; and/or
(2) when L is $-(L_1)_m-(L_2)_{n-}(L_3)_p-$, $L_1$ is selected from $-N(R_{12})-$, m is 1, $L_2$ is selected from $C_1$-$C_6$ alkylene, and n is 1,

then $L_3$ is not $-N(R_{12})C(O)-$; and/or

(3) when L is $-(L_1)_m-(L_2)_n-(L_3)_p-$, $L_1$ is selected from $-N(R_{12})-$, m is 1, $L_2$ is selected from $C_1$-$C_6$ alkylene, n is 1, p is 0, $R_9$ is 3-12-membered heterocyclyl substituted by $R_{14}$, and at least one $R_{14}$ is an oxo substituent, then $R_2$ is selected from $C_2$-$C_6$ alkynyl substituted by one or more $R_6$, or $R_2$ and $R_4$ together with the groups that they are attached to, form 7-12-membered spiro heterocycle optionally substituted by one or more $R_6$; and/or

(4) when L is $-(L_1)_m-(L_2)_n-(L_3)_p-$, $L_1$ is selected from $-N(R_{12})-$, m is 1, $L_2$ is selected from $C_1$-$C_6$ alkylene, n is 1, p is 0, and $R_9$ is 5-10-membered heteroaryl, then $R_2$ is not morpholinyl; and/or

(5) when L is $-(L_1)_m-(L_2)_n-(L_3)_p-$, $L_1$ is selected from $-N(R_{12})-$, m is 1, n is 0, p is 0, and $R_9$ is a 3-12-membered heterocyclyl, then the 3-12-membered heterocyclyl is a monocyclic ring, and is at least substituted by one oxo group.

**20.** The compound or pharmaceutically acceptable salt, stereoisomer, tautomer, polymorph, solvate, hydrate, *N*-oxide, isotope labeled compound, metabolite, ester, or prodrug thereof of anyone of claims 1 - 19, wherein the compound is selected from:

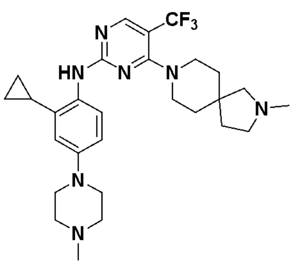
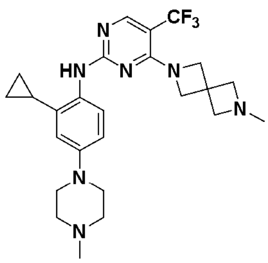
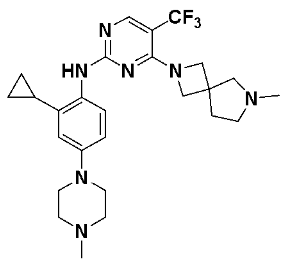
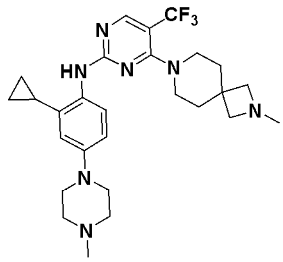

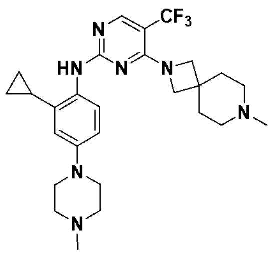
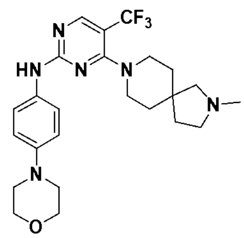
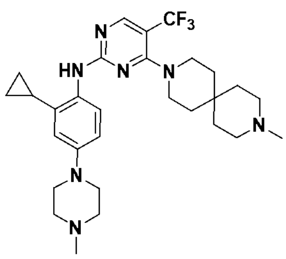
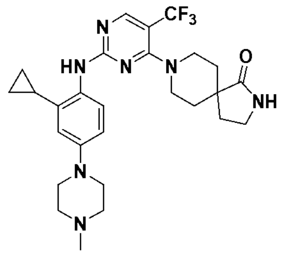

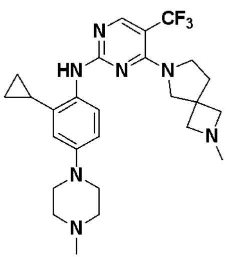
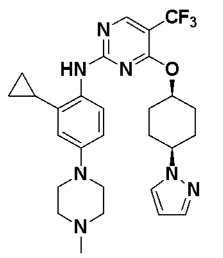
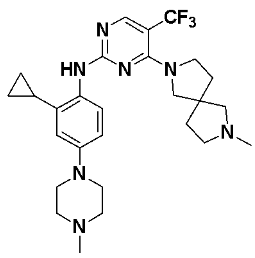
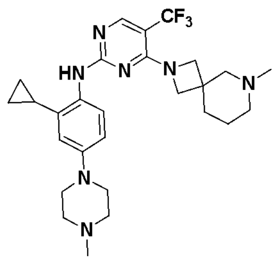

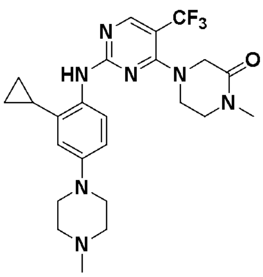
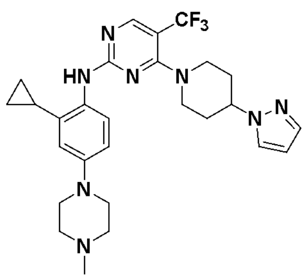
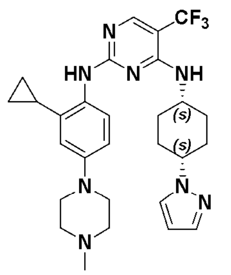
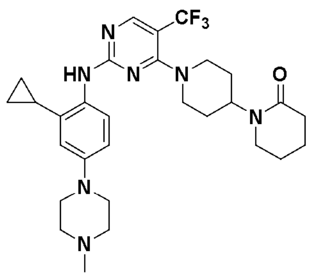

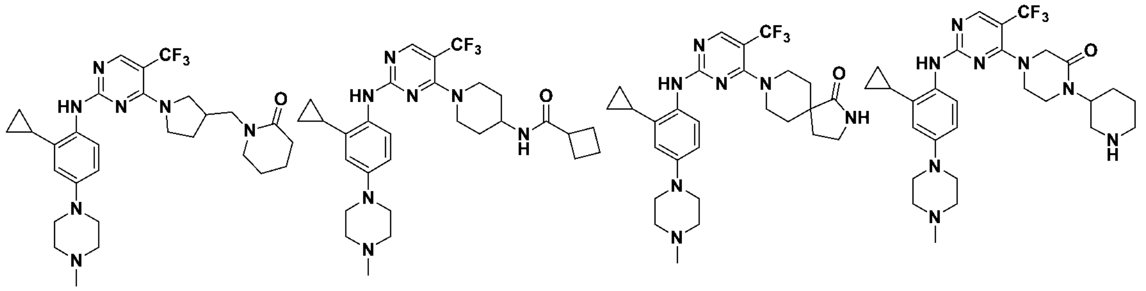

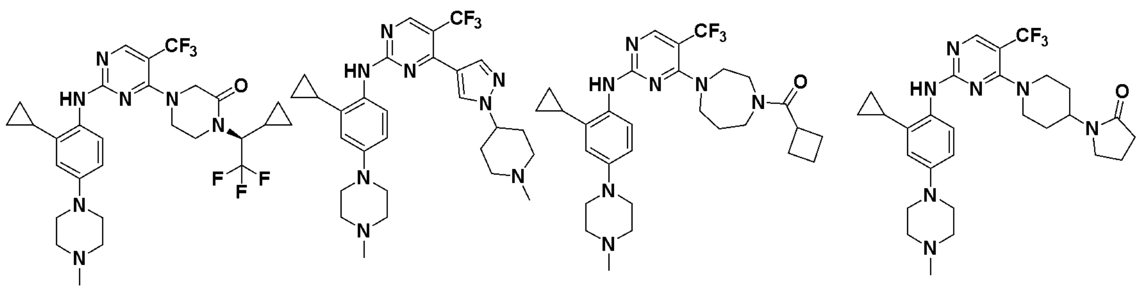

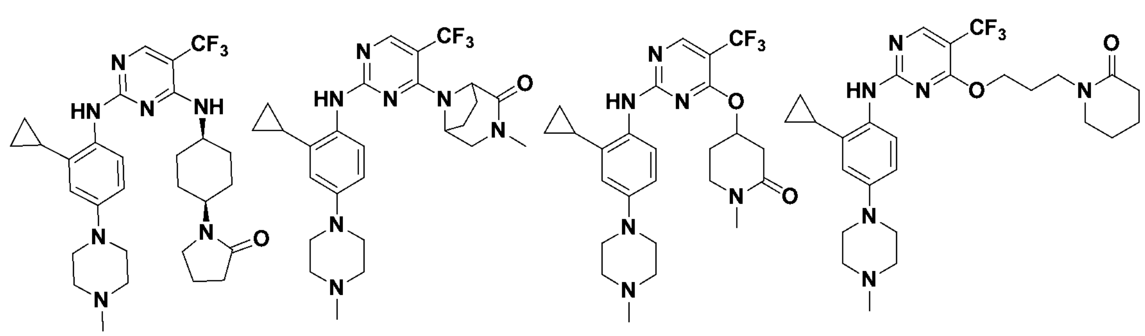

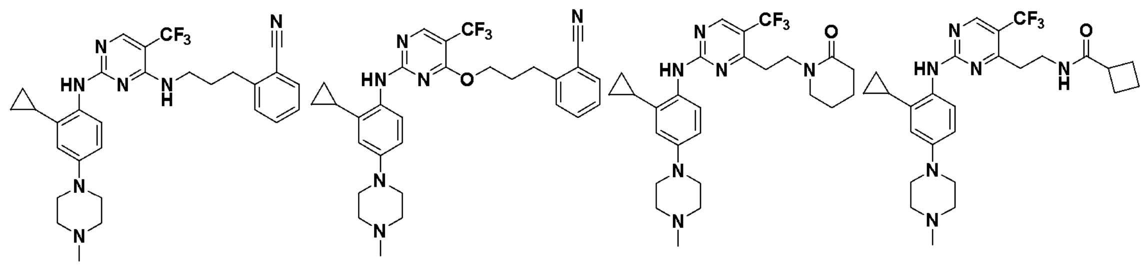

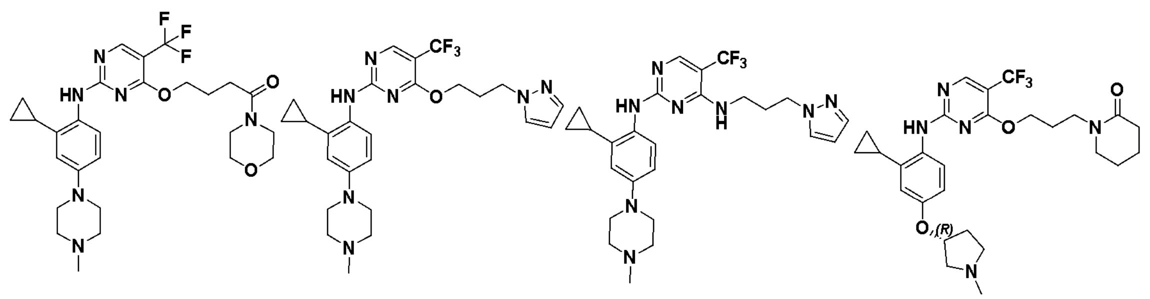

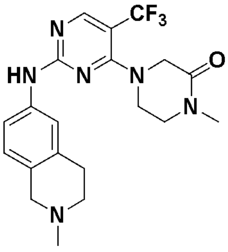
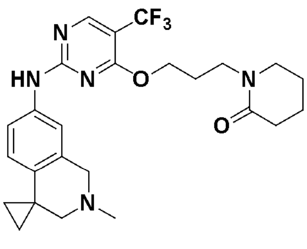
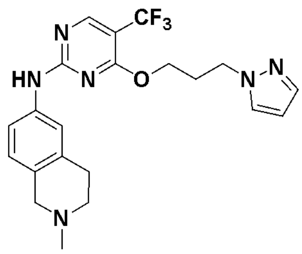
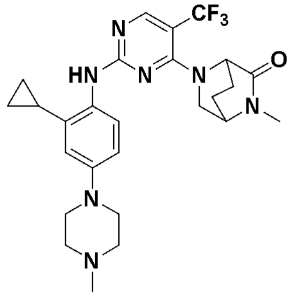

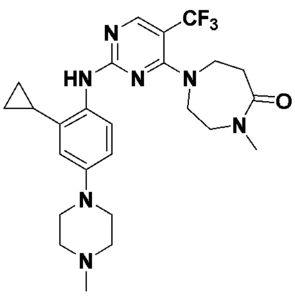
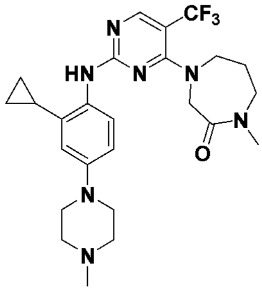
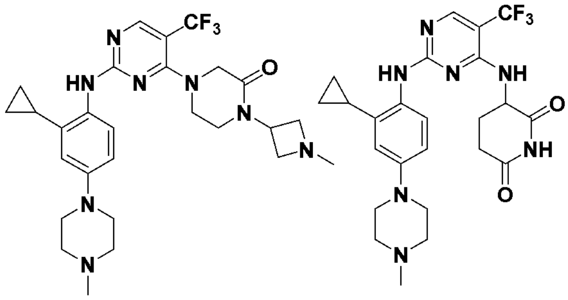

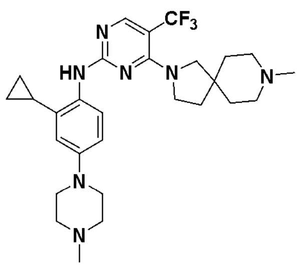
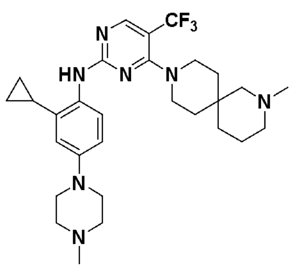
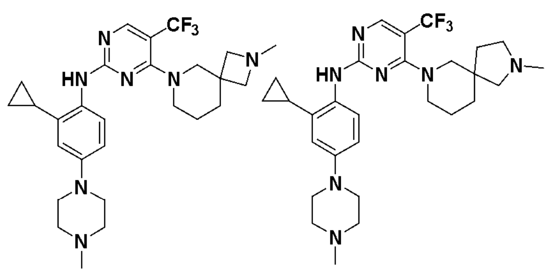

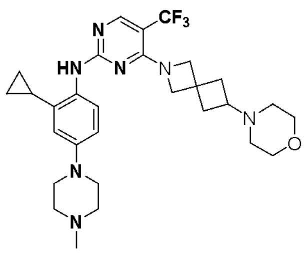
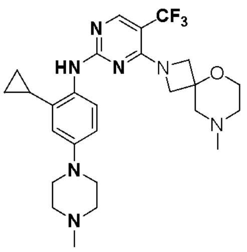
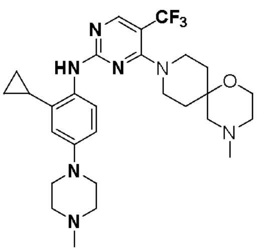
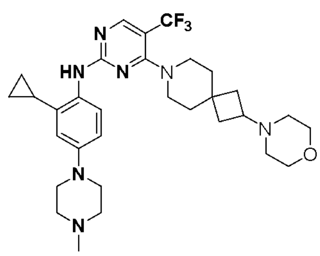

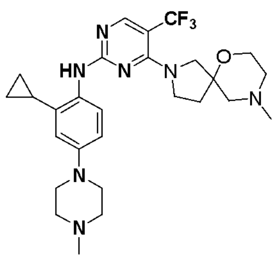
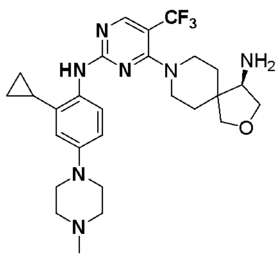
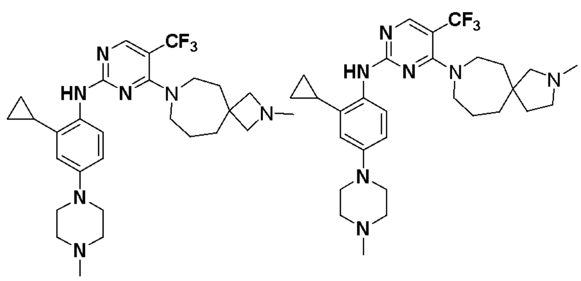

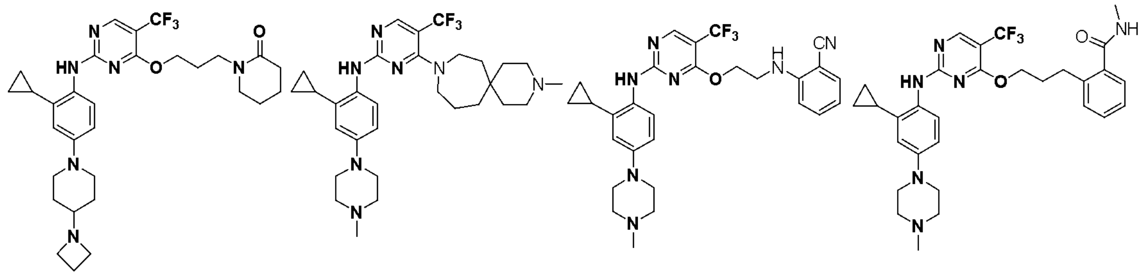

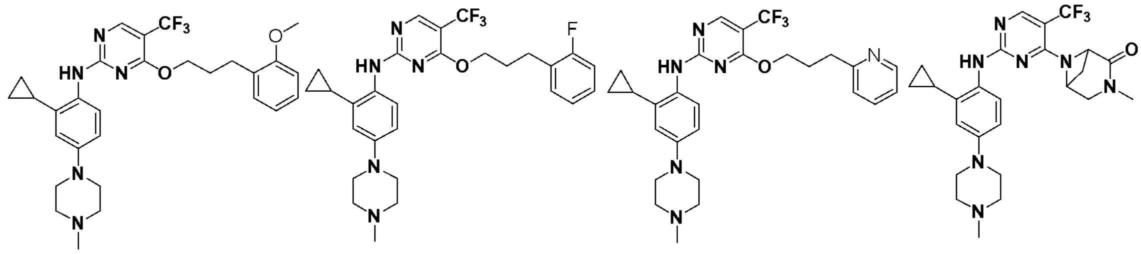

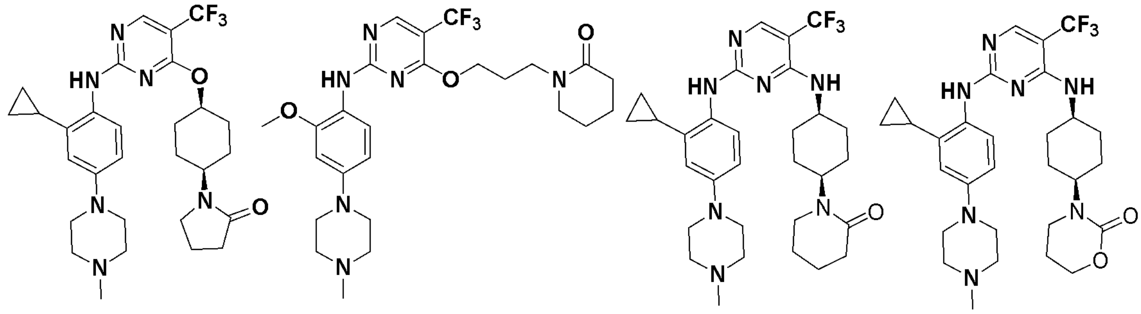

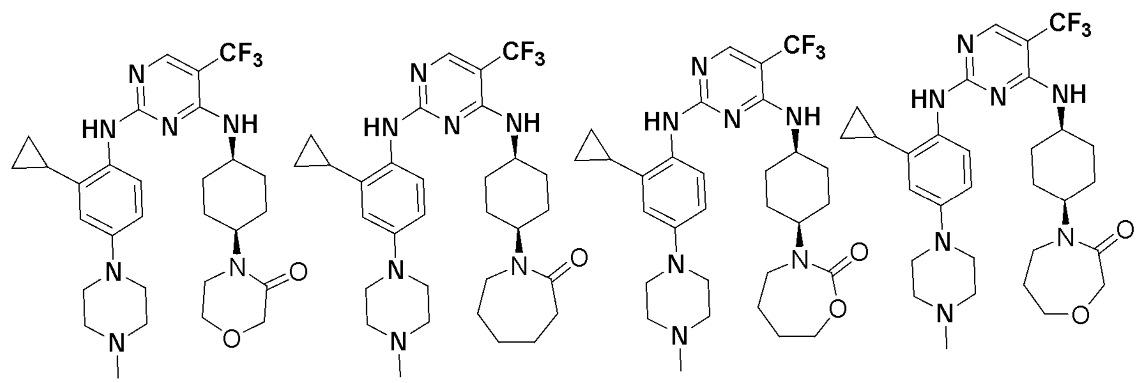

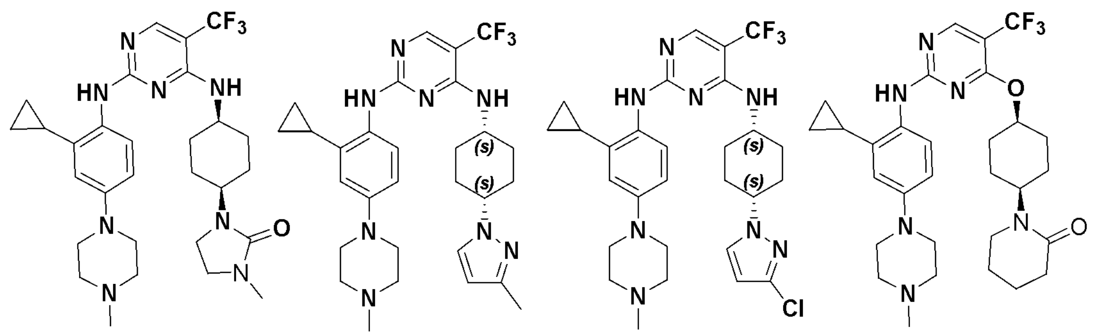

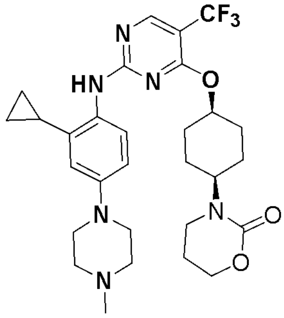

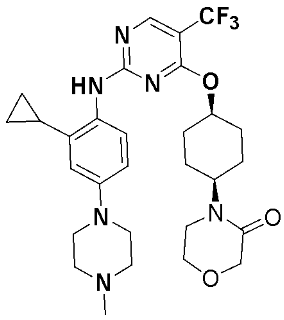

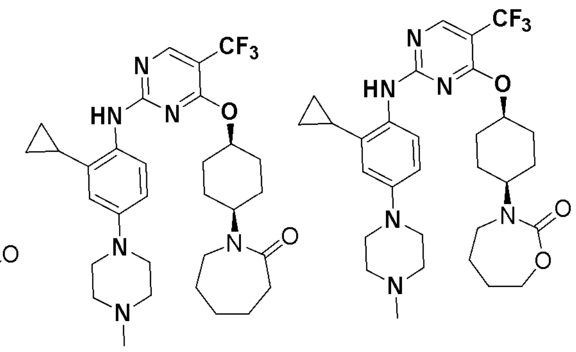

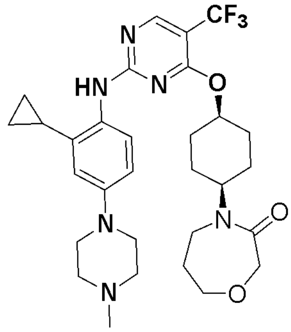

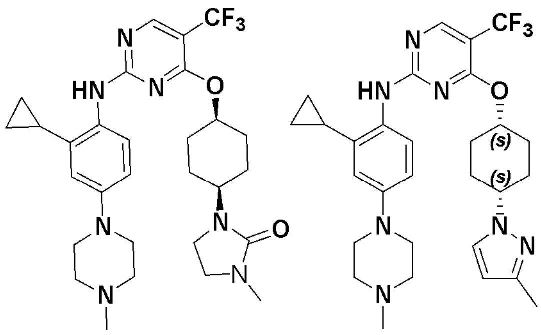

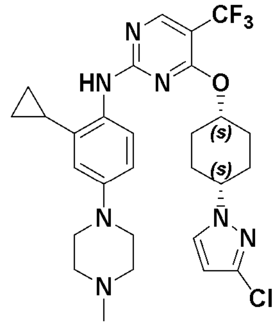

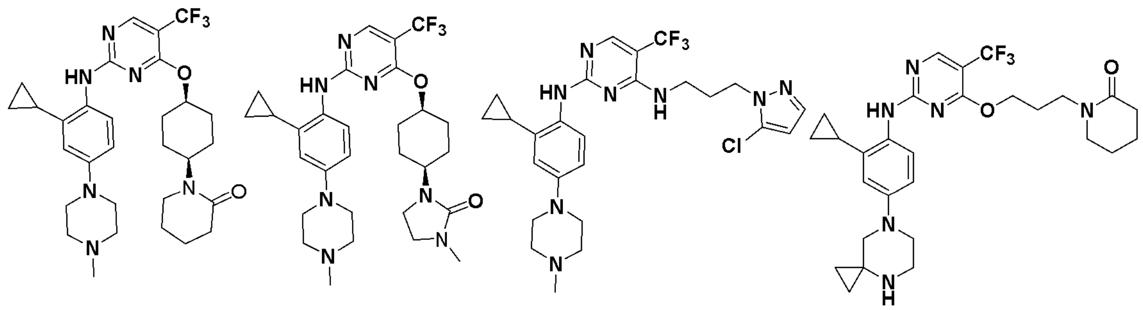

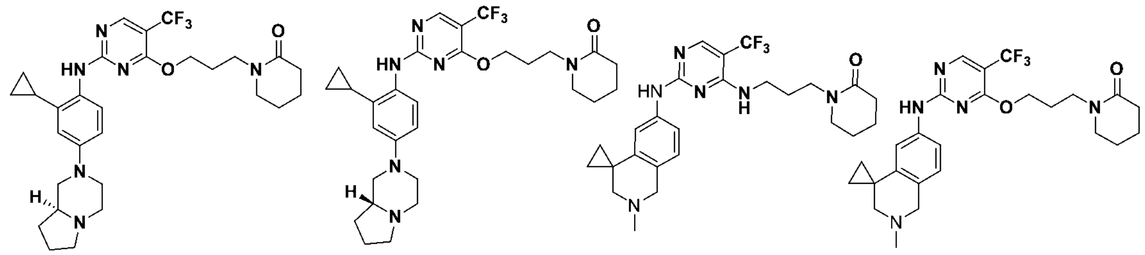

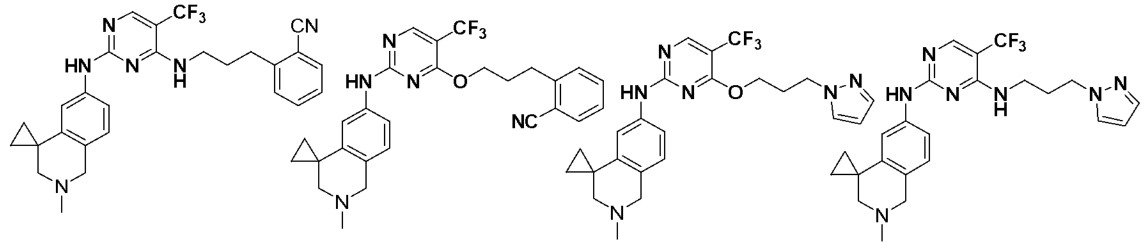

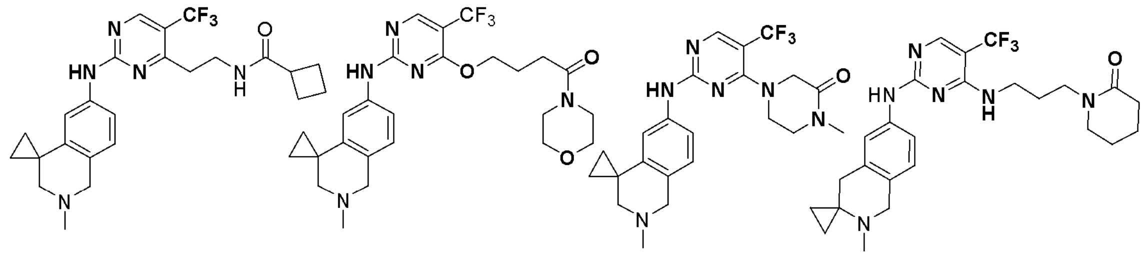

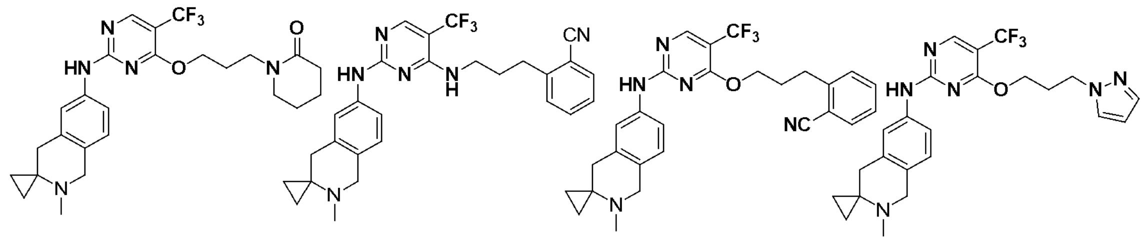

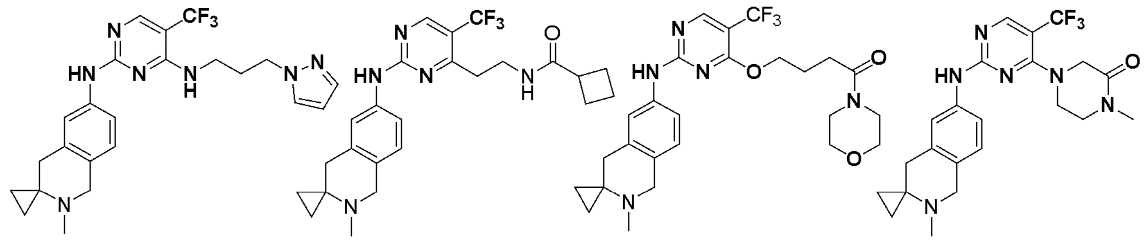

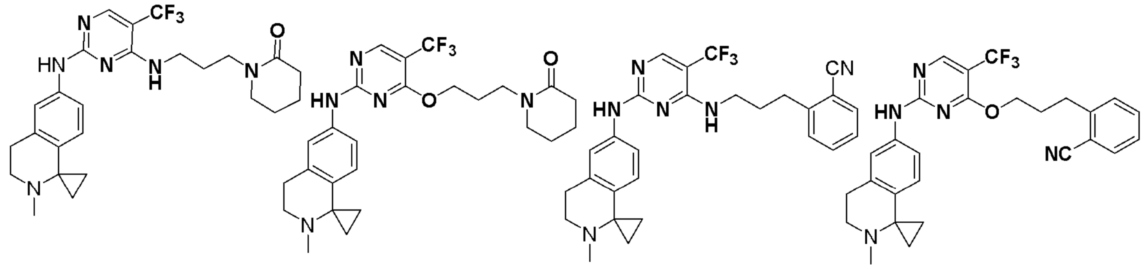

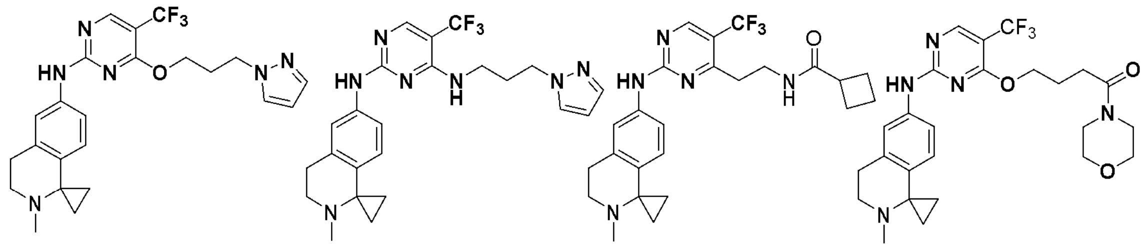

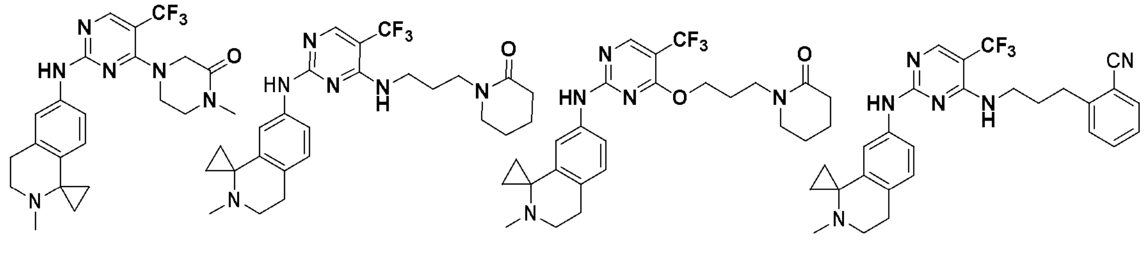

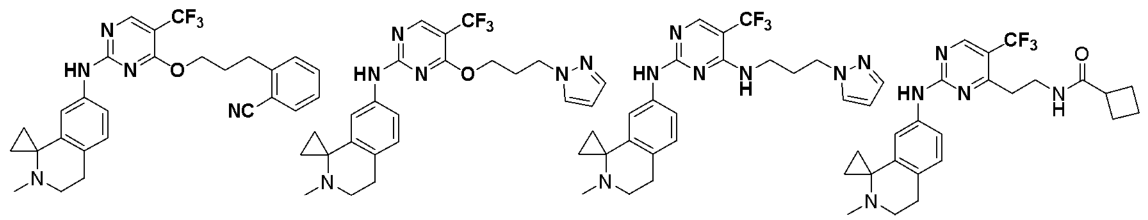

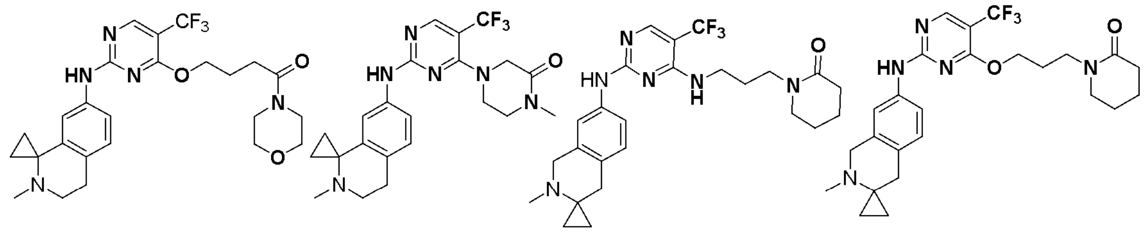

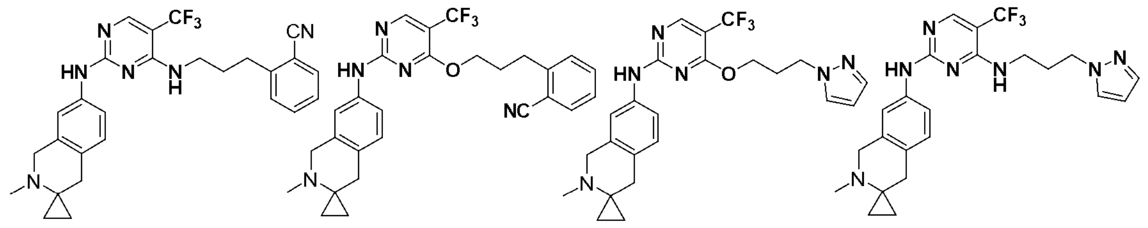

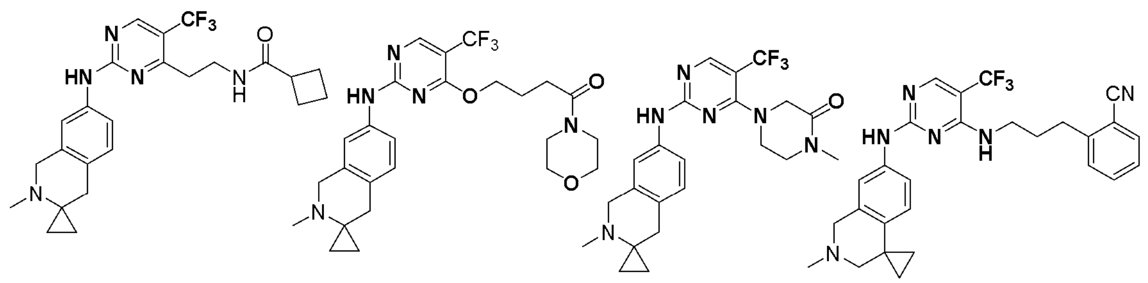

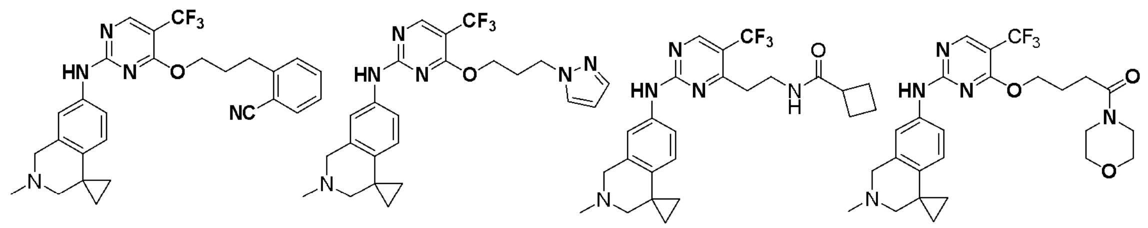

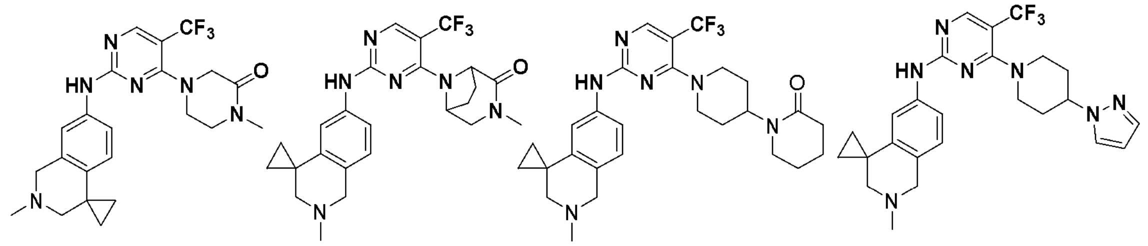

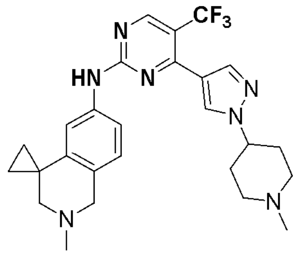
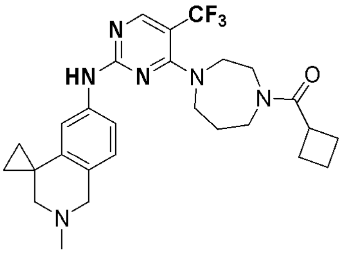
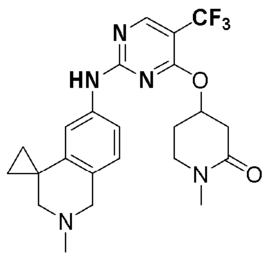
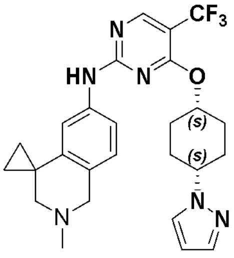

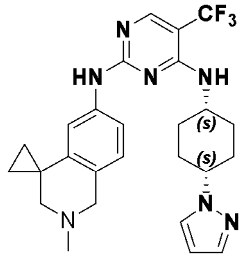
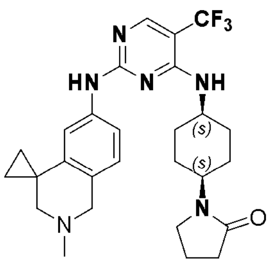
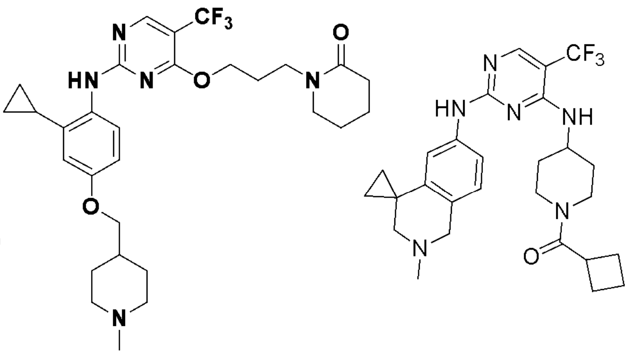

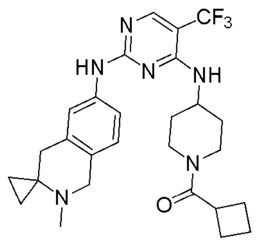
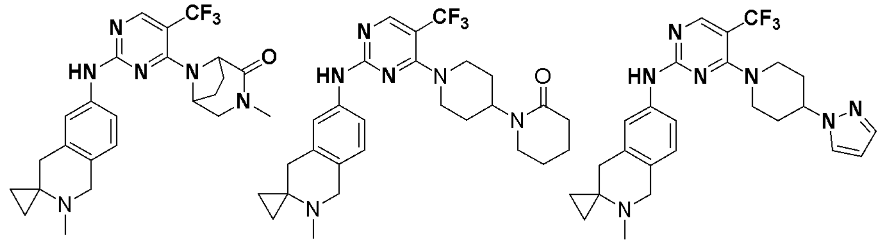

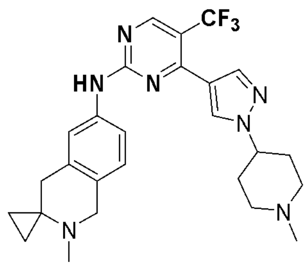
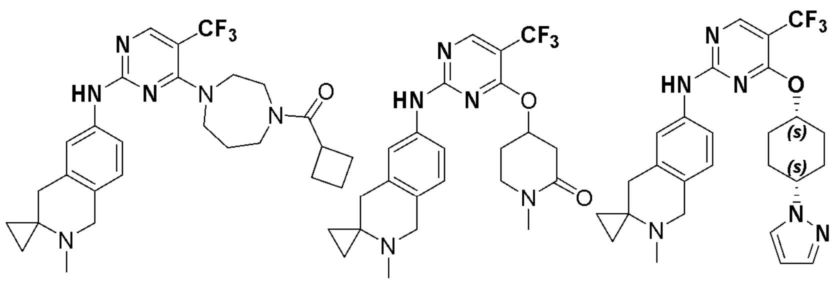

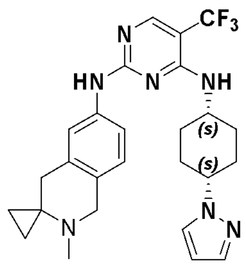
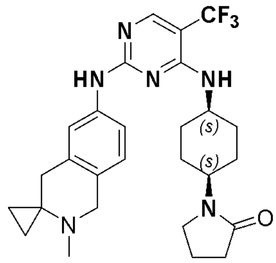
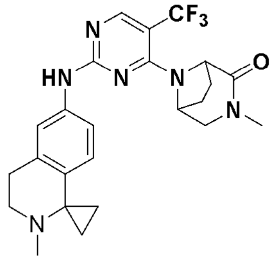
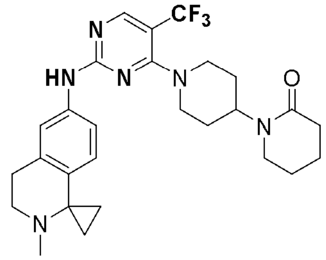

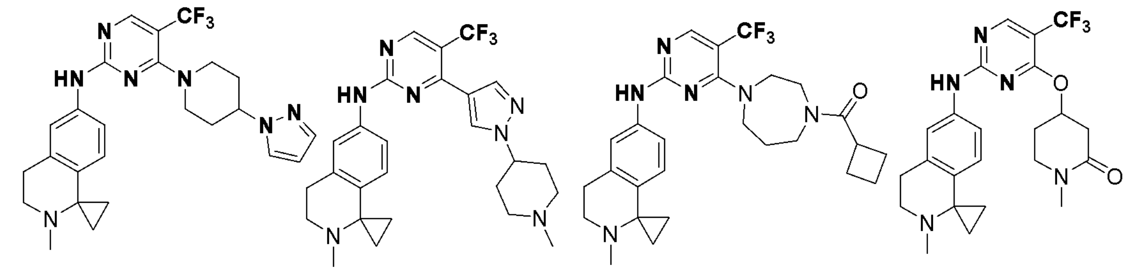

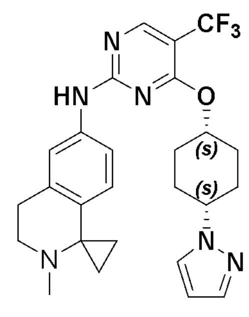
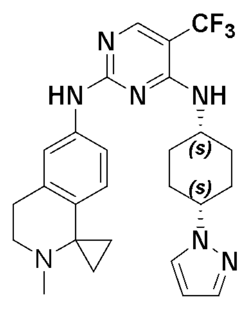
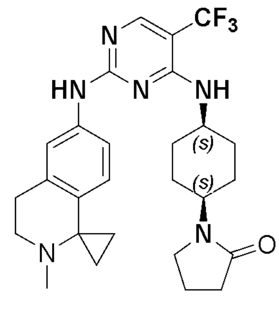
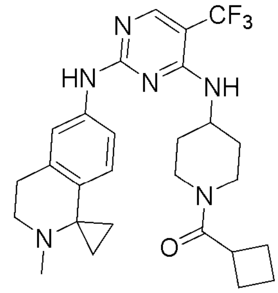

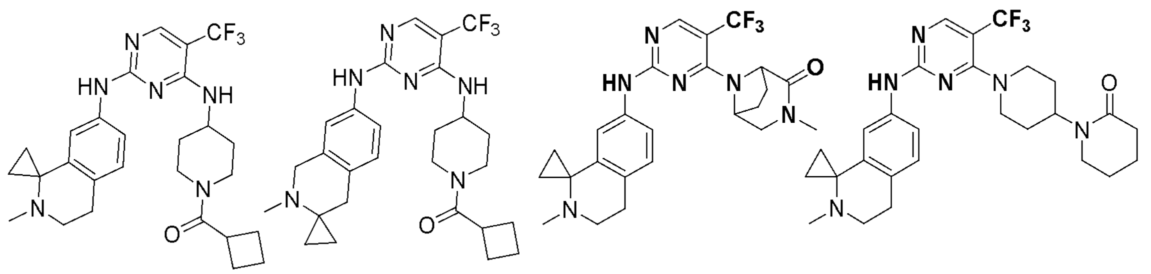

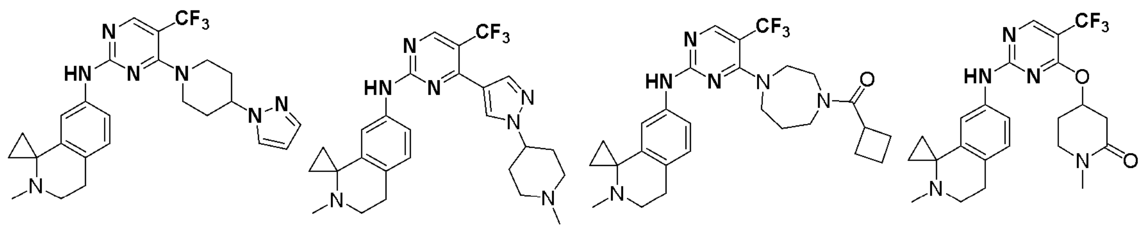

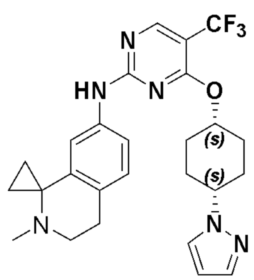
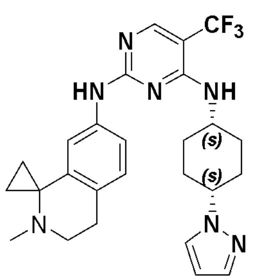
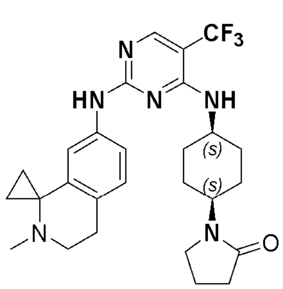
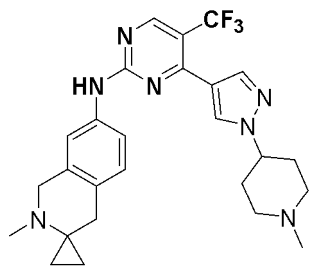

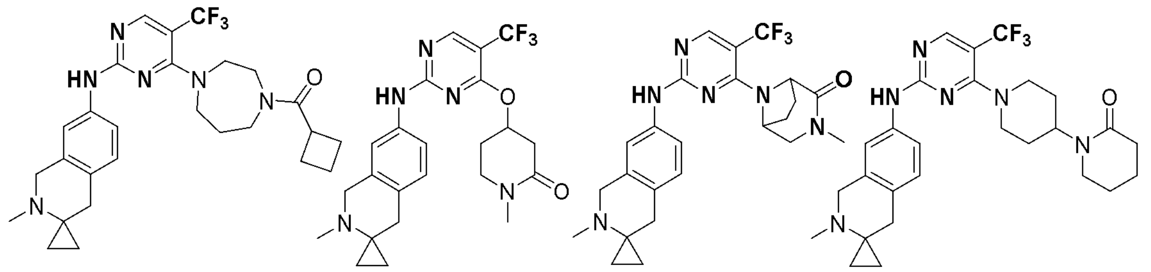

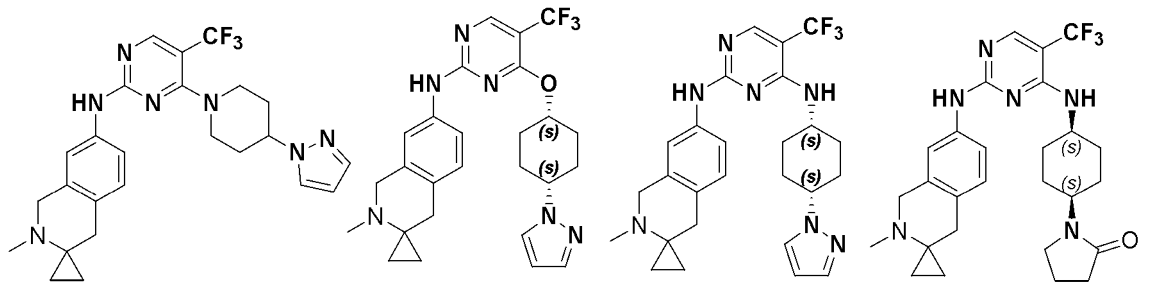

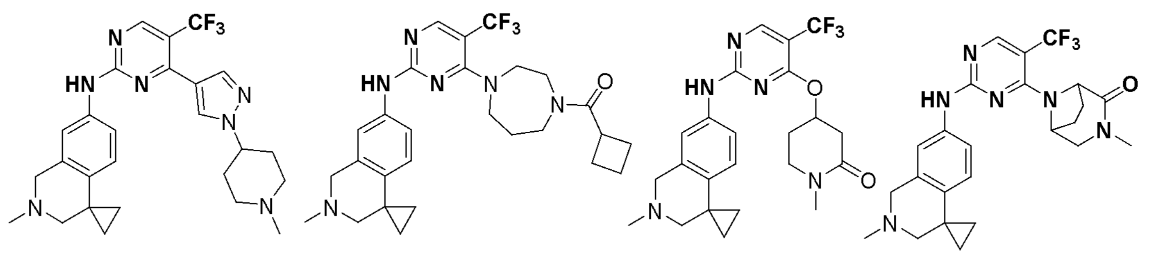

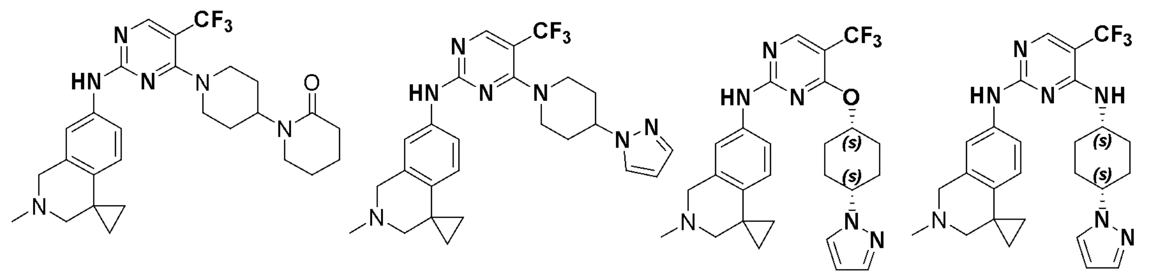

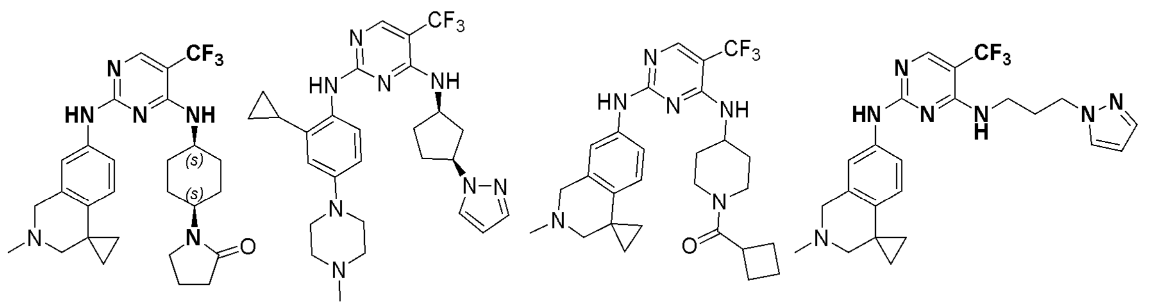

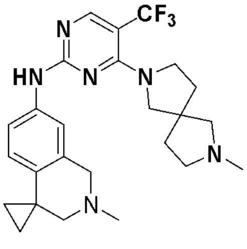
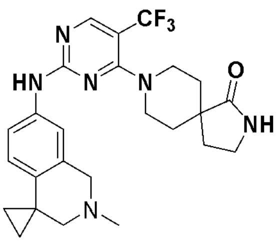
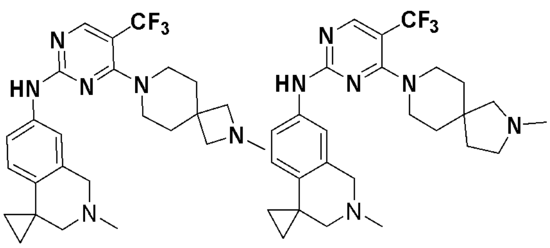

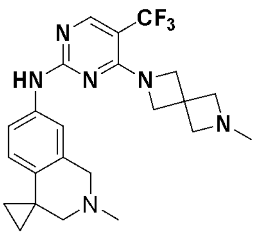
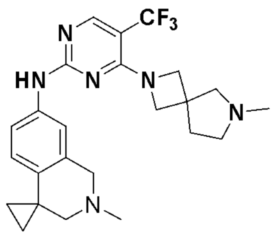
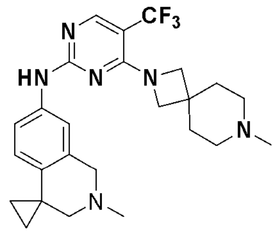
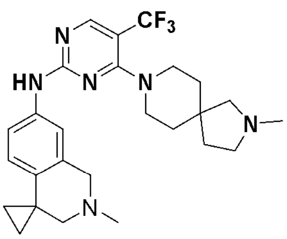

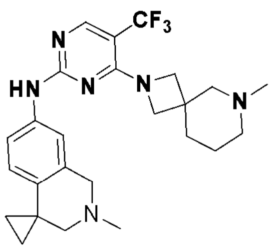
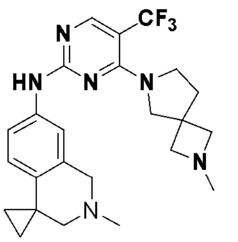
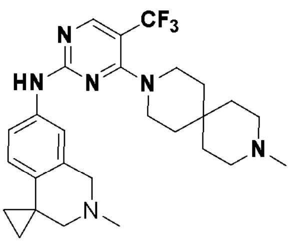
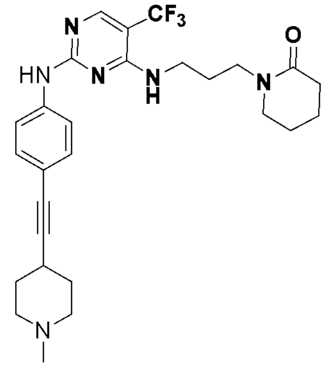

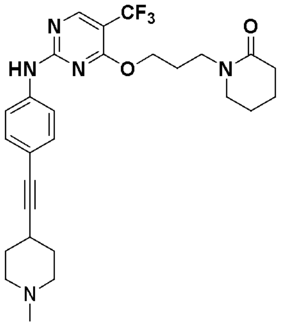
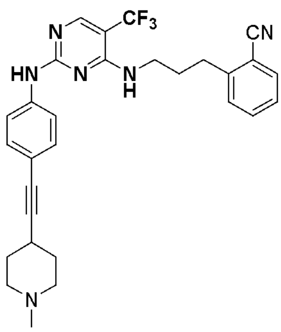
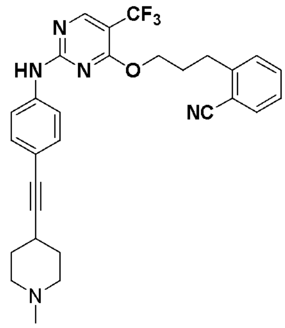
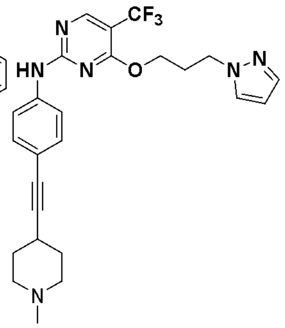

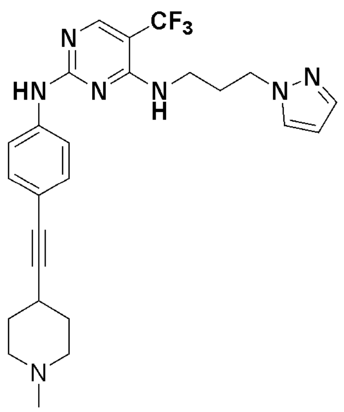
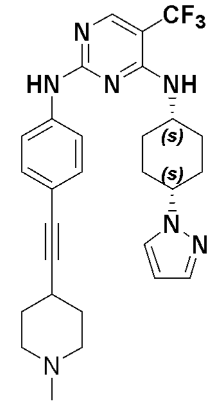
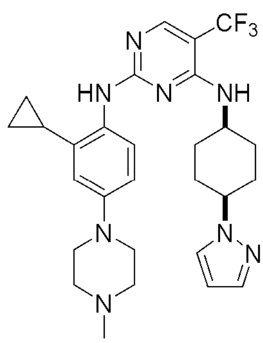
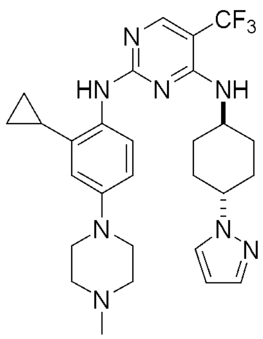

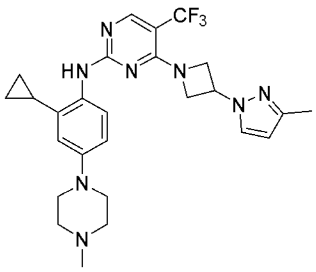
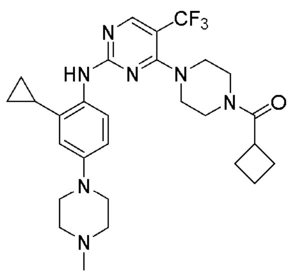
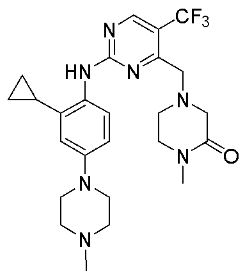
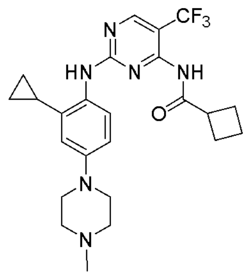

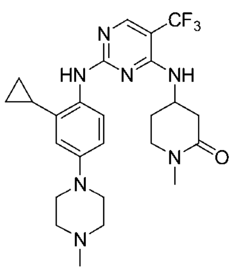
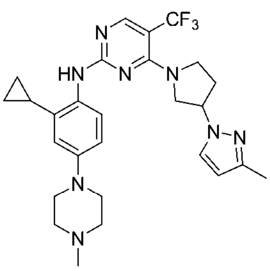
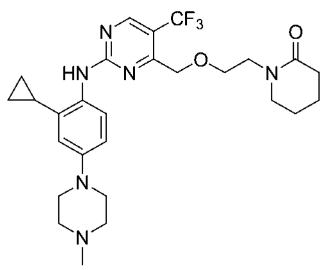
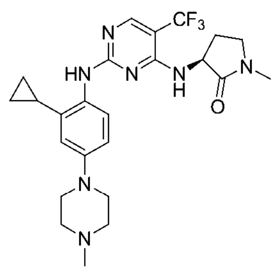

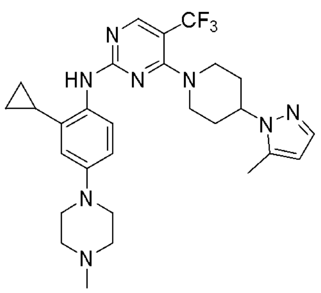
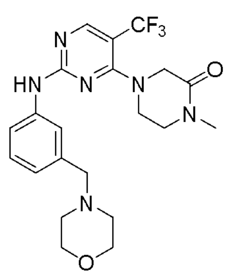
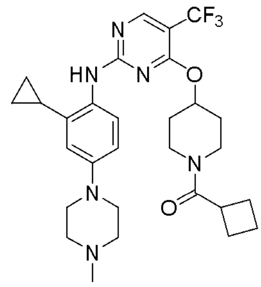
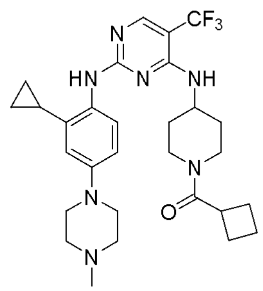

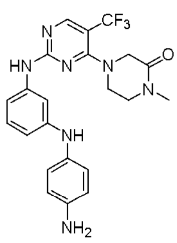
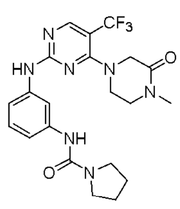
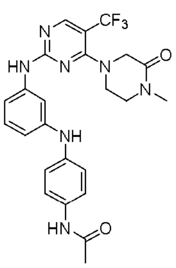
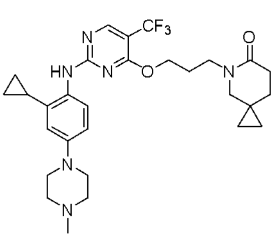
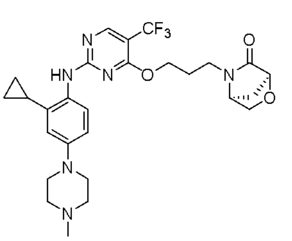.

**21.** A pharmaceutical composition, comprising an effective amount of the compound or pharmaceutically acceptable salt, stereoisomer, tautomer, polymorph, solvate, hydrate, N-oxide, isotope labeled compound, metabolite, ester, or

prodrug thereof of anyone of claims 1 - 20, and one or more pharmaceutically acceptable carriers.

**22.** Use of the compound or pharmaceutically acceptable salt, stereoisomer, tautomer, polymorph, solvate, hydrate, N-oxide, isotope labeled compound, metabolite, ester, or prodrug thereof of anyone of claims 1 - 20, or the pharmaceutical composition of claim 21 in the preparation of a medicament for treating a ULK1-mediated disease in a subject in need thereof; preferably, wherein the ULK1-mediated disease is **characterized by** an abnormal autophagy; preferably, wherein the abnormal autophagy is induced by treatment; preferably, wherein the ULK1-mediated disease is a cancer, preferably lung cancer, breast cancer, or pancreatic cancer, for example non-small cell lung cancer, triple negative breast cancer, or pancreatic ductal adenocarcinoma, or the disease is tuberous sclerosis (TSC) or lymphangioleiomyomatosis (LAM).

**23.** The use according to claim 22, wherein the medicament is used for co-administration with an additional therapeutic agent; preferably, wherein the additional therapeutic agent is a standard care therapy, for example an mTOR inhibitor, carboplatin, a MEK inhibitor or a PARP inhibitor.

**24.** The use according to claim 22 or 23, wherein the medicament is used to degrade ATG13 in the subject.

**25.** A method for preparing the compound of claim 1, comprising the steps shown in the following synthetic route A or B:

Synthetic route A

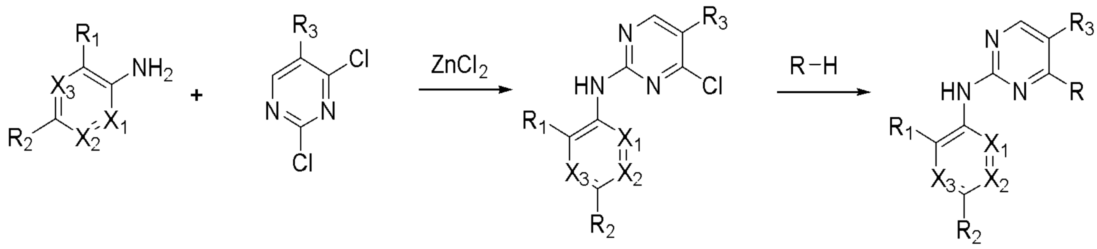


1-0 1-1 1-2 (1)

or

Synthetic route B

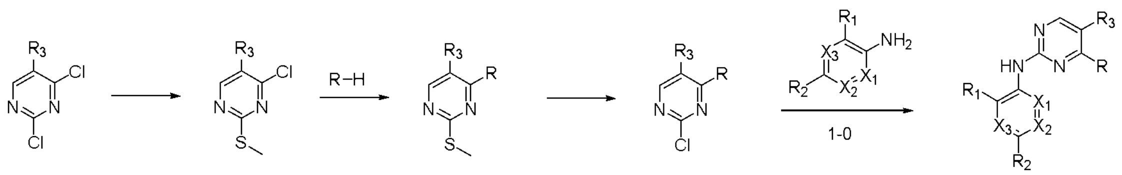


2-0 2-1 2-2 2-3 (1)

wherein: $R_1$, $R_2$, $R_3$, R, $X_1$, $X_2$, and $X_3$ are as defined in claim 1.

**INTERNATIONAL SEARCH REPORT**

International application No.
**PCT/CN2024/115733**

**A. CLASSIFICATION OF SUBJECT MATTER**

C07D239/48(2006.01)i; C07D417/14(2006.01)i; C07D417/02(2006.01)i; C07D401/12(2006.01)i; C07D417/12(2006.01)i; C07D 471/10(2006.01)i; C07D239/34(2006.01)i; C07D405/14(2006.01)i; A61K31/506(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: C07D, A61K, A61P

Documentation searched other than minimum documentation to the extent that such documents are included in the fields searched

Electronic data base consulted during the international search (name of data base and, where practicable, search terms used)

CNTXT, WPABS, ENTXT, VEN, Registry(STN), Caplus(STN): 嘧啶, 肿瘤, 杂环, 抑制剂, ULK1, fused ring, cancer, tumor, inhibitor+, heterocycle, 结构检索, structural search

**C. DOCUMENTS CONSIDERED TO BE RELEVANT**

| Category* | Citation of document, with indication, where appropriate, of the relevant passages | Relevant to claim No. |
|---|---|---|
| PX | WO 2023215494 A1 (ERASCA, INC.) 09 November 2023 (2023-11-09)<br>description, pages 1-611 | 1-25 |
| PX | WO 2024140401 A1 (JS INNOMED HOLDINGS LTD. et al.) 04 July 2024 (2024-07-04)<br>description, pages 1-35 | 1-25 |
| X | WO 2016033100 A1 (SALK INSTITUTE FOR BIOLOGICAL STUDIES et al.) 03 March 2016 (2016-03-03)<br>description, pages 2-6, 31-93, and 101-246 | 1-25 |
| X | WO 2023087027 A1 (ERASCA, INC.) 19 May 2023 (2023-05-19)<br>description, pages 1-6, 15-135, and 162-402 | 1-25 |
| X | CN 114127057 A (DECIPHERA PHARMACEUTICALS, LLC) 01 March 2022 (2022-03-01)<br>description, pages 6-49, 52-70, and 106-132 | 1-25 |
| X | CN 115484956 A (SALK INSTITUTE FOR BIOLOGICAL STUDIES et al.) 16 December 2022 (2022-12-16)<br>description, pages 1-4, 20-82, 84-86, and 91-95 | 1-25 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 November 2024** | **03 December 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

INTERNATIONAL SEARCH REPORT

International application No.
PCT/CN2024/115733

C. DOCUMENTS CONSIDERED TO BE RELEVANT

| Category* | Citation of document, with indication, where appropriate, of the relevant passages | Relevant to claim No. |
|---|---|---|
| X | WO 2022214869 A2 (LIFEARC) 13 October 2022 (2022-10-13)<br>description, pages 1-7, 36, and 39-106 | 1-25 |
| X | CN 114258318 A (DECIPHERA PHARMACEUTICALS, LLC) 29 March 2022 (2022-03-29)<br>description, pages 3-6 and 51-122 | 1-25 |
| X | CN 115515589 A (SALK INSTITUTE FOR BIOLOGICAL STUDIES et al.) 23 December 2022 (2022-12-23)<br>description, pages 1-27, 33-127, 129-130, and 133-179 | 1-25 |

Form PCT/ISA/210 (second sheet) (July 2022)

**INTERNATIONAL SEARCH REPORT**
**Information on patent family members**

International application No.
**PCT/CN2024/115733**

| Patent document cited in search report | Publication date (day/month/year) | Patent family member(s) | Publication date (day/month/year) |
|---|---|---|---|
| WO 2023215494 A1 | 09 November 2023 | TW 202400168 A | 01 January 2024 |
| WO 2024140401 A1 | 04 July 2024 | None | |
| WO 2016033100 A1 | 03 March 2016 | JP 2017526674 A | 14 September 2017 |
| | | JP 6684780 B2 | 22 April 2020 |
| | | CA 2959347 A1 | 03 March 2016 |
| | | CA 2959347 C | 07 March 2023 |
| | | US 2019284207 A1 | 19 September 2019 |
| | | EP 3185868 A1 | 05 July 2017 |
| | | EP 3185868 A4 | 10 January 2018 |
| | | EP 3185868 B1 | 06 April 2022 |
| | | ES 2914099 T3 | 07 June 2022 |
| | | US 2019152989 A1 | 23 May 2019 |
| | | US 10689397 B2 | 23 June 2020 |
| | | JP 2022017384 A | 25 January 2022 |
| | | EP 4070795 A1 | 12 October 2022 |
| | | JP 2020059731 A | 16 April 2020 |
| | | JP 6968141 B2 | 17 November 2021 |
| | | US 2017342088 A1 | 30 November 2017 |
| | | US 10266549 B2 | 23 April 2019 |
| | | DK 3185868 T3 | 23 May 2022 |
| | | US 2019248806 A1 | 15 August 2019 |
| | | US 10774092 B2 | 15 September 2020 |
| WO 2023087027 A1 | 19 May 2023 | EP 4433059 A1 | 25 September 2024 |
| | | TW 202332448 A | 16 August 2023 |
| | | AU 2022383957 A1 | 30 May 2024 |
| | | CA 3238655 A1 | 19 May 2023 |
| CN 114127057 A | 01 March 2022 | HUE 065486 T2 | 28 May 2024 |
| | | US 2023039712 A1 | 09 February 2023 |
| | | US 12071432 B2 | 27 August 2024 |
| | | ES 2966807 T3 | 24 April 2024 |
| | | CO 2021016768 A2 | 08 April 2022 |
| | | US 2020354352 A1 | 12 November 2020 |
| | | US 11530206 B2 | 20 December 2022 |
| | | JP 2022531801 A | 11 July 2022 |
| | | KR 20220008873 A | 21 January 2022 |
| | | EP 3966207 A1 | 16 March 2022 |
| | | EP 3966207 B1 | 01 November 2023 |
| | | BR 112021022504 A2 | 12 April 2022 |
| | | LT 3966207 T | 11 December 2023 |
| | | MX 2021013661 A | 11 March 2022 |
| | | AU 2020275392 A1 | 02 December 2021 |
| | | AU 2020275392 B2 | 14 September 2023 |
| | | HRP 20231730 T1 | 10 May 2024 |
| | | SG 11202112336 PA | 30 December 2021 |
| | | IL 287787 A | 01 January 2022 |
| | | PE 20220597 A1 | 22 April 2022 |
| | | CL 2021002952 A1 | 15 July 2022 |
| | | CA 3139708 A1 | 19 November 2020 |
| | | RS 65019 B1 | 31 January 2024 |
| | | EP 4342469 A2 | 27 March 2024 |

Form PCT/ISA/210 (patent family annex) (July 2022)

**INTERNATIONAL SEARCH REPORT**
**Information on patent family members**

International application No.
**PCT/CN2024/115733**

| Patent document cited in search report | Publication date (day/month/year) | Patent family member(s) | Publication date (day/month/year) |
|---|---|---|---|
| | | EP 4342469 A3 | 19 June 2024 |
| | | TW 202108573 A | 01 March 2021 |
| | | SI 3966207 T1 | 29 December 2023 |
| | | DK 3966207 T3 | 18 December 2023 |
| | | PL 3966207 T3 | 04 March 2024 |
| | | PT 3966207 T | 20 December 2023 |
| | | AU 2023251416 A1 | 09 November 2023 |
| | | FI 3966207 T3 | 30 November 2023 |
| | | WO 2020231806 A1 | 19 November 2020 |
| CN 115484956 A | 16 December 2022 | CA 3171335 A1 | 19 August 2021 |
| | | EP 4103186 A1 | 21 December 2022 |
| | | EP 4103186 A4 | 06 March 2024 |
| | | US 2023130766 A1 | 27 April 2023 |
| | | KR 20220154094 A | 21 November 2022 |
| | | WO 2021163633 A1 | 19 August 2021 |
| | | AU 2021218740 A1 | 22 September 2022 |
| | | JP 2023513797 A | 03 April 2023 |
| WO 2022214869 A2 | 13 October 2022 | EP 4320121 A2 | 14 February 2024 |
| | | US 2024208934 A1 | 27 June 2024 |
| | | CA 3214567 A1 | 13 October 2022 |
| | | KR 20240025504 A | 27 February 2024 |
| | | JP 2024516359 A | 15 April 2024 |
| | | WO 2022214869 A3 | 24 November 2022 |
| | | AU 2022255073 A1 | 23 November 2023 |
| CN 114258318 A | 29 March 2022 | PE 20221083 A1 | 05 July 2022 |
| | | KR 20220024605 A | 03 March 2022 |
| | | CA 3143489 A1 | 24 December 2020 |
| | | CL 2021003356 A1 | 19 August 2022 |
| | | EP 3983081 A1 | 20 April 2022 |
| | | JP 2022536540 A | 17 August 2022 |
| | | IL 288996 A | 01 February 2022 |
| | | US 2021128556 A1 | 06 May 2021 |
| | | US 11590134 B2 | 28 February 2023 |
| | | CO 2022000260 A2 | 29 April 2022 |
| | | US 2023338376 A1 | 26 October 2023 |
| | | BR 112021025544 A2 | 17 May 2022 |
| | | MA 56191 A | 20 April 2022 |
| | | AU 2020297422 A1 | 27 January 2022 |
| | | AU 2020297422 B2 | 21 March 2024 |
| | | WO 2020257180 A1 | 24 December 2020 |
| | | TW 202115012 A | 16 April 2021 |
| | | AU 2024204210 A1 | 11 July 2024 |
| | | MX 2021015628 A | 18 April 2022 |
| CN 115515589 A | 23 December 2022 | EP 4103182 A1 | 21 December 2022 |
| | | EP 4103182 A4 | 21 February 2024 |
| | | AU 2021218739 A1 | 22 September 2022 |
| | | JP 2023513794 A | 03 April 2023 |
| | | US 2023135635 A1 | 04 May 2023 |
| | | WO 2021163629 A1 | 19 August 2021 |
| | | CA 3171187 A1 | 19 August 2021 |

Form PCT/ISA/210 (patent family annex) (July 2022)

**INTERNATIONAL SEARCH REPORT**
**Information on patent family members**

International application No.
**PCT/CN2024/115733**

| Patent document cited in search report | Publication date (day/month/year) | Patent family member(s) | Publication date (day/month/year) |
|---|---|---|---|
| | | KR 20220153581 A | 18 November 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202311138740


### Non-patent literature cited in the description


- **STAHL** ; **WERMUTH**. Handbook of Pharmaceutical Salts: Properties, Selection, and Use. Wiley-VCH, 2002
- **T. L. GILCHRIST**. Comprehensive Organic Synthesis. Academic Press, vol. 7, 748-750
- **G. W. H. CHEESEMAN** ; **E. S. G. WERSTIUK**. Advances in Heterocyclic Chemistry. Academic Press, vol. 22, 390-392
- **T. HIGUCHI** ; **V. STELLA**. Pro-drugs as Novel Delivery Systems. ACS Symposium Series, vol. 14
- Bioreversible Carriers in Drug Design. Pergamon Press, 1987
- **H. BUNDGAARD**. Design of Prodrugs. Elsevier, 1985
- Protective Groups in Organic Chemistry. Plenum Press, 1973
- **T.W. GREENE** ; **P.G.M. WUTS**. Protective Groups in Organic Synthesis. John Wiley & Sons, 1991
- Remington's Pharmaceutical Sciences. 1990